# EUROPEAN PATENT SPECIFICATION

(11) **EP 1 928 461 B1**
(45) Date of publication and mention of the grant of the patent: **04.01.2012**
(21) Application number: 06788808.1
(22) Date of filing: 27.07.2006
(51) Int. Cl.: A61K 31/505, C07D 487/04, C07D 239/74, C07D 239/26, C07D 471/18, C07D 487/18, C07D 471/04, A61K 31/506, A61K 31/517, A61K 31/519

(54) **PYRIMIDINE COMPOUNDS AS SEROTONIN RECEPTOR MODULATORS**
PYRIMIDIN-VERBINDUNGEN ALS SEROTONIN-REZEPTOR-MODULATOREN
COMPOSES DE PYRIMIDINE UTILES COMME MODULATEURS DES RECEPTEURS DE LA SEROTONINE

(30) Priority: 04.08.2005 US 705719 P
(43) Date of publication of application: 11.06.2008
(73) Proprietor: Janssen Pharmaceutica NV, 2340 Beerse (BE)
(72) Inventor: DVORAK, Curt, A., San Diego, CA 92126 (US); RUDOLPH, Dale, A., San Diego, CA 92128 (US); SHIREMAN, Brock, T., San Diego, CA 92130 (US)
(74) Representative: Warner, James Alexander
(86) International application number: PCT/US2006/029437
(87) International publication number: WO 2007/019083

(56) References cited:
- EP-A1- 1 211 246
- EP-A1- 1 264 820
- WO-A-00/59510
- WO-A-95/29909
- WO-A-97/47601
- WO-A-02/072558
- JP-A- 3 148 265
- US-A- 3 969 355
- US-A- 5 137 890
- HERRERA A ET AL: "On the mechanism of reaction between ketones and nitriles. Unexpected results from benzyl nitriles" TETRAHEDRON, ELSEVIER SCIENCE PUBLISHERS, AMSTERDAM, NL, vol. 58, no. 19, 6 May 2002 (2002-05-06), pages 3755-3764, XP004350399 ISSN: 0040-4020
- DUBE, HENRY ET AL: "Synthesis of chiral .alpha.-aminoalkylpyrimidines using an enantioselective three-component reaction" SYNTHESIS , (12), 2015-2025 CODEN: SYNTBF; ISSN: 0039-7881, 2004, XP002411606
- KAWAMURA, SHINICHI ET AL: "Fused heterocycles, furo[3,2-d]pyrimidines and dihydrocylopenta[d]pyrimidines, as potential new herbicides" BIOSCIENCE, BIOTECHNOLOGY, AND BIOCHEMISTRY , 56(11), 1897-9 CODEN: BBBIEJ; ISSN: 0916-8451, 1992, XP009076153
- HERRERA, ANTONIO ET AL: "1H and 13C NMR spectral assignments of 2,4-diaryl-substituted cycloalkyl[d]pyrimidines" MAGNETIC RESONANCE IN CHEMISTRY , 40(4), 293-299 CODEN: MRCHEG; ISSN: 0749-1581, 2002, XP001248275
- VOITENKO, Z. V. ET AL: "Conversions of 2-(2-oxocyclohexylcarbonyl)benzoic acid derivatives to pyrazolo[5,1-a]isoindole and pyrimidine rings" PHOSPHORUS, SULFUR AND SILICON AND THE RELATED ELEMENTS , 180(1), 163-177 CODEN: PSSLEC; ISSN: 1042-6507, 2005, XP001248745
- APPELL, MICHAEL ET AL: "An Analysis of the Binding of Cocaine Analogues to the Monoamine Transporters Using Tensor Decomposition 3-D QSAR" BIOORGANIC & MEDICINAL CHEMISTRY , 10(5), 1197-1206 CODEN: BMECEP; ISSN: 0968-0896, 2002, XP002411608
- BONHAUS D W ET AL: "RS-127445: A SELECTIVE, HIGH AFFINITY, ORALLY BIOAVAILABLE 5-HT 2B RECEPTOR ANTAGONIST" BRITISH JOURNAL OF PHARMACOLOGY, BASINGSTOKE, HANTS, GB, vol. 127, no. 5, July 1999 (1999-07), pages 1075-1082, XP000980373 ISSN: 0007-1188

## Description

### FIELD OF THE INVENTION

There is provided by the present invention compounds that are serotonin receptor modulators. More particularly, there is provided by the present invention pyrimidine compounds that are serotonin receptor modulators useful for the treatment of disease states mediated by serotonin receptor activity.

### BACKGROUND OF THE INVENTION

Serotonin (5-hydroxytryptamine, 5-HT) is a major neurotransmitter eliciting effects via a multiplicity of receptors. To date, at least fifteen different 5-HT receptors have been identified, largely as the result of cloning cDNA's, and these receptors have been grouped into seven families (5-HT, through 5-HT₇) (Hoyer, D. et al. Pharmacol. Biochem. Behav. 2002, 71, 533-554). Fourteen of the fifteen cloned 5-HT receptors are expressed in the brain. 5-HT is implicated in many disease states, particularly conditions of the central nervous system including; depression, anxiety, schizophrenia, eating disorders, obsessive compulsive disorder, learning and memory dysfunction, migraine, chronic pain, sensory perception, motor activity, temperature regulation, nociception, sexual behavior, hormone secretion, and cognition. The identification of multiple 5-HT receptors has provided the opportunity to characterize existing therapeutic agents thought to act via the serotonergic system. Consequently, this has led to the realization that many drugs have non-selective properties (Roth, B.L et al. Neuroscientist 2000, 6(4), 252-262*).* For example, the antipsychotic drugs, clozapine, chlorpromazine, haloperidol and olanzapine exhibit affinities for multiple serotonin receptors in addition to other families of receptors. Similar behavior has been noted for antidepressants, including imipramine, nortriptaline, fluoxetine and sertraline. Similarly, the anti-migraine agent sumatriptan exhibits high affinity for several serotonin receptors. While the lack of selectivity often contributes to a favourable therapeutic outcome, it can also cause undesirable and dose-limiting side effects (Stahl, S.M. Essential Psychopharmacology, 2nd ed., Cambridge University Press, Cambridge, U.K., 2000). Thus, the inhibition of serotonin and norepinephrine uptake together with 5-HT₂ receptor blockade is responsible for the therapeutic effects of the tricyclic antidepressants. In contrast, their blockade of histamine H₁, muscarinic and alpha-adrenergic receptors can lead to sedation, blurred vision and orthostatic hypertension respectively. Likewise, the atypical antipsychotics, including olanzapine and clozapine, are considered to have positive therapeutic effects attributable to their actions at 5-HT₂, D₂ and 5-HT₇ receptors. Conversely, their side effect liability is due to their affinities for a range of dopaminergic, serotonergic and adrenergic receptors.

More selective ligands therefore have the potential to ameliorate untoward pharmacologies and provide novel therapies. More importantly the ability to obtain compounds with known receptor selectivities affords the prospect to target multiple therapeutic mechanisms and improve clinical responses with a single drug.

4-Phenyltetrahydropyrido[4,3-d]pyrimidines with utility in the treatment of gastrointestinal diseases have been described in U.S. Pat. No. 5,137,890 (Sanfilippo et al.):

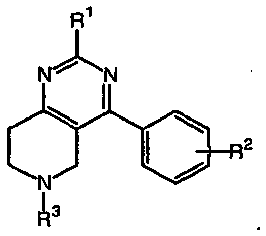

The features and advantages of the invention are apparent to one of ordinary skill in the art. Based on this disclosure, including the summary, detailed description, background, examples, and claims, one of ordinary skill in the art will be able to make modifications and adaptations to various conditions and usages.

Described herein is a series of pyrimidine compounds with the ability to modulate the activity of serotonin receptors.

### SUMMARY OF THE INVENTION

The invention features a compound of Formulae (I) or (II): 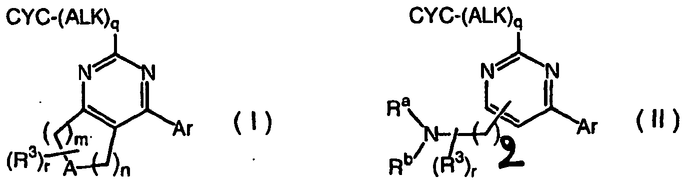 wherein
m is 1, 2, or 3;
n is 1,2, or 3;
where when m and n are both present, m + n is greater than or equal to 2, and is less than or equal to 4;
R^{a} and R^{b} are independently -H, -C₁₋₇alkyl, or -C₃₋₇cycloalkyl, or R^{a} and R^{b} taken together with the nitrogen of attachment form piperidinyl, pyrrolidinyl, morpholinyl, thiomorpholinyl, or piperazinyl, where each R^{a} and R^{b} is optionally and independently substituted with -C₁₋₄alkyl;
q is 0 or 1;
A is >NR¹, >CHNR^{c}R^{d}, >CHOH, or -CH₂-, wherein
- R¹: is selected from the group consisting of -H, -C₁₋₇alkyl, -C₃₋₇Cycloalkyl, and benzyl, where each alkyl, cycloalkyl, or benzyl is optionally mono-, di-, or tri-substituted with R^{e};
R^{e} is selected from the group consisting of -C₁₋₄alkyl, -C₂₋₄alkenyl, -C₂₋₄alkynyl, -C₃₋₆cycloalkyl, halo, -CF₃, -OH, -OC₁₋₄alkyl, -OCF₃, -N(R^{f})R^{g} (wherein R^{f} and R^{g} are independently -H or -C₁₋₄alkyl, or R^{f} and R^{g} taken together with the nitrogen of attachment form piperidinyl, pyrrolidinyl, morpholinyl, thiomorpholinyl, or piperazinyl), -C(O)N(R^{f})R^{g}, -N(R^{h})C(O)R^{h}, -N(R^{h})SO₂C₁₋₇alkyl (wherein R^{h} is -H or -C₁₋₄alkyl, or two R^{h} in the same substituent taken together with the amide of attachment form an otherwise aliphatic 4- to 6-membered ring), -S(O)₀₋₂-C₁₋₄alkyl, -SO₂N(R^{f})R^{g}, -SCF₃, -C(O)C₁₋₄alkyl, -CN, -COOH, and -COOC₁₋₄alkyl;
- R^{c} and R^{d}: are independently selected from the group consisting of -H, -C₁₋₇alkyl, -C₃₋₇alkenyl, -C₃₋₇alkynyl, -C₃₋₇cycloalkyl, -C₁₋₇alkylC₃₋₇cycloalkyl, and -C₃₋₇cycloalkylC₁₋₇alkyl, or R^{c} and R^{d} taken together with the nitrogen of attachment form piperidinyl, pyrrolidinyl, morpholinyl, thiomorpholinyl, or piperazinyl, where each R^{c} and R^{d} is optionally and independently substituted with R^{e};
R³ is -C₁₋₄alkyl, -C₁₋₄alkenyl, or benzyl, each optionally substituted with -C₁₋₃alkyl, -OH, or halo, or two R³ substituents taken together form C₂₋₅alkylene optionally substituted with -C₁₋₃alkyl, -OH, or halo;
r is 0 or is an integer less than or equal to m + n + 1;
Ar is an aryl or heteroaryl ring selected from the group consisting of:
a) phenyl, optionally mono-, di-, or tri-substituted with R¹ or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
   - R¹: is selected from the group consisting of -C₁₋₇alkyl, -C₂₋₇alkenyl, -C₂₋₇alkynyl, -C₃₋₇cycloalkyl, halo, -CF₃, -OH, -OC₁₋₇alkyl, -OCF₃, -OC₃₋₇alkenyl, -OC₃₋₇alkynyl, -N(R^{j})R^{k} (wherein R^{j} and R^{k} are independently -H or -C₁₋₄alkyl), -C(O)N(R^{j})R^{k}, -N(R^{j})C(O)R^{k}, -N(R^{j})SO₂C₁₋₆alkyl, -S(O)_{0- 2}-C₁₋₆alkyl, -SO₂N(R^{j})R^{k}, -SCF₃, -C(O)C₁₋₆alkyl, -NO₂, -CN, -COOH, and -COOC₁₋₇alkyl;
b) a monocyclic aromatic hydrocarbon group having five ring atoms, having a carbon atom which is the point of attachment, having one carbon atom replaced by >O, >S, >NH, or >N(C₁₋₄alkyl), having up to two additional carbon atoms optionally replaced by -N=, optionally mono- or di-substituted with R¹;
c) a monocyclic aromatic hydrocarbon group having six ring atoms, having a carbon atom which is the point of attachment, having one or two carbon atoms replaced by -N=, optionally mono- or di-substituted with R^{j}; and
d) phenyl or pyridyl, substituted with a substituent selected from the group consisting of phenyl, phenoxy, pyridyl, thiophenyl, oxazolyl, and tetrazolyl, where the resultant substituted moiety is optionally further mono-, di-, or tri-substituted with R^{j};
ALK is a branched or unbranched C₁₋₇alkylene, C₂₋₇alkenylene, C₂₋₇alkynylene, C₃₋₇cycloalkylene, or C₃₋₇cycloalkenylene, optionally mono-, di-, or tri-substituted with R^{m};
- R^{m}: is selected from the group consisting of halo, -CF₃, -OH, -OC₁₋₇alkyl, -OC₃₋₇cycloalkyl, -OCF₃, -N(R^{p})R^{s} (wherein R^{p} and R^{s} are independently -H or -C₁₋₇alkyl), -C(O)N(R^{p})R^{s}, -N(R^{t})C(O)R^{t}, -N(R^{t})SO₂C₁₋₆alkyl (wherein R^{t} is -H or -C₁₋₇alkyl), -S(O)₀₋₂-C₁₋₆alkyl, -SO₂N(R^{p})R^{s}, -SCF₃, -CN, -NO₂, -C(O)C₁₋₇alkyl, -COOH, and -COOC₁₋₇alkyl;
CYC is -H or is a ring system selected from the group consisting of:
i) phenyl, optionally mono-, di-, or tri-substituted with R^{u} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
   - R^{u}: is selected from the group consisting of -C₁₋₇alkyl, -C₃₋₇cycloalkyl, phenyl, benzyl, halo, -CF₃, -OH, -OC₁₋₇alkyl, -OC₃₋₇cycloalkyl, -Ophenyl, -Obenzyl, -OCF₃, -N(R^{v})R^{w} (wherein R^{v} and R^{w} are independently -H or -C₁₋₇alkyl, or R^{v} and R^{w} taken together with the nitrogen of attachment form piperidinyl, pyrrolidinyl, morpholinyl, thiomorpholinyl, or piperazinyl, where each R^{v} and R^{w} is optionally and independently substituted with -OH or -C₁₋₇alkyl), -C(O)N(R^{v})R^{w}, -N(R^{x})C(O)R^{x}, -N(R^{x})SO₂C₁₋₆alkyl (wherein R^{x} is -H or -C₁₋₇alkyl, or two R^{x} in the same substituent taken together with the amide of attachment form an otherwise aliphatic 4- to 6-membered ring), -N-(SO₂C₁₋₆alkyl)₂, -S(O)₀₋₂-C₁₋₆alkyl, -SO₂N(R^{v})A^{w}, -SCF₃, -C(O)C₁₋₆alkyl, -NO₂, -CN, -COOH, and -COOC₁₋₇alkyl;
ii) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atoms optionally replaced by -N=, optionally mono- or di-substituted with R^{u};
iii) a monocyclic aromatic hydrocarbon group having six ring atoms, having a carbon atom which is the point of attachment, having one or two carbon atoms replaced by -N=, optionally mono- or di-substituted with R^{u}; and
iv) a non-aromatic heterocyclic ring having 4 to 8 members, said ring having 0, 1, or 2 non-adjacent heteroatom members selected from the group consisting of O, S, -N=, >NH, and >N(C₁₋₄alkyl), having 0, 1, or 2 double bonds, having 0, 1, or 2 carbon members which is a carbonyl, optionally, having one carbon member which forms a bridge, having 0 to 5 substituents R^{u}, and where when q is 0, said ring has a carbon atom which is the point of attachment;
and enantiomers, diastereomers, hydrates, solvates, and pharmaceutically acceptable salts, esters and amides thereof;
with the proviso that in Formula (I):
(a) when ALK is methylene, ethylene, propylene, or isopropylene, CYC is -H, Ar is phenyl or mono-substituted phenyl, m is 2, n is 1, and A is >NR¹, then R¹ is not -C₁₋₄alkyl or benzyl;
(b) when q is 0, CYC is phenyl, Ar is phenyl or 3-chlorophenyl, m is 2, and n is 1, then A is not unsubstituted -CH₂-; and
(c) when q is 0, CYC is 2-pyridyl, Ar is 2-pyridyl, m is 2, and n is 1, then A is not unsubstituted -CH₂-.

Isomeric forms of the compounds of Formulae (I) and (II) and of their pharmaceutically acceptable salts, esters, and amides, are encompassed within the present invention, and reference herein to one of such isomeric forms is meant to refer to at least one of such isomeric forms. One of ordinary skill in the art will recognize that compounds according to this invention may exist, for example in a single isomeric form whereas other compounds may exist in the form of a regioisomeric mixture.

The present invention provides compounds for use in methods of treating or preventing diseases and conditions mediated by the serotonin receptors, particularly, 5-HT₇ and/or 5-HT₂ receptor subtypes.

The invention also features pharmaceutical compositions containing such compounds and methods of using such compositions in the treatment or prevention of disease states mediated by the serotonin receptors, particularly, 5-HT₇ and/or 5-HT₂ receptor subtypes.

Compounds of the present invention are useful in combination with other therapeutic agents as a combination therapy method, including use in combination with selective serotonin reuptake inhibitors (SSRIs), anti-psychotics, norepinephrine reuptake inhibitors (NRIs), sedatives, monoamine oxidase inhibitors (MAOs), or tricyclic antidepressants (TCAs).

Additional features and advantages of the invention will become apparent from the detailed description and examples below, and the appended claims.

### DETAILED DESCRIPTION

Particular preferred compounds of the invention comprise a compound of Formula (I) or (II), or an enantiomer, diastereomer, hydrate, solvate thereof, or a pharmaceutically acceptable salt, amide or ester thereof, wherein m, n, R^{a}, R^{b}, q, A, R³, r, Ar, ALK, and CYC have any of the meanings defined hereinabove and equivalents thereof, or at least one of the following assignments and equivalents thereof. Such assignments may be used where appropriate with any of the definitions, claims or embodiments defined herein:
Preferably, m is 1 and n is 1.
Preferably, m is 1 and n is 2.
Preferably, m is 2 and n is 1.
Preferably, m is 2 and n is 2.
Preferably, m is 1 and n is 3.
Preferably, m is 3 and n is 1.
Preferably, q is 1.

Preferably, -N(R^{a})R^{b} is amino, methylamino, ethylamino, isopropylamino, dimethylamino, diethylamino, diisopropylamino, cyclopropylamino, cyclopentylamino, piperidinyl, pyrrolidinyl, morpholinyl, thiomorpholinyl, or piperazinyl.

More preferably, -N(R^{a})R^{b} is amino, methylamino, dimethylamino, or N-methylpiperazinyl.

Preferably, A is >NR¹.

Preferably, R¹ is selected from the group consisting of hydrogen, methyl, ethyl, isopropyl, butyl, hexyl, cyclopropyl, cyclobutyl, cyclopentyl, and benzyl, each optionally mono-, di-, or tri-substituted with R^{e}.

More preferably, R¹, optionally R^{e} substituted, is selected from the group consisting of hydrogen, methyl, ethyl, isopropyl, and benzyl.

Even more preferably, R¹ is hydrogen or methyl.

Preferably, R³, optionally substituted, is selected from the group consisting of methyl, ethyl, propyl, isopropyl, butyl, methylene, allyl, and benzyl. Alternatively, two R³ substituents taken together form ethylene.

More preferably, R³ is methyl.

Preferably, r is 0, 1, or 2.

Preferably Ar, optionally substituted, is selected from the group consisting of:
a) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
b) furanyl, oxazolyl, isoxazolyl, 1,2,3-oxadiazolyl, 1,2,4-oxadiazolyl, 1,2,5-oxadiazolyl, 1 ,3,4-oxadiazolyl, thiophenyl, thiazolyl, isothiazolyl, pyrrolyl, imidazolyl, pyrazolyl, 1,2,3-triazolyl, 1,2,4-triazolyl,
c) pyridinyl, pyridinyl-N-oxide, pyrazinyl, pyrimidinyl, pyridazinyl, and
d) biphenyl, and 4-tetrazolylphenyl.

More preferably, Ar, optionally substituted, is selected from the group consisting of phenyl, pyridyl, thiophen-2-yl, and thiophen-3-yl.

Specific Ar may be selected from the group consisting of phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl; 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 3-nitrophenyl, 4-nitrophenyl, 3-chloro-4-fluorophenyl, 3-fluoro-4-chlorophenyl, benzo[1,3]dioxol-4 or 5-yl, 3-hydroxyphenyl, 4-hydroxyphenyl, 4-hydroxy-2-methylphenyl, 4-hydroxy-3-fluorophenyl, 3,4-dihydroxyphenyl,4-dimethylaminophenyl, 4-carbamoylphenyl, 4-fluoro-3-methylphenyl, 2-phenoxyphenyl, furan-2-yl, furan-3-yl, 5-methyl-furan-2-yl, thiophen-2-yl, thiophen-3-yl, 5-chlorothiophen-2-yl, 5-methylthiophen-2-yl, 5-chlorothiophen-3-yl, 5-methylthiophen-3-yl, oxazol-2-yl, 4,5-dimethyl-oxazol-2-yl, thiazol-2-yl, 3H-[1,2,3]triazol-4-yl, 2H-pyrazol-3-yl, 1H-pyrazol-4-yl, 4-pyridyl, 5-fluoro-pyridin-2-yl, 4'-chlorobiphenyl, and 4-tetrazolylphenyl.

Preferably, ALK, optionally substituted, is selected from the group consisting of methylene, ethylene, propylene, butylene, sec-butylene, tert-butylene, pentylene, 1-ethylpropylene, 2-ethylpropylene, 2-ethylbutylene, isopropylene, but-3-enylene, isobutylene, 3-methylbutylene, allylene, prop-2-ynylene, cyclopropylene, cyclobutylene, cyclopentylene, cyclohexylene, and cycloheptylene.

Specific ALK may be selected from the group consisting of methylene, hydroxymethylene, fluoromethylene, difluoromethylene, trifluoromethylmethylene, 2,2,2-trifluoro-1-trifluoromethyl-ethylene, methoxycarbonylmethyl, methylcarbamoylmethyl, ethylene, 2-dimethylaminoethylene, 2-cyanoethylene, 2-methoxyethylene, 1-carboxy-ethylene, propylene, 3-methoxycarbonyl propylene, 3-carboxy propylene, isopropylene, 1-fluoro-1-methyl-ethylene, 1-hydroxy-1-methylethylene, 1-carboxy-1-methyl-ethylene, 1-ethylpropylene, 2-ethylpropylene, butylene, tert-butylene, sec-butylene, isobutylene, 4-hydroxybutylene, 4-methoxycarbonyl butylene, 4-carboxy butylene, 2-ethylbutylene, isobutylene, 3-methylbutylene, prop-2-ynylene, but-3-enylene, pentylene, 5-hydroxypentylene, cyclopropylene, cyclobutylene, cyclopentylene, cyclopentenylene, 3,3-difluoro-cyclopentylene, 3-hydroxy-cyclohexylene, 4-fluorocyclohexylene, 4,4-difluoro-cyclohexylene, and 1-methyl-cyclopropylene.

Preferably CYC, optionally substituted, is hydrogen or is a ring system selected from the group consisting of:
i) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
ii) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1,2,4-triazolyl,
iii) pyridinyl, pyridinyl-N-oxide, pyrazinyl, pyrimidinyl, pyridazinyl, and
iv) pyrrolinyl, pyrrolidinyl, pyrazolinyl, piperidinyl, homopiperidinyl, azepanyl, tetrahydrofuranyl, tetrahydropyranyl, piperazinyl, morpholinyl, thiomorpholinyl, and piperidinonyl.

More preferably, CYC, optionally substituted, is selected from the group consisting of hydrogen, phenyl, thiophen-2-yl, thiophen-3-yl, furan-2-yl, furan-3-yl, pyridinyl, piperidin-1, 2, 3 or 4-yl, 2-pyrrolin-2, 3, 4, or 5-yl, 3-pyrrolin-2 or 3-yl, 2-pyrazolin-3, 4 or 5-yl, tetrahydrofuran-3-yl, tetrahydropyran-4-yl, morpholin-2, 3, or 4-yl, thiomorpholin-2, 3, or 4-yl, piperazin-1, 2, 3, or 4-yl, pyrrolidin-1, 2, or 3-yl, and homopiperidinyl.

Most preferably, CYC, optionally substituted, is selected from the group consisting of hydrogen, phenyl, pyridyl, thiophen-2-yl, thiophen-3-yl, tetrahydropyranyl, furan-2-yl, furan-3-yl, tetrahydrofuran-3-yl, and piperidinyl.

Specific CYC may be selected from the group consisting of hydrogen, phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 2-cyanophenyl; 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 2,6-difluorophenyl, 2,6-dichlorophenyl, 2,6-dimethylphenyl, 2,4,6-trifluorophenyl, 2,4,6-trichlorophenyl, 3,4,5-trimethoxyphenyl, 4-fluoro-3-methylphenyl, 3-nitrophenyl, 4-nitrophenyl, 4-methyl-3-fluorophenyl, 3,4-dimethylphenyl, 4-methoxy-3-fluorophenyl, 4-methoxy-2-methylphenyl, 3-aminophenyl, 4-aminophenyl, 4-carbomethoxyphenyl; 3-methanesulfonylamino-phenyl, 4-methanesulfonylamino-phenyl, 3-dimethanesulfonylamino-phenyl, 4-dimethanesulfonylamino-phenyl, thiophen-2-yl, thiophen-3-yl, 5-chlorothiophen-2-yl, benzo[1,3]dioxol-4 or 5-yl, tetrahydrofuran-3-yl, tetrahydropyran-2,3 or 4-yl, furan-2-yl, furan-3-yl, 5-carboxyethyl-furan-2-yl, piperidinyl, 3,4-bisbenzyloxyphenyl, 2-hydroxyphenyl, 3-hydroxyphenyl, 4-hydroxyphenyl, 4-hydroxy-2-methylphenyl, 4-hydroxy-3-fluorophenyl, 3,4-dihydroxyphenyl, 1-piperidinyl, 4-piperidinyl, and 1-methyl-4-piperidinyl.

In one embodiment of Formula (I), CYC-(ALK)q- is -C₃₋₈cycloalkyl.

In another embodiment of Formula (I), CYC-(ALK)_{q} is not methyl, ethyl, propyl, or isopropyl where A is >NR¹. In another embodiment of Formula (I), CYC-(ALK)q- is not methyl, ethyl, propyl, or isopropyl. In another embodiment of Formula (I), m is not 2. In another embodiment of Formula (I), A is not unsubstituted -CH₂- where q is 0.

Compounds of Formulae (I) and (II) comprise compounds that satisfy any one of the combinations of definitions given herein and equivalents thereof.

It is understood that the symbol ">" when used herein immediately prior to an atom means that the atom immediately following this symbol is divalent.

It is understood that some compounds referred to herein are chiral and/or have geometric isomeric centers, for example E- and Z- isomers. The present invention encompasses all such optical isomers, including diastereomers and racemic mixtures, atropisomers, and geometric isomers, and mixtures thereof, that possess the activity that characterizes the compounds of this invention. In addition, certain compounds referred to herein can exist in solvated as well as unsolvated forms. It is understood that this invention encompasses all such solvated and unsolvated forms that possess the activity that characterizes the compounds of this invention.

Compounds according to the present invention that have been modified to be detectable by some analytic technique are also within the scope of this invention. The compounds of the present invention may be labeled with radioactive elements such as ¹²⁵I, ¹⁸F, ¹¹C, ⁶⁴Cu, ³H, ¹⁴C, and the like for use in imaging or for radioactive treatment of patients. An example of such compounds is an isotopically labeled compound, such as an ¹⁸F isotopically labeled compound that may be used as a probe in detection and/or imaging techniques, such as positron emission tomography (PET) and single-photon emission computed tomography (SPECT). Preferably, compounds of the present invention labeled with ¹⁸F or ¹¹C may be used as a positron emission tomography (PET) molecular probe for studying serotonin-mediated disorders. Alternatively, compounds of the present invention labeled with ¹⁴C may be used in metabolic studies. Another example of such compounds is an isotopically labeled compound, such as a deuterium and/or tritium labeled compound, that may be used in reaction kinetic studies. The compounds described herein may be reacted with an appropriate functionalized radioactive reagent using conventional chemistry to provide radiolabeled compounds.

Preferred compounds, which are pyrimidines, are selected from the group consisting of:

| **Ex.** | **Chemical Name** |
|---|---|
| **1** | 2-tert-Butyl-4-(4-fluoro-phenyl)-5,6,7,8-tetrahydro-pyrido[4,3-d]pyrimidine; |
| **2** | 2-Benryl-4-(4-fluoro-phenyl)-5,6,7,8-tetrahydro-pyrido[4,3-d]pyrimidine; |
| **3** | 2-sec-Butyl-4-(4-fluoro-phenyl)-5,6,7,8-tetrahydro-pyrido[4,3-d]pyrimidine hydrochloride; |
| **4** | 2-sec-Butyl-4-p-tolyl-5,6,7,8-tetrahydro-pyrido[4,3-d]pyrimidine hydrochloride; |
| **5** | 2-Cyclobutyl-4-(4-fluoro-phenyl)-5,6,7,8-tetrahydro-pyrido[4,3-d]pyrimidine hydrochloride; |
| **6** | 2-Cyclobutyl-4-p-tolyl-5,6,7,8-tetrahydro-pyrido[4,3-d]pyrimidine; |
| **7** | 2-Cyclopropyl-4-(4-fluoro-phenyl)-5,6,7,8-tetrahydro-pyrido[4,3-d]pyrimidine; |
| **8** | 2-Benzyl-4-p-tolyl-5,6,7,8-tetrahydro-pyrido[4,3-d]pyrimidine; |
| **9** | 2-Benzyl-4-(4-trifluoromethyl-phenyl)-5,6,7,8-tetrahydro-pyrido[4,3-d]pyrimidine; |
| **10** | 2-Benzyl-4-(3,4-difluoro-phenyl)-5,6,7,8-tetrahydro-pyrido[4,3-d]pyrimidine; |
| **11** | 2-Benzyl-4-phenyl-5,6,7,8-tetrahydro-pyrido[4,3-d]pyrimidine; |
| **12** | 2-Benryl-4-(3-fluoro-phenyl)-5,6,7,8-tetrahydro-pyrido[4,3-d]pyrimidine; |
| **13** | 2-(4-Fluoro-benryl)-4-(4-fluoro-phenyl)-5,6,7,8-tetrahydro-pyrido[4,3-d]pyrimidine; |
| **14** | 2-(4-Fluoro-benzyl)-4-(4-fluoro-phenyl)-6-methyl-5,6,7,8-tetrahydro-pyrido[4,3-d]pyrimidine; |
| **15** | 4-[2-(4-Fluoro-benzyl)-6-methyl-5,6,7,8-tetrahydro-pyrido[4,3-d]pyrimidin-4-yl]-benzonitrile; |
| **16** | 4-[2-(4-Fluoro-benzyl)-5,6,7,8-tetrahydro-pyrido[4,3-d]pyrimidin-4-yl]-benzonitrile; |
| **17** | 2-Cyclopentyl-4-(4-fluoro-phenyl)-5,6,7,8-tetrahydro-pyrido[4,3-d]pyrimidine; |
| **18** | 2-Cyclopentyl-4-p-tolyl-5,6,7,8-tetrahydro-pyrido[4,3-d]pyrimidine; |
| **19** | 2-Cyclopentyl-4-(4-methoxy-phenyl)-5,6,7,8-tetrahydro-pyrido[4,3-d]pyrimidine; |
| **20** | 4-(2-Cyclopentyl-5,6,7,8-tetrahydro-pyrido[4,3-d]pyrimidin-4-yl)-benzonitrile; |
| **21** | 4-(4-Fluoro-phenyl)-2-isopropyl-5,6,7,8-tetrahydro-pyrido[4,3-d]pyrimidine hydrochloride; |
| **22** | 4-(4-Fluoro-phenyl)-2-isopropyl-6-methyl-5,6,7,8-tetrahydro-pyrido[4,3-d]pyrimidine; |
| **23** | 4-(3,4-Dichloro-phenyl)-2-isopropyl-5,6,7,8-tetrahydro-pyrido[4,3-d]pyrimidine hydrochloride; |
| **24** | 4-(3,4-Difluoro-phenyl)-2-isopropyl-5,6,7,8-tetrahydro-pyrido[4,3-d]pyrimidine hydrochloride; |
| **25** | 4-(3-Fluoro-phenyl)-2-isopropyl-5,6,7,8-tetrahydro-pyrido[4,3-d]pyrimidine; |
| **26** | 4-(2-Fluoro-phenyl)-2-isopropyl-5,6,7,8-tetrahydro-pyrido[4,3-d]pyrimidine; |
| **27** | 4-(2,4-Difluoro-phenyl)-2-isopropyl-5,6,7,8-tetrahydro-pyrido[4,3-d]pyrimidine; |
| **28** | 2-Isopropyl-4-p-tolyl-5,6,7,8-tetrahydro-pyrido[4,3-d]pyrimidine; |
| **29** | 4-(4-Chloro-phenyl)-2-isopropyl-5,6,7,8-tetrahydro-pyrido[4,3-d]pyrimidine; |
| **30** | 2-Isopropyl-4-(4-methoxy-phenyl)-5,6,7,8-tetrahydro-pyrido[4,3-d]pyrimidine; |
| **31** | 2-Isopropyl-4-phenyl-5,6,7,8-tetrahydro-pyrido[4,3-d]pyrimidine; |
| **32** | 2-Isopropyl-4-(4-trifluoromethyl-phenyl)-5,6,7,8-tetrahydro-pyrido[4,3-d]pyrimidine; |
| **33** | 2-Isopropyl-4-(2-phenoxy-phenyl)-5,6,7,8-tetrahydro-pyrido[4,3-d]pyrimidine; |
| **34** | 2-isobutyl-4-thiophen-3-yl-5,6,7,8-tetrahydro-pyrido[4,3-d]pyrimidine; |
| **35** | 2-Isobutyl-4-thiophen-2-yl-5,6,7,8-tetrahydro-pyrido[4,3-d]pyrimidine; |
| **36** | 2-Isobutyl-4-pyridin-4-yl-5,6,7,8-tetrahydro-pyrido[4,3-d]pyrimidine; |
| **37** | 4-(4-Fluoro-phenyl)-2-isobutyl-5,6,7,8-tetrahydro-pyrido[4,3-d]pyrimidine; |
| **38** | 2-Isobutyl-4-p-tolyl-5,6,7,8-tetrahydro-pyrido[4,3-d]pyrimidine; |
| **39** | 4-(4-Fluoro-3-methyl-phenyl)-2-isobutyl-5,6,7,8-tetrahydro-pyrido[4,3-d]pyrimidine; |
| **40** | 4-(2-Isobutyl-5,6,7,8-tetrahydro-pyrido[4,3-d]pyrimidin-4-yl)-benzonitrile; |
| **41** | 2-Isobutyl-4-(4-methoxy-phenyl)-5,6,7,8-tetrahydro-pyrido[4,3-d]pyrimidine; |
| **42** | 2-sec-Butyl-4-(2-fluoro-phenyl)-5,6,7,8-tetrahydro-pyrido[4,3-d]pyrimidine hydrochloride; |
| **43** | 2-sec-Butyl-4-(3-fluoro-phenyl)-5,6,7,8-tetrahydro-pyrido[4,3-d]pyrimidine; |
| **44** | 2-sec-Butyl-4-(4-methoxy-phenyl)-5,6,7,8-tetrahydro-pyrido[4,3-d]pyrimidine; |
| **45** | 2-sec-Butyl-4-(4-trifluoromethoxy-phenyl)-5,6,7,8-tetrahydro-pyrido[4,3-d]pyrimidine; |
| **46** | 2-Cyclopentyl-4-(4-fluoro-phenyl)-6,7,8,9-tetrahydro-5H-pyrimido[4,5-d]azepine; |
| **47** | 2-Cyclopentyl-4-p-tolyl-6,7,8,9-tetrahydro-5H-pyrimido[4,5-d]azepine; |
| **48** | 2-Cyclopentyl-4-(4-methoxy-phenyl)-6,7,8,9-tetrahydro-5H-pyrimido[4,5-d]azepine; |
| **49** | 4-(2-Cyclopentyl-6,7,8,9-tetrahydro-5H-pyrimido[4,5-d]azepin-4-yl)-benzonitrile; |
| **50** | 4-(4-Fluoro-phenyl)-2-isopropyl-6,7,8,9-tetrahydro-5H-pyrimido[4,5-d]azepine hydrochloride; |
| **51** | 4-(4-Chloro-phenyl)-2-methyl-6,7,8,9-tetrahydro-5H-pyrimido[4,5-d]azepine; |
| **52** | 2-Methyl-4-phenyl-6,7,8,9-tetrahydro-5H-pyrimido[4,5-d]azepine; |
| **53** | 4-(3-Chloro-phenyl)-2-methyl-6,7,8,9-tetrahydro-5H-pyrimido[4,5-d]azepine; |
| **54** | 2-Benryl-4-(4-fluoro-phenyl)-6,7,8,9-tetrahydro-5H-pyrimido[4,5-d]azepine; |
| **55** | 2-Benzyl-4-p-tolyl-6,7,8,9-tetrahydro-5H-pyrimido[4,5-d]azepine; |
| **56** | 2-Benzyl-4-(4-trifluoromethyl-phenyl)-6,7,8,9-tetrahydro-5H-pyrimido[4,5-d]azepine; |
| **57** | 2-(4-Fluoro-benzyl)-4-(4-fluoro-phenyl)-6,7,8,9-tetrahydro-5H-pyrimido[4,5-d]azepine; |
| **58** | 2-Cyclopentyl-4-(4-fluoro-phenyl)-7-methyl-6,7,8,9-tetrahydro-5H-pyrimido[4,5-d]azepine; |
| **59** | 2-Cyclopentyl-7-methyl-4-p-tolyl-6,7,8,9-tetrahydro-5H-pyrimido[4,5-d]azepine; |
| **60** | 2-Cyclopentyl-4-(4-methoxy-phenyl)-7-methyl-6,7,8,9-tetrahydro-5H-pyrimido[4,5-d]azepine; |
| **61** | 2-Benzyl-7-methyl-4-p-tolyl-6,7,8,9-tetrahydro-5H-pyrimido[4,5-d]azepine; |
| **62** | 2-(4-Fluoro-benzyl)-4-(4-fluoro-phenyl)-7-methyl-6,7,8,9-tetrahydro-5H-pyrimido[4,5-d]azepine; |
| **63** | 2-(4-Fluoro-benzyl)-4-(4-fluoro-phenyl)-7-methyl-9-methylene-6,7,8,9-tetrahydro-5H-pyrimido[4,5-d]azepine; |
| **64** | 2-Benzyl-4-(4-fluoro-phenyl)-6,7,8,9-tetrahydro-5H-pyrimido[4,5-c]azepine hydrochloride; |
| **65** | 4-(4-Fluoro-phenyt)-2-isopropyl-6,7,8,9-tetrahydro-5H-pyrimido[4,5-c]azepine hydrochloride; |
| **66** | 2-Isopropyl-4-p-tolyl-6,7,8,9-tetrahydro-5H-pyrimido[4,5-c]azepine hydrochloride; |
| **67** | 2-Isopropyl-4-(4-methoxy-phenyl)-6,7,8,9-tetrahydro-5H-pyrimido[4,5-c]azepine; |
| **68** | 2-Isopropyl-4-phenyl-6,7,8,9-tetrahydro-5H-pyrimido[4,5-c]azepine; |
| **69** | 2-Benzyl-4-(4-fluoro-phenyl)-6,7,8,9-tetrahydro-5H-1,3,6-triaza-benzocycloheptene hydrochloride; |
| **70** | 2,7-Dibenzyl-4-(4-fluoro-phenyl)-5,6,7,8-tetrahydro-pyrido[3,4-d]pyrimidine hydrochloride; |
| **71** | 2,7-Dibenryl-4-p-tolyl-5,6,7,8-tetrahydro-pyrido[3,4-d]pyrimidine; |
| **72** | 2,7-Dibenzyl-4-phenyl-5,6,7,8-tetrahydro-pyrido[3,4-d]pyrimidine; |
| **73** | 2,7-Dibenryl-4-(4-methoxy-phenyl)-5,6,7,8-tetrahydro-pyrido[3,4-d]pyrimidine; |
| **74** | 7-Benzyl-4-(4-fluoro-phenyl)-2-isopropyl-5,6,7,8-tetrahydro-pyrido[3,4-d]pyrimidine; |
| **75** | 7-Benzyl-2-isopropyl-4-phenyl-5,6,7,8-tetrahydro-pyrido[3,4-d]pyrimidine; |
| **76** | 2-Benzyl-4-(4-fluoro-phenyl)-5,6,7,8-tetrahydro-pyrido[3,4-d]pyrimidine hydrochloride; |
| **77** | 2-Benzyl-4-p-tolyl-5,6,7,8-tetrahydro-pyrido[3,4-d]pyrimidine hydrochloride; |
| **78** | 2-Benzyl-4-phenyl-5,6,7,8-tetrahydro-pyrido[3,4-d]pyrimidine; |
| **79** | 2-Benzyl-4-(4-methoxy-phenyl)-5,6,7,8-tetrahydro-pyrido[3,4-d]pyrimidine; |
| **80** | 4-(4-Fluoro-phenyl)-2-isopropyl-5,6,7,8-tetrahydro-pyrido[3,4-d]pyrimidine hydrochloride; |
| **81** | 2-Isopropyl-4-phenyl-5,6,7,8-tetrahydro-pyrido[3,4-d]pyrimidine; |
| **82** | 2-Benzyl-4-(4-fluoro-phenyl)-7-methyl-5,6,7,8-tetrahydro-pyrido[3,4-d]pyrimidine hydrochloride; |
| **83** | 2-Benzyl-4-(4-fluoro-phenyl)-7-isopropyl-5,6,7,8-tetrahydro-pyrido[3,4-d]pyrimidine; |
| **84** | 4-(4-Fluoro-phenyl)-2-isopropyl-7-methyl-5,6,7,8-tetrahydro-pyrido[3,4-d]pyrimidine hydrochloride; |
| **85** | 2-Isopropyl-7-methyl-4-phenyl-5,6,7,8-tetrahydro-pyrido[3,4-d]pyrimidine; |
| **86** | 7-Benzyl-2-isopropyl-4-(5-methyl-thiophen-3-yl)-5,6,7,8-tetrahydro-pyrido[3,4-d]pyrimidine hydrochloride; |
| **87** | 7-Benzyl-2-isopropyl-4-thiophen-3-yl-5,6,7,8-tetrahydro-pyrido[3,4-d]pyrimidine hydrochloride; |
| **88** | 2-Isopropyl-4-(5-methyl-thiophen-3-yl)-5,6,7,8-tetrahydro-pyrido[3,4-d]pyrimidine hydrochloride; |
| **89** | 2-Isopropyl-4-thiophen-3-yl-5,6,7,8-tetrahydro-pyrido[3,4-d]pyrimidine hydrochloride; |
| **90** | 2-Isopropyl-7-methyl-4-(5-methyl-thiophen-3-yl)-5,6,7,8-tetrahydro-pyrido[3,4-d]pyrimidine hydrochloride; |
| **91** | 2-Isopropyl-7-methyl-4-thiophen-3-yl-5,6,7,8-tetrahydro-pyrido[3,4-d]pyrimidine hydrochloride; |
| **92** | 6-Benryl-4-(4-fluoro-phenyl)-2-isopropyl-8-methyl-5,6,7,8-tetrahydro-pyrido[4,3-d]pyrimidine; |
| **93** | 6-Benzyl-4-(3-chloro-4-fluoro-phenyl)-2-isopropyl-8-methyl-5,6,7,8-tetrahydro-pyrido[4,3-d]pyrimidine; |
| **94** | 6-Benzyl-2-isopropyl-8-methyl-4-p-tolyl-5,6,7,8-tetrahydro-pyrido[4,3-d]pyrimidine; |
| **95** | 6-Benzyl-2-isopropyl-8-methyl-4-phenyl-5,6,7,8-tetrahydro-pyrido[4,3-d]pyrimidine; |
| **96** | 6-Benzyl-2-isopropyl-8-methyl-4-(4-trifluoromethyl-phenyl)-5,6,7,8-tetrahydro-pyrido[4,3-d]pyrimidine; |
| **97** | 6-Benzyl-4-(4-chloro-phenyl)-2-isopropyl-8-methyl-5,6,7,8-tetrahydro-pyrido[4,3-d]pyrimidine; |
| **98** | 6-Benzyl-2-isopropyl-8-methyl-4-thiophen-3-yl-5,6,7,8-tetrahydro-pyrido[4,3-d]pyrimidine; |
| **99** | 6-Benryl-4-(4'-chloro-biphenyl-4-yl)-2-isopropyl-8-methyl-5,6,7,8-tetrahydro-pyrido[4,3-d]pyrimidine; |
| **100** | 4-(4-Fluoro-phenyl)-2-isopropyl-8-methyl-5,6,7,8-tetrahydro-pyrido[4,3-d]pyrimidine hydrochloride; |
| **101** | 4-(3-Chloro-4-fluoro-phenyl)-2-isopropyl-8-methyl-5,6,7,8-tetrahydro-pyrido[4,3-d]pyrimidine hydrochloride; |
| **102** | 2-Isopropyl-8-methyl-4-p-tolyl-5,6,7,8-tetrahydro-pyrido[4,3-d]pyrimidine hydrochloride; |
| **103** | 2-Isopropyl-8-methyl-4-phenyl-5,6,7,8-tetrahydro-pyrido[4,3-d]pyrimidine; |
| **104** | 2-Isopropyl-8-methyl-4-(4-trifluoromethyl-phenyl)-5,6,7,8-tetrahydro-pyrido[4,3-d]pyrimidine; |
| **105** | 2-Isopropyl-8-methyl-4-thiophen-3-yl-5,6,7,8-tetrahydro-pyrido[4,3-d]pyrimidine; |
| **106** | 4-(4-Fluoro-phenyl)-2-isopropyl-6,7-dihydro-5H-pyrrolo[3,4-d]pyrimidine hydrochloride; |
| **107** | 4-(4-Fluoro-phenyl)-2-isopropyl-7-pyrrolidin-1-yl-5,6,7,8-tetrahydro-quinazoline; |
| **108** | [4-(4-Fluoro-phenyl)-2-isopropyl-5,6,7,8-tetrahydro-quinazolin-7-yl]-methyl-amine hydrochloride; |
| **109** | [4-(4-Fluoro-phenyl)-2-isopropyl-5,6,7,8-tetrahydro-quinazolin-6-yl]-methyl-amine hydrochloride; |
| **110** | 4-(4-Fluoro-phenyl)-2-isopropyl-5,6,7,8-tetrahydro-quinazolin-7-ol; |
| **111** | 4-(4-Fluoro-phenyl)-2-isopropyl-5,6,7,8-tetrahydro-quinazoline; |
| **112** | (2-Benzyl-6-p-tolyl-pyrimidin-4-ylmethyl)-dimethyl-amine; |
| **113** | 2-Benzyl-4-(4-methyl-piperazin-1-ylmethyl)-6-p-tolyl-pyrimidine; |
| **114** | [6-(4-Fluoro-phenyl)-2-isopropyl-pyrimidin-4-ylmethyl]-methyl-amine; |
| **115** | 2-(2-Benzyl-6-p-tolyl-pyrimidin-4-yl)-ethylamine; |
| **116** | [2-(4-Fluoro-benzyl)-4-p-tolyl-pyrimidin-5-ylmethyl]-dimethyl-amine; |
| **117** | 4-(4-Fluoro-phenyl)-2-phenyl-5,6,7,8-tetrahydro-pyrido[4,3-d]pyrimidine; |
| **118** | 2-(3,3-Difluoro-cyclopentyl)-4-(4-fluoro-phenyl)-5,6,7,8-tetrahydro-pyrido[4,3-d]pyrimidine; |
| **119** | 4-(4-Fluoro-phenyl)-2-(tetrahydro-furan-3-yl)-5,6,7,8-tetrahydro-pyrido[4,3-d]pyrimidine; |
| **120** | 4-(4-Fluoro-phenyl)-2-(2-piperidin-1-yl-ethyl)-5,6,7,8-tetrahydro-pyrido[4,3-d]pyrimidine; |
| **121** | 2-(1-Fluoro-1-methyl-ethyl)-4-(4-fluoro-phenyl)-5,6,7,8-tetrahydro-pyrido[4,3-d]pyrimidine; |
| **122** | 3-(4-Fluoro-phenyl)-5-isopropyl-4,6,12-triaza-tricyclo[7.2.1.0^{2,7}]dodeca-2,4,6-triene; |
| **123** | 7-(4-Fluoro-phenyl)-5-isopropyl-4,6,13-triaza-tricyclo[8.2.1.0^{3,8}]trideca-3,5,7-triene; |
| **124** | 4-(4-Fluoro-phenyl)-2-(tetrahydro-pyran-4-yl)-5,6,7,8-tetrahydro-pyrido[4,3-d]pyrimidine; |
| **125** | 4-(4-Fluoro-phenyl)-2-(tetrahydro-pyran-3-yl)-5,6,7,8-tetrahydro-pyrido[4,3-d]pyrimidine; |
| **126** | 4-(4-Fluoro-phenyl)-2-(2-methoxy-ethyl)-5,6,7,8-tetrahydro-pyrido[4,3-d]pyrimidine; |
| **127** | 2-[4-(4-Fluoro-phenyl)-5,6,7,8-tetrahydro-pyrido[4,3-d]pyrimidin-2-yl]-propan-2-ol; |
| **128** | 4-(4-Fluoro-phenyl)-2-(1-methyl-1-phenyl-ethyl)-5,6,7,8-tetrahydro-pyrido[4,3-d]pyrimidine; |
| **129** | 2-Cyclopent-3-enyl-4-(4-fluoro-phenyl)-5,6,7,8-tetrahydro-pyrido[4,3-d]pyrimidine; |
| **130** | 3-[4-(4-Fluoro-phenyl)-5,6,7,8-tetrahydro-pyrido[4,3-d]pyrimidin-2-yl]-cyclohexanol; |
| **131** | 4-(4-Fluoro-phenyl)-2-piperidin-4-yl-5,6,7,8-tetrahydro-pyrido[4,3-d]pyrimidine; |
| **132** | 4-(4-Fluoro-phenyl)-2-(1-methyl-piperidin-4-yl)-5,6,7,8-tetrahydro-pyrido[4,3-d]pyrimidine; |
| **133** | [4-(4-Fluoro-phenyl)-5,6,7,8-tetrahydro-pyrido[4,3-d]pyrimidin-2-yl]-phenyl-methanol; |
| **134** | 4-(4-Fluoro-phenyl)-2-(fluoro-phenyl-methyl)-5,6,7,8-tetrahydro-pyrido[4,3-d]pyrimidine; |
| **135** | 2-(Difluoro-phenyl-methyl)-4-(4-fluoro-phenyl)-5,6,7,8-tetrahydro-pyrido[4,3-d]pyrimidine; |
| **136** | 4-(4-Fluoro-phenyl)-2-phenyl-5,6,7,8-tetrahydro-pyrido[4,3-d]pyrimidine; |
| **137** | 4-(4-Fluoro-phenyl)-2-(3-fluoro-phenyl)-5,6,7,8-tetrahydro-pyrido[4,3-d]pyrimidine; |
| **138** | 4-(4-Fluoro-phenyl)-2-(4-methoxy-phenyl)-5,6,7,8-tetrahydro-pyrido[4,3-d]pyrimidine; |
| **139** | 4-(4-Fluoro-phenyl)-2-o-tolyl-5,6,7,8-tetrahydro-pyrido[4,3-d]pyrimidine; |
| **140** | 3-[4-(4-Fluoro-phenyl)-5,6,7,8-tetrahydro-pyrido[4,3-d]pyrimidin-2-yl]-benzonitrile; |
| **141** | 4-(4-Fluoro-phenyl)-2-(2,2,2-trifluoro-1-trifluoromethyl-ethyl)-5,6,7,8-tetrahydro-pyrido[4,3-d]pyrimidine; |
| **142** | 4-(4-Fluoro-phenyl)-2-(1-methyl-cyclopropyl)-5,6,7,8-tetrahydro-pyrido[4,3-d]pyrimidine; |
| **143** | 2-[4-(4-Fluoro-phenyl)-5,6,7,8-tetrahydro-pyrido[4,3-d]pyrimidin-2-yl]-2-methyl-propionic acid; |
| **144** | 2-[4-(4-Fluoro-phenyl)-5,6,7,8-tetrahydro-pyrido[4,3-d]pyrimidin-2-yl]-propionic acid; |
| **145** | 2-(4-Fluoro-cyclohexyl)-4-(4-fluoro-phenyl)-5,6,7,8-tetrahydro-pyrido[4,3-d]pyrimidine; |
| **146** | 2-(4,4-Diffluoro-cyclohexyl)-4-(4-fluoro-phenyl)-5,6,7,8-tetrahydro-pyrido[4,3-d]pyrimidine; |
| **147** | 4-(4-Fluoro-phenyl)-2-phenethyl-5,6,7,8-tetrahydro-pyrido[4,3-d]pyrimidine; |
| **148** | 4-Furan-2-yl-2-isopropyl-5,6,7,8-tetrahydro-pyrido[4,3-d]pyrimidine; |
| **149** | 2-Isopropyl-4-(5-methyl-furan-2-yl)-5,6,7,8-tetrahydro-pyrido[4,3-d]pyrimidine; |
| **150** | 4-Furan-3-yl-2-isopropyl-5,6,7,8-tetrahydro-pyrido[4,3-d]pyrimidine; |
| **151** | 4-(5-Fluoro-pyridin-2-yl)-2-isopropyl-5,6,7,8-tetrahydro-pyrido[4,3-d]pyrimidine; |
| **152** | 2-Isopropyl-4-oxazol-2-yl-5,6,7,8-tetrahydro-pyrido[4,3-d]pyrimidine; |
| **153** | 4-(4,5-Dimethyl-oxazol-2-yl)-2-isopropyl-5,6,7,8-tetrahydro-pyrido[4,3-d]pyrimidine; |
| **154** | 2-Isopropyl-4-thiazol-2-yl-5,6,7,8-tetrahydro-pyrido[4,3-d]pyrimidine; |
| **155** | 2-Isopropyl-4-(3H-[1,2,3]triazol-4-yl)-5,6,7,8-tetrahydro-pyrido[4,3-d]pyrimidine; |
| **156** | 2-Isopropyl-4-(2H-pyrazol-3-yl)-5,6,7,8-tetrahydro-pyrido[4,3-d]pyrimidine; |
| **157** | 2-Isopropyl-4-(1H-pyrazol-4-yl)-5,6,7,8-tetrahydro-pyrido[4,3-d]pyrimidine; |
| **158** | 4-(4-Fluolo-phenyl)-2,6-diisopropyl-5,6,7,8-tetrahydro-pyrido[4,3-d]pyrimidine; |
| **159** | 6-Ethyl-4-(4-fluoro-phenyl)-2-isopropyl-5,6,7,8-tetrahydro-pyrido[4,3-d]pyrimidine; |
| **160** | 6-Cyclopropyl-4-(4-fluoro-phenyl)-2-isopropyl-5,6,7,8-tetrahydro-pyrido[4,3-d]pyrimidine; |
| **161** | 6-Cyclobutyl-4-(4-fluoro-phenyl)-2-isopropyl-5,6,7,8-tetrahydro-pyrido[4,3-d]pyrimidine; |
| **162** | 6-Cyclopentyl-4-(4-fluoro-phenyl)-2-isopropyl-5,6,7,8-tetrahydro-pyrido[4,3-d]pyrimidine; |
| **163** | 6-Butyl-4-(4-fluoro-phenyl)-2-isopropyl-5,6,7,8-tetrahydro-pyrido[4,3-d]pyrimidine; |
| **165** | 4-(4-Fluoro-phenyl)-2-isopropyl-5,6,7,8-tetrahydro-pyrido[4,3-d]pyrimidine, citrate salt; |
| **166** | {2-[2-tert-Butyl-6-(4-fluoro-phenyl)-pyrimidin-4-yl]-ethyl}-methyl-amine; and |
| **167** | {2-[2-tert-Butyl-6-(4-fluoro-phenyl)-pyrimidin-4-yl]-ethyl}-dimethyl-amine. |

Preferably, the compound is 4-(4-fluoro-phenyl)-2-isopropyl-5,6,7,8-tetrahydro-pyrido[4,3-d]pyrimidine or a pharmaceutically acceptable salt thereof.

The features and advantages of the invention are apparent to one of ordinary skill in the art. Based on this disclosure, including the summary, detailed description, background, examples, and claims, one of ordinary skill in the art will be able to make modifications and adaptations to various conditions and usages. Publications described herein are incorporated by reference in their entirety. Where chemical symbols are used, it is understood that they are read from left to right, and that otherwise their spatial orientation has no significance.

The compounds as described above may be made according to processes within the skill of the art and/or that are described in the schemes and examples that follow. To obtain the various compounds herein, starting materials may be employed that carry the ultimately desired substituents though the reaction scheme with or without protection as appropriate. This may be achieved by means of conventional protecting groups, such as those described in "Protective Groups in Organic Chemistry", ed. J.F.W. McOmie, Plenum Press, 1973; and T.W. Greene & P.G.M. Wuts, "Protective Groups in Organic Synthesis", 3rd ed., John Wiley & Sons, 1999. The protecting groups may be removed at a convenient subsequent stage using methods known from the art. Alternatively, it may be necessary to employ, in the place of the ultimately desired substituent, a suitable group that may be carried through the reaction scheme and replaced as appropriate with the desired substituent. Such compounds, precursors, or prodrugs are also within the scope of the invention. Reactions may be performed between the melting point and the reflux temperature of the solvent, and preferably between 0 °C and the reflux temperature of the solvent.

The pyrimidine compounds of Formulae (I) and (II) may be prepared by a number of reaction schemes. Access to compounds of Formulae (I) and (II) is described in Schemes 1-5. Persons skilled in the art will recognize that certain compounds are more advantageously produced by one scheme as compared to the other. In addition to the Schemes shown below, alternative methods may be used to prepare compounds of Formulae (I) or (II). Such methods are described in U.S. Patent Appl. No. 10/941,664 (Carruthers et al.).

**Table of Acronyms**

| **Term** | **Acronym** |
|---|---|
| Tetrahydrofuran | THF |
| N,N-Dimethylformamide | DMF |
| N,N-Dimethylacetamide | DMA |
| Dimethyl sulfoxide | DMSO |
| tert-Butylcarbamoyl | Boc |
| High-pressure liquid chromatography | HPLC |
| Thin layer chromatography | TLC |
| N,N-Diisopropylethylamine | DIEA |
| 1,2-Dichloroethane | DCE |
| Ethylene glycol dimethyl ether | DME |
| Acetyl | Ac |
| Diisobutylaluminum hydride | DIBAL-H |
| Ethyl acetate | EtOAc |
| Trifluoroacetic acid | TFA |
| Methanesulfonyl chloride | MsCl |

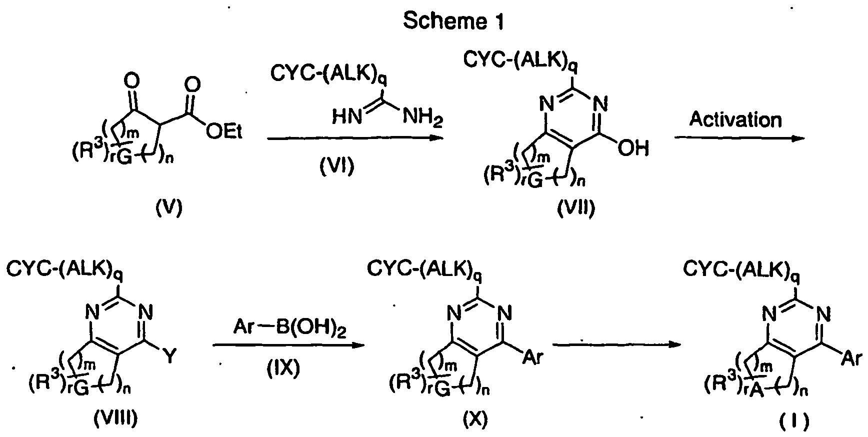

Referring to Scheme 1, compounds of Formula (I) may be preprared from beta-ketoesters (V), where G may be A or a protected form of A. Where A contains an amine group, the amine moiety may be suitably protected as an alkyl or benzyl amine, amide, carbamate, or other suitable group. Preferred protecting groups for amines include the t-butyl carbamate (Boc) or benzyl groups. Beta-ketoesters (V) are available according to methods known to one skilled in the art. Compounds of formula (V) are reacted with amidines (VI), prepared, for example, in the presence of KOtBu or a tertiary amine base such as Et₃N, in a solvent such as tBuOH, at temperatures ranging from room temperature to the reflux temperature of the solvent, to form hydroxy pyrimidines (VII). (See also: U.S. Patent Appl. 60/326,662; Tetrahedron 1989, 45(20), 6511). Pyrimidines (VII) can be converted into precursors for transition metal-catalyzed cross-coupling reactions, such as Stille, Suzuki, Negishi or other such coupling reactions known to one skilled in the art. For example, treatment with POCl₃, PCl₃, PCl₅, PBr₃ or POBr₃ can afford the corresponding halopyrimidines, where Y is bromide or chloride. Preferably, pyrimidines (VII) are treated with a triflating agent such as trifluoromethane-sulfonic anhydride or N-phenyl-bis(trifluoromethane-sulfonimide) in DCE, CH₂Cl₂, THF, or the like, in the presence of a base such as pyridine, Et₃N, DIEA, or KOtBu, to provide triflates of formula (VII) where Y is OTf. Coupling of halides or triflates (VIII) with aryl boronic acids (IX), or their boronic ester analogs, in the presence of a catalyst such as Pd(PPh₃)₄, PdCl₂(PPh₃)₂, PdCl₂(P*o-*tol₃)₂, PdCl₂(dppe) or PdCl₂(dppf), in a solvent such as THF, 1,4-dioxane, DMA, DMF, DME, toluene, toluene/ethanol, or toluene/H₂O mixtures, in the presence of a base such as Na₂CO₃, K₂CO₃, Cs₂CO₃, K₃PO₄, KF, CsF, or KOAc, affords pyrimidines (X). Preferred catalysts are Pd(PPh₃)₄ and PdCl₂(dppf), with or without additives such as dppf and catalytic Bu₄NBr. Preferred conditions include PdCl₂(dppf), catalytic dppf, and K₃PO₄ in 1,4-dioxane.

Where G contains a protecting group, it may be removed using generally accepted methods, or may be otherwise converted into A of Formula (I). More specifically, a group such as a t-butyl carbamate may be removed with an acid such as trifluoroacetic acid or HCl, in a solvent such as CH₂Cl₂, dioxane, EtOH, or MeOH to afford compounds of Formula (I). Where G contains a benzyl group, said group may be removed according to standard methods, including hydrogenation in the presence of a palladium catalyst such as Pd/C, in a solvent such as EtOH, or through reaction with 1-chloroethylchloroformate in DCE.

Compounds of formula (X) where G is >NH may be further converted into additional embodiments of Formula (I) wherein A is >NR¹ using conventional synthetic methods such as reductive amination or alkylation protocols. Thus, treatment of amines (X) with a suitable aldehyde in the presence of a reductant such as NaBH₄, NaBH₃CN, NaBH(OAc)₃, or H_{2(g)}, in the presence of a catalyst, in a solvent such as CH₂Cl₂, DCE, THF, EtOH, or MeOH affords compounds of Formula (I) where A is >NR¹. One skilled in the art will recognize that the addition of an acid such as AcOH, Ti(O-iPr)₄, trifluoroacetic acid, or HCl, may be required. Alternatively, compounds (X) where G is >NH may be treated with an alkylating agent, such as an alkyl chloride, bromide, iodide, mesylate or tosylate, in a solvent such as DMF, DMA, THF, or EtOH, and in the presence of a base such as NaHCO₃, Na₂CO₃, K₂CO₃ or Cs₂CO₃ will produce compounds of Formula (I) where A is >NR¹.

In the following Schemes, the R³ substituents of Formula (I) and intermediates have been removed to simplify the structural depictions, but one skilled in the art will recognize that the procedures shown provide access to compounds of Formula (I) containing the R³ substituents.

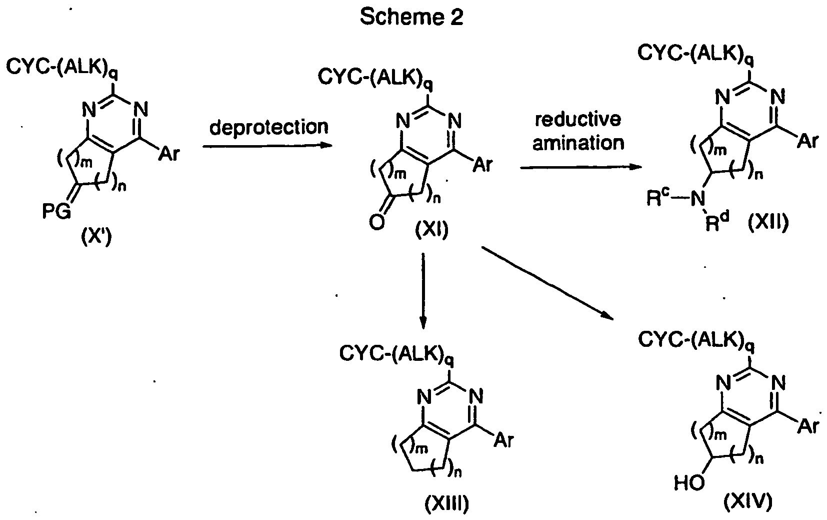

Referring to Scheme 2, compounds of formula (X'), where PG is a ketone protecting group, may be prepared according to the methods described in Scheme 1. Compounds (X') may subsequently be converted into additional embodiments of Formula (I), exemplified by compounds (XII), (XIII), and (XIV). Reductive amination of ketones (XI) may be accomplished as described in Scheme 1. Alternatively, ketones (XI) may be reduced using conventional methods such as NaBH₄ in EtOH or DIBAL-H in THF to the corresponding secondary alcohols (XIV), or reduced completely via hydrogenation, Wolff-Kishner reduction, or other protocols to form carbocycles (XIII).

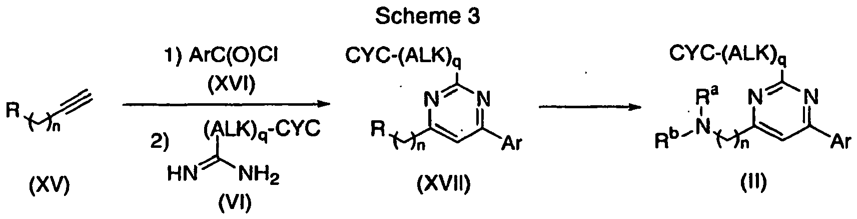

Compounds of Formula (II) may be accessed according to Scheme 3. Alkynes (XV) where R is a suitable protected alcohol or amine are first coupled with a suitable acid chloride (XVI), in the presence of a palladium catalyst such as Pd(PPh₃)₂Cl₂, a base such as Et₃N, an additive such as Cul, in a solvent such as THF to form intermediate alkynyl ketones. Said ketones are reacted in situ with amidines (VI), under conditions as described in Scheme 1, to form pyrimidines (XVII). Alcohol and amino protecting groups may then be removed under standard conditions. The resulting free amines are themselves compounds of Formula (II), but may be further processed to additional embodiments of Formula (II) via reductive amination as described in Scheme 2. Where a free alcohol is liberated, said alcohol may be converted to -N(R^{a})R^{b} by: 1) formation of a suitable leaving group (an alkyl halide, mesylate, or tosylate); and 2) displacement with HN(R^{a})R^{b}. Alternatively, the leaving group may be displaced by treatment with sodium azide. Subsequent reduction of the azido group under Staudinger conditions gives a free amine. In another embodiment, the free alcohol may be oxidized to the corresponding aldehyde using, for example, Dess-Martin periodinane or Swern oxidation conditions, and the resulting aldehyde converted to -N(R^{a})R^{b} using reductive amination methods as described in Scheme 1.

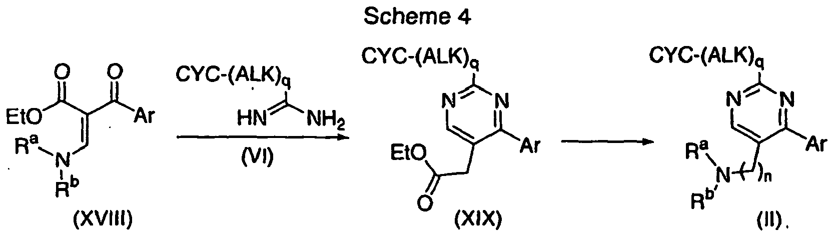

Alternatively, compounds of Formula (II) may be prepared according to Scheme 4. Acrylate esters (XVIII) may be condensed with amidines (VI) as described in Scheme 1 to form pyrimidines (XIX). The pendant ester group may be transformed into amines where n = 1 by reduction to the alcohol, and either: 1) oxidizing to the corresponding aldehyde, and performing a reductive amination to install the -N(R^{a})R^{b} substituent; or 2) activating the alcohol as a leaving group such as a tosylate, bromide, or chloride, and displacing with a suitable HN(R^{a})R^{b} reagent. For n = 2, the ester may be converted to an amide through peptide coupling methods, and the amide reduced to the corresponding amine. For n = 3, homologation procedures known to one skilled in the art may be used to install two carbon units.

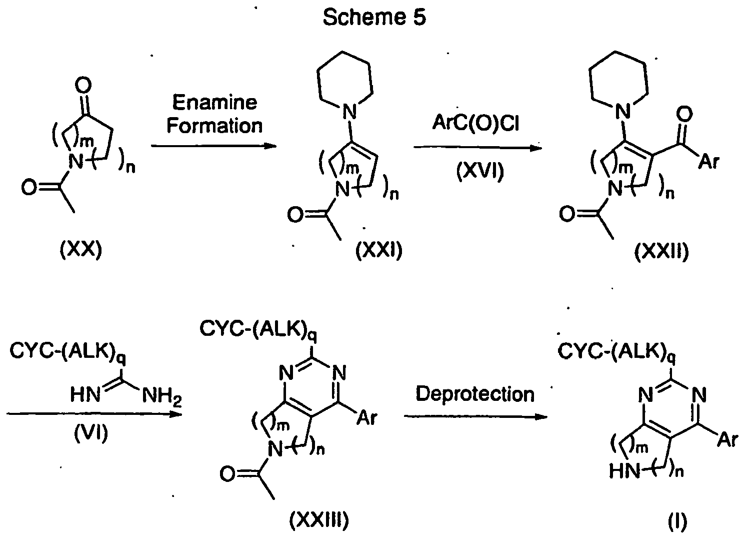

Compounds of Formula (I) where A is >NH, and m and n are as defined in Formula (I), may be prepared according to Scheme 5. Ketones (XX) are commercially available or may be prepared using methods known to one skilled in the art. The nitrogen protecting group may be an acyl group or carbamoyl group (such as a Boc group). Preferably, the nitrogen protecting group is acetyl. Conversion to enamines of formula (XXI) is performed by reaction with a secondary amine under standard water removal conditions. Preferably, the reaction is done with piperidine as the secondary amine, and using a Dean Stark trap, with a catalyst such as p-toluenesulfonic acid, in a solvent such as toluene. Elevated temperatures are preferred. Enamines are transformed into 1,3-diketones (XXII) by reaction with acyl chlorides (XVI), in the presence of a suitable base such as Et₃N, in a solvent such as CH₂Cl₂. See also: Breitenbucher, et al. PCT Intl. Appl. WO02/014314. Condensation with amidines of formula (VI) to form pyrimidines (XXIII) may be accomplished as described in Scheme 1. Preferably, condensations are accomplished in the presence of Et₃N, in a solvent such as t-amyl alcohol, at temperatures between room temperature and reflux temperature of the solvent. Deprotection of the nitrogen protecting group may be effected using conditions known to one skilled in the art. Preferably, where the protecting group is acetyl, deprotection is done in the presence of 10% aqueous HCl at reflux temperature. One skilled in the art will recognize that compounds of Formula (I) prepared in Scheme 5 may be subsequently converted to other embodiments where A is >NR¹ as described in Scheme 1.

Compounds of Formulae (I) or (II) may be converted to their corresponding salts using methods known to those skilled in the art. For example, amines of Formulae (I) or (II) may be treated with trifluoroacetic acid, HCl, or citric acid in a solvent such as MeOH to provide the corresponding salt forms.

Compounds prepared according to the schemes described above may be obtained as single enantiomers, diastereomers, or regioisomers, or as racemic mixtures or mixtures of enantiomers, diastereomers, or regioisomers. Where regioisomeric or diastereomeric mixtures are obtained, isomers may be separated using conventional methods such as chromatography or crystallization. Where racemic (1:1) and non-racemic (not 1:1) mixtures of enantiomers are obtained, single enantiomers may be isolated using conventional separation methods known to one skilled in the art. Particularly useful separation methods may include chiral chromatography, recrystallization, resolution, diastereomeric salt formation, or derivatization into diastereomeric adducts followed by separation.

Also described are prodrugs of the compounds of this invention. In general, such prodrugs will be functional derivatives of the compounds that are readily convertible *in vivo* into the required compound. Thus, in the methods of treatment of the present invention, the term "administering" shall encompass the treatment of the various disorders described with a compound of Formula (I) or (II) or with a compound that converts to a compound of Formula (I) or (II) *in vivo* after administration to the patient. Conventional procedures for the selection and preparation of suitable prodrug derivatives are described, for example, in "Design of Prodrugs", ed. H. Bundgaard, Elsevier, 1985. In addition to salts, the invention provides the esters, amides, and other protected or derivatized forms of the described compounds.

For therapeutic use, salts of the compounds of the present invention are those that are pharmaceutically acceptable. However, salts of acids and bases that are non-pharmaceutically acceptable may also find use, for example, in the preparation or purification of a pharmaceutically acceptable compound. All salts, whether pharmaceutically acceptable or not are included within the ambit of the present invention.

Pharmaceutically acceptable salts, esters, and amides of compounds according to the present invention refer to those salt, ester, and amide forms of the compounds of the present invention which would be apparent to the pharmaceutical chemist, *i.*e., those that are non-toxic and that would favorably affect the pharmacokinetic properties of said compounds of the present invention. Those compounds having favorable pharmacokinetic properties would be apparent to the pharmaceutical chemist, *i.e*., those which are non-toxic and which possess such pharmacokinetic properties to provide sufficient palatability, absorption, distribution, metabolism and excretion. Other factors, more practical in nature, which are also important in the selection, are cost of raw materials, ease of crystallization, yield, stability, hygroscopicity and flowability of the resulting bulk drug.

Examples of acids that may be used in the preparation of pharmaceutically acceptable salts include the following: acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, boric acid, (+)-camphoric acid, camphorsulfonic acid, (+)-(1 S)-camphor-10-sulfonic acid, capric acid, caproic acid, caprylic acid, cinnamic acid, citric acid, cyclamic acid, cyclohexanesulfamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (±)-DL-lactic acid, lactobionic acid, lauric acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, perchloric acid, phosphoric acid, L-pyroglutamic acid, saccharic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, undecylenic acid, and valeric acid.

Compounds of the present invention containing acidic protons may be converted into their therapeutically active non-toxic metal or amine addition salt forms by treatment with appropriate organic and inorganic bases. Appropriate base salt forms comprise, for example, the ammonium salts; the alkali and earth alkaline metal salts (e.g. lithium, sodium, potassium, magnesium, calcium salts, which may be prepared by treatment with, for example, magnesium hydroxide, calcium hydroxide, potassium hydroxide, zinc hydroxide, or sodium hydroxide); and amine salts made with organic bases (e.g. primary, secondary and tertiary aliphatic and aromatic amines such as L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, N-methylglucamine, hydrabamine, 1*H*-imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, secondary amines, triethanolamine, trimethylamine, triethylamine, *N*-methyl-D-glucamine, 2-amino-2-(hydroxymethyl)-1,3-propanediol, and tromethamine). *See, e.g.,* S.M. Berge, et al., "Pharmaceutical Salts", J. Pharm. Sci., 1977, 66:1-19, and Handbook of Pharmaceutical Salts, Propertions, Selection, and Use; Stahl; P.H., Wermuth, C.G., Eds.; Wiley-VCH and VHCA: Zurich, 2002, which are incorporated herein by reference.

Pharmaceutically acceptable esters and amides are those that are within a reasonable benefit/risk ratio, pharmacologically effective and suitable for contact with the tissues of patients without undue toxicity, irritation, or allergic response. Representative pharmaceutically acceptable amides of the invention include those derived from ammonia, primary C₁₋₆alkyl amines and secondary di(C₁₋₆alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C₁₋₃alkyl primary amines, and di(C₁₋₂alkyl)amines.

Representative pharmaceutically acceptable esters of the invention include C₁₋₇alkyl, C₅₋₇cycloalkyl, phenyl, substituted phenyl, and phenylC₁₋₆alkyl- esters. Preferred esters include methyl esters. Furthermore, examples of suitable esters include such esters where one or more carboxyl substituents is replaced with *p*-methoxybenzyloxy-carbonyl, 2,4,6-trimethylbenzyloxy-carbonyl, 9-anthryloxycarbonyl, CH₃SCH₂COO-, tetrahydrofur-2-yloxycarbonyl, tetrahydropyran-2-yloxy-carbonyl, fur-2-yloxycarbonyl, benzoylmethoxy-carbonyl, p-nitrobenzyloxy-carbonyl, 4-pyridylmethoxycarbonyl, 2,2,2-trichloro-ethoxycarbonyl, 2,2,2-tribromoethoxycarbonyl, t-butyloxycarbonyl, t-amyloxy-carbonyl, diphenylmethoxycarbonyl, triphenylmethoxycarbonyl, adamantyloxy-carbonyl, 2-benzyloxyphenyloxycarbonyl, 4-methylthiophenyloxycarbonyl, or tetrahydropyran-2-yloxycarbonyl.

The compounds of the present invention are serotonin receptor modulators, and as such, the compounds are useful in the treatment of serotonin-mediated disease states. Particularly, the compounds may be used in methods for treating or preventing CNS disorders, such as sleep disorders, depression/anxiety, generalized anxiety disorder, schizophrenia, bipolar disorders, cognitive disorders, mild cognitive impairment, Alzheimer's disease, Parkinson's disease, psychotic disorders, phobic disorders, obsessive-compulsive disorder, mood disorders, post-traumatic stress and other stress-related disorders, migraine, pain, eating disorders, obesity, sexual dysfunction, metabolic disturbances, hormonal imbalance, hot flushes associated with menopause, alcohol abuse, drug abuse, addictive disorders including drug addiction and alcohol addiction, nausea, inflammation, centrally mediated hypertension, sleep/wake disturbances, jetlag, and circadian rhythm abnormalities. The compounds may also be used in the treatment and prevention of hypotension, peripheral vascular disorders, cardiovascular shock, renal disorders, gastric motility, diarrhea, spastic colon, irritable bowel disorders, ischemias, septic shock, urinary incontinence, and other disorders related to the gastrointestinal and vascular systems. In addition, compounds of the present invention may be used in methods for treating or preventing a range of ocular disorders including glaucoma, optic neuritis, diabetic retinopathy, retinal edema, and age-related macular degeneration.

The compounds of the present invention are 5-HT₇ modulators and many are 5-HT₇ antagonists. As such, the compounds are useful in methods for treating or preventing 5-HT₇-mediated disease states. Where the compounds possess substantial 5-HT₇ antagonist activity, they may be particularly useful in methods for treating or preventing depression/anxiety, sleep/wake disturbances, jetlag, migraine, urinary incontinence, gastric motility, and irritable bowel disorders.

Many of the compounds of the present invention are 5-HT₂ modulators and many are 5-HT₂ antagonists. As such, the compounds are useful in methods for treating or preventing 5-HT₂-mediated diseases and conditions. Where the compounds possess substantial 5-HT₂ antagonist activity, they may be particularly useful in methods for treating or preventing depression/anxiety, generalized anxiety disorder, schizophrenia, bipolar disorders, psychotic disorders, obsessive-compulsive disorder, mood disorders, post-traumatic stress disorders, sleep disturbances, sexual dysfunction, hot flushes associated with menopause, eating disorders, migraine, addictive disorders, and peripheral vascular disorders.

The compounds of the present invention are 5-HT₆ modulators and many are 5-HT₆ antagonists. As such, the compounds are useful in methods for treating or preventing 5-HT₆-mediated disease states. Where the compounds possess substantial 5-HT₆ antagonist activity, they may be particularly useful in methods for treating or preventing schizophrenia, cognitive disorders, mild cognitive impairment, Alzheimer's disease, and Parkinson's disease.

Said methods of treating and preventing comprise the step of administering to a mammal suffering therefrom an effective amount of at least one compound of the present invention.

The present invention also relates to a method of treating or preventing a serotonin-mediated disease or condition with a combination therapy, comprising administering at least one compound of the present invention in combination with one or more neuroactive agents. Suitable neuroactive agents include: selective serotonin reuptake inhibitors (SSRIs), anti-psychotics, norepinephrine reuptake inhibitors (NRIs), sedatives, monoamine oxidase inhibitors (MAOs), and tricyclic antidepressants (TCAs). In another embodiment, the present invention includes compositions comprising at least one compound of the present invention and one or more neuroactive agent.

Compounds of the present invention may be administered in pharmaceutical compositions to treat patients (humans and other mammals) with disorders mediated by the serotonin receptor. Thus, the invention features pharmaceutical compositions containing at least one compound of the present invention and a pharmaceutically acceptable carrier. A composition of the invention may further include at least one other therapeutic agent (for example, a combination formulation or combination of differently formulated active agents for use in a combination therapy method).

The present invention also features methods of using or preparing or formulating such pharmaceutical compositions. The pharmaceutical compositions can be prepared using conventional pharmaceutical excipients and compounding techniques known to those skilled in the art of preparing dosage forms. It is anticipated that the compounds of the invention can be administered by oral, parenteral, rectal, topical, or ocular routes, or by inhalation. Preparations may also be designed to give slow release of the active ingredient. The preparation may be in the form of tablets, capsules, sachets, vials, powders, granules, lozenges, powders for reconstitution, liquid preparations, or suppositories. Preferably, compounds may be administered by intravenous infusion or topical administration, but more preferably by oral administration.

For oral administration, the compounds of the invention can be provided in the form of tablets or capsules, or as a solution, emulsion, or suspension. Tablets for oral use may include the active ingredient mixed with pharmaceutically acceptable excipients such as inert diluents, disintegrating agents, binding agents, lubricating agents, sweetening agents, flavoring agents, coloring agents and preservatives. Suitable inert fillers include sodium and calcium carbonate, sodium and calcium phosphate, lactose, starch, sugar, glucose, methyl cellulose, magnesium stearate, mannitol, sorbitol, and the like; typical liquid oral excipients include ethanol, glycerol, water and the like. Starch, polyvinyl-pyrrolidone, sodium starch glycolate, microcrystalline cellulose, and alginic acid are suitable disintegrating agents. Binding agents may include starch and gelatin. The lubricating agent, if present, will generally be magnesium stearate, stearic acid or talc. If desired, the tablets may be coated with a material such as glyceryl monostearate or glyceryl distearate to delay absorption in the gastrointestinal tract, or may be coated with an enteric coating. Capsules for oral use include hard gelatin capsules in which the active ingredient is mixed with a solid, semi-solid, or liquid diluent, and soft gelatin capsules wherein the active ingredient is mixed with water, an oil such as peanut oil or olive oil, liquid paraffin, a mixture of mono and di-glycerides of short chain fatty acids, polyethylene glycol 400, or propylene glycol.

Liquids for oral administration may be suspensions, solutions, emulsions or syrups or may be presented as a dry product for reconstitution with water or other suitable vehicles before use. Compositions of such liquid may contain pharmaceutically-acceptable excipients such as suspending agents (for example, sorbitol, methyl cellulose, sodium alginate, gelatin, hydroxyethylcellulose, carboxymethylcellulose, aluminium stearate gel and the like); non-aqueous vehicles, which include oils (for example, almond oil or fractionated coconut oil), propylene glycol, ethyl alcohol or water; preservatives (for example, methyl or propyl p-hydroxybenzoate or sorbic acid); wetting agents such as lecithin; and, if needed, flavoring or coloring agents.

The compounds of this invention may also be administered by non-oral routes. The compositions may be formulated for rectal administration as a suppository. For parenteral use, including intravenous, intramuscular, intraperitoneal, or subcutaneous routes, the compounds of the invention will generally be provided in sterile aqueous solutions or suspensions, buffered to an appropriate pH and isotonicity or in parenterally acceptable oil. Suitable aqueous vehicles include Ringer's solution and isotonic sodium chloride. Such forms will be presented in unit dose form such as ampules or disposable injection devices, in multi-dose forms such as vials from which the appropriate dose may be withdrawn, or in a solid form or pre-concentrate that can be used to prepare an injectable formulation. Another mode of administration of the compounds of the invention may utilize a patch formulation to affect transdermal delivery. The compounds of this invention may also be administered by inhalation, via the nasal or oral routes using a spray formulation consisting of the compound of the invention and a suitable carrier.

Methods are known in the art for determining effective doses for therapeutic (treatment) and prophylactic (preventative) purposes for the pharmaceutical compositions or the drug combinations of the present invention, whether or not formulated in the same composition. The specific dosage level required for any particular patient will depend on a number of factors, including severity of the condition being treated, the route of administration, and the weight of the patient. For therapeutic purposes, "effective dose" or "effective amount" refers to that amount of each active compound or pharmaceutical agent, alone or in combination, that elicits the biological or medicinal response in a tissue system, animal, or human that is being sought by a researcher, veterinarian, medical doctor, or other clinician, which includes alleviation of the symptoms of the disease or disorder being treated. For prophylactic purposes (i.e., preventing or inhibiting the onset or progression of a disorder), the term "effective dose" or "effective amount" refers to that amount of each active compound or pharmaceutical agent, alone or in combination, that inhibits in a subject the onset or progression of a disorder as being sought by a researcher, veterinarian, medical doctor, or other clinician, the delaying of which disorder is mediated, at least in part, by the modulation of the serotonin receptor. Methods of combination therapy include coadministration of a single formulation containing all active agents; essentially contemporaneous administration of more than one formulation; and administration of two or more active agents separately formulated.

It is anticipated that the daily dose (whether administered as a single dose or as divided doses) will be in the range 0.01 to 1000 mg per day, more usually from 1 to 500 mg per day, and most usually from 10 to 200 mg per day. Expressed as dosage per unit body weight, a typical dose will be expected to be between 0.0001 mg/kg and 15 mg/kg, especially between 0.01 mg/kg and 7 mg/kg, and most especially between 0.15 mg/kg and 2.5 mg/kg.

Preferably, oral doses range from about 0.05 to 200 mg/kg, daily, taken in 1 to 4 separate doses. Some compounds of the invention may be orally dosed in the range of about 0.05 to about 50 mg/kg daily, others may be dosed at 0.05 to about 20 mg/kg daily, while still others may be dosed at 0.1 to about 10 mg/kg daily. Infusion doses can range from about 1 to 1000 µg/kg/min of inhibitor, admixed with a pharmaceutical carrier over a period ranging from several minutes to several days. For topical administration compounds of the present invention may be mixed with a pharmaceutical carrier at a concentration of about 0.1% to about 10% of drug to vehicle.

### Examples

In order to illustrate the invention, the following examples are included. These examples do not limit the invention. They are only meant to suggest a method of practicing the invention.

Preparative Reversed-Phase HPLC was performed as follows:
Method A. Instrument, Hewlett Packard Series 1100; Column, Agilent ZORBAX® Bonus RP, 5 µm, 4.6x250 mm; Flow rate, 1 mL/min; Detection, λ = 220 & 254 nm; Gradient, 1 to 99% acetonitrile/water, 0.05% trifluoroacetic acid over 20 min.
Method B. Instrument, Hewlett Packard HPLC; Column, Agilent ZORBAX® Eclipse XDB-C8, 5 µm, 4.6x150 mm; Flow rate, 1 mL/min; Detection, λ = 220 & 254 nm; Gradient, 1 to 99% acetonitrile/water, 0.05% trifluoroacetic acid over 8 min.

Mass spectra were obtained on an Agilent series 1100 MSD using electrospray ionization (ESI) in either positive or negative modes as indicated.

Thin-layer chromatography was performed using Merck silica gel 60 F₂₅₄ 2.5 cm x 7.5 cm 250 µm or 5.0 cm x 10.0 cm 250 µm pre-coated silica gel plates. Preparative thin-layer chromatography was performed using EM Science silica gel 60 F₂₅₄ 20 cm x 20 cm 0.5 mm pre-coated plates with a 20 cm x 4 cm concentrating zone.

NMR spectra were obtained on either a Bruker model DPX400 (400 MHz), DPX500 (500 MHz), or DPX600 (600 MHz) spectrometer. The format of the ¹H NMR data below is: chemical shift in ppm down field of the tetramethylsilane reference (multiplicity, coupling constant *J* in Hz, integration).

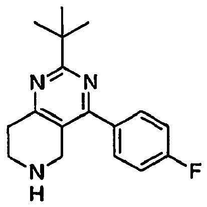

### Example 1; 2-tert-Butyl-4-(4-fluoro-phenyl)-5,6,7,8-tetrahydro-pyrido[4,3-d]pyrimidine.

Step A. 2-tert-Butyl-4-(4-fluoro-phenyl)-5,6,7,8-tetrahydro-pyrido[4,3-d]pyrimidine hydrochloride. To a tert-BuOH (17 mL) solution of 4-oxo-piperidine-1,3-dicarboxylic acid-1-tert-butyl ester-3-ethyl ester (2.18 g, 8.05 mmol), and 2,2-dimethyl-propionamidine hydrochloride (1.0 g, 7.3 mmol) was added Et₃N (3.0 mL, 22.0 mmol). The reaction solution was heated at reflux for 48 h, cooled to rt, and concentrated. The resulting solid was dissolved in CH₂Cl₂ and washed with water. The aqueous layer was extracted with CH₂Cl₂. The combined organic layers were dried and concentrated to give a yellow solid that was triturated with Et₂O to give 1.74 g (70%) of the title compound as a white solid. MS (ESI): exact mass calcd. for C₁₆H₂₅N₃O₃, 307.19; m/z found, 308.4 [M+H]⁺. ¹H NMR (CDCl₃): 4.35 (s, 2H), 3.68-3.67 (m, 2H), 2.74-2.65 (m, 2H), 1.49 (s, 9H), 1.37 (s, 9H).

Step B. 2-tert-Butyl-4-trifluoromethanesulfonyloxy-7,8-dihydro-5H-pyrido[4,3-d]pyrimidine-6-carboxylic acid tert-butyl ester. To a 0 °C solution of the product from Step A (1.0 g, 3.25 mmol) in CH₂Cl₂ (16 mL) was added Et₃N (0.53 mL, 3.80 mmol) and trifluoromethanesulfonic anhydride (0.64 mL, 3.8 mmol) dropwise over 10 min. After 2 h at 0 °C, the mixture was diluted with CH₂Cl₂ and washed with water. The aqueous layer was extracted with CH₂Cl₂. The combined organic layers were dried and concentrated. The resulting residue was purified via SiO₂ chromatography (10-30% EtOAc/hexanes) to give 1.28 g (91%) of the title compound. MS (ESI): exact mass calcd. for C₁₇H₂₄F₃N₃O₃S, 439.14; m/z found, 440.3 [M+H]⁺. ¹H NMR (CDCl₃): 4.56 (s, 2H), 3.77 (t, J = 5.7, 2H), 2.99-2.95 (m, 2H), 1.50 (s, 9H), 1.36 (s, 9H).

Step C. 2-tert-Butyl-4-(4-fluoro-phenyl)-7,8-dihydro-5H-pyrido[4,3-d]pyrimidine-6-carboxylic acid tert-butyl ester. To the product from Step B (0.17 g, 0.39 mmol) was added 4-fluorophenylboronic acid (0.082 g, 0.586 mmol), K₃PO₄ (0.124 g, 0.584 mmol), Pd(Cl)₂dppf•CH₂Cl₂ (0.018 g, 0.022 mmol) and dppf (0.008 g, 0.014 mmol). The mixture was evacuated with N₂, dioxane (4 mL) was added and the mixture was heated at reflux for 2 h. After cooling to room temperature (rt), the mixture was diluted with Et₂O, filtered through a small SiO₂ plug, and the filtrate was concentrated. The resulting residue was purified via SiO₂ chromatography (5-30% EtOAc/hexanes) to give 0.134 g (89%) of the title compound. MS (ESI): exact mass calcd. for C₂₂H₂₈FN₃O₂, 385.22; m/z found, 386.4 [M+H]⁺. ¹H NMR (CDCl₃): 7.60 (dd, J = 5.4, 8.8, 2H), 7.17-7.14 (m, 2H), 4.59 (s, 2H), 3.76 (t, J = 6.1, 2H), 3.09 (t, J = 6.1, 2H), 1.44 (s, 9H), 1.41 (s, 9H).

Step D. 2-tert-Butyl-4-(4-fluoro-phenyl)-5,6,7,8-tetrahydro-pyrido[4,3-d]pyrimidine hydrochloride. To an EtOAc solution of the product from Step D (0.130 g, 0.337 mmol) was added 4 M HCl in dioxane. After stirring for 18 h the volatiles were removed and the solid partitioned between water and EtOAc. The aqueous layer was made basic with 1 N NaOH and extracted with EtOAc (2X). The combined organic layers were dried with MgSO₄, filtered and concentrated. The resulting residue was purified via SiO₂ chromatography (1-7% 2 M NH₃ in MeOH/CH₂Cl₂) to give 0.079 g (82%) of the title compound. The corresponding HCl salt was obtained upon treatment of the free base in Et₂O with 1 M HCl in Et₂O. MS (ESI): exact mass calcd. for C₁₇H₂₀FN₃, 285.16; m/z found, 286.4 [M+H]⁺. ¹H NMR (DMSO-d₆): 9.74 (s, 2H), 7.71 (dd, J = 5.5, 8.6, 2H), 7.42 (d, J = 8.8, 2H), 4.31 (m, 2H), 3.49-3.48 (m, 2H), 3.16 (t, J = 6.2, 2H), 1.38 (s, 9H).

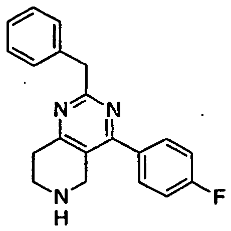

### Example 2; 2-Benzyl-4-(4-fluoro-phenyl)-5,6,7,8-tetrahydro-pyrido[4,3-d]pyrimidine.

Step A. 2-Benzyl-4-trifluoromethanesulfonyloxy-7.8-dihydro-5H-pyrido[4,3-d]yrimidine-6-carboxylic acid tert-butyl ester. To a solution of 2-benzyl-4-hydroxy-7,8-dihydro-5H-pyrido[4,3-d]pyrimidine-6-carboxylic acid tert-butyl ester (2.0 g, 5.9 mmol; prepared from 2-phenyl-acetamidine hydrochloride as described in Example 1, Step A) in THF (15 mL) was added KOtBu (0.408 g, 3.6 mmol). After 15 min, the mixture was treated with *N*-phenyl-bis(trifluoromethanesulfonimide) (1.18 g, 3.3 mmol) and the mixture was stirred for 18 h. The mixture was diluted with water and extracted with EtOAc (2x). The combined organic layers were dried and concentrated. The resulting residue was purified via SiO₂ chromatography (10-40% EtOAc/hexanes) to give 1.15 g (81%) of the title compound which was contaminated with byproducts from *N*-phenyl-bis(trifluoromethanesulfonimide).

Step B. 2-Benzyl-(4-fluoro-pheny)-7,8-dihydro-5H-pyrido[4,3-d]pyrimidine-6-carboxylic acid tert-butyl ester. The title compound was prepared as described in Example 1, Step C. MS (ESI): exact mass calcd. for C₂₅H₂₆FN₃O₂, 419.20; m/z found, 420.5 [M+H]⁺. ¹H NMR (CDCl₃): 7.53 (dd, J = 5.3, 8.7, 2H), 7.43-7.41 (m, 2H), 7.31-7.28 (m, 2H), 7.23-7.14 (m, 3H), 4.55 (s, 2H), 4.27 (s, 2H), 3.75 (t, J = 6.1, 2H), 3.00 (t, J = 6.0, 2H), 1.43 (s, 9H).

Step C. 2-Benzyl-4-(4-fluoro-phenyl)-5,6,7,8-tetrahydro-pyrido[4,3-d]pyrimidine. A solution of the product from Step B (0.131 g, 0.312 mmol) in CH₂Cl₂ was treated with TFA. After stirring for 4 h, the mixture was concentrated and partitioned between saturated (satd.) aq. NaHCO₃ and CH₂Cl₂ (2x). The combined organic layers were dried and concentrated. The resulting residue was purified via SiO₂ chromatography (1-7% 2 M NH₃ in MeOH/CH₂Cl₂) to give 0.084 g (84%) of the title compound. MS (ESI): exact mass calcd. for C₂₀H₁₈FN₃, 319.15; m/z found, 320.4 [M+H]⁺. ¹H NMR (CDCl₃): 7.51 (dd, J = 5.4, 8.8, 2H), 7.41 (d, J = 7.4, 2H), 7.31-7.27 (m, 2H), 7.22-7.19 (m, 1 H), 7.15 (dd, J = 8.7, 2H), 4.27 (s, 2H), 3.95 (s, 2H), 3.24 (t, J = 6.1, 2H), 2.97 (t, J = 6.1, 2H).

Unless otherwise specified, the compounds in Examples 3-57 were prepared using methods similar to those described in Examples 1 and 2, utilizing the appropriate β-ketoesters, amidine hydrochlorides, and arylboronic acids.

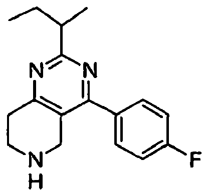

### Example 3; 2-sec-Butyl-4-(4-fluoro-phenyl)-5,6,7,8-tetrahydro-pyrido[4,3-d]pyrimidine hydrochloride.

MS (ESI): exact mass calcd. for C₁₇H₂₀FN₃, 285.16; m/z found, 286.4 [M+H]⁺. ¹H NMR (DMSO-d₆): 9.72 (s, 2H), 7.69 (dd, J = 5.5, 8.8, 2H), 7.42 (dd, J = 8.8, 2H), 4.33-4.27 (m, 2H), 3.52-3.44 (m, 2H), 3.16 (t, J = 6.4, 2H), 2.96-2.88 (m, 1 H), 1.88-1.77 (m, 1 H), 1.66-1.56 (m, 1H). 1.26 (d, J = 6.9, 3H), 0.83 (t, J = 7.4, 3H).

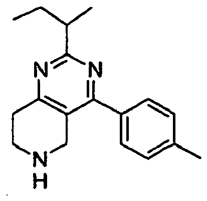

### Example 4; 2-sec-Butyl-4-p-tolyl-5,6,7,8-tetrahydro-pyrido[4,3-d]pyrimidine hydrochloride.

MS (ESI): exact mass calcd. for C₂₁H₂₁N₃, 281.19; m/z found, 282.5 [M+H]⁺. ¹H NMR (DMSO-d₆): 9.69 (s, 2H), 7.52 (d, J = 8.1, 2H); 7.38 (d, J = 7.4?, 2H), 4.33-4.26 (m, 2H), 3.52-3.54 (m, 2H), 3.15 (t, J = 6.3, 2H), 2.96-2.87 (m, 1 H), 2.92 (m, 1 H), 2.40 (s, 3H), 1.88-1.77 (m, 1 H), 1.67-1.55 (m, 1 H), 1.26 (d, J = 6.9, 3H), 0.83 (t, J = 7.4, 3H).

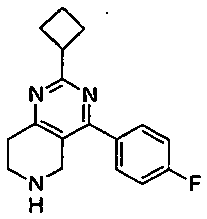

### Example 5; 2-Cyclobutyl-4-(4-fluoro-phenyl)-5,6,7,8-tetrahydro-pyrido[4,3-d]pyrimidine hydrochloride.

MS (ESI): exact mass calcd. for C₁₇H₁₈FN₃, 283.15; m/z found, 284.3 [M+H]⁺. ¹H NMR (DMSO-d₆): 9.72 (m, 2H), 7.70 (dd, J = 5.5, 8.8, 2H), 7.44-7.40 (m, 2H), 4.32-4.26 (m, 2H), 3.79-3.73 (m, 1 H), 3.50-3.45 (m, 2H), 3.16 (t, J = 6.4, 2H), 2.42-2.27 (m, 4H), 2.08-1.99 (m, 1 H), 1.91-1.83 (m, 1H).

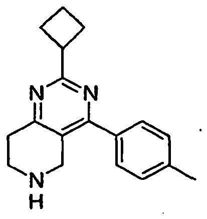

### Example 6; 2-Cyclobutyl-4-p-tolyl-5,6,7,8-tetrahydro-pyrido[4,3-d]pyrimidine.

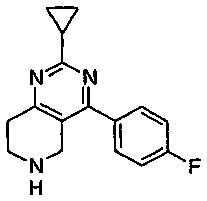

### Example 7; 2-Cyclopropyl-4-(4-fluoro-phenyl)-5,6,7,8-tetrahydro-pyrido[4,3-d]pyrimidine.

MS (ESI): exact mass calcd. for C₁₆H₁₆FN₃, 269.13; m/z found, 270.3 [M+H]⁺. ¹H NMR (DMSO-d₆): 9.81 (m, 2H), 7.67 (dd, J = 5.4, 8.6, 2H), 7.40 (dd, J = 8.8, 2H), 4.26-4.22 (m, 2H), 3.48-3.42 (m, 2H), 3.11 (t, J=6.3, 2H), 2.24-2.19 (m, 1 H), 1.08-1.01 (m, 4H).

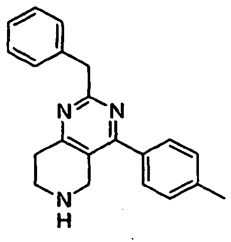

### Example 8; 2-Benzyl-4-p-tolyl-5,6,7,8-tetrahydro-pyrido[4,3-d]pyrimidine.

MS (ESI): exact mass calcd. for C₂₁H₂₁N₃, 315.17; m/z found, 316.4 [M+H]⁺. ¹H NMR (CDCl₃): 7.43-7.41 (m, 4H), 7.30-7.25 (m, 4H), 7.21-7.18 (m, 1H), 4.27 (s, 2H), 3.97 (s, 2H), 3.23 (t, J = 6.1, 2H), 2.96 (t, J = 6.1, 2H), 2.40 (s, 3H).

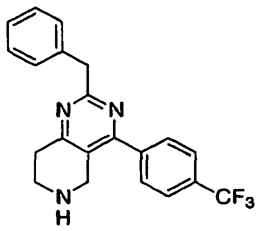

### Example 9; 2-Benzyl-4-(4-trifluoromethyl-phenyl)-5,6,7,8-tetrahydro-pyrido[4,3-d]pyrimidine.

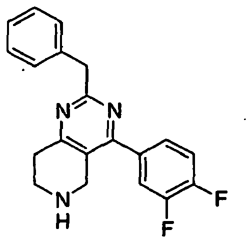

### Example 10; 2-Benzyl-4-(3,4-difluoro-phenyl)-5,6,7,8-tetrahydro-pyrido[4,3-d]pyrimidine.

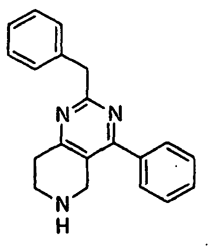

### Example 11; 2-Benzyl-4-phenyl-5,6,7,8-tetrahydro-pyrido[4,3-d]pyrimidine.

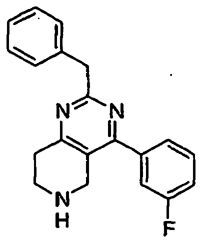

### Example 12; 2-Benzyl-4-(3-fluoro-phenyl)-5,6,7,8-tetrahydro-pyrido[4,3-d]pyrimidine.

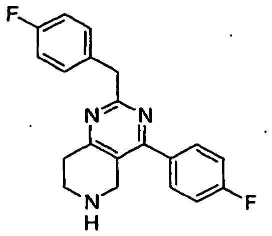

### Example 13; 2-(4-Fluoro-benzyl)-4-(4-fluoro-phenyl)-5,6,7,8-tetrahydro-pyrido[4,3-d]pyrimidine.

MS (ESI): exact mass calcd. for C₂₀H₁₇F₂N₃, 337.14; m/z found, 338.4 [M+H]⁺. ¹H NMR (CDCl₃): 7.53-7.49 (m, 2H), 7.38-7.35 (m, 2H), 7.18-7.13 (m, 2H), 6.99-6.95 (m, 2H), 4.23 (s, 2H), 3.96 (s, 2H), 3.25 (t, J = 6.1, 2H), 2.97 (t, J = 6.1, 2H).

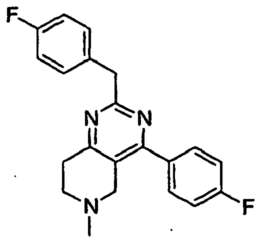

### Example 14; 2-(4-Fluoro-benzyl)-4-(4-fluoro-phenyl)-6-methyl-5,6,7,8-tetrahydro-pyrido[4,3-d]pyrimidine.

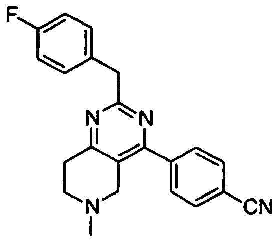

### Example 15; 4-[2-(4-Fluoro-benzyl)-6-methyl-5,6,7,8-tetrahydro-pyrido[4,3-d]pyrimidin-4-yl]-benzonitrile.

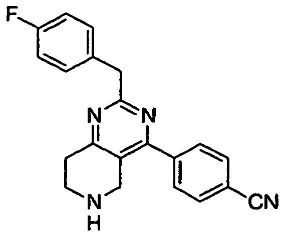

### Example 16; 4-[2-(4-Fluoro-benzyl)-5,6,7,8-tetrahydro-pyrido[4,3-d]pyrimidin-4-yl]-benzonitrile.

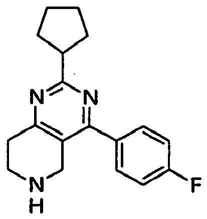

### Example 17; 2-Cyclopentyl-4-(4-fluoro-phenyl)-5,6,7,8-tetrahydro-pyrido[4,3-d]pyrimidine.

MS (ESI): exact mass calcd. for C₁₈H₂₀FN₃, 297.16; m/z found, 298.4 [M+H]⁺. ¹H NMR (CDCl₃): 7.55 (dd, J = 5.4, 8.8, 2H), 7.15 (t, J = 8.8, 2H), 3.97 (s, 2H), 3.36-3.28 (m, 1 H), 3.26 (t, J = 6.1, 2H), 2.97 (t, J = 6.1, 2H), 2.12-1.64 (m, 8H).

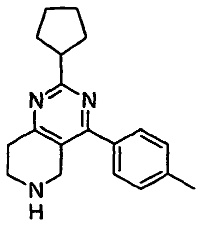

### Example 18; 2-Cyclopentyl-4-p-tolyl-5,6,7,8-tetrahydro-pyrido[4,3-d]pyrimidine.

MS (ESI): exact mass calcd. for C₁₉H₂₃N₃, 293.19; m/z found, 294.5 [M+H]⁺. ¹H NMR (CDCl₃): 7.55 (dd, J = 5.4, 8.8, 2H), 7.15 (t, J = 8.8, 2H), 3.97 (s, 2H), 3.36-3.27 (m, 1H), 3.26 (t, J = 6.1, 2H), 2.97 (t, J = 6.1, 2H), 2.12-1.64 (m, 8H).

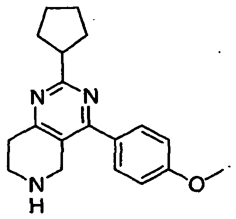

### Example 19; 2-Cyclopentyl-4-(4-methoxy-phenyl)-5,6,7,8-tetrahydro-pyrido[4,3-d]pyrimidine.

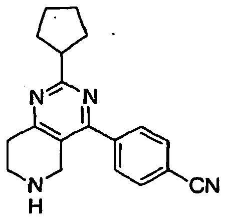

### Example 20; 4-(2-Cyclopentyl-5,6,7,8-tetrahydro-pyrido[4,3-d]pyrimidin-4-yl)-benzonitrile.

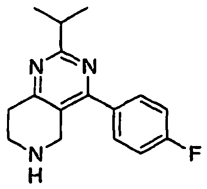

### Example 21; 4-(4-Fluoro-phenyl)-2-isopropyl-5,6,7,8-tetrahydro-pyrido[4,3-d]pyrimidine hydrochloride.

MS (ESI): exact mass calcd. for C₁₆H₁₈FN₃, 271.15; m/z found, 272.4 [M+H]⁺. ¹H NMR (MeOH-d₄): 7.75-7.70 (m, 2H), 7.37-7.31 (m, 2H), 4.47 (s, 2H), 3.72-3.68 (m, 2H), 3.37-3.32 (m, 2H), 3.30-3.22 (m, 1H), 1.39 (d, J = 6.9, 6H).

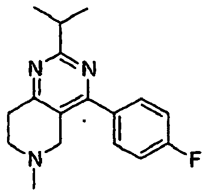

### Example 22; 4-(4-Fluoro-phenyl)-2-isopropyl-6-methyl-5,6,7,8-tetrahydro-pyrido[4,3-d]pyrimidine.

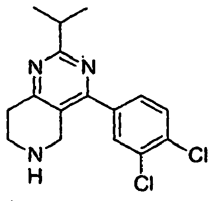

### Example 23; 4-(3,4-Dichloro-phenyl)-2-isopropyl-5,6,7,8-tetrahydro-pyrido[4,3-d]pyrimidine hydrochloride.

MS (ESI): exact mass calcd. for C₁₆H₁₇Cl₂N₃, 321.08; m/z found, 322.3 [M+H]⁺. ¹H NMR (MeOH-d₄): 7.86-7.84 (m, 1 H), 7.77-7.74 (m, 1 H), 7.60-7.57 (m, 1H), 4.47 (s, 2H), 3.71-3.67 (m, 2H), 3.35-3.32 (m, 2H), 3.29-3.21 (m, 1H), 1.38 (d, J = 6.9, 6H).

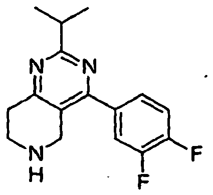

### Example 24; 4-(3,4-Difluoro-phenyl)-2-isopropyl-5,6,7,8-tetrahydro-pyrido[4,3-d]pyrimidine hydrochloride.

MS (ESI): exact mass calcd. for C₁₆H₁₇F₂N₃, 289.14; m/z found, 290.4 [M+H]⁺. ¹H NMR (MeOH-d₄): 7.67-7.62 (m, 1H), 7.54-7.47 (m, 2H), 4.48 (s, 2H), 3.71-3.67 (m, 2H), 3.35-3.32 (m, 2H), 3.29-3.21 (m, 1 H), 1.38 (d, J = 6.9, 6H).

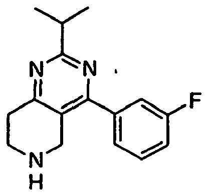

### Example 25; 4-(3-Fluoro-phenyl)-2-isopropyl-5,6,7,8-tetrahydro-pyrido[4,3-d]pyrimidine.

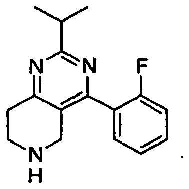

### Example 26; 4-(2-Fluoro-phenyl)-2-isopropyl-5,6,7,8-tetrahydro-pyrido[4,3-d]pyrimidine.

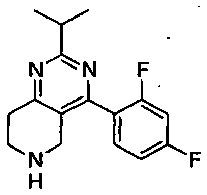

### Example 27; 4-(2,4-Difluoro-phenyl)-2-isopropyl-5,6,7,8-tetrahydro-pyrido[4,3-d]pyrimidine.

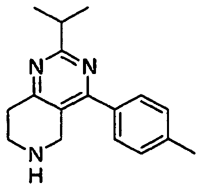

### Example 28; 2-Isopropyl-4-p-tolyt-5,6,7,8-tetrahydro-pyrido[4,3-d]pyrimidine.

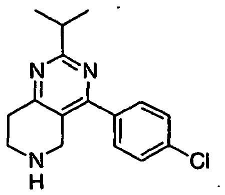

### Example 29; 4-(4-Chloro-phenyl)-2-isopropyl-5,6,7,8-tetrahydro-pyrido[4,3-d]pyrimidine.

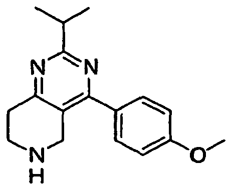

### Example 30; 2-Isopropyl-4-(4-methoxy-phenyl)-5,6,7,8-tetrahydro-pyrido[4,3-d]pyrimidine.

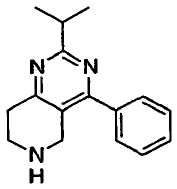

### Example 31; 2-Isopropyl-4-phenyl-5,6,7,8-tetrahydro-pyrido[4,3-d]pyrimidine.

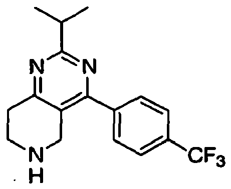

### Example 32; 2-Isopropyl-4-(4-trifluoromethyl-phenyl)-5,6,7,8-tetrahydro-pyrido[4,3-d]pyrimidine.

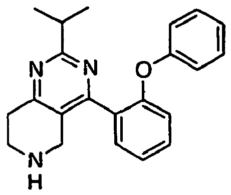

### Example 33; 2-Isopropyl-4-(2-phenoxy-phenyl)-5,6,7,8-tetrahydro-pyrido[4,3-d]pyrimidine.

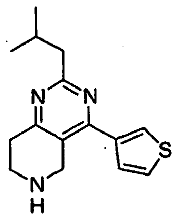

### Example 34; 2-Isobutyl-4-thiophen-3-yl-5,6,7,8-tetrahydro-pyrido[4,3-d]pyrimidine.

MS (ESI): exact mass calcd. for C₁₅H₁₉N₃S, 273.13; m/z found, 274.1 [M+H]⁺. ¹H NMR (CDCl₃): 7.67 (dd, J =1.3, 2.9, 1 H), 7.52 (dd, J = 1.3, 5.0, 1 H), 7.40 (dd, J = 2.9, 5.0, 1 H), 4.11 (s, 2H), 3.2 (t, J = 6.1, 2H), 2.96 (t, J = 6.1, 2H), 2.80 (d, J = 7.3, 2H), 2.35-2.25 (m, 1 H), 0.98 (d, J = 6.7, 6H).

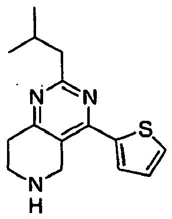

### Example 35; 2-Isobutyl-4-thiophen-2-yl-5,6,7,8-tetrahydro-pyrido[4,3-d]pyrimidine.

MS (ESI): exact mass calcd. for C₁₅H₁₉N₃S, 273.13; m/z found, 274.1 [M+H]⁺. ¹H NMR (CDCl₃): 7.52 (dd, J = 1.0, 5.1, 1H), 7.48 (dd, J = 1.0, 3.8, 1 H), 7.16 (dd, J = 3.8, 5.1, 1 H), 4.22, (s, 2H), 3.27 (t, J = 6.0, 2H), 2.96 (t, J = 6.0, 2H), 2.78 (d, J = 7.3, 2H), 2.36-2.26 (m, 1H), 0.99 (d, J = 6.7, 6H).

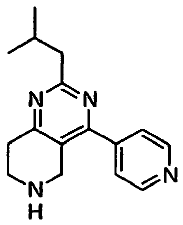

### Example 36; 2-Isobutyl-4-pyridin-4-yl-5,6,7,8-tetrahydro-pyrido[4,3-d]pyrimidine.

MS (ESI): exact mass calcd. for C₁₆H₂₀N₄, 268.17; m/z found, 269.2 [M+H]⁺. ¹H NMR (CDCl₃): 8.75-8.74 (m, 2H), 7.44-7.43 (m, 2H), 3.97 (s, 2H), 3.28 (t, J = 6.0, 2H), 3.01 (t, J = 6.0, 2H), 2.83 (d, J = 7.3, 2H), 2.34-2.23 (m, 1 H), 0.98 (d, J = 6.7, 6H).

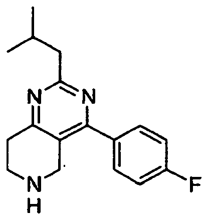

### Example 37; 4-(4-Fluoro-phenyl)-2-isobutyl-5,6,7,8-tetrahydro-pyrido[4,3-d]pyrimidine.

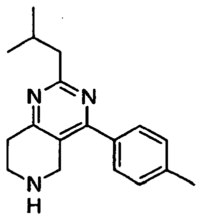

### Example 38; 2-Isobutyl-4-p-tolyl-5,6,7,8-tetrahydro-pyrido[4,3-d]pyrimidine.

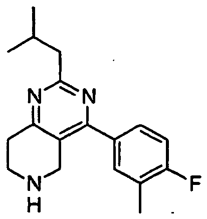

### Example 39; 4-(4-Fluoro-3-methyl-phenyl)-2-isobutyl-5,6,7,8-tetrahydro-pyrido[4,3-d]pyrimidine.

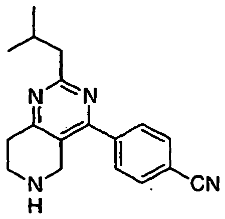

### Example 40; 4-(2-Isobutyl-5,6,7,8-tetrahydro-pyrido[4,3-d]pyrimidin-4-yl)-benzonitrile.

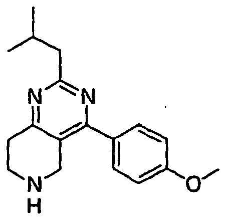

### Example 41; 2-Isobutyl-4-(4-methoxy-phenyl)-5,6,7,8-tetrahydro-pyrido[4,3-d]pyrimidine.

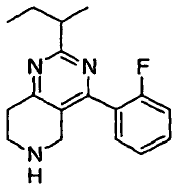

### Example 42; 2-sec-Butyl-4-(2-fluoro-phenyl)-5,6,7,8-tetrahydro-pyrido[4,3-d]pyrimidine hydrochloride.

MS (ESI): exact mass calcd. for C₁₇H₂₀FN₃, 285.16; m/z found, 286.4 [M+H]⁺. ¹H NMR (DMSO-d₆): 9.62 (s, 2H), 7.66-7.61 (m, 1 H), 7.51-7.39 (m, 3H), 4.08-4.06 (m, 2H), 3.55-3.47 (m, 2H), 3.17 (dd, J = 6.3, 2H), 2.93 (tq, J= 6.9, 7.4, 1 H), 1.86-1.75 (m, 1 H), 1.66-1.55 (m, 1 H), 1.25 (d, J = 6.9, 3H), 0.82 (t, J = 7.4, 3H).

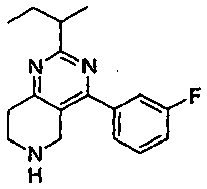

### Example 43; 2-sec-Butyl-4-(3-fluoro-phenyl)-5,6,7,8-tetrahydro-pyrido[4,3-d]pyrimidine.

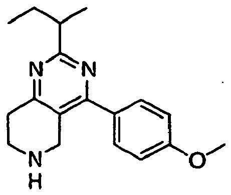

### Example 44; 2-sec-Butyl-4-(4-methoxy-phenyl)-5,6,7,8-tetrahydro-pyrido[4,3-d]pyrimidine.

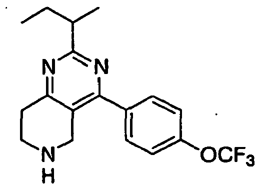

### Example 45; 2-sec-Butyl-4-(4-trifluoromethoxy-phenyl)-5,6,7,8-tetrahydro-pyrido[4,3-d]pyrimidine.

The following Examples 46-57 were prepared as described in Examples 1 and 2, substituting 5-oxo-azepane-1,4-dicarboxylic acid 1-tert-butyl ester 4-ethyl ester (J. Het. Chem. 1992, 29(4), 779-786) for 4-oxo-piperidine-1,3-dicarboxylic acid 1-tert-butyl ester 3-ethyl ester.

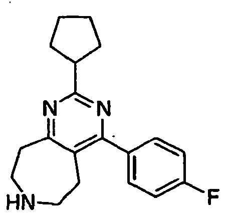

### Example 46; 2-Cyclopentyl-4-(4-fluoro-phenyl)-6,7,8,9-tetrahydro-5H-pyrimido[4,5-d]azepine.

MS (ESI): exact mass calcd. for C₁₉H₂₂FN₃, 311.18; m/z found, 312.4 [M+H]⁺. ¹H NMR (CDCl₃): 7.46 (dd, J = 5.4, 8.6, 2H), 7.14 (dd, J = 8.7, 2H), 3.32-3.26 (m, 1H), 3.18-3.16 (m, 2H), 3.07-3.05 (m, 2H), 2.96-2.92 (m, 4H), 2.12-2.06 (m, 2H), 1.99-1.91 (m, 2H), 1.87-1.80 (m, 2H), 1.72-1.64 (m, 2H).

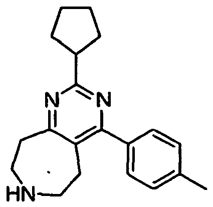

### Example 47; 2-Cyclopentyl-4-p-tolyl-6,7,8,9-tetrahydro-5H-pyrimido[4,5-d]azepine.

MS (ESI): exact mass calcd. for C₂₀H₂₅N₃, 307.2; m/z found, 308.4 [M+H]⁺. ¹H NMR (CDCl): 7.37 (d, J = 8.1, 2H), 7.26 (m, 2H), 3.32-3.26 (m, 1H), 3.17-3.15 (m, 2H), 3.07-3.05 (m, 2H), 2.94 (m, 4H), 2.41 (s, 3H), 2.11-2.05 (m, 2H), 2.0-1.93 (m, 2H), 1.86-1.79 (m, 2H), 1.71-1.62 (m, 2H).

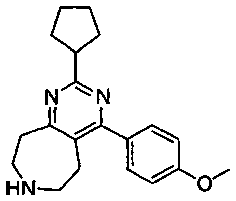

### Example 48; 2-Cyclopentyl-4-(4-methoxy-phenyl)-6,7,8,9-tetrahydro-5H-pyrimido[4,5-d]azepine.

MS (ESI): exact mass calcd. for C₂₀H₂₅N₃O,323.2; m/z found, 324.5 [M+H]⁺. ¹H NMR (CDCl₃): 7.44 (d, J = 8.9, 2H), 6.98 (d, J = 8.9, 2H), 3.86 (s, 3H), 3.35-3.26 (m, 1 H), 3.17-3.15 (m, 2H), 3.07-3.05 (m, 2H), 2.12-2.05 (m, 2H), 2.00-1.92 (m, 2H), 1.87-1.80 (m, 2H), 1.71-1.64 (m, 2H).

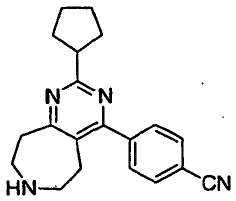

### Example 49; 4-(2-Cyclopentyl-6,7,8,9-tetrahydro-5H-pyrimido[4,5-d]azepin-4-yl)-benzonitrile.

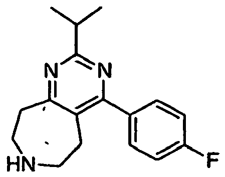

### Example 50; 4-(4-Fluoro-phenyl)-2-isopropyl-6,7,8,9-tetrahydro-5H-pyrimido[4,5-d]azepine hydrochloride.

MS (ESI): exact mass calcd. for C₁₇H₂₀FN₃, 285.16; m/z found, 286.2 [M+H]⁺. ¹H NMR (MeOH-d₄): 7.76-7.71 (m, 2H), 7.41-7.36 (m, 2H), 3.69-3.65 (m, 2H), 3.63-3.59 (m, 2H), 3.49-3.45 (m, 2H), 3.42-3.34 (m, 3H), 1.45 (d, J = 6.9, 6H).

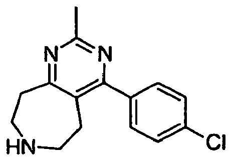

### Example 51; 4-(4-Chloro-phenyl)-2-methyl-6,7,8,9-tetrahydro-5H-pyrimido[4,5-d]azepine.

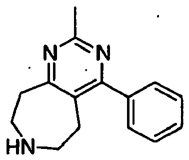

### Example 52; 2-Methyl-4-phenyl-6,7,8,9-tetrahydro-5H-pyrimido[4,5-d]azepine.

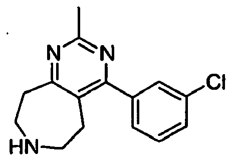

### Example 53; 4-(3-Chloro-phenyl)-2-methyl-6,7,8,9-tetrahydro-5H-pyrimido[4,5-d]azepine.

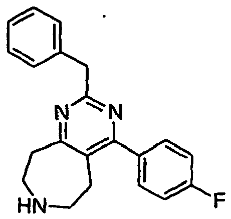

### Example 54; 2-Benzyl-4-(4-fluoro-phenyl)-6,7,8,9-tetrahydro-5H-pyrimido[4,5-d]azepine.

MS (ESI): exact mass calcd. for C₂₁H₂₀FN₃, 333.16; m/z found, 334.4 [M+H]⁺. ¹H NMR (CDCl₃): 7.44-7.42 (m, 4H), 7.31-7.28 (m, 2H), 7.23-7.20 (m, 1 H), 7.16-7.13 (m, 2H), 4.25 (s, 2H), 3.18-3.16 (m, 2H), 3.06-3.03 (m, 2H), 2.94-2.90 (m, 4H).

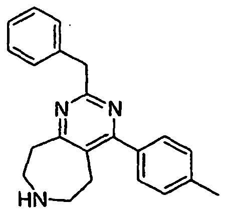

### Example 55; 2-Benzyl-4-p-tolyl-6,7,8,9-tetrahydro-5H-pyrimido[4,5-d]azepine.

MS (ESI): exact mass calcd. for C₂₂H₂₃N₃, 329.19; m/z found, 330.5 [M+H]⁺. ¹H NMR (CDCl₃): 7.45-7.43 (m, 2H), 7.35-7.33 (m, 2H), 7.30-7.25 (m, 4H), 7.22-7.19 (m, 1 H), 4.25 (s, 2H), 3.17-3.15 (m, 2H), 3.05-3.03 (m, 2H), 2.41 (s, 3H).

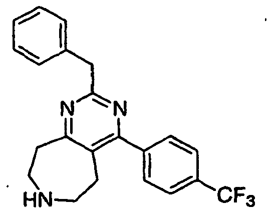

### Example 56; 2-Benzyl-4-(4-trifluoromethyl-phenyl)-6,7,8,9-tetrahydro-5H-pyrimido[4,5-d]azepine.

MS (ESI): exact mass calcd. for C₂₂H₂₀F₃N₃, 383.16; m/z found, 384.4 [M+H]⁺. ¹H NMR (CDCl₃): 7.72 (d, J = 8.1, 2H), 7.55 (d, J = 8.0, 2H), 7.43 (d, J = 7.5, 2H), 7.31-7.28 (m, 2H), 7.23-7.20 (m, 1 H), 4.26 (s, 2H), 3.20-3.18 (m, 2H), 3.06-3.04 (m, 2H), 2.95-2.93 (m, 2H), 2.90-2.88 (m, 2H).

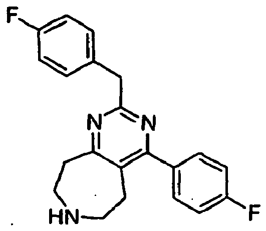

### Example 57; 2-(4-Fluoro-benzyl)-4-(4-fluoro-phenyl)-6,7,8,9-tetrahydro-5H-pyrimido[4,5-d]azepine.

MS (ESI): exact mass calcd. for C₂₁H₁₉F₂N₃, 351.15; m/z found, 352.4 [M+H]⁺. ¹H NMR (CDCl₃): 7.44-7.41 (m, 2H), 7.40-7.36 (m, 2H), 7.18-7.12 (m, 2H), 7.01-6.95 (m, 2H), 4.21 (s, 2H), 3.19-3.17 (m, 2H), 3.07-3.04 (m, 2H), 2.96-2.92 (m, 4H).

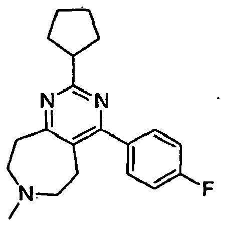

### Example 58; 2-Cyclopentyl-4-(4-fluoro-phenyl)-7-methyl-6,7,8,9-tetrahydro-5H-pyrimido[4,5-d]azepine.

To a solution of 2-cyclopentyl-4-(4-fluoro-phenyl)-6,7,8,9-tetrahydro-5H-pyrimido[4,5-d]azepine (0.035 g, 0.112 mmol) in MeOH (1 mL) was added formaldehyde (37% in water; 0.10 mL) and NaBH(OAc)₃ (0.032 g, 0.151 mmol). After the reaction was judged complete, the mixture was diluted with 1 N NaOH and extracted with CH₂Cl₂ (3X). The combined organic layers were dried and concentrated. The resulting residue was purified via SiO₂ chromatography (1-7% 2 M NH₃ in MeOH/CH₂Cl₂) to give 0.031 g (87%) of the title compound. MS (ESI): exact mass calcd. for C₂₀H₂₄FN₃, 325.20; m/z found, 326.4 [M+H]⁺. ¹H NMR (CDCl₃): 7.47 (dd, J = 5.4, 8.8, 2H), 7.15 (t, J = 8.7, 2H), 3.38-3.30 (m, 1 H), 3.20 (dd, J = 4.1, 6.3, 2H), 2.97 (dd, J = 4.2, 5.9, 2H), 2.71-2.70 (m, 2H), 2.60 (m, 2H), 2.41 (s, 3H), 2.12-2.05 (m, 2H), 1.99-1.91 (m, 2H), 1.87-1.79 (m, 2H), 1.73-1.63 (m, 2H).

The following compounds in Examples 59-62 were prepared using methods similar to those described in Example 58, starting with the corresponding unmethylated azepines from the preceding examples.

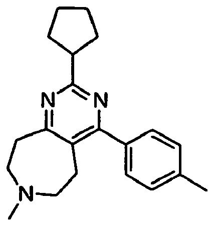

### Example 59; 2-Cyclopentyl-7-methyl-4-p-tolyl-6,7,8,9-tetrahydro-5H-pyrimido[4,5-d]azepine.

MS (ESI): exact mass calcd. for C₂₁H₂₇N₃, 321.22; m/z found, 322.5 [M+H]⁺. ¹H NMR (CDCl₃): 7.39-7.37 (m, 2H), 7.27-7.25 (m, 2H), 3.35-3.26 (m, 1 H), 3.20-3.18 (m, 2H), 3.0-2.97 (m, 2H), 2.7-2.68 (m, 2H), 2.59 (m, 2H), 2.41 (s, 3H), 2.40 (s, 3H), 2.12-2.04 (m, 2H), 2.0-1.91 (m, 2H), 1.88-1.79 (m, 2H), 1.72-1.61 (m, 2H).

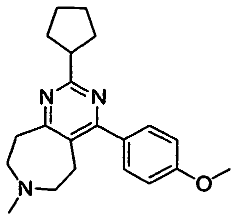

### Example 60; 2-Cyclopentyl-4-(4-methoxy-phenyl)-7-methyl-6,7,8,9-tetrahydro-5H-pyrimido[4,5-d]azepine.

MS (ESI): exact mass calcd. for C₂₁H₂₇N₃O, 337.22; m/z found, 338.5 [M+H]⁺. ¹H NMR (CDCl₃): 7.44 (d, J = 8.8, 2H), 6.98 (d, J = 8.8, 2H), 3.86 (s, 3H), 3.34-3.26 (m, 1 H), 3.19 (dd, J = 4.2, 6.2, 2H), 3.01 (dd, J = 4.3, 5.5, 2H), 2.71-2.69 (m, 2H), 2.61 (m, 2H), 2.41 (s, 3H), 2.12-2.05 (m, 2H), 2.0-1.91 (m, 2H), 1.87-1.79 (m, 2H), 1.71-1.63 (m, 2H).

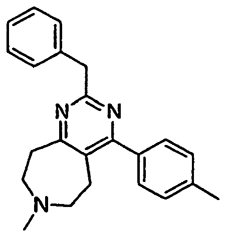

### Example 61; 2-Benzyl-7-methyl-4-p-tolyl-6,7,8,9-tetrahydro-5H-pyrimido[4,5-d]azepine.

MS (ESI): exact mass calcd. for C₂₃H₂₅N₃, 343.2; m/z found, 344.5 [M+H]⁺. ¹H NMR (CDCl₃): 7.43 (d, J = 7.3, 2H), 7.35 (d, J = 8.0, 2H), 7.30-7.25 (m, 4H), 7.21-7.19 (m, 1 H), 4.26 (s, 2H), 3.18-3.16 (m, 2H), 2.97-2.95 (m, 2H), 2.66-2.65 (m, 2H), 2.56 (m, 2H), 2.41 (s, 3H), 2.38 (s, 3H).

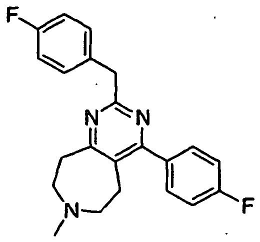

### Example 62; 2-(4-Fluoro-benzyl)-4-(4-fluoro-phenyl)-7-methyl-6,7,8,9-tetrahydro-5H-pyrimido[4,5-d]azepine.

MS (ESI): exact mass calcd. for C₂₂H₂₁F₂N₃, 365.17; m/z found, 366.4 [M+H]⁺. ¹H NMR (CDCl₃): 7.46-7.42 (m, 2H), 7.39-7.36 (m, 2H), 7.18-7.13 (m, 2H), 7.0-6.95 (m, 2H), 4.22 (s, 2H), 3.20-3.17 (m, 2H), 2.96-2.93 (m, 2H), 2.67-2.65 (m, 2H), 2.58-2.55 (m, 2H), 2.39 (s, 3H).

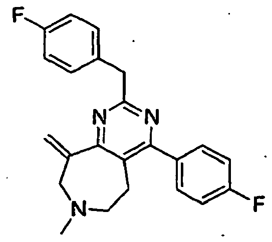

### Example 63; 2-(4-Fluoro-benzyl)-4-(4-fluoro-phenyl)-7-methyl-9-methylene-6,7,8,9-tetrahydro-5H-pyrimido[4,5-d]azepine.

To a solution of 2-(4-fluoro-benzyl)-4-(4-fluoro-phenyl)-5,6,8,9-tetrahydro-pyrimido[4,5-d]azepine-7-carboxylic acid tert-butyl ester in formic acid was added paraformaldehyde (10 equiv.). The mixture was heated at 80°C for 6 h. The mixture was diluted with water and basified to pH -10 with 1 M NaOH. The mixture was extracted with CH₂Cl₂, dried and concentrated. Chromatography on SiO₂ (0-5% 2 M NH₃ in MeOH/CH₂Cl₂) afforded the desired compound. 2-(4-Fluoro-benzyl)-4-(4-fluoro-phenyl)-7-methyl-6,7,8,9-tetrahydro-5H-pyrimido[4,5-d]azepine was also obtained. MS (ESI): exact mass calcd. for C₂₃H₂₁F₂N₃, 377.17; m/z found, 378.4 [M+H]⁺. ¹H NMR (CDCl₃): 7.50-7.46 (m, 2H), 7.42-7.37 (m, 2H), 7.17-7.12 (m, 2H), 6.99-6.94 (m, 4H), 6.03-6.01 (m, 1H), 5.47-5.46 (m, 1 H), 4.25 (s, 2H), 3.51 (s, 2H), 2.87-2.83 (m, 2H), 2.76-2.72 (m, 2H), 2.41 (s, 3H).

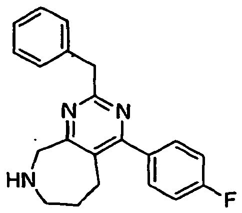

### Example 64; 2-Benzyl-4-(4-fluoro-phenyl)-6,7,8,9-tetrahydro-5H-pyrimido[4,5-c]azepine hydrochloride.

Step A. 3-Oxo-azepane-1,4-dicarboxylic acid 1-tert-butyl ester 4-ethyl ester and 4-oxo-azepane-1,3-dicarboxylic acid 1-tert-butvl ester 3-ethyl ester. To a 0 °C solution of 3-oxo-piperidine-1-carboxylic acid tert-butyl ester (11.3 g, 56.7 mmol) in Et₂O (170 mL) was added BF₃•Et₂O (7.2 mL, 56.7 mmol) and ethyl diazoacetate (7.2 mL, 68.0 mmol) dropwise over 30 min. After an additional 1 h, satd. aq. NaHCO₃ was added and the solution was stirred for 1 h, then was extracted with Et₂O (2x). The combined organic layers were washed with brine, dried and concentrated. The resulting residue was purified via SiO₂ chromatography (10-30% EtOAc/hexanes) to give 5.48 g (34%) of 3-oxo-azepane-1,4-dicarboxylic acid 1-tert-butyl ester 4-ethyl ester. In addition, 5.25 g (32%) of the more polar 4-oxo-azepane-1,3-dicarboxylic acid 1-tert-butyl ester 3-ethyl ester was isolated.

Step B. 2-Benzyl-4-(4-fluoro-phenyl)-6,7,8,9-tetrahydro-5H-pyrimido[4,5-c]azepine. The title compound was prepared from 3-oxo-azepane-1,4-dicarboxylic acid 1-tert-butyl ester 4-ethyl ester according to the methods described in Example 1. MS (ESI): exact mass calcd. for C₂₁H₂₀FN₃, 333.16; m/z found, 334.4 [M+H]⁺. ¹H NMR (DMSO-d₆): 9.81 (m, 2H), 7.55 (dd, J = 5.5, 8.7, 2H), 7.40-7.35 (m, 4H), 7.31-7.27 (m, 2H), 7.22-7.19 (m, 1H), 4.46-4.43 (m, 2H), 4.21 (s, 2H), 3.43-3.33 (m, 2H), 3.00-2.93 (m, 2H), 1.98-1.88 (m, 2H).

The following compounds in Examples 65-68 were prepared using methods similar to those described in Example 64.

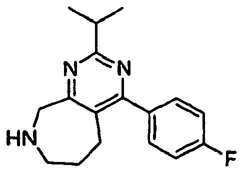

### Example 65; 4-(4-Fluoro-phenyl)-2-isopropyl-6,7,8,9-tetrahydro-5H-pyrimido[4,5-c]azepine hydrochloride.

MS (ESI): exact mass calcd. for C₁₇H₂₀FN₃, 285.16; m/z found, 286.4 [M+H]⁺. ¹H NMR (DMSO-d₆): 9.77 (s, 2H), 7.57 (dd, J = 5.5, 8.7, 2H), 7.40-7.37 (m, 2H), 4.49-4.44 (m, 2H), 3.43-3.36 (m, 2H), 3.19-3.10 (m, 1 H), 2.99-2.97 (m, 2H), 1.99-1.92 (m, 2H), 1.30 (d, J = 6.9, 6H).

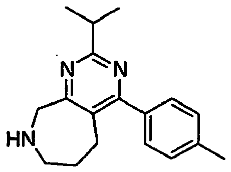

### Example 66; 2-Isopropyl-4-p-tolyl-6,7,8,9-tetrahydro-5H-pyrimido[4,5-c]azepine hydrochloride.

MS (ESI): exact mass calcd. for C₁₈H₂₃N₃, 281.19; m/z found, 282.4 [M+H]⁺. ¹H NMR (DMSO-d₆): 9.80 (s, 2H), 7.41 (d, J = 8.0, 2H), 7.35 (d, J = 8.0, 2H), 4.46 (m, 2H), 3.50-3.36 (m, 2H), 3.18-3.10 (m, 1H), 3.90-2.98 (m, 2H), 2.8 (s, 3H), 1.98-1.90 (m, 2H), 1.29 (d, J = 6.9, 6H).

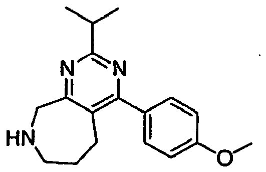

### Example 67; 2-Isopropyl-4-(4-methoxy-phenyl)-6,7,8,9-tetrahydro-5H-pyrimido[4,5-c]azepine.

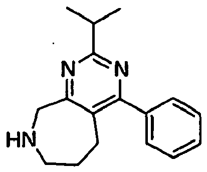

### Example 68; 2-Isopropyl-4-phenyl-6,7,8,9-tetrahydro-5H-pyrimido[4,5-c]azepine.

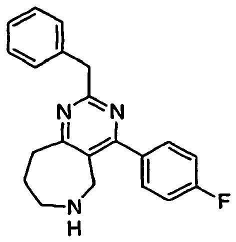

### Example 69; 2-Benzyl-4-(4-fluoro-phenyl)-6,7,8,9-tetrahydro-5H-1,3,6-triazabenzocycloheptene hydrochloride.

The title compound was synthesized as described in Example 64 using 4-oxo-azepane-1,3-dicarboxylic acid 1-tert-butyl ester 3-ethyl ester. MS (ESI): exact mass calcd. for C₂₁H₂₀FN₃, 333.16; m/z found, 334.4 [M+H]⁺. ¹H NMR (DMSO-d₆): 9.59 (s, 2H), 7.66 (dd, J = 5.5, 8.8, 2H), 7.42-7.27 (m, 6H), 7.22-7.19 (m, 1 H), 4.29-4.26 (m, 2H), 4.21 (s, 2H), 3.42-.36(m, 2H), 3.22-3.20 (m, 2H), 2.01-1.95 (m, 2H).

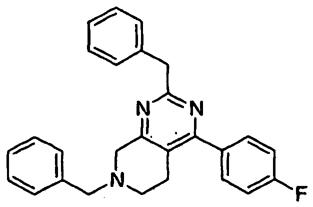

### Example 70; 2,7-Dibenzyl-4-(4-fluoro-phenyl)-5,6,7,8-tetrahydro-pyrido[3,4-d]pyrimidine hydrochloride.

The title compound was synthesized as described in Example 1, Steps A-C, using 1-benzyl-3-oxo-piperidine-4-carboxylic acid ethyl ester hydrochloride. Purification was performed using SiO₂ chromatography (2 M NH₃ in MeOH/CH₂Cl₂). MS (ESI): exact mass calcd. for C₂₇H₂₄FN₃, 409.2; m/z found, 410.5 [M+H]⁺. ¹H NMR (MeOH-d₄): 7.74-7.70 (m, 2H), 7.66-7.63 (m, 2H), 7.56-7.53 (m, 3H), 7.36-7.18 (m, 7H), 4.59 (s, 2H), 4.49 (br s, 2H), 4.29 (s, 2H).

The compounds in Examples 71-75 were prepared using methods similar to those described in Example 70.

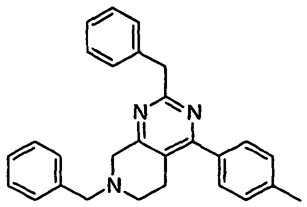

### Example 71; 2,7-Dibenzyl-4-p-tolyl-5,6,7,8-tetrahydro-pyrido[3,4-d]pyrimidine.

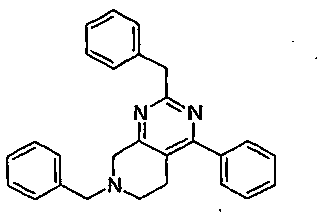

### Example 72; 2,7-Dibenzyl-4-phenyl-5,6,7,8.tetrahydro-pyrido[3,4.d]pyrimidine.

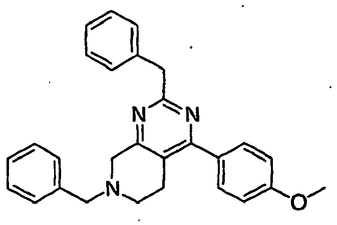

### Example 73; 2,7-Dibenzyl-4-(4-methoxy-phenyl)-5,6,7,&tetrahydro-pyrido[3,4-d]pyrimidine.

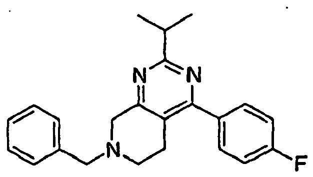

### Example 74; 7-Benzyl-4-(4-fluoro-phenyl)-2-isopropyl-5,6,7,8-tetrahydro-pyrido[3,4-d]pyrimidine.

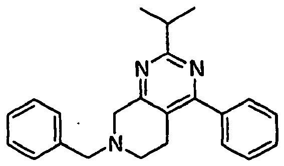

### Example 75; 7-Benzyl-2-isopropyl-4-phenyl-5,6,7,8-tetrahydro-pyrido[3,4-d]pyrimidine.

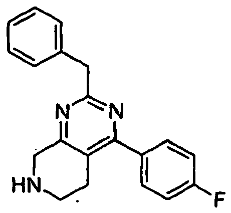

### Example 76; 2-Benryl-4-(4-fluoro-phenyl)-5,6,7,8-tetrahydro-pyrido[3,4-d]pyrimidine hydrochloride.

To a solution of 2,7-dibenzyl-4-(4-fluoro-phenyl)-5,6,7,8-tetrahydro-pyrido[3,4-d]pyrimidine hydrochloride in EtOH was added 10% Pd/C (1 equiv) followed by 1,4-cyclohexadiene (5 equiv). The mixture was heated at 85°C for 5 h, filtered and concentrated. The residue was dissolved in CH₂Cl₂ and treated with Dowex 550A resin. After 1 h, the resin was removed by filtration and the filtrate was concentrated. Chromatography on SiO₂ (2 M NH₃ in MeOH/CH₂Cl₂) afforded the title compound. MS (ESI): exact mass calcd. for C₂₀H₁₈FN₃, 319.15; m/z found, 320.4 (M+H)⁺. ¹H NMR (MeOH-d₄): 7.70-7.67 (m, 2H), 7.36-7.35 (m, 2H), 7.31-7.25 (m, 4H), 7.20-7.17 (m, 1 H), 4.42 (s, 2H), 4.26 (s, 2H), 3.48 (t, J = 6.1, 2H), 3.09 (t, J = 6.1, 2H).

The compounds in Examples 77-81 were prepared using methods similar to those described in Example 76.

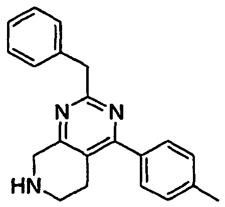

### Example 77; 2-Benryl-4-p-tolyl-5,6,7,8-tetrahydro-pyrido[3,4-d]pyrimidine hydrochloride.

MS (ESI): exact mass calcd. for C₂₁H₂₁N₃, 315.17; m/z found, 316.4 [M+H]⁺. ¹H NMR (MeOH-d₄): 7.55-7.54 (m, 2H), 7.44-7.36 (in, 4H), 7.29-7.26 (m, 2H), 7.22-7.19 (m, 1 H), 4.46 (s, 2H), 4.29 (s, 2H), 3.48 (t, J = 6.1, 2H), 3.12 (t, J = 6.1, 2H), 2.44 (s, 3H).

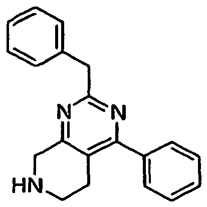

### Example 78; 2-Benzyl-4-phenyl-5,6,7,8-tetrahydro-pyrido[3,4-d]pyrimidine.

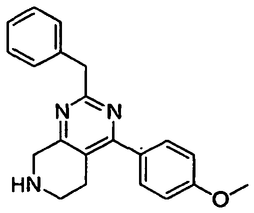

### Example 79; 2-Benzyl-4-(4-methoxy-phenyl)-5,6,7,8-tetrahydro-pyrido[3,4-d]pyrimidine.

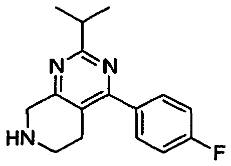

### Example 80; 4-(4-Fluoro-phenyl)-2-isopropyl-5,6,7,8-tetrahydro-pyrido[3,4-d]pyrimidine hydrochloride.

MS (ESI): exact mass calcd. for C₁₆H₁₈FN₃, 271.15; m/z found, 272.4 [M+H]⁺. ¹H NMR (MeOH-d₄): 7.74-7.72 (m, 2H), 7.33-7.30 (m, 2H), 4.48 (s, 2H), 3.51 (t, J = 6.0, 2H), 3.26-3.20 (m, 1 H), 3.12 (t, J = 6.0, 2H), 1.37 (d, J = 7.2, 6H).

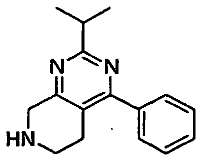

### Example 81; 2-Isopropyl-4-phenyl-5,6,7,8-tetrahydro-pyrido[3,4-d]pyrimidine.

The compounds in Examples 82-85 were prepared using methods similar to those described in Example 58.

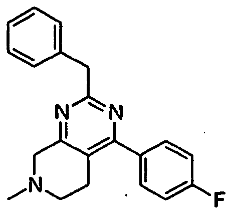

### Example 82; 2-Benzyl-4-(4-fluoro-phenyl)-7-methyl-5,6,7,8-tetrahydro-pyrido[3,4-d]pyrimidine hydrochloride.

MS (ESI): exact mass calcd. for C₂₁H₂₀FN₃, 333.16; m/z found, 334.4 [M+H]⁺. ¹H NMR (MeOH-d₄): 7.70-7.68 (m, 2H), 7.36-7.17 (m, 7H), 4.70-4.60 (m, 1 H), 4.45-4.35 (m, 1 H), 4.25 (s, 2H), 3.76 (br s, 1 H), 3.11-3.01 (m, 4H).

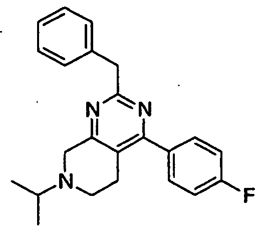

### Example 83; 2-Benzyl-4-(4-fluoro-phenyl)-7-isopropyl-5,6,7,8-tetrahydro-pyrido[3,4-d]pyrimidine.

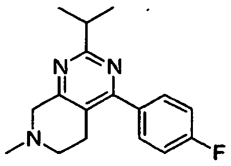

### Example 84; 4-(4-Fluoro-phenyl)-2-isopropyl-7-methyl-5,6,7,8-tetrahydro-pyrido[3,4-d]pyrimidine hydrochloride.

MS (ESI): exact mass calcd. for C₁₇H₂₀FN₃, 285.16; m/z found, 286.3 [M+H]⁺. ¹H NMR (MeOH-d₄): 7.74-7.71 (m, 2H), 7.32-7.29 (m, 2H), 4.72-4.62 (m, 1 H), 4.49-4.37 (m, 1H), 3.79 (br s, 1 H), 3.43-3.32 (m, 2H), 3.25-3.19 (m, 1 H), 3.13 (s, 3H), 3.09-2.99 (m, 1 H), 1.35 (d, J = 6.6, 6H).

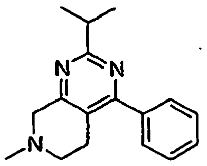

### Example 85; 2-Isopropyl-7-methyl-4-phenyl-5,6,7,8-tetrahydro-pyrido[3,4-d]pyrimidine.

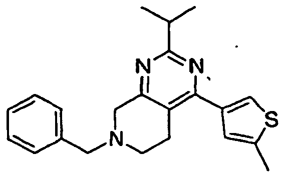

### Example 86; 7-Benzyl-2-isopropyl-4-(5-methyl-thiophen-3-yl)-5,6,7,8-tetrahydro-pyrido[3,4-d]pyrimidine hydrochloride.

Step A. 4-(7-Benzyl-2-isopropyl-5,6,7,8-tetrahydro-pyrido[3,4-d]pyrimidin-4-yl)-thiophene-2-carbaldehyde. The title compound was prepared as described in Example 1, Steps A-C.

Step B. 7-Benzyl-2-isopropyl-4-(5-methyl-thiophen-3-yl)-5,6,7,8-tetrahydro-pyrido[3,4-d]pyrimidine. To a solution of the product of Step A (0.230 g) in ethylene glycol was added hydrazine hydrate (0.1 mL). The mixture was heated at 200°C for 1 h, then KOH (0.150 g) was added and the heating continued for 6 h. The mixture was allowed to cool then was diluted with water and extracted with Et₂O. The combined organic extracts were dried and concentrated to 0.210 g of pale yellow solid. Chromatography on SiO₂ (EtOAc/hexanes) afforded 0.146 g of the title compound. MS (ESI): exact mass calcd. for C₂₂H₂₅N₃S, 363.18; found m/z 364.4 [M+H]⁺. ¹H NMR (MeOH-d₄): 7.85-7.84 (m, 1 H), 7.65-7.63 (m, 2H), 7.56-7.55 (m, 3H), 7.37-7.36 (m, 1 H), 4.60 (br s, 2H), 4.44 (br s, 2H), 3.46-3.32 (m, 2H), 3.22-3.17 (m, 1H), 2.55 (s, 3H), 1.34 (d, J = 7.2, 6H).

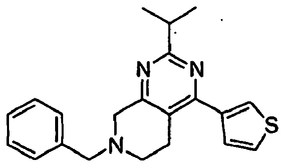

### Example 87; 7-Benzyl-2-isopropyl-4-thiophen-3-yl-5,6,7,8-tetrahydro-pyrido[3,4-d]pyrimidine hydrochloride.

The title compound was prepared according to the methods described in Example 86. MS (ESI): exact mass calcd. for C₂₁H₂₃N₃S, 349.16; m/z found, 350.4 [M+H]⁺. ¹H NMR (MeOH-d₄): 8.29-8.28 (m, 1 H), 7.72-7.69 (m, 4H), 7.56-7.55 (m, 3H), 4.65 (s, 2H), 4.59 (br s, 2H), 3.89 (br s, 1H), 3.57-3.32 (m, 4H), 1.40 (d, J = 6.6, 6H).

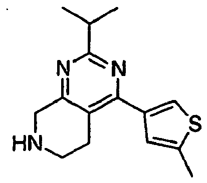

### Example 88; 2-Isopropyl-4-(5-methyl-thiophen-3-yl)-5,6,7,8-tetrahydro-pyrido[3,4-d]pyrimidine hydrochloride.

A solution of 7-benzyl-2-isopropyl-4-(5-methyl-thiophen-3-yl)-5,6,7,8-tetrahydro-pyrido[3,4-d]pyrimidine hydrochloride (0.133 g) in 1,2-dichloroethane (7 mL) was treated with 1-chloroethylchloroformate (0.105 mL). The mixture was heated at 95 °C for 16 h, concentrated, dissolved in MeOH, and heated at 50° C for an additional 2 h. The mixture was concentrated and chromatographed on SiO₂ (2 M NH₃ in MeOH/CH₂Cl₂). MS (ESI): exact mass calcd. for C₁₅H₁₉N₃S, 273.13; m/z found, 274.3 [M+H]⁺. ¹H NMR (MeOH-d₄): 7.81-7.80 (m, 1H), 7.36-7.35 (m, 1H), 4.42 (s, 2H), 3.55 (t, J = 6.0, 2H), 3.25 (t, J = 6.0, 2H), 3.19 (m, 1 H), 2.55 (s, 3H), 1.35 (d, J = 7.2, 6H).

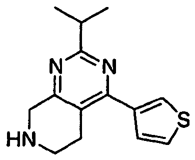

### Example 89; 2-Isopropyl-4-thiophen-3-yl-5,6,7,8-tetrahydro-pyrido[3,4-d]pyrimidine hydrochloride.

The title compound was prepared according to the methods described in Example 88. MS (ESI): exact mass calcd. for C₁₄H₁₇N₃S, 259.11; m/z found, 260.3 [M+H]⁺. ¹H NMR (MeOH-d₄): 8.16-8.15 (m, 1 H), 7.70-7.65 (m, 2H), 4.49 (s, 2H), 3.57 (t, J = 6.0, 2H), 3.31-3.23 (m, 3H), 1.39 (d, J = 7.2, 6H).

The following Examples 90-91 were prepared using methods similar to those described in Example 58.

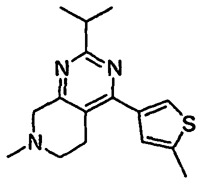

### Example 90; 2-Isopropyl-7-methyl-4-(5-methyl-thiophen-3-yl)-5,6,7,8-tetrahydro-pyrido[3,4-d]pyrimidine hydrochloride.

MS (ESI): exact mass calcd. for C₁₆H₂₁N₃S, 287.15; m/z found, 288.3 [M+H]⁺. ¹H NMR (MeOH-d₄): 7.79 (s, 1 H), 7.36 (s, 1H), 4.65-4.54 (m, 1 H), 4.45-4.35 (m, 1H), 3.83 (br s, 1H), 3.41 (br s, 1H), 3.27 (br s, 1 H), 3.20-3.13 (m, 4H), 2.55 (s, 3H), 1.34 (d, J = 7.2, 6H).

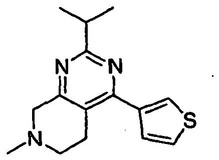

### Example 91; 2-Isopropyl-7-methyl-4-thiophen-3-yl-5,6,7,8-tetrahydro-pyrido[3,4-d]pyrimidine hydrochloride.

MS (ESI): exact mass calcd. for C₁₅H₁₉N₃S, 273.13; m/z found, 274.3 [M+H]⁺. ¹H NMR (MeOH-d₄): 8.27 (t, J = 1.8, 1 H), 7.71 (d, J = 1.8, 2H), 4.82-4.71 (m, 1H), 4.63-4.49 (m, 1 H), 3.95-3.81 (m, 1 H), 3.57-3.41 (m, 1H), 3.34-3.32 (m, 1H), 3.16 (s, 3H), 1.41 (d, J = 6.6, 6H).

The following compounds in Examples 92-99 were prepared using the methods similar to those described for Example 70 utilizing 1-benzyl-5-methyl-4-oxo-piperidine-3-carboxylic acid ethyl ester in place of 1-benzyl-3-oxo-piperidine-4-carboxylic acid ethyl ester hydrochloride.

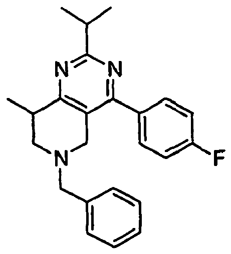

### Example 92; 6-Benzyl-4-(4-fluoro-phenyl)-2-isopropyl-8-methyl-5,6,7,8-tetrahydro-pyrido[4,3-d]pyrimidine.

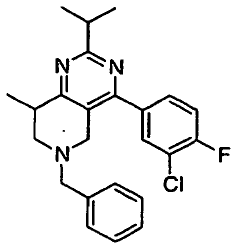

### Example 93; 6-Benzyl-4-(3-chloro-4-fluoro-phenyl)-2-isopropyl-8-methyl-5,6,7,8-tetrahydro-pyrido[4,3-d]pyrimidine.

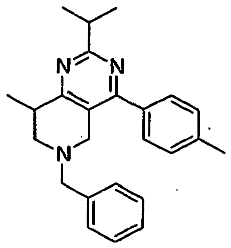

### Example 94; 6-Benzyl-2-isopropyl-8-methyl-4-p-tolyl-5,6,7,8-tetrahydro-pyrido[4,3-d]pyrimidine.

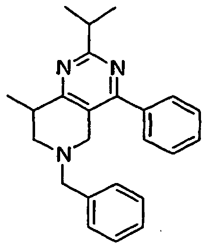

### Example 95; 6-Benryl-2-isopropyl-8-methyl-4-phenyl-5,6,7,8-tetrahydro-pyrido[4,3-d]pyrimidine.

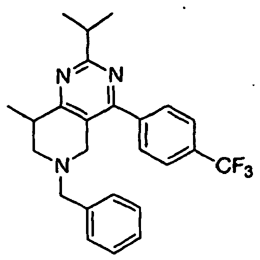

### Example 96; 6-Benzyl-2-isopropyl-8-methyl-4-(4-trifluoromethyl-phenyl)-5,6,7,8-tetrahydro-pyrido[4,3.d]pyrimidine.

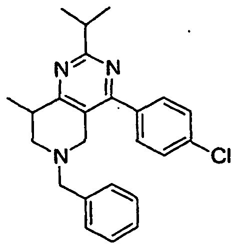

### Example 97; 6-Benzyl-4-(4-chloro-phenyl)-2-isopropyl-8-methyl-5,6,7,8-tetrahydro-pyrido[4,3-d]pyrimidine.

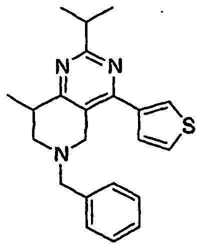

### Example 98; 6-Benzyl-2-isopropyl-8-methyl-4-thiophen-3-yl-5,6,7,8-tetrahydro-pyrido[4,3-d]pyrimidine.

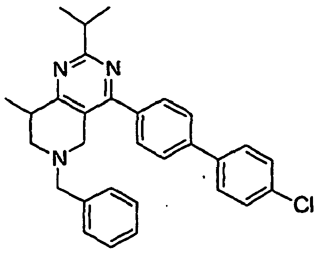

### Example 99; 6-Benzyl-4-(4'-chloro-biphenyl-4-yl)-2-isopropyl-8-methyl-5,6,7,8-tetrahydro-pyrido[4,3-d]pyrimidine.

The following compounds in Examples 100-105 were prepared using methods similar to those described in Example 76.

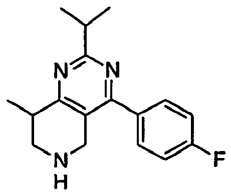

### Example 100; 4-(4-Fluoro-phenyl)-2-isopropyl-8-methyl-5,6,7,8-tetrahydro-pyrido[4,3-d]pyrimidine hydrochloride.

MS (ESI): exact mass calcd. for C₁₇H₂₀FN₃, 285.16; m/z found, 286.4 [M+H]⁺. ¹H NMR (MeOH-d₄): 7.69-7.66 (m, 2H), 7.34-7.30 (m, 2H), 4.56-4.53 (m, 1H), 4.32-4.29 (m, 1 H), 3.81-3.78 (m, 1H), 3.45-3.38 (m, 1 H), 3.36-3.30 (m, 1 H), 3.26-3.24 (m, 1 H), 1.55 (d, J = 7.2, 3H), 1.37 (d, J = 6.6, 6H).

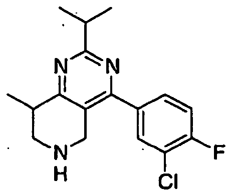

### Example 101; 4-(3-Chloro-4-fluoro-phenyl)-2-isopropyl-8-methyl-5,6,7,8-tetrahydro-pyrido[4,3-d]pyrimidine hydrochloride.

MS (ESI): exact mass calcd. for C₁₇H₁₉CIFN₃, 319.13; m/z found, 320.3 [M+H]⁺. ¹H NMR (MeOH-d₄): 7.79-7.77 (m, 1 H), 7.60-7.58 (m, 1 H), 7.46-7.44 (m, 1 H), 4.56-4.54 (m, 1 H), 4.32-4.29 (m, 1H), 3.80-3.77 (m, 1 H), 3.40-3.32 (m, 2H), 3.26-3.20 (m, 1H), 1.54 (d, J = 7.2, 3H), 1.36 (d, J = 7.2, 6H).

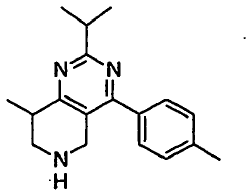

### Example 102; 2-Isopropyl-8-methyl-4-p-tolyl-5,6,7,8-tetrahydro-pyrido[4,3-d]pyrimidine hydrochloride.

MS (ESI): exact mass calcd. for C₁₈H₂₃N₃, 281.19; m/z found, 282.4 [M+H]⁺. ¹H NMR (MeOH-d4): 7.57-7.56 (m, 2H), 7.45-7.44 (m, 2H), 4.59-4.56 (m, 1H), 4.35-4.33 (m, 1 H), 3.83-3.80 (m, 1 H), 3.52-3.49 (m, 1 H), 3.40-3.30 (m, 2H), 2.46 (s, 3H), 1.58 (d, J = 7.2, 3H), 1.41 (d, J = 6.6, 6H).

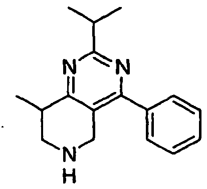

### Example 103; 2-Isopropyl-8-methyl-4-phenyl-5,6,7,8-tetrahydro-pyrido[4,3-d]pyrimidine.

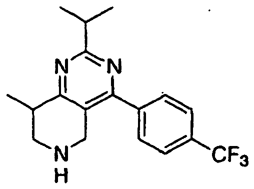

### Example 104; 2-Isopropyl-8-methyl-4-(4-trifluoromethyl-phenyl)-5-,6,7,8-tetrahydro-pyrido[4,3-d]pyrimidine.

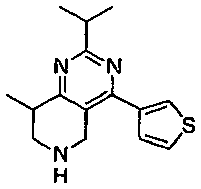

### Example 105; 2-Isopropyl-8-methyl-4-thiophen-3-yl-5,6,7,8-tetrahydro-pyrido[4,3-d]pyrimidine.

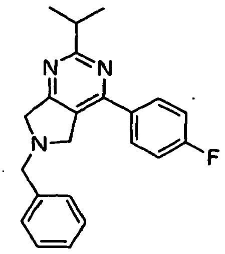

### Example 106; 4-(4-Fluoro-phenyl)-2-isopropyl-6,7-dihydro-5H-pyrrolo[3,4-d]pyrimidine hydrochloride.

Step A. 6-Benzyl-2-isopropyl-6,7-dihydro-5H-pyrrolo[3,4-d]pyrimidin-4-ol. To a solution of 1-benzyl-4-oxo-pyrrolidine-3-carboxylic acid ethyl ester hydrochloride (U.S. Pat. No. 3,312,716; 0.568 g, 2.30 mmol) in tert-BuOH was added isobutyramidine hydrochloride (0.282 g, 2.30 mmol) and KOtBu (0.516 g, 4.6 mmol). After heating for 6 h at 100°C, the reaction was cooled to rt, concentrated, diluted with water and washed with Et₂O. The organic layer discarded. The aqueous layer was adjusted to pH 7 and extracted with Et₂O. The organic layers were then dried and concentrated to give 0.145 g (23%) of the title compound of yellow solid that was used without further purification.

Steps B and C. The title compound was prepared according to the methods described in Example 1, Steps B and C. MS (ESI): exact mass calcd. for C₂₂H₂₂FN₃, 347.18; m/z found, 348.3 [M+H]⁺. ¹H NMR (MeOH-d₄): 7.99-7.96 (m, 2H), 7.61-7.60 (m, 2H), 7.53-7.51 (m, 3H), 7.34-7.32 (m, 2H), 5.09 (br s, 2H), 4.71 (br s, 4H), 3.31-3.25 (m, 1 H), 1.38 (d, J = 7.2, 6H).

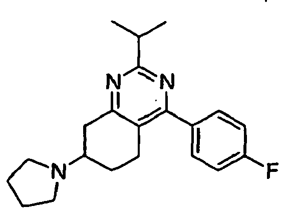

### Example 107; 4-(4-Fluoro-phenyl)-2-isopropyl-7-pyrrolidin-1-yl-5,6,7,8-tetrahydroquinazoline.

Step A. 4-Ethoxy-2-oxo-cyclohex-3-enecarboxylic acid ethyl ester. To a -78 °C solution of LDA (192 mmol) in THF (200 mL) was added 3-ethoxy-cyclohex-2-enone (23 mL) dropwise. After stirring for 1 h at -78 °C, ethyl cyanoformate (16 mL) was added. The mixture was stirred at -78°C for 4 h and then was warmed to rt and stirred for 1 h. The mixture was concentrated, diluted with aq. NH₄Cl (300 mL), and poured into water. The resulting solids were collected by suction filtration and washed with hexanes followed by water, then was dried and concentrated to provide 17.1 g of a brown solid. TLC (SiO₂, 33% EtOAc/hexanes): R_{f} = 0.43.

Step B. 7-Oxo-1,4-dioxa-spiro[4.5]decane-8-carboxylic acid ethyl ester. To a solution of the product of Step A (25.4 g resulting from iterative reactions) in toluene (500 mL) was added ethylene glycol (8.5 mL) and p-TsOH (1.9 g). The mixture was heated at reflux for 4 h in a flask fitted with a Dean-Stark trap. The mixture was then cooled and concentrated. Chromatography on SiO₂ (0 to 15% EtOAc/hexanes) afforded 7.76 g of the desired compound. TLC (SiO₂, 25% EtOAc/hexanes): R_{f} = 0.42.

Step C. 2-Isopropyl-7-(2-[1,3]dioxolane)-5,6,7,8-tetrahydro-quinazolin-4-ol. The title compound was prepared according to the method described in Example 1, Step A. MS (ESI): exact mass calcd. for C₁₃H₁₈N₂O₃, 250.13; m/z found, 251.3 [M+H]⁺.

Step D. Trifluoro-methanesulfonic acid 2-isopropyl-7-(2-[1,3]Dioxolane)-5,6,7,8-tetrahydro-quinazolin-4-yl ester. The title compound was prepared according to the method described in Example 1, Step B. TLC (SiO₂, 25% EtOAc/hexanes): R_{f} = 0.46. MS (ESI): exact mass calcd. for C₁₄H₁₇F₃N₂O₅S, 382.08; m/z found, 383.2 [M+H]⁺. Step E. 4-(4-Fluoro-phenyl)-2-isopropyl-7-(2-[1,3]dioxolane)-5,6,7,8-tetrahydroquinazoline. The title compound was prepared according to the method described in Example 1, Step C. TLC (SiO₂, 25% EtOAc/hexanes): R_{f} = 0.40. MS (ESI): exact mass calcd. for C₁₉H₂₁ FN₂O₂, 328.16; m/z found, 329.3 [M+H]⁺.

Step F. 4-(4-Fluoro-phenyl)-2-isopropyl-5,8-dihydro-6H-quinazolin-7-one. To a solution of the product of Step E (1.15 g) in THF (70 mL) was added 1 M HCl (6 mL). The mixture was heated at 60°C for 10 h, cooled to rt, and poured into 350 mL of water. The aqueous mixture was basified to pH -9 with 1 M NaOH, and was extracted with CH₂Cl₂. The organic layer was dried and concentrated to afford 0.98 g of the desired compound, which was used in the next step without purification. MS (ESI): exact mass calcd. for C₁₇H₁₇FN₂O, 284.13; m/z found, 285.3 [M+H]⁺.

Step G. 4-(4-Fluoro-phenyl)-2-isopropyl-7-pyrrolidin-1-yl-5,6,7,8-tetrahydroquinazoline. To a solution of the product of Step F (0.128 g) in MeOH (4 mL) was added of bromocresol green (0.003 g), pyrrolidine (0.06 mL), and NaBH₃CN (0.20 g). To this mixture was added 1 M HCl in MeOH until a persistent color change to yellow was observed. After 30 min, the mixture was quenched with 1 M NaOH and poured into water (50 mL). The mixture was extracted with CH₂Cl₂, dried and concentrated. Chromatography on SiO₂ (0 to 5% 2 M NH₃ in MeOH/CH₂Cl₂) afforded 0.015 g of the desired compound. MS (ESI): exact mass calcd. for C₂₁H₂₆FN₃, 339.21; m/z found, 340.4 [M+H]⁺. ¹H NMR (CDCl₃): 7.59-7.54 (m, 2H), 7.18-7.13 (m, 2H), 3.76-3.68 (m, 1 H), 3.50-3.44 (m, 1H), 3.41-3.35 (m, 1H), 3.28-3.14 (m, 2H), 3.0-2.93 (m, 1 H), 2.88-2.70 (m, 4H), 2.36-2.23 (m, 3H), 2.19-2.09 (m, 1H), 1.98-1.87 (m, 2H).

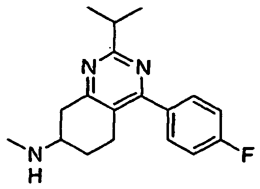

### Example 108; [4-(4-Fluoro-phenyl)-2-isopropyl-5,6,7,8-tetrahydro-quinazolin-7-yl]-methyl-amine hydrochloride.

The title compound was prepared according to the method described for Example 107. MS (ESI): exact mass calcd. for C₁₈H₂₂FN₃, 299.18; m/z found, 300.4 [M+H]⁺. ¹H NMR (MeOH-d₄): 7.80-7.72 (m, 2H), 7.37-7.30 (m, 2H), 3.81-3.71 (m, 1 H), 3.65-3.56 (m, 1H), 3.35-3.26 (m, 1H), 3.22-3.14 (m, 1H), 3.11-3.00 (m, 1H), 2.99-2.91 (m, 1H), 2.85 (s, 3H), 2.42-2.33 (m, 1H), 1.92-1.82 (m, 1H), 1.43-1.38 (m, 6H).

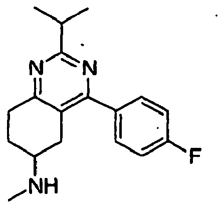

### Example 109; [4-(4-Fluoro-phenyl)-2-isopropyl-5,6,7,8-tetrahydro-quinazolin-6-yl]-methyl-amine hydrochloride.

The title compound was prepared according to the methods described for Example 107, using 1,4-dioxa-spiro[4.5]decan-8-one. MS (ESI): exact mass calcd. for C₁₈H₂₂FN₃, 299.18; m/z found, 300.4 [M+H]⁺. ¹H NMR (MeOH-d₄): 7.87-7.82 (m, 2H), 7.40-7.35 (m, 2H), 3.59-3.52 (m, 1H), 3.41-3.24 (m, 4H), 3.20-3.13 (m, 1 H), 2.76 (s, 3H), 2.54-2.47 (m, 1 H), 2.21-2.11 (m, 1 H), 1.45 (d, J = 6.9, 3H), 1.44 (d, J = 6.9, 3H).

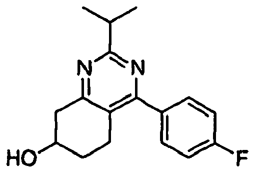

### Example 110; 4-(4-Fluoro-phenyl)-2-isopropyl-5,6,7,8-tetrahydro-quinazolin-7-ol.

To a solution of 4-(4-fluoro-phenyl)-2-isopropyl-5,8-dihydro-6H-quinazolin-7-one (0.126 g) in EtOH (3 mL) was added NaBH₄ (0.053 g). After 16 h, the mixture was treated with 1 M NaOH (5 mL) and water (10 mL). The mixture was stirred for 30 min then extracted with CH₂Cl₂. The organic layer was dried and concentrated. Chromatography on SiO₂ (10 to 35% EtOAc/hexanes) afforded 0.98 g of the title compound. TLC (SiO₂, 50% EtOAc/hexanes): R, = 0.18. MS (ESI): exact mass calcd. for C₁₇H₁₉FN₂O, 286.15; m/z found, 287.3 [M+H]⁺.

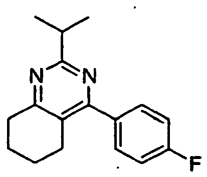

### Example 111; 4-(4-Fluoro-phenyl)-2-isopropyl-5,6,7,8-tetrahydro-quinazoline.

The title compound was obtained as a minor product from the hydrogenation of of 4-(4-fluoro-phenyl)-2-isopropyl-5,8-dihydro-6H-quinazolin-7-one with 10% Pd/C in the presence of pyrrolidine. MS (ESI): exact mass calcd. for C₁₇H₁₉FN₂, 270.15; m/z found, 271.3 [M+H]⁺. ¹H NMR (CDCl₃): 7.59-7.55 (m, 2H), 7.16-7.12 (m, 2H), 3.21-3.13 (m, 1H), 2.95-2.91 (m, 2H), 2.71-2.67 (m, 2H), 1.95-1.89 (m, 2H), 1.77-1.72 (m, 2H), 1.35 (d, J = 6.9, 6H).

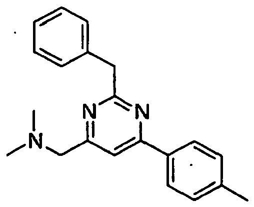

### Example 112; (2-Benzyl-6-p-tolyl-pyrimidin-4-ylmethyl)-dimethyl-amine.

Step A. 2-Benzyl-4-(tetrahydro-pyran-2-yloxymethyl)-6-p-tolyl-pyrimidine. To a solution of Pd(PPh₃)₂Cl₂ (0.285 g, 0.41 mmol) and Cul in THF (50 mL) (0.148 g, 0.777 mmol) was added Et₃N (1.5 mL, 11.0 mmol), p-toluoyl chloride (1.3 mL, 10.0 mmol) and tetrahydro-2-(2-propynyloxy)-2H-pyran (1.4 mL, 10.0 mmol). After stirring for 2.5 h, a solution of 2-phenylacetamidine hydrochloride (2.0 g, 11.7 mmol) in THF/MeOH (1:1, 10 mL) was added followed by additional MeOH (5 mL) and Na₂CO₃ (3.2 g, 30.0 mmol). The reaction mixture was heated at reflux for 15 h, cooled to rt, diluted with Et₂O and filtered through a small pad of diatomaceous earth. The filtrate was concentrated and purified via SiO₂ chromatography (10-45% EtOAc/hexanes) to give 2.0 g (53%) of the title compound. ¹H NMR (CDCl₃): 8.02-8.00 (m, 2H), 7.68 (s, 1 H), 7.46-7.44 (m, 2H), 7.30-7.27 (m, 4H), 7.21-7.19 (m, 1 H), 4.87 (d, J = 14.7, 1H). 4.77 (t, J = 3.5, 1 H), 4.62 (d, J = 15.1, 1 H), 4.31 (s, 2H), 3.91-3.86 (m, 1 H), 3.57-3.52 (m, 1H), 2.41 (s, 3H), 1.96-1.87 (m, 1 H), 1.84-1.72 (m, 2H), 1.66-1.55 (m, 3H).

Step B. (2-Benzyl-6-p-tolyl-pyrimidin-4-yl)-methanol. To a solution of the product from Step A (2.0 g, 5.3 g) in MeOH (30 mL) was added *p*-TsOH•H₂O. After 18 h, the reaction was diluted with satd. aq. NaHCO₃ and extracted with EtOAc (2X). The combined organic layers were dried and concentrated to give the 1.53 g (99%) of the title compound. MS (ESI): exact mass calcd. for C₁₉H₁₈N₂O, 290.14; m/z found, 291.4 [M+H]⁺. ¹H NMR (CDCl₃): 8.00-7.98 (m, 2H), 7.45-7.43 (m, 3H), 7.32-7.28 (m, 4H), 7.23-7.20 (m, 1 H), 4.74 (d, J = 4.8, 2H), 4.33 (s, 2H), 3.62 (t, J = 5.1, 1H), 2.42 (s, 3H).

Step C. (2-Benzyl-6-p-tolyl-pyrimidin-4-ylmethyl)-dimethyl-amine. To a solution of the product from Step B (0.102 g, 0.35 mmol) in CH₂Cl₂ (2 mL) was added the Dess-Martin periodinane (0.228, 0.53 mmol). After 30 min, the mixture was diluted satd. aq. NaHCO₃ and extracted with CH₂Cl₂ (2X). The combined organic layers were dried, concentrated, and filtered through a small plug of SiO₂ (25% EtOAc in hexanes). The filtrate was concentrated to give 2-benzyl-6-p-tolyl-pyrimidine-4-carbaldehyde (0.55g, 54%). To a solution of this aldehyde in CH₂Cl₂ (3 mL) was added dimethylamine (2 M in THF; 0.15 mL, 0.30 mmol) and NaBH(OAc)₃ (0.058 mg, 0.27 mmol). After 15 h, the reaction was diluted with CH₂Cl₂ and washed with 1 N NaOH. The aqueous layer was extracted with CH₂Cl₂ (1X). The combined organic layers were dried and concentrated. The resulting residue was purified via SiO₂ chromatography (1-7% 2 M NH₃ in MeOH/CH₂Cl₂) to give 0.050 g (79%) of the title compound. MS (ESI): exact mass calcd. for C₂₁H₂₃N₃, 317.19; m/z found, 318.4 [M+H]⁺. ¹H NMR (CDCl₃): 8.02 (d, J = 8.2, 2H), 7.66 (s, 1 H), 7.22 (d, J = 7.5, 2H), 7.31-7.26 (m, 4H), 7.22-7.18 (m, 1H), 4.33 (s, 2H), 3.58 (s, 2H), 2.41 (s, 3H), 2.32 (s, 6H).

The compounds in Examples 113-114 were prepared using methods similar to those described in Example 112, with the appropriate substituent changes.

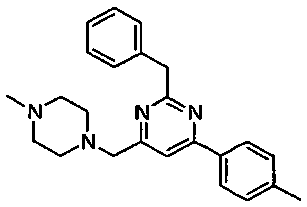

### Example 113; 2-Benzyl-4-(4-methyl-piperazin-1-ylmethyl)-6-p-tolyl-pyrimidine.

MS (ESI): exact mass calcd. for C₂₄H₂₈N₄, 372.23; m/z found, 373.5 [M+H]⁺. ¹H NMR (CDCl₃): 8.02-7.99 (m, 2H), 7.66 (s, 1H), 7.46-7.44 (m, 2H), 7.31-7.27 (m, 4H), 7.22-7.18 (m, 1 H), 4.32 (s, 2H), 3.66 (s, 2H), 2.58-2.50 (m, 8H), 2.42 (s, 3H), 2.32 (s, 3H).

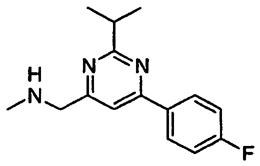

### Example 114; [6-(4-Ftuoro-phenyl)-2-isopropyl-pyrimidin-4-ylmethyl]-methyl-amine.

Prop-2-ynyl-carbamic acid tert-butyl ester was converted to [6-(4-fluoro-phenyl)-2-isopropyl-pyrimidin-4-ylmethyl]-methyl-carbamic acid tert-butyl ester using methods described in Example 112, Step A. This ester was deprotected according to the methods described in Example 1, Step D, to provide the title compound. MS (ESI): exact mass calcd. for C₁₅H₁₈FN₃, 259.15; m/z found, 260.3 [M+H]⁺. ¹H NMR (DMSO-d₈): 9.45 (s, 2H); 8.31-8.28 (m, 2H), 8.10 (s, 1H), 7.45-7.41 (m, 2H), 4.35 (t, J = 6.0, 2H), 3.26-3.18 (m, 1H), 2.69 (t, J = 5.4, 3H), 1.37 (d, J = 6.9, 6H).

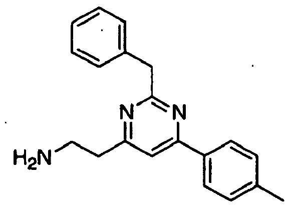

### Example 115; 2-(2-Benzyl-6-p-tolyl-pyrimidin-4-yl)-ethylamine.

Step A and B. 2-(2-Benzyl-6-p-tolyl-pyrimidin-4-yl)-ethanol. The title compound was prepared from 2-(3-butynyloxy)tetrahydro-2H-pyran using the methods described in Example 112, Steps A and B. MS (ESI): exact mass calcd. for C₂₀H₂₀N₂O, 304.16; m/z found, 305.4 [M+H]⁺. ¹H NMR (CDCl₃): 7.98 (d, J = 8.2, 2H), 7.42-7.42 (m, 2H), 7.34-7.29 (m, 4H), 7.25-7.22 (m, 1 H), 4.31 (s, 2H), 3.98 (t, J = 5.3, 2H), 2.97 (t, J = 5.3, 2H), 2.42 (s, 3H).

Step C. 4-(2-Azido-ethyl)-2-benzyl-6-p-tolyl-pyrimidine. To a 0 °C solution of the product of Step B (0.150 g, 0.493 mmol) in THF (2.5 mL) was added MsCI (0.042 mL, 0.54 mmol) followed by Et₃N (0.76 mL, 0.54 mmol). After 1 h, EtOAc was added and the mixture washed with brine, dried and concentrated to give methanesulfonic acid 2-(2-benzyl-6-p-tolyl-pyrimidin-4-yl)-ethyl ester (0.185 g). To a solution of this mesylate (0.120 g, 0.32 mmol) in DMF (1 mL) was added sodium azide (0.105 g, 1.6 mmol). The flask was heated at 40 °C for 10 h, then was cooled to rt, diluted with water, and extracted with EtOAc (3X). The combined organic layers were dried and concentrated. The resulting residue was purified via SiO₂ chromatography (5-15% EtOAc/hexanes) to give 0.080 g (76%) of the title compound. MS (ESI): exact mass calcd. for C₂₀H₁₉N₅, 329.16; m/z found, 330.4 [M+H]⁺. ¹H NMR (CDCl₃): 7.98 (d, J = 8.2, 2H), 7.46-7.43 (m, 2H), 7.38 (s, 1 H), 7.32-7.28 (m, 4H), 7.23-7.19 (m, 2H), 4.31 (s, 2H), 3.76 (t, J = 6.8, 2H), 3.02 (t, J = 6.8, 2H), 2.42 (s, 3H).

Step D. To a solution of the product of Step C (0.066 g, 0.2 mmol) in THF (2 mL) was added PPh₃ (0.059 g, 2.2 mmol). After 18 h, water was added (0.10 mL) and the mixture was stirred for 48 h. The mixture was diluted with water and extracted with CH₂Cl₂ (2X). The combined organic layers were dried and concentrated. The resulting residue was purified via SiO₂ chromatography (5-15% EtOAc/hexanes) to give 0.060 g (99%) of the title compound. MS (ESI): exact mass calcd. for C₂₀H₂₁N₃, 303.17; m/z found, 304.4 [M+H]⁺. ¹H NMR (CDCl₃): 7.98-7.96 (m, 2H), 7.45-7.44 (m, 2H), 7.36 (s, 1 H), 7.32-7.28 (m, 4H), 7.23-7.19 (m, 1H), 4.31 (s, 2H), 3.13 (t, J = 6.5, 2H), 2.91 (t, J = 6.5, 2H), 2.42 (s, 3H).

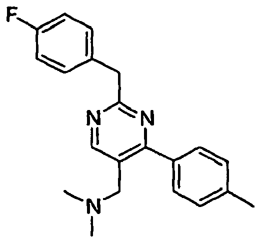

### Example 116; [2-(4-Fluoro-benzyl)-4-p-tolyl-pyrimidin-5-ylmethyl]-dimethyl-amine.

Step A. 2-(4-Fluoro-benzyl)-4-p-tolyl-pyrimidine-5-carboxylic acid ethyl ester. To solution of 3-dimethylamino-2-(4-methyl-benzoyl)-acrylic acid ethyl ester (Tetrahedron, 2002, 58, 8581-8589; 0.567 g, 2.15 mmol) in EtOH (10 mL) was added 2-(4-fluorophenyl)-acetamidine hydrochloride (0.405 g, 2.15 mmol) and Et₃N (0.90 mL, 6.5 mmol). The mixture was heated at reflux for 18 h, and then was diluted with water and extracted with EtOAc (2X). The combined organic layers were dried and concentrated. The resulting residue was purified via SiO₂ chromatography (5-20% EtOAc/hexanes) to give 0.560 g (74%) of the title compound. MS (ESI): exact mass calcd. for C₂₁H₁₉FN₂O₂, 350.14; m/z found, 351.4 [M+H]⁺. ¹H NMR (CDCl₃): 8.97 (s, 1 H), 7.51 (d, J = 8.2, 2H), 7.38-7.35 (m, 2H), 7.27-7.26 (m, 2H), 7.01-6.97 (m, 2H), 4.32 (s, 2H), 4.23 (q, J = 7.1, 2H), 2.42 (s, 3H), 1.16 (t, J = 7.1, 3H).

Ster B. [2-(4-Fluoro-benzyl)-4-p-tolyl-pyrimidin-5-yl]-methanol. To a 0 °C solution of the product from Step A (0.606 g, 1.73 mmol) in THF (8 mL) was added DIBAL-H (1.5 M in toluene; 2.5 mL, 3.8 mmol). The mixture was allowed to warm to rt, and was stirred for 18 h. The mixture was diluted with 20% aq. sodium potassium tartrate and extracted with EtOAc (2X). The combined organic layers were dried and concentrated. The resulting residue was purified via SiO₂ chromatography (40-60% EtOAc/hexanes) to give 0.330 g (62%) of the title compound. MS (ESI): exact mass calcd. for C₁₉H₁₇FN₂O, 308.13; m/z found, 309.4 [M+H]⁺. ¹H NMR (CDCl₃): 8.77 (s, 1 H), 7.58 (d, J = 8.0; 2H), 7.38-7.35 (m, 2H), 7.29 (d, J = 7.9, 2H), 6.99-6.96 (m, 2H), 4.70 (s, 2H), 4.29 (s, 2H), 2.42 (s, 3H).

Step C. The title compound was prepared using the methods described in Example 112, Step C. MS (ESI): exact mass calcd. for C₂₁H₂₂FN₃, 335.18; m/z found, 336.4 [M+H]⁺. ¹H NMR (CDCl₃): 8.67 (s, 1H), 7.69 (d, J = 8.1, 2H), 7.40-7.36 (m, 2H), 7.29 (d, J = 7.9, 2H), 7.01-6.96 (m, 2H), 4.29 (s, 2H), 3.36 (s, 2H), 2.42 (s, 3H), 2.22 (s, 6H).

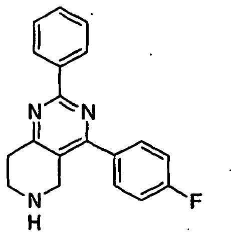

### Example 117; 4-(4-Fluoro-phenyl)-2-phenyl-5,6,7,8-tetrahydro-pyrido[4,3-d]pyrimidine.

The title compound was prepared as described in the preceding examples. MS (ESI)_{:} exact mass calcd. for C₁₉H₁₆FN₃, 305.35; m/z found, 306.4 [M+H]⁺. ¹H NMR (CDCl₃)_{:} 8.48-8.46 (m, 2H), 7.66 (dd, J = 5.4, 8.7, 2H), 7.49-7.45 (m, 3H), 7.21-7.17, (m, J = 8.7, 2H), 4.06 (s, 2H), 3.31 (t, J = 6.1, 2H), 3.08 (t, J = 6.1, 2H).

The following Examples 118-163 may be prepared according to the methods described in the preceding examples.

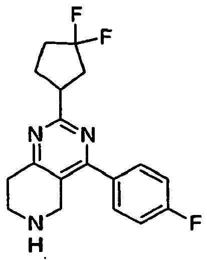

### Example 118; 2-(3,3-Difluoro-cyclopentyl)-4-(4-fluoro-phenyl)-5,6,7,8-tetrahydro-pyrido[4,3-d]pyrimidine.

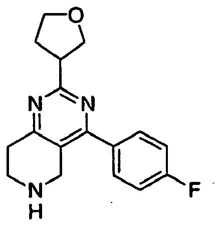

### Example 119; 4-(4-Fluoro-phenyl)-2-(tetrahydro-furan-3-yl)-5,6,7,8-tetrahydro-pyrido[4,3-d]pyrimidine.

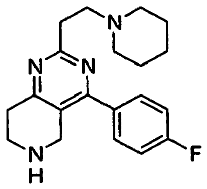

### Example 120; 4-(4-Fluoro-phenyl)-2-(2-piperidin-1-yl-ethyl)-5,6,7,8-tetrahydro-pyrido[4,3-d]pyrimidine.

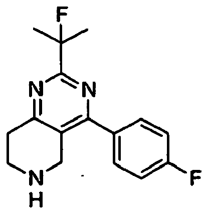

### Example 121; 2-(1-Fluoro-1-methyl-ethyl)-4-(4-fluoro-phenyl)-5,6,7,8-tetrahydro-pyrido[4,3-d]pyrimidine.

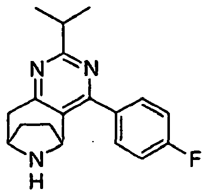

### Example 122; 3-(4-Fluoro-phenyl)-5-isopropyl-4,6,12-triaza-tricyclo[7.2.1.02^{2,7}]dodeca-2,4,6-triene.

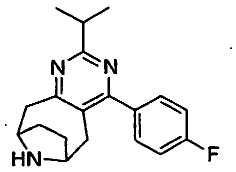

### Example 123; 7-(4-Fluoro-phenyl)-5-isopropyl-4,6,13-triaza-tricyclo[8.2.1.0^{3,8}]trideca-3,5,7-triene.

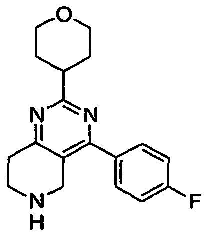

### Example 124; 4-(4-Fluoro-phenyl)-2-(tetrahydro-pyran-4-yl)-5,6,7,8-tetrahydro-pyrido[4,3-d]pyrimidine.

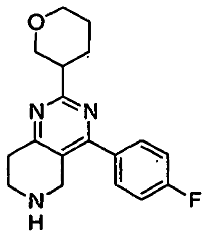

### Example 125; 4-(4-Fluoro-phenyl)-2-(tetrahydro-pyran-3-yl)-5,6,7,8-tetrahydro-pyrido[4,3-d]pyrimidine.

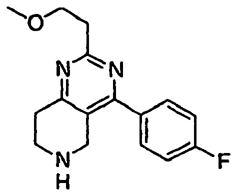

### Example 126; 4-(4-Fluoro-phenyl)-2-(2-methoxy-ethyl)-5,6,7,8-tetrahydro-pyrido[4,3-d]pyrimidine.

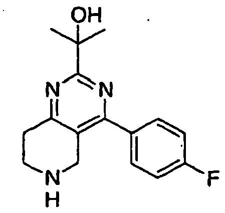

### Example 127; 2-[4-(4-Fluoro-phenyl)-5,6,7,8-tetrahydro-pyrido[4,3-d]pyrimidin-2-yl]-propan-2-ol.

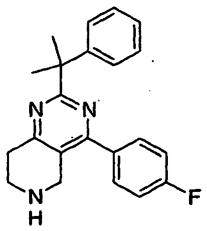

### Example 128; 4-(4-Fluoro-phenyl)-2-(1-methyl-1-phenyl-ethyl)-5,6,7,8-tetrahydro-pyrido[4,3-d]pyrimidine.

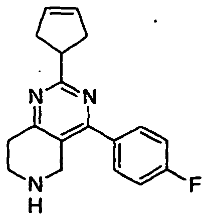

### Example 129; 2-Cyclopent-3-enyl-4-(4-fluoro-phenyl)-5,6,7,8-tetrahydro-pyrido[4,3-d]pyrimidine.

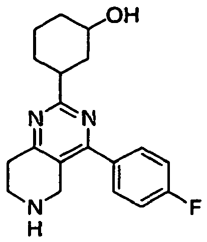

### Example 130; 3-[4-(4-Fluoro-phenyl)-5,6,7,8-tetrahydro-pyrido[4,3-d]pyrimidin-2-yl]-cyclohexanol.

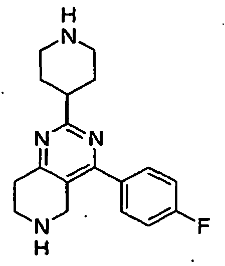

### Example 131; 4-(4-Fluoro-phenyl)-2-piperidin-4-yl-5,6,7,8-tetrahydro-pyrido[4,3-d]oyrimidine.

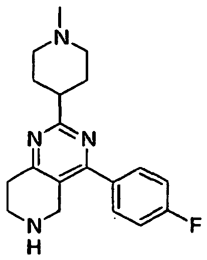

### Example 132; 4-(4-Fluoro-phenyl)-2-(1-methyl-piperidin-4-yl)-5,6,7,8-tetrahydro-pyrido[4,3-d]pyrimidine.

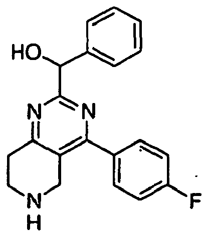

### Example 133; [4-(4-Fluoro-phenyl)-5,6,7,8-tetrahydro-pyrido[4,3-d]pyrimidin-2-yl]-phenyl-methanol.

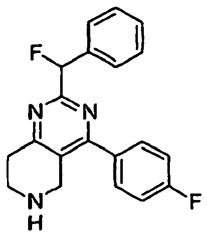

### Example 134; 4-(4-Fluoro-phenyl)-2-(fluoro-phenyl-methyl)-5,6,7,8-tetrahydro-pyrido[4,3-d]pyrimidine.

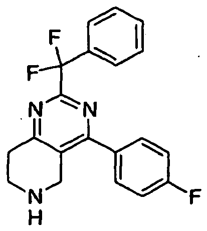

### Example 135; 2-(Difluoro-phenyl-methyl)-4-(4-fluoro-phenyl)-5,6,7,8-tetrahydro-pyrido[4,3-d]pyrimidine.

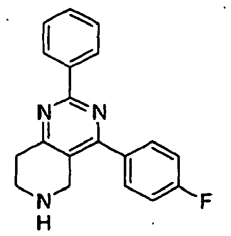

### Example 136; 4-(4-Fluoro-phenyl)-2-phenyl-5,6,7,8-tetrahydro-pyrido[4,3-d]pyrimidine.

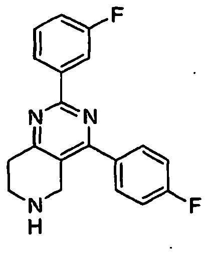

### Example 137; 4-(4-Fluoro-phenyl)-2-(3-fluoro-phenyl)-5,6,7,8-tetrahydro-pyrido[4,3-d]pyrimidine.

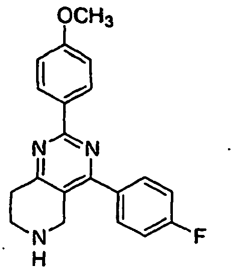

### Example 138; 4-(4-Fluoro-phenyl)-2-(4-methoxy-phenyl)-5,6,7,8-tetrahydro-pyrido[4,3-d]pyrimidine.

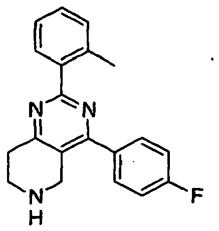

### Example 139; 4-(4-Fluoro-phenyl)-2-o-tolyl-5,6,7,8-tetrahydro-pyrido[4,3-d]pyrimidine.

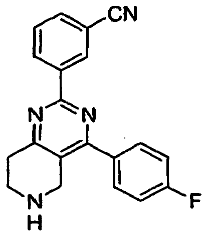

### Example 140; 3-[4-(4-Fluoro-phenyl)-5,6,7,8-tetrahydro-pyrido[4,3-d]pyrimidin-2-yl]-benzonitrile.

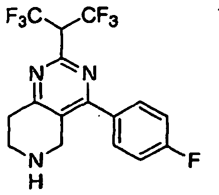

### Example 141; 4-(4-Fluoro-phenyl)-2-(2,2,2-trifluoro-1-trifluoromethyl-ethyl)-5,6,7,8-tetrahydro-pyrido[4,3-d]pyrimidine.

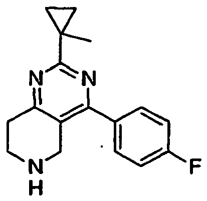

### Example 142; 4-(4-Fluoro-phenyl)-2-(1-methyl-cyclopropyl)-5,6,7,8-tetrahydro-pyrido[4,3-d]pyrimidine.

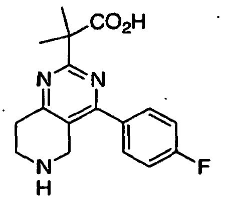

### Example 143; 2-[4-(4-Fluoro-phenyl)-5,6,7,8-tetrahydro-pyrido[4,3-d]pyrimidin-2-yl]-2-methyl-propionic acid.

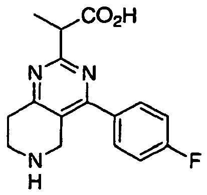

### Example 144; 2-[4-(4-Fluoro-phenyl)-5,6,7,8-tetrahydro-pyrido[4,3-d]pyrimidin-2-yl]-propionic acid.

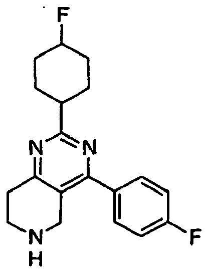

### Example 145; 2-(4-Fluoro-cyclohexyl)-4-(4-fluoro-phenyl)-5,6,7,8-tetrahydro-pyrido[4,3-d]pyrimidine.

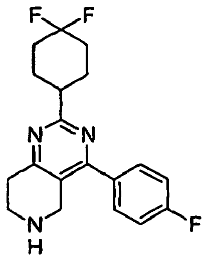

### Example 146; 2-(4,4-Difluoro-cycloheoyl)-4-(4-fluoro-phenyl)-5,6,7,8-tetrahydro-pyrido[4,3-d]pyrimidine.

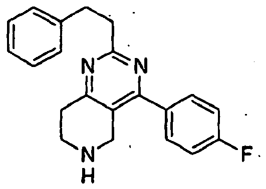

### Example 147; 4-(4-Fluoro-phenyl)-2-phenethyl-5,6,7,8-tetrahydro-pyrido[4,3-d]pyrimidine.

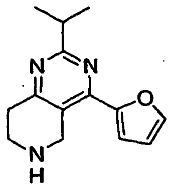

### Example 148; 4-Furan-2-yl-2-isopropyl-5,6,7,8-tetrahydro-pyrido[4,3-d]pyrimidine.

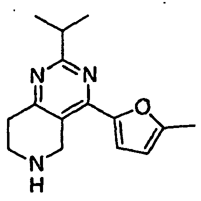

### Example 149; 2-Isopropyl-4-(5-methyl-furan-2-yl)-5,6,7,8-tetrahydro-pyrido[4,3-d]pyrimidine.

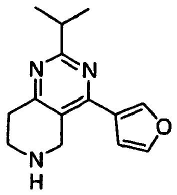

### Example 150; 4-Furan-3-yl-2-isopropyl-5,6,7,8-tetrahydro-pyrido[4,3-d]pyrimidine.

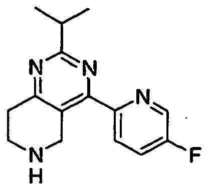

### Example 151; 4-(5-Fluoro-pyridin-2-yl)-2-isopropyl-5,6,7,8-tetrahydro-pyrido[4,3-d]pyrimidine.

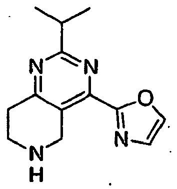

### Example 152; 2-Isopropyl-4-oxazol-2-yl-5,6,7,8-tetrahydro-pyrido[4,3-d]pyrimidine.

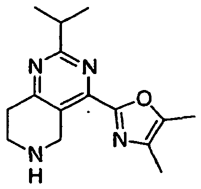

### Example 153; 4-(4,5-Dimethyl-oxazol-2-yl)-2-isopropyl-5,6,7,8-tetrahydro-pyrido[4,3-d]pyrimidine.

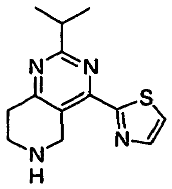

### Example 154; 2-isopropyl-4-thiazol-2-yl-5,6,7,8-tetrahydro-pyrido[4,3-d]pyrimidine.

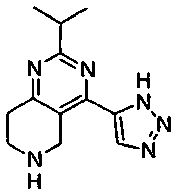

### Example 155; 2-Isopropyl-4-(3H-[1,2,3]triazol-4-yl)-5,6,7,8-tetrahydro-pyrido[4,3-d]pyrimidine.

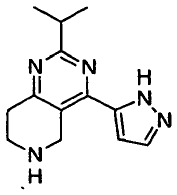

### Example 156; 2-Isopropyl-4-(2H-pyrazol-3-yl)-5,6,7,8-tetrahydro-pyrido[4,3-d]pyrimidine.

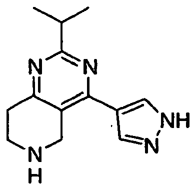

### Example 157; 2-Isopropyl-4-(1H-pyrazol-4-yl)-5,6,7,8-tetrahydro-pyrido[4,3-d]pyrimidine.

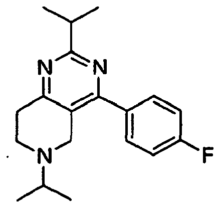

### Example 158; 4-(4-Fluoro-phenyl)-2,6-diisopropyl-5,6,7,8-tetrahydro-pyrido[4,3-d]pyrimidine.

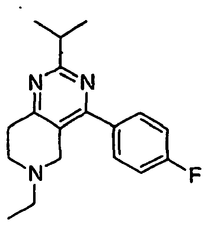

### Example 159; 6-Ethyl-4-(4-fluoro-phenyl)-2-isopropyl-5,6,7,8-tetrahydro-pyrido[4,3-d]pyrimidine.

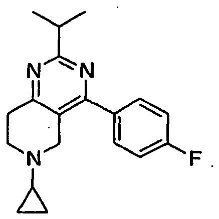

### Example 160; 6-Cyclopropyt-4-(4-fluoro-phenyl)-2-isopropyl-5,6,7,8-tetrahydro-pyrido[4,3-d]pyrimidine.

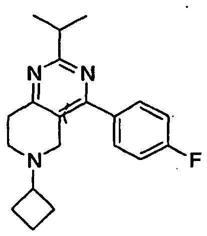

### .Example 161; 6-Cyclobutyl-4-(4-fluoro-phenyl)-2-isopropyl-5,6,7,8-tetrahydro-pyrido[4,3-d]pytimidine. -

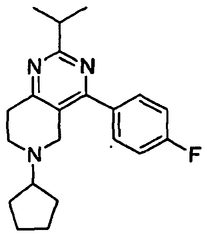

### Example 162; 6-Cyclopentyl-4-(4-fluoro-phenyl)-2-isopropyl-5,6,7,8-tetrahydro-pyrido[4,3-d]pyrimidine.

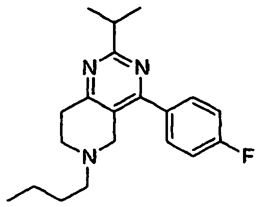

### Example 163; 6-Butyl-4-(4-fluoro-phenyl)-2-isopropyl-5,6,7,8-tetrahydro-pyrido[4,3-d]pyrimidine.

### Example 164; Alternative preparation of 4-(4-fluoro-phenyl)-2-isopropyl-5,6,7,8-tetrahydro-pyrido[4,3-d]pyrimidine.

Step A. 1-(3,4,5,6,3',6'-Hexahydro-2H,2'H-[1,4']bipyridinyl-1'-yl)-ethanone. A flask equipped with a Dean-Stark trap and a reflux condenser was charged with 1-acetyl-piperdin-4-one (100 g, 0.71 mol) and toluene (1 L). Piperidine (63.4 g, 0.75 mol) and p-toluenesulfonic acid monohydrate (0.27 g, 1.4 mmol, 0.2 mol %) were added, and the resulting solution was heated at reflux for 8 h. The mixture was cooled to rt and concentrated to give a crude product which was used directly in the next reaction.

Step B. 1-[5'-(4-Fluoro-benzoyl)-3,4,5,6,3',6'-hexahydro-2H,2'H-[1,4']bipyridinyl-1'-yl]-ethanone. A solution of crude 1-(3,4,5,6,3',6'-hexahydro-2H,2'H-[1,4']bipyridinyl-1'-yl)-ethanone in CH₂Cl₂ (1.5 L) was treated with Et₃N (108 mL, 0.78 mol) and then cooled to 0 °C. A solution of 4-fluorobenzoyl chloride (107 g, 0.68 mol) in CH₂Cl₂ (150 mL) was added over 1 h. The reaction mixture was stirred at rt for 2 h, then was concentrated to give a crude material which was directly used on next reaction. HPLC: R_{T} = 7.52 min. MS (ESI): exact mass calcd. for C₁₉H₂₃FN₂O₂, 330.17; m/z found, 331.0 [M+H]⁺.

Step C. 1-[4-(4-Fluoro-phenyl)-2-isopropyl-7,8-dihydro-5H-pyrido[4,3-d]pyrimidin-6-yl]-ethanone. A solution of crude 1-[5'-(4-fluoro-benzoyl)-3,4,5,6,3',6'-hexahydro-2H,2'H-[1,4']bipyridinyl-1'-yl]-ethanone in t-amyl alcohol (1.5 L) was treated with Et₃N (108 mL, 0.78 mol) and 2-methyl propanimidamide hydrochloride (82.6 g, 0.67 mol). The reaction mixture was heated at reflux for 16 h and then was cooled to rt. The mixture was concentrated, and the residue was diluted with water (2 L) and extracted with EtOAc (2x). The combined organic layers were dried (MgSO₄) and concentrated to afford the crude title compound, which was used in next reaction without further purification. HPLC: R_{T} = 8.11 min. MS (ESI): exact mass calcd. for C₁₈H₂₀FN₃O, 313.16; m/z found, 314.0 [M+H]⁺. ¹H NMR (CDCl₃; mixture of rotamers): 7.60-7.50 (m, 2H), 7.24-7.12 (m, 2H), 4.71 (s, 1.4H), 4.56 (s, 0.6H), 3.93 (t, J = 6.2, 0.6H), 3.80 (t, J = 6.2, 1.4H), 3.18 (sept, J = 6.8, 1 H), 3.07 (t, J = 6.2, 1.4H), 3.02 (t, J = 6.2, 0.6H), 2.15 (s, 2.1 H), 2.00 (s, 0.9H), 1.34 (d, J = 6.8, 6H).

Step D. A mixture of crude 1-[4-(4-fluoro-phenyl)-2-isopropyl-7,8-dihydro-5H-pyrido[4,3-d]pyrimidin-6-yl]-ethanone in 10% aq. HCl (800 mL) was heated at reflux for 2 h and then cooled to rt. The aqueous solution was washed with EtOAc (400 mL) and then was basified with NaOH pellets (-120 g) to pH > 12. The basic solution was extracted with CH₂Cl₂ (2 x 500 mL). The combined organic layers were washed with 1 N NaOH (400 mL), dried (MgSO₄), and concentrated to give the crude product (100 g), which was used in the next reaction without further purification. HPLC: RT = 6.89 min. MS (ESI): exact mass calcd. for C₁₆H₁₈FN₃, 271.15; m/z found, 271.9 [M+H]⁺.

### Example 165; 4-(4-Fluoro-phenyl)-2-isopropyl-5,6,7,8-tetrahydro-pyrido[4,3-d]pyrimidine, citrate salt.

A solution of crude 4-(4-fluoro-phenyl)-2-isopropyl-5,6,7,8-tetrahydro-pyrido[4,3-d]pyrimidine (Example 164) in EtOH (1 L) was treated with citric acid (71 g, 0.37 mol). The solution was warmed to 50 °C to form a homogeneous solution, then was cooled to rt and stirred for 16 h. The mixture was diluted with Et₂O (750 mL) and was stirred for 1 h. The precipitated white solid was collected by filtration and washed with cold EtOH (ca. 100 mL). The white solid was dried in a vacuum oven at 55 ºC to give an off-white solid (157.3 g, 50% overall yield). HPLC: RT = 6.86 min. MS (ESI): exact mass calcd. for C₁₆H₁₈FN₃, 271.15; m/z found, 271.9 [M+H]⁺. ¹H NMR (D₂O): 7.49-7.43 (m, 2H), 7.27-7.19 (m, 2H), 4.28 (s, 2H), 3.60 (t, J = 6.5, 2H), 3.20 (t, J = 6.5, 2H), 3.11 (sept, J = 6.9, 1 H), 2.77 (d, J = 15.4, 2H), 2.65 (d, J = 15.4, 2H), 1.23 (d, J = 6.9, 6H). ¹³C NMR (D₂O): 176.59, 172.69, 171.99, 162.59 (d, J_{C-F} =11.6), 160.67, 159.41, 129.42, 128.53 (d, J_{C-F} = 8.8), 115.91, 113.89 (d, J_{C-F} = 22.4), 71.78, 41,61, 40.10, 38.77, 34.78, 25.33, 18.64.

The compounds in Examples 166-167 were prepared using methods analogous to those described in the preceding examples.

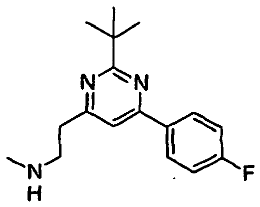

### Example 166; {2-[2-tert-Butyl-6-(4-fluoro-phenyl)-pyrimidin-4-yl]-ethyl}-methyl-amine.

MS (ESI): exact mass calcd. for C₁₇H₂₂FN₃, 287.18; m/z found, 288.7 [M+H]⁺.

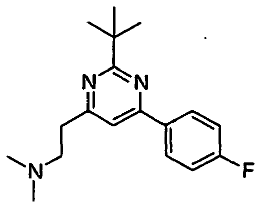

### Example 167; {2-[2-tert-Butyl-6-(4-fluoro-phenyl)-pyrimidin-4-yl]-ethyl}-dimethyl-amine. MS (ESI): exact mass calcd. for C₁₈H₂₄FN₃, 301.20; m/z found, 302.7 [M+H]⁺.

### Assay Methods

### In vitro pharmacology

Stock drug solutions (10 mM) were prepared in DMSO (the final assay concentration of DMSO not exceeding 0.4%). Drug dilutions were prepared in assay buffer.

Sigmoidal inhibition curves were generated and fitted by nonlinear regression analysis (GraphPad Prism). Kᵢ values were calculated according to the Cheng and Prussoff equation (Biochem. Pharmacol. 1973, 22, 3099-3108), IC₅₀/(1+[S]/K_{d}), where the following values were used: 5-HT₇ ([S] = 1 nM; K_{d} = 0.42); 5-HT_{2A} ([S] = 1 nM; K_{d} = 0.4 nM); 5-HT_{2B} ([S] = 4 nM; K_{d} = 3.5 nM); 5-HT_{2C} ([S] = 3 nM; K_{d} = 3 nM); 5-HT₆ ([S] = 1.7 nM; K_{d} = 1.7 nM).

Data obtained for compounds tested in Assays 1-4 are presented in Table 1 below. -

### 1. Affinity for 5-HT₇ receptor binding sites

The affinity of the compounds described in this invention for the 5-HT₇ receptor binding site was evaluated by single competition radioligand binding assay. The assay was performed on membranes prepared from HEK-293 cells that had been subjected to stable transfection with the rat 5-HT₇ₐ receptor (GB: NM022938). Cells were scraped from the culture plates, suspended in Tris-HCI 50 mM pH 7.5 and collected through centrifugation (1000 rpm for 5 min). The cell pellets were homogenized (Polytron, 15 s, setting 5) in 50 mM Tris-HCI (pH 7.5), 5 mM EDTA. Following centrifugation (15,000 rpm for 25 min), membranes (135 µg protein/mL) were resuspended in the same buffer and incubated for 60 min at RT with 1 nM [³H]5-CT in the presence of increasing concentration of test compounds. Nonspecific binding was defined in the presence of 10 µM 5-HT. Incubation was stopped by rapid filtration using the cell harvester (Packard). Radioactivity was counted in a TopCount-NXT (Packard). Experiments were conducted in triplicate.

### 2. Affinity for 5-HT_{2A} receptor binding sites

The affinity of the compounds for the rat 5-HT_{2A} receptor was evaluated by competitive radioligand binding assay using [³H]ketanserine as the radioligand. The assay was performed on membranes from rat cortex (Schotte, A. et al. Psychopharmacology 1996, 124, 57-73). Brain tissue (rat cortex) was homogenized in 20 volumes per wet weight tissue of Tris-HCI buffer (50 mM, pH 7.4). The total membrane fraction was collected by centrifugation and washed by subsequent centrifugation runs (25 min at 25,000 g at 4 °C). Membranes were re-suspended in Tris-HCI buffer (50 mM, pH 7.4) containing 1 nM [³H]ketanserin. Non-specific binding was estimated in the presence of 10 µM risperidone. The incubation was terminated by rapid filtration over Whatman GF/B filters pre-soaked in 0.1% polyethylenimine, and one washing step with 1 mL ice-cold Tris-HCI buffer, pH 7.4.

### 3. Affinity for 5HT₂ receptor binding sites

Receptor binding was performed using the human recombinant 5-HT_{2A} (GB: X57830), 5-HT_{2B} (GB: Z36748) and 5-HT_{2C} (GB: M81778) receptors. The affinity of the compounds for the 3 different human 5-HT₂ receptor subtypes was evaluated by competitive radioligand binding assays using [³H]ketanserin (h5-HT_{2A}) or [³H]mesulergine (h5-HT_{2B} and h5-HT_{2C}). The assays were performed on membranes prepared from NIH3T3 stably transfected with h5-HT_{2A} or CHO stably transfected with h5-HT_{2B} and h5-HT_{2C}.

### 4. Affinity for 5-HT₆ receptor binding sites

Receptor binding was performed using the human recombinant 5-HT₆ (GB: BC0794995) receptor. The affinity of the compounds for the human 5-HT₆ receptor was evaluated by competitive radioligand binding assays using [³H]LSD. The assays were performed on membranes prepared from HEK-293 stably transfected with h5-HT₆. Non-specific binding was estimated in the presence of 1 µM clozapine.

**Table 1. Binding Affinities (nM)**

| **Ex.** | **Kᵢ 5-HT₇** | **Kᵢ 5-HT2_{A}** | **Kᵢ 5-HT_{2B}** | **Kᵢ 5-HT_{2C}** | **Kᵢ 5-HT₆** |
|---|---|---|---|---|---|
| **1** | 540 | 4 | 70 | 90 | NT |
| **2** | >10000 | 20 | 5000 | 510 | NT |
| **3** | >10000 | 11 | 2250 | 1020 | >10000 |
| **4** | >10000 | 20 | 250 | 200 | NT |
| **5** | >10000 | 13 | 350 | 250 | NT |
| **6** | >10000 | 50 | 200 | 300 | NT |
| **7** | >10000 | 25 | 900 | 900 | NT |
| **8** | 1000 | 20 | 1000 | 320 | 200 |
| **9** | 5000 | 1350 | >10000 | 7500 | NT |
| **10** | >10000 | 200 | >10000 | 5000 | NT |
| **11** | >10000 | 100 | >10000 | 2000 | NT |
| **12** | >10000 | 300 | >10000 | 5000 | NT |
| **13** | >10000 | 12.5 | 2200 | 1000 | NT |
| **14** | >10000 | 30 | 4000 | 4000 | NT |
| **15** | >10000 | 1000 | >10000 | >10000 | NT |
| **16** | >10000 | 2000 | >10000 | >10000 | NT |
| **17** | >10000 | 4 | 250 | 70 | NT |
| **18** | 525 | 10 | 80 | 60 | 330 |
| **19** | >10000 | 100 | 140 | 200 | NT |
| **20** | >10000 | 700 | 1200 | >10000 | NT |
| **21** | >10000 | 25 | 1185 | 1170 | >10000 |
| **22** | >10000 | 32 | 500 | 1300 | NT |
| **23** | >10000 | 500 | >10000 | >10000 | NT |
| **24** | >10000 | 240 | >10000 | >10000 | NT |
| **25** | >10000 | 300 | >10000 | >10000 | NT |
| **26** | >10000 | 300 | >10000 | >10000 | NT |
| **27** | >10000 | 60 | >10000 | >10000 | NT |
| **28** | >10000 | 100 | 400 | 3000 | NT |
| **29** | >10000 | 240 | 1600 | 2000 | >10000 |
| **30** | >10000 | 800 | 1000 | 2500 | NT |
| **31** | >10000 | 70 | 2000 | >10000 | NT |
| **32** | >10000 | 2000 | >10000 | >10000 | NT |
| **33** | >10000 | >10000 | >10000 | >10000 | NT |
| **34** | >10000 | 150 | 400 | 1000 | NT |
| **35** | 500 | 70 | 400 | 2000 | NT |
| **36** | >10000 | >10000 | >10000 | >10000 | NT |
| **37** | >10000 | 40 | 400 | 1000 | NT |
| **38** | >10000 | 40 | 50 | 800 | NT |
| **39** | >10000 | 150 | >10000 | >10000 | NT |
| **40** | >10000 | 500 | >10000 | >10000 | NT |
| **41** | >10000 | 2000 | 5000 | 5000 | NT |
| **42** | >10000 | 120 | >10000 | >10000 | NT |
| **43** | >10000 | 200 | >10000 | >10000 | NT |
| **44** | >10000 | 400 | 500 | 1000 | NT |
| **45** | >10000 | >10000 | >10000 | >10000 | NT |
| **46** | 120 | 14 | 30 | 200 | 100 |
| **47** | 30 | 2.5 | 1 | 22 | 12 |
| **48** | 40 | 20 | 3 | 100 | 24 |
| **49** | 300 | 50 | 25 | 400 | 200 |
| **50** | 240 | 3.7 | 20 | 80 | 260 |
| **51** | >10000 | 470 | 140 | 9000 | NT |
| **52** | >10000 | 725 | 1150 | 9000 | NT |
| **53** | >10000 | 1000 | >10000 | >10000 | NT |
| **54** | 5000 | 0.55 | 30 | 20 | 30 |
| **55** | 35 | 0.42 | 2.5 | 16 | 8.5 |
| **56** | 180 | 0.80 | 22 | 38 | 30 |
| **57** | 550 | 0.70 | 40 | 50 | 80 |
| **58** | >10000 | 2 | 40 | 30 | 100 |
| **59** | 100 | 1.5 | 7.8 | 29 | 20 |
| **60** | 400 | 6 | 10 | 60 | 60 |
| **61** | 250 | 1.9 | 15 | 35 | 20 |
| **62** | 5275 | 0.75 | 21 | 21 | 27 |
| **63** | >10000 | 1.5 | 52 | 46 | 60 |
| **64** | >10000 | 10 | 1300 | 70 | 1300 |
| **65** | >10000 | 20 | 300 | 570 | >10000 |
| **66** | 300 | 8 | 30 | 145 | NT |
| **67** | 5400 | 56 | 80 | 495 | NT |
| **68** | >10000 | 80 | 660 | 7100 | NT |
| **69** | >10000 | 140 | >10000 | 650 | 7100 |
| **70** | >10000 | 117 | >10000 | 3733 | 250 |
| **71** | >10000 | 1027 | 4333 | 3583 | 300 |
| **72** | >10000 | 130 | >10000 | >10000 | NT |
| **73** | >10000 | 140 | >10000 | >10000 | NT |
| **74** | >10000 | 47 | 1900 | >10000 | NT |
| **75** | 5000 | 70 | 3000 | >10000 | NT |
| **76** | 150 | 9 | 315 | 75 | 300 |
| **77** | 22 | 15 | 200 | 130 | 400 |
| **78** | 400 | 25 | 900 | 380 | NT |
| **79** | 300 | 115 | 435 | 900 | NT |
| **80** | >10000 | 11 | 158 | 940 | 2800 |
| **81** | >10000 | 25 | 450 | 6000 | NT |
| **82** | >10000 | 2 | 500 | 20 | 230 |
| **83** | >10000 | 30 | 1000 | 300 | 230 |
| **84** | >10000 | 14 | 400 | 5000 | NT |
| **85** | >10000 | 82 | 1150 | >10000 | NT |
| **86** | 9000 | 100 | 4100 | >10000 | NT |
| **87** | >10000 | 600 | 2000 | >10000 | NT |
| **88** | 550 | 13 | 60 | 185 | NT |
| **89** | >10000 | 25 | 40 | 1400 | NT |
| **90** | 2300 | 17 | 130 | 1000 | NT |
| **91** | >10000 | 75 | 200 | 9000 | NT |
| **92** | >10000 | 300 | >10000 | >10000 | NT |
| **93** | >10000 | >10000 | >10000 | >10000 | NT |
| **94** | >10000 | >10000 | >10000 | >10000 | NT |
| **95** | >10000 | 2000 | >10000 | >10000 | NT |
| **96** | >10000 | >10000 | >10000 | >10000 | NT |
| **97** | >10000 | >10000 | >10000 | >10000 | NT |
| **98** | >10000 | >10000 | >10000 | >10000 | NT |
| **99** | >10000 | >10000 | >10000 | >10000 | NT |
| **100** | >10000 | 14 | 900 | 300 | NT |
| **101** | >10000 | 400 | >10000 | >10000 | NT |
| **102** | >10000 | 20 | 150 | 230 | 370 |
| **103** | >10000 | 40 | 1000 | 1000 | NT |
| **104** | >10000 | 700 | 500 | 3000 | NT |
| **105** | >10000 | 40 | 100 | 400 | NT |
| **106** | >10000 | 90 | 6300 | >10000 | NT |
| **107** | >10000 | 12 | 275 | 500 | 4000 |
| **108** | >10000 | 18 | 42 | 1000 | NT |
| **109** | >10000 | 400 | >10000 | >10000 | >10000 |
| **110** | >10000 | 220 | 1200 | 4000 | NT |
| **111** | >10000 | 300 | 3500 | >10000 | NT |
| **112** | >10000 | 130 | >10000 | >10000 | NT |
| **113** | >10000 | 650 | >10000 | >10000 | NT |
| **114** | >10000 | 35 | 300 | 1000 | NT |
| **115** | 5000 | 1000 | 5000 | >10000 | NT |
| **116** | >10000 | 2000 | >10000 | >10000 | NT |
| **117** | >10000 | 50 | 1000 | 4000 | NT |
| **166** | NT | 11 | 18 | 170 | NT |
| **167** | 450 | 7 | 14 | 200 | NT |

| | | | | | |
|---|---|---|---|---|---|
| NT = not tested | | | | | |

### 5. In Vitro Functional Assay for 5-HTg Receptor (Intracellular Calcium)

In vitro functional properties of these compounds on the different 5-HT₂ receptor subtypes were determined using fluorometric imaging plate reader (FLIPR) based calcium assay as previously described (Porter, R.H. et al. Br. J. Pharmacol. 1999, 128, 13-20; Jerman, J.C. et al. Eur. J. Pharmacol. 2001, 414, 23-30). The 5-HT₂ receptors are linked to the Gq family of G proteins and to subsequent activation of phospholipase C, induction of phosphoinositide metabolism and to an increase in intracellular calcium concentration. The same cell lines as described in the previous section (receptor binding) were used for the FLIPR experiments. Data obtained for compounds tested are presented in Table 2.

**Table 2.**

| **Ex.** | **pK_{b} 5-HT_{2A}** | **pK_{b} 5-HT_{2B}** | **pK_{b} 5-HT_{2C}** |
|---|---|---|---|
| **13** | 7.1 | 5 | NT |
| **17** | 8.1 | 5.4 | NT |
| **18** | 7.6 | 6.7 | NT |
| **21** | 7.5 | 5 | 5 |
| **50** | 8.3 | 8.3 | NT |
| **57** | 8.2 | 6.9 | NT |
| **62** | 8.8 | 7.3 | NT |
| **63** | 7.8 | 6.3 | NT |
| **64** | 7.1 | 5 | NT |
| **69** | 6 | 5 | NT |
| **76** | 7 | 5 | NT |
| **78** | 7.1 | 5 | NT |
| **82** | 8.1 | 5.4 | NT |
| **100** | 7 | 5 | NT |

| | | | |
|---|---|---|---|
| NT = not tested | | | |

## Claims

1. A compound of Formulae (I) or (II): 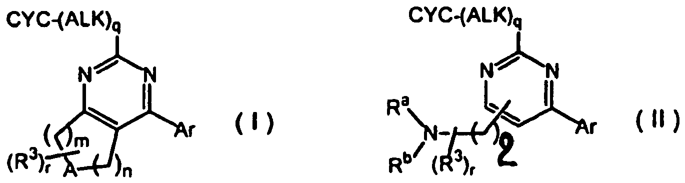 wherein
m is 1, 2, or 3;
n is 1, 2, or 3;
where m and n are present, m + n is greater than or equal to 2, and is less than or equal to 4;
R^{a} and R^{b} are independently -H, -C₁₋₇alkyl, or -C₃₋₇cycloalkyl, or R^{a} and R^{b} taken together with the nitrogen of attachment form piperidinyl, pyrrolidinyl, morpholinyl, thiomorpholinyl, or piperazinyl, where each R^{a} and R^{b} is optionally and independently substituted with -C₁₋₄alkyl;
q is 0 or 1;
A is >NR¹, >CHNR^{c}R^{d}, >CHOH, or -CH₂-, wherein
R¹ is selected from the group consisting of -H, -C₁₋₇alkyl, -C₃₋₇cycloalkyl, and benzyl, where each alkyl, cycloalkyl, or benzyl is optionally mono-, di-, or tri-substituted with R^{e};
R^{e} is selected from the group consisting of -C₁₋₄alkyl, -C₂₋₄alkenyl, -C₂₋₄alkynyl, -C₃₋₆cycloalkyl, halo, -CF₃, -OH, -OC₁₋₄alkyl, -OCF₃, -N(R^{f})R^{g} (wherein R^{f} and R⁹ are independently -H or -C₁₋₄alkyl, or R^{f} and R^{g} taken together with the nitrogen of attachment form piperidinyl, pyrrolidinyl, morpholinyl, thiomorpholinyl, or piperazinyl), -C(O)N(R^{f})R^{g}, -N(R^{h})C(O)R^{h}, -N(R^{h})SO₂C₁₋₇alkyl (wherein R^{h} is -H or -C₁₋₄alkyl, or two R^{h} in the same substituent taken together with the amide of attachment form an otherwise aliphatic 4- to 6-membered ring), -S(O)₀₋₂-C₁₋₄alkyl, -SO₂N(R^{f})R^{g}, -SCF₃, -C(O)C₁₋₄alkyl, -CN, -COOH, and -COOC₁₋₄alkyl;
R^{c} and R^{d} are independently selected from the group consisting of -H, -C₁₋₇alkyl, -C₃₋₇alkenyl, -C₃₋₇alkynyl, -C₃₋₇cycloalkyl, -C₁₋₇alkylC₃₋₇cycloalkyl, and -C₃₋₇cycloalkylC₁₋₇alkyl, or R^{c} and R^{d} taken together with the nitrogen of attachment form piperidinyl, pyrrolidinyl, morpholinyl, thiomorpholinyl, or piperazinyl, where each R^{c} and R^{d} is optionally and independently substituted with R^{e};
R³ is -C₁₋₄alkyl, -C₁₋₄alkenyl, or benzyl, each optionally substituted with -C₁₋₃alkyl, -OH, or halo, or two R³ substituents taken together form C₂₋₅alkylene optionally substituted with -C₁₋₃alkyl, -OH, or halo;
r is 0 or is an integer less than or equal to m + n + 1;
Ar is an aryl or heteroaryl ring selected from the group consisting of:
a) phenyl, optionally mono-, di-, or tri-substituted with Rⁱ or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
Rⁱ is selected from the group consisting of -C₁₋₇alkyl, -C₂₋₇alkenyl, -C₂₋₇alkynyl, -C₃₋₇cycloalkyl, halo, -CF₃, -OH, -OC₁₋₇alkyl, -OCF₃, -OC₃₋₇alkenyl, -OC₃₋₇alkynyl, -N(R^{j})R^{k} (wherein R^{j} and R^{k} are independently -H or - C₁₋₄alkyl), -C(O)N(R^{j}R^{k}, -N(R^{j})C(O)R^{k}, -N(R^{j})SO₂C₁₋₆alkyl, -S(O)₀₋₂-C₁₋₆alkyl, -SO₂N(R^{j})R^{k}, -SCF₃, -C(O)C₁₋₆alkyl, -NO₂, -CN, -COOH, and -COOC₁₋₇alkyl;
b) a monocyclic aromatic hydrocarbon group having five ring atoms, having a carbon atom which is the point of attachment, having one carbon atom replaced by >O, >S, >NH, or >N(C₁₋₄alkyl), having up to two additional carbon atoms optionally replaced by -N=, optionally mono- or di-substituted with Rⁱ;
c) a monocyclic aromatic hydrocarbon group having six ring atoms, having a carbon atom which is the point of attachment, having one or two carbon atoms replaced by -N=, optionally mono- or di-substituted with Rⁱ; and
d) phenyl or pyridyl, substituted with a substituent selected from the group consisting of phenyl, phenoxy, pyridyl, thiophenyl, oxazolyl, and tetrazolyl, where the resultant substituted moiety is optionally further mono-, di-, or tri-substituted with R';
ALK is a branched or unbranched C₁₋₇alkylene, C₂₋₇alkenylene, C₂₋₇alkynylene, C₃₋₇cycloalkylene, or C₃₋₇cycloalkenylene, optionally mono-, di-, or tri-substituted with R^{m};
R^{m} is selected from the group consisting of halo, -CF₃, -OH, -OC₁₋₇alkyl, -OC₃₋₇cycloalkyl, -OCF₃, -N(R^{p})R^{s} (wherein R^{p} and R^{s} are independently -H or -C₁₋₇alkyl), -C(O)N(R^{p})R^{s}, -N(R')C(O)R', -N(R¹)SO₂C₁₋₆alkyl (wherein R^{t} is -H or - C₁₋₇alkyl), -S(O)₀₋₂-C₁₋₆alkyl, -SO₂N(R^{p})R^{s}, -SCF₃, -CN, -NO₂, -C(O)C₁₋₇alkyl, -COOH, and -COOC₁₋₇alkyl;
CYC is -H or is a ring system selected from the group consisting of:
i) phenyl, optionally mono-, di-, or tri-substituted with R^{u} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
R^{u} is selected from the group consisting of -C₁₋₇alkyl, -C₃₋₇cycloalkyl, phenyl, benzyl, halo, -CF₃, -OH, -OC₁₋₇alkyl, -OC₃₋₇cycloalkyl, -Ophenyl, -Obenzyl, -OCF₃, -N(R^{v})R^{w} (wherein R^{v} and R^{w} are independently -H or -C₁₋₇alkyl, or R^{v} and R^{w} taken together with the nitrogen of attachment form piperidinyl, pyrrolidinyl, morpholinyl, thiomorpholinyl, or piperazinyl, where each R^{v} and R^{w} is optionally and independently substituted with -OH or -C₁₋₇alkyl), -C(O)N(R^{v})R^{w}, -N(R^{x})C(O)R^{x}, -N(R^{x})SO₂C₁₋₆alkyl (wherein R^{x} is -H or -C₁₋₇alkyl, or two R^{x} in the same substituent taken together with the amide of attachment form an otherwise aliphatic 4- to 6-membered ring), -N-(SO₂C₁₋₆alkyl)₂, -S(O)₀₋₂-C₁₋₆alkyl, -SO₂N(R^{v})R^{w}, -SCF₃, -C(O)C₁₋₆alkyl, -NO₂, -CN, -COOH, and -COOC₁₋₇alkyl;
ii) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atoms optionally replaced by -N=, optionally mono- or di-substituted with R^{u};
iii) a monocyclic aromatic hydrocarbon group having six ring atoms, having a carbon atom which is the point of attachment, having one or two carbon atoms replaced by -N=, optionally mono- or di-substituted with R^{u}; and
iv) a non-aromatic heterocyclic ring having 4 to 8 members, said ring having 0, 1, or 2 non-adjacent heteroatom members selected from the group consisting of O, S, -N=, >NH, and >N(C₁₋₄alkyl), having 0, 1, or 2 double bonds, having 0, 1, or 2 carbon members which is a carbonyl, optionally having one carbon member which forms a bridge, having 0 to 5 substituents R^{u}, and where when q is 0, said ring has a carbon atom which is the point of attachment;
and enantiomers, diastereomers, hydrates, solvates and pharmaceutically acceptable salts, esters and amides thereof;
with the proviso that in Formula (I):
(a) when ALK is methylene, ethylene, propylene, or isopropylene, CYC is -H, Ar is phenyl or mono-substituted phenyl, m is 2, n is 1, and A is >NR¹, then R¹ is not -C₁₋₄alkyl or benzyl;
(b) when q is 0, CYC is phenyl, Ar is phenyl or 3-chlorophenyl, m is 2, and n is 1, then A is not unsubstituted -CH₂-; and
(c) when q is 0, CYC is 2-pyridyl, Ar is 2-pyridyl, m is 2, and n is 1, then A is not unsubstituted -CH₂-;wherein said compound is not: 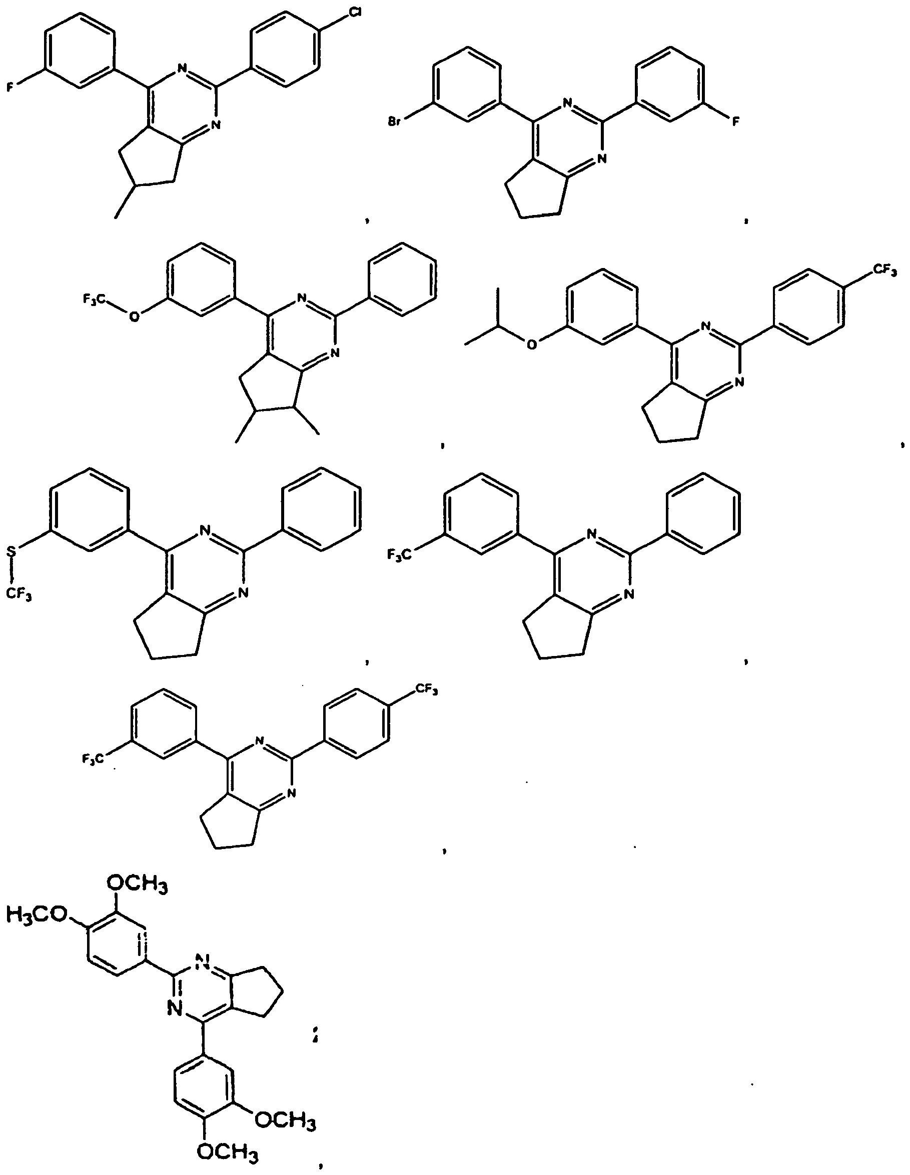 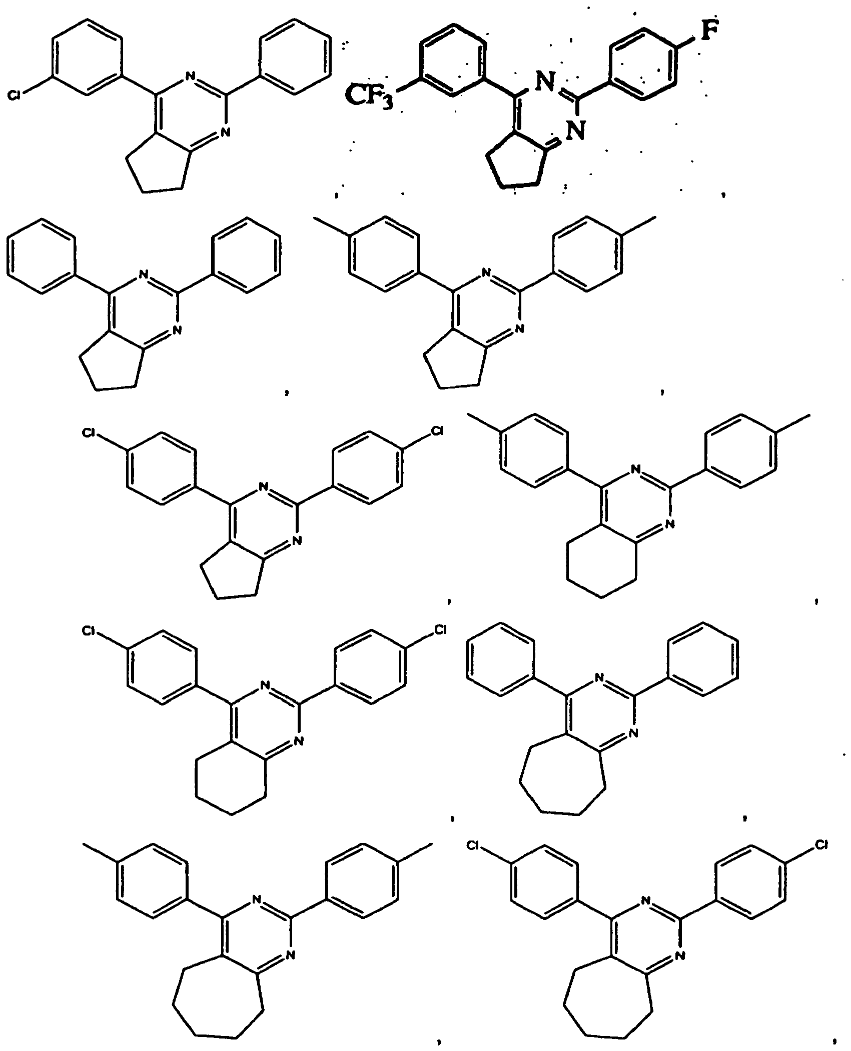 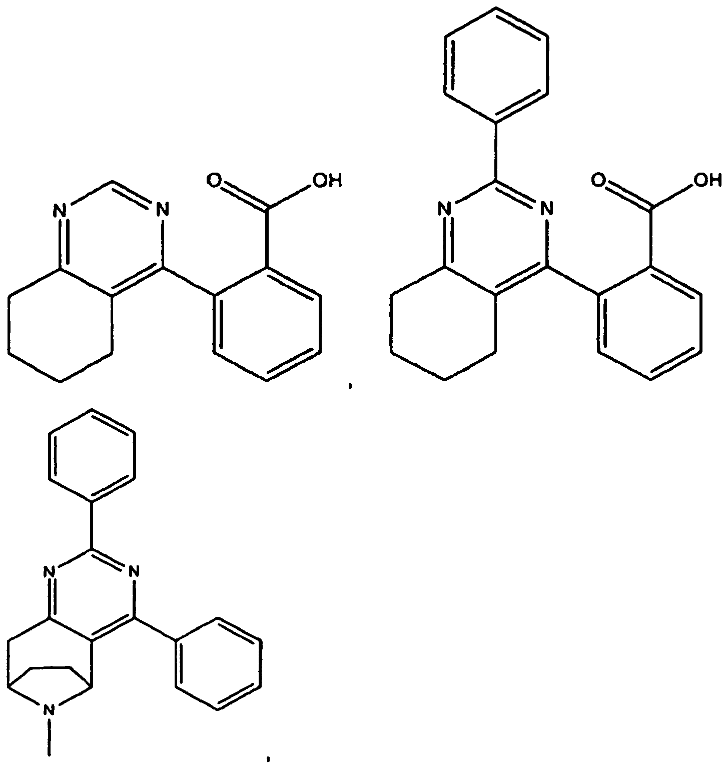
7-benzyl-2-methyl-4-phenyl-5,8-dihydro-6H-pyrido[3,4-d]pyrimidine.
2-methyl-4-phenyl-5,8-dihydro-6H-pyrido[3,4-d]pyrimidine, or
4-(2-Methyl-4-nitro-phenyl)-6,7-dihydro-5H-cyclopentapyrimidine.

2. The compound of claim 1 wherein m is 1 and n is 1.

3. The compound of claim 1 wherein m is 1 and n is 2.

4. The compound of claim 1 wherein m is 2 and n is 1.

5. The compound of claim 1 wherein m is 2 and n is 2.

6. The compound of claim 1 wherein m is 1 and n is 3.

7. The compound of claim 1 wherein m is 3 and n is 1.

8. The compound of claim 1 wherein q is 1.

9. The compound of claim 1 wherein -N(R^{a})R^{b} is amino, methylamino, ethylamino, isopropylamino, dimethylamino, diethylamino, diisopropylamino, cyclopropylamino, cyclopentylamino, piperidinyl, pyrrolidinyl, morpholinyl, thiomorpholinyl, or piperazinyl.

10. The compound of claim 1 wherein -N(R^{a})R^{b} is amino, methylamino, dimethylamino, or N-methylpiperazinyl.

11. The compound of claim 1 wherein A is >NR¹.

12. The compound of claim 1 wherein R¹ is selected from the group consisting of hydrogen, methyl, ethyl, isopropyl, butyl, hexyl, cyclopropyl, cyclobutyl, cyclopentyl, and benzyl, each optionally mono-, di-, or tri-substituted with R^{e}.

13. The compound of claim 1 wherein R¹, optionally R^{e} substituted, is selected from the group consisting of hydrogen, methyl, ethyl, isopropyl, and benzyl.

14. The compound of claim 1 wherein R¹ is hydrogen or methyl.

15. The compound of claim 1 wherein R³, optionally substituted, is selected from the group consisting of methyl, ethyl, propyl, isopropyl, butyl, methylene, allyl, and benzyl.

16. The compound of claim 1 wherein two R³ substituents taken together form ethylene.

17. The compound of claim 1 wherein R³ is methyl.

18. The compound of claim 1 wherein r is 0, 1, or 2.

19. The compound of claim 1 wherein Ar, optionally substituted, is selected from the group consisting of:
a) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
b) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1,2,4-triazolyl,
c) pyridinyl, pyridinyl-N-oxide, pyrazinyl, pyrimidinyl, pyridazinyl, and
d) biphenyl, and 4-tetrazolylphenyl.

20. The compound of claim 1 wherein Ar, optionally substituted, is selected from the group consisting of phenyl, pyridyl, thiophen-2-yl, and thiophen-3-yl.

21. The compound of claim 1 wherein Ar is selected from the group consisting of phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 3-nitrophenyl, 4-nitrophenyl, 3-chloro-4-fluorophenyl, 3-fluoro-4-chlorophenyl, benzo[1,3]dioxol-4 or 5-yl, 3-hydroxyphenyl, 4-hydroxyphenyl, 4-hydroxy-2-methylphenyl, 4-hydroxy-3-fluorophenyl, 3,4-dihydroxyphenyl,4-dimethylaminophenyl, 4-carbamoylphenyl, 4-fluoro-3-methylphenyl, 2-phenoxyphenyl, furan-2-yl, furan-3-yl, 5-methyl-furan-2-yl, thiophen-2-yl, thiophen-3-yl, 5-chlorothiophen-2-yl, 5-methylthiophen-2-yl, 5-chlorothiophen-3-yl, 5-methylthiophen-3-yl, oxazol-2-yl, 4,5-dimethyl-oxazol-2-yl, thiazol-2-yl, 3H-[1,2,3]triazol-4-yl, 2H-pyrazol-3-yl, 1H-pyrazol-4-yl, 4-pyridyl, 5-fluoropyridin-2-yl, 4'-chlorobiphenyl, and 4-tetrazolylphenyl.

22. The compound of claim 1 wherein ALK, optionally substituted, is selected from the group consisting of methylene, ethylene, propylene, butylene, sec-butylene, tert-butylene, pentylene, 1-ethylpropylene, 2-ethylpropylene, 2-ethylbutylene, isopropylene, but-3-enylene, isobutylene, 3-methylbutylene, allylene, prop-2-ynylene, cyclopropylene, cyclobutylene, cyclopentylene, cyclohexylene, and cycloheptylene.

23. The compound of claim 1 wherein ALK is selected from the group consisting of methylene, hydroxymethylene, fluoromethylene, difluoromethylene, trifluoromethylmethylene, 2,2,2-trifluoro-1-trifluoromethyl-ethylene, methoxycarbonylmethyl, methylcarbamoylmethyl, ethylene, 2-dimethylaminoethylene, 2-cyanoethylene, 2-methoxyethylene, 1-carboxy-ethylene, propylene, 3-methoxycarbonyl propylene, 3-carboxy propylene, isopropylene, 1-fluoro-1-methylethylene, 1-hydroxy-1-methyl-ethylene, 1-carboxy-1-methyl-ethylene, 1-ethylpropylene, 2-ethylpropylene, butylene, tert-butylene, sec-butylene, isobutylene, 4-hydroxybutylene, 4-methoxycarbonyl butylene, 4-carboxy butylene, 2-ethylbutylene, isobutylene, 3-methylbutylene, prop-2-ynylene, but-3-enylene, pentylene, 5-hydroxypentylene, cyclopropylene, cyclobutylene, cyclopentylene, cyclopentenylene, 3,3-difluoro-cyclopentylene, 3-hydroxy-cyclohexylene, 4-fluorocyclohexylene, 4,4-difluoro-cyclohexylene, and 1-methyl-cyclopropylene.

24. The compound of claim 1 wherein CYC, optionally substituted, is hydrogen or is a ring system selected from the group consisting of:
i) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
ii) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1,2,4-triazolyl,
iii) pyridinyl, pyridinyl-N-oxide, pyrazinyl, pyrimidinyl, pyridazinyl, and
iv) pyrrolinyl, pyrrolidinyl, pyrazolinyl, piperidinyl, homopiperidinyl, azepanyl, tetrahydrofuranyl, tetrahydropyranyl, piperazinyl, morpholinyl, thiomorpholinyl, and piperidinonyl.

25. The compound of claim 1 wherein CYC, optionally substituted, is selected from the group consisting of hydrogen, phenyl, thiophen-2-yl, thiophen-3-yl, furan-2-yl, furan-3-yl, pyridinyl, piperidin-1, 2, 3 or 4-yl, 2-pyrrolin-2, 3, 4, or 5-yl, 3-pyrrolin-2 or 3-yl, 2-pyrazolin-3, 4 or 5-yl, tetrahydrofuran-3-yl, tetrahydropyran-4-yl, morpholin-2, 3, or 4-yl, thiomorpholin-2, 3, or 4-yl, piperazin-1, 2, 3, or 4-yl, pyrrolidin-1, 2, or 3-yl, and homopiperidinyl.

26. The compound of claim 1 wherein CYC, optionally substituted, is selected from the group consisting of hydrogen, phenyl, pyridyl, thiophen-2-yl, thiophen-3-yl, tetrahydropyranyl, furan-2-yl, furan-3-yl, tetrahydrofuran-3-yl, and piperidinyl.

27. The compound of claim 1 wherein CYC is selected from the group consisting of hydrogen, phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 2-cyanophenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 2,6-difluorophenyl, 2,6-dichlorophenyl, 2,6-dimethylphenyl, 2,4,6-trifluorophenyl, 2,4,6-trichlorophenyl, 3,4,5-trimethoxyphenyl, 4-fluoro-3-methylphenyl, 3-nitrophenyl, 4-nitrophenyl, 4-methyl-3-fluorophenyl, 3,4-dimethylphenyl, 4-methoxy-3-fluorophenyl, 4-methoxy-2-methylphenyl, 3-aminophenyl, 4-aminophenyl, 4-carbomethoxyphenyl, 3-methanesulfonylamino-phenyl, 4-methanesulfonylamino-phenyl, 3-dimethanesulfonylamino-phenyl, 4-dimethanesulfonylamino-phenyl, thiophen-2-yl, thiophen-3-yl, 5-chlorothiophen-2-yl, benzo[1,3]dioxol-4 or 5-yl, tetrahydrofuran-3-yl, tetrahydropyran-2,3 or 4-yl, furan-2-yl, furan-3-yl, 5-carboxyethyl-furan-2-yl, piperidinyl, 3,4-bisbenzyloxyphenyl, 2-hydroxyphenyl, 3-hydroxyphenyl, 4-hydroxyphenyl, 4-hydroxy-2-methylphenyl, 4-hydroxy-3-fluorophenyl, 3,4-dihydroxyphenyl, 1-piperidinyl, 4-piperidinyl, and 1-methyl-4-piperidinyl.

28. A compound selected from the group consisting of:
2-tert-Butyl-4-(4-fluoro-phenyl)-5,6,7,8-tetrahydro-pyrido[4,3-d]pyrimidine;
2-Benzyl-4-(4-fluoro-phenyl)-5,6,7,8-tetrahydro-pyrido[4,3-d]pyrimidine;
2-sec-Butyl-4-(4-fluoro-phenyl)-5,6,7,8-tetrahydro-pyrido[4,3-d]pyrimidine hydrochloride;
2-sec-Butyl-4-p-tolyl-5,6,7,8-tetrahydro-pyrido[4,3-d]pyrimidine hydrochloride;
2-Cyclobutyl-4-(4-fluoro-phenyl)-5,6,7,8-tetrahydro-pyrido[4,3-d]pyrimidine hydrochloride;
2-Cyclobutyl-4-p-tolyl-5,6,7,8-tetrahydro-pyrido[4,3-d]pyrimidine;
2-Cyclopropyl-4-(4-fluoro-phenyl)-5,6,7,8-tetrahydro-pyrido[4,3-d]pyrimidine;
2-Benzyl-4-p-tolyl-5,6,7,8-tetrahydro-pyrido[4,3-d]pyrimidine;
2-Benzyl-4-(4-trifluoromethyl-phenyl)-5,6,7,8-tetrahydro-pyrido[4,3-d]pyrimidine;
2-Benzyl-4-(3,4-difluoro-phenyl)-5,6,7,8-tetrahydro-pyrido[4,3-d]pyrimidine;
2-Benzyl-4-phenyl-5,6,7,8-tetrahydro-pyrido[4,3-d]pyrimidine;
2-Benzyl-4-(3-fluoro-phenyl)-5,6,7,8-tetrahydro-pyrido[4,3-d]pyrimidine;
2-(4-Fluoro-benzyl)-4-(4-fluoro-phenyl)-5,6,7,8-tetrahydro-pyrido[4,3-d]pyrimidine;
2-(4-Fluoro-benzyl)-4-(4-fluoro-phenyl)-6-methyl-5,6,7,8-tetrahydro-pyrido[4,3-d]pyrimidine;
4-[2-(4-Fluoro-benzyl)-6-methyl-5,6,7,8-tetrahydro-pyrido[4,3-d]pyrimidin-4-yl]-benzonitrile;
4-[2-(4-Fluoro-benzyl)-5,6,7,8-tetrahydro-pyrido[4,3-d]pyrimidin-4-yl]-benzonitrile;
2-Cyclopentyl-4-(4-fluoro-phenyl)-5,6,7,8-tetrahydro-pyrido[4,3-d]pyrimidine;
2-Cyclopentyl-4-p-tolyl-5,6,7,8-tetrahydro-pyrido[4,3-d]pyrimidine;
2-Cyclopentyl-4-(4-methoxy-phenyl)-5,6,7,8-tetrahydro-pyrido[4,3-d]pyrimidine;
4-(2-Cyclopentyl-5,6,7,8-tetrahydro-pyrido[4,3-d]pyrimidin-4-yl)-benzonitrile;
4-(4-Fluoro-phenyl)-2-isopropyl-5,6,7,8-tetrahydro-pyrido[4,3-d]pyrimidine hydrochloride;
4-(4-Fluoro-phenyl)-2-isopropyl-6-methyl-5,6,7,8-tetrahydro-pyrido[4,3-d]pyrimidine;
4-(3,4-Dichloro-phenyl)-2-isopropyl-5,6,7,8-tetrahydro-pyrido[4,3-d]pyrimidine hydrochloride;
4-(3,4-Difluoro-phenyl)-2-isopropyl-5,6,7,8-tetrahydro-pyrido[4,3-d]pyrimidine hydrochloride;
4-(3-Fluoro-phenyl)-2-isopropyl-5,6,7,8-tetrahydro-pyrido[4,3-d]pyrimidine;
4-(2-Fluoro-phenyl)-2-isopropyl-5,6,7,8-tetrahydro-pyrido[4,3-d]pyrimidine;
4-(2,4-Difluoro-phenyl)-2-isopropyl-5,6,7,8-tetrahydro-pyrido[4,3-d]pyrimidine;
2-Isopropyl-4-p-tolyl-5,6,7,8-tetrahydro-pyrido[4,3-d]pyrimidine;
4-(4-Chloro-phenyl)-2-isopropyl-5,6,7,8-tetrahydro-pyrido[4,3-d]pyrimidine;
2-Isopropyl-4-(4-methoxy-phenyl)-5,6,7,8-tetrahydro-pyrido[4,3-d]pyrimidine;
2-Isopropyl-4-phenyl-5,6,7,8-tetrahydro-pyrido[4,3-d]pyrimidine;
2-Isopropyl-4-(4-trifluoromethyl-phenyl)-5,6,7,8-tetrahydro-pyrido[4,3-d]pyrimidine;
2-Isopropyl-4-(2-phenoxy-phenyl)-5,6,7,8-tetrahydro-pyrido[4,3-d]pyrimidine;
2-Isobutyl-4-thiophen-3-yl-5,6,7,8-tetrahydro-pyrido[4,3-d]pyrimidine;
2-Isobutyl-4-thiophen-2-yl-5,6,7,8-tetrahydro-pyrido[4,3-d]pyrimidine;
2-Isobutyl-4-pyridin-4-yl-5,6,7,8-tetrahydro-pyrido[4,3-d]pyrimidine;
4-(4-Fluoro-phenyl)-2-isobutyl-5,6,7,8-tetrahydro-pyrido[4,3-d]pyrimidine;
2-Isobutyl-4-p-tolyl-5,6,7,8-tetrahydro-pyrido[4,3-d]pyrimidine;
4-(4-Fluoro-3-methyt-phenyl)-2-isobutyl-5,6,7,8-tetrahydro-pyrido[4,3-d]pyrimidine;
4-(2-Isobutyl-5,6,7,8-tetrahydro-pyrido[4,3-d]pyrimidin-4-yl)-benzonitrile;
2-Isobutyl-4-(4-methoxy-phenyl)-5,6,7,8-tetrahydro-pyrido[4,3-d]pyrimidine;
2-sec-Butyl-4-(2-fluoro-phenyl)-5,6,7,8-tetrahydro-pyrido[4,3-d]pyrimidine hydrochloride;
2-sec-Butyl-4-(3-fluoro-phenyl)-5,6,7,8-tetrahydro-pyrido[4,3-d]pyrimidine;
2-sec-Butyl-4-(4-methoxy-phenyl)-5,6,7,8-tetrahydro-pyrido[4,3-d]pyrimidine;
2-sec-Butyl-4-(4-trifluoromethoxy-phenyl)-5,6,7,8-tetrahydro-pyrido[4,3-d]pyrimidine;
2-Cyclopentyl-4-(4-fluoro-phenyl)-6,7,8,9-tetrahydro-5H-pyrimido[4,5-d]azepine;
2-Cyclopentyl-4-p-tolyl-6,7,8,9-tetrahydro-5H-pyrimido[4,5-d]azepine;
2-Cyclopentyl-4-(4-methoxy-phenyl)-6,7,8,9-tetrahydro-5H-pyrimido[4,5-d]azepine;
4-(2-Cyclopentyl-6,7,8,9-tetrahydro-5H-pyrimido[4,5-d]azepin-4-yl)-benzonitrile;
4-(4-Fluoro-phenyl)-2-isopropyl-6,7,8,9-tetrahydro-5H-pyrimido[4,5-d]azepine hydrochloride;
4-(4-Chloro-phenyl)-2-methyl-6,7,8,9-tetrahydro-5H-pyrimido[4,5-d]azepine;
2-Methyl-4-phenyl-6,7,8,9-tetrahydro-5H-pyrimido[4,5-d]azepine;
4-(3-Chloro-phenyl)-2-methyl-6,7,8,9-tetrahydro-5H-pyrimido[4,5-d]azepine;
2-Benzyl-4-(4-fluoro-phenyl)-6,7,8,9-tetrahydro-5H-pyrimido[4,5-d]azepine;
2-Benzyl-4-p-tolyl-6,7,8,9-tetrahydro-5H-pyrimido[4,5-d]azepine;
2-Benzyl-4-(4-trifluoromethyl-phenyl)-6,7,8,9-tetrahydro-5H-pyrimido[4,5-d]azepine;
2-(4-Fluoro-benzyl)-4-(4-fluoro-phenyl)-6,7,8,9-tetrahydro-5H-pyrimido[4,5-d]azepine;
2-Cyclopentyl-4-(4-fluoro-phenyl)-7-methyl-6,7,8,9-tetrahydro-5H-pyrirnido[4,5-d]azepine;
2-Cyclopentyl-7-methyl-4-p-tolyl-6,7,8,9-tetrahydro-5H-pyrimido[4,5-d]azepine;
2-Cyclopentyl-4-(4-methoxy-phenyl)-7-methyl-6,7,8,9-tetrahydro-5H-pyrimido[4,5-d]azepine;
2-Benzyl-7-methyl-4-p-tolyl-6,7,8,9-tetrahydro-5H-pyrimido[4,5-d]azepine;
2-(4-Fluoro-benzyl)-4-(4-fluoro-phenyl)-7-methyl-6,7,8,9-tetrahydro-5H-pyrimido[4,5-d]azepine;
2-(4-Fluoro-benzyl)-4-(4-fluoro-phenyl)-7-methyl-9-methylene-6,7,8,9-tetrahydro-5H-pyrimido[4,5-d]azepine;
2-Benzyl-4-(4-fluoro-phenyl)-6,7,8,9-tetrahydro-5H-pyrimido[4,5-c]azepine hydrochloride;
4-(4-Fluoro-phenyl)-2.isopropyl-6,7,8,9-tetrahydro-5H-pyrimido[4,5-c]azepine hydrochloride;
2-Isopropyl-4-p-tolyl-6,7,8,9-tetrahydro-5H-pyrimido[4,5-c]azepine hydrochloride;
2-Isopropyl-4-(4-methoxy-phenyl)-6,7,8,9-tetrahydro-5H-pyrimido[4,5-c]azepine;
2-Isopropyl-4-phenyl-6,7,8,9-tetrahydro-5H-pyrimido[4,5-c]azepine;
2-Benzyl-4-(4-fluoro-phenyl)-6,7,8,9-tetrahydro-5H-1,3,6-triaza-benzocycloheptene hydrochloride;
2,7-Dibenzyl-4-(4-fluoro-phenyl)-5,6,7,8-tetrahydro-pyrido[3,4-d]pyrimidine hydrochloride;
2,7-Dibenzyl-4-p-tolyl-5,6,7,8-tetrahydro-pyrido[3,4-d]pyrimidine;
2,7-Dibenzyl-4-phenyl-5,6,7,8-tetrahydro-pyrido[3,4-d]pyrimidine;
2,7-Dibenzyl-4-(4-methoxy-phenyl)-5,6,7,8-tetrahydro-pyrido[3,4-d]pyrimidine;
7-Benzyl-4-(4-fluoro-phenyl)-2-isopropyl-5,6,7,8-tetrahydro-pyrido[3,4-d]pyrimidine;
7-Benzyl-2-isopropyl-4-phenyl-5,6,7,8-tetrahydro-pyrido[3,4-d]pyrimidine;
2-Benzyl-4-(4-fluoro-phenyl)-5,6,7,8-tetrahydro-pyrido[3,4-d]pyrimidine hydrochloride;
2-Benzyl-4-p-tolyl-5,6,7,8-tetrahydro-pyrido[3,4-d]pyrimidine hydrochloride;
2-Benzyl-4-phenyl-5,6,7,8-tetrahydro-pyrido[3,4-d]pyrimidine;
2-Benzyl-4-(4-methoxy-phenyl)-5,6,7,8-tetrahydro-pyrido[3,4-d]pyrimidine;
4-(4-Fluoro-phenyl)-2-isopropyl-5,6,7,8-tetrahydro-pyrido[3,4-d]pyrimidine hydrochloride;
2-lsopropyl-4-phenyl-5,6,7,8-tetrahydro-pyrido[3,4-d]pyrimidine;
2-Benzyl-4-(4-fluoro-phenyl)-7-methyl-5,6,7,8-tetrahydro-pyrido[3,4-d]pyrimidine hydrochloride;
2-Benzyl-4-(4-fluoro-phenyl)-7-isopropyl-5,6,7,8-tetrahydro-pyrido[3,4-d]pyrimidine;
4-(4-Fluoro-phenyl)-2-isopropyl-7-methyl-5,6,7,8-tetrahydro-pyrido[3,4-d]pyrimidine hydrochloride;
2-Isopropyl-7-methyl-4-phenyl-5,6,7,8-tetrahydro-pyrido[3,4-d]pyrimidine;
7-Benzyl-2-isopropyl-4-(5-methyl-thiophen-3-yl)-5,6,7,8-tetrahydro-pyrido[3,4-d]pyrimidine hydrochloride;
7-Benzyl-2-isopropyl-4-thiophen-3-yl-5,6,7,8-tetrahydro-pyrido[3,4-d]pyrimidine hydrochloride;
2-Isopropyl-4-(5-methyl-thiophen-3-yl)-5,6,7,8-tetrahydro-pyrido[3,4-d]pyrimidine hydrochloride;
2-Isopropyl-4-thiophen-3-yl-5,6,7,8-tetrahydro-pyrido[3,4-d]pyrimidine hydrochloride;
2-Isopropyl-7-methyl-4-(5-methyl-thiophen-3-yl)-5,6,7,8-tetrahydro-pyrido[3,4-d]pyrimidine hydrochloride;
2-Isopropyl-7-methyl-4-thiophen-3-yl-5,6,7,8-tetrahydro-pyrido[3,4-d]pyrimidine hydrochloride;
6-Benzyl-4-(4-fluoro-phenyl)-2-isopropyl-8-methyl-5,6,7,8-tetrahydro-pyrido[4,3-d]pyrimidine;
6-Benzyl-4-(3-chloro-4-fluoro-phenyl)-2-isopropyl-8-methyl-5,6,7,8-tetrahydro-pyrido[4,3-d]pyrimidine;
6-Benzyl-2-isopropyl-8-methyl-4-p-tolyl-5,6,7,8-tetrahydro-pyrido[4,3-d]pyrimidine;
6-Benzyl-2-isopropyl-8-methyl-4-phenyl-5,6,7,8-tetrahydro-pyrido[4,3-d]pyrimidine;
6-Benzyl-2-isopropyl-8-methyl-4-(4-trifluoromethyl-phenyl)-5,6,7,8-tetrahydro-pyrido[4,3-d]pyrimidine;
6-Benzyl-4-(4-chloro-phenyl)-2-isopropyl-8-methyl-5,6,7,8-tetrahydro-pyrido[4,3-d]pyrimidine;
6-Benzyl-2-isopropyl-8-methyl-4-thiophen-3-yl-5,6,7,8-tetrahydro-pyrido[4,3-d]pyrimidine;
6-Benzyl-4-(4'-chloro-biphenyl-4-yl)-2-isopropyl-8-methyl-5,6,7,8-tetrahydro-pyrido[4,3-d]pyrimidine;
4-(4-Fluoro-phenyl)-2-isopropyl-8-methyl-5,6,7,8-tetrahydro-pyrido[4,3-d]pyrimidine hydrochloride;
4-(3-Chloro-4-fluoro-phenyl)-2-isopropyl-8-methyl-5,6,7,8-tetrahydro-pyrido[4,3-d]pyrimidine hydrochloride;
2-Isopropyl-8-methyl-4-p-tolyl-5,6,7,8-tetrahydro-pyrido[4,3-d]pyrimidine hydrochloride;
2-Isopropyl-8-methyl-4-phenyl-5,6,7,8-tetrahydro-pyrido[4,3-d]pyrimidine;
2-Isopropyl-8-methyl-4-(4-trifluoromethyl-phenyl)-5,6,7,8-tetrahydro-pyrido[4,3-d]pyrimidine;
2-Isopropyl-8-methyl-4-thiophen-3-yl-5,6,7,8-tetrahydro-pyrido[4,3-d]pyrimidine;
4-(4-Fluoro-phenyl)-2-isopropyl-6,7-dihydro-5H-pyrrolo[3,4-d]pyrimidine hydrochloride;
4-(4-Fluoro-phenyl)-2-isopropyl-7-pyrrolidin-1-yl-5,6,7,8-tetrahydro-quinazoline;
[4-(4-Fluoro-phenyl)-2-isopropyl-5,6,7,8-tetrahydro-quinazolin-7-yl]-methyl-amine hydrochloride;
[4-(4-Fluoro-phenyl)-2-isopropyl-5,6,7,8-tetrahydro-quinazolin-6-yl]-methyl-amine hydrochloride;
4-(4-Fluoro-phenyl)-2-isopropyl-5,6,7,8-tetrahydro-quinazolin-7-ol;
4-(4-Fluoro-phenyl)-2-isopropyl-5,6,7,8-tetrahydro-quinazoline;
(2-Benzyl-6-p-tolyl-pyrimidin-4-ylmethyl)-dimethyl-amine;
2-Benzyl-4-(4-methyl-piperazin-1-ylmethyl)-6-p-tolyl-pyrimidine;
[6-(4-Fluoro-phenyl)-2-isopropyl-pyrimidin-4-ylmethyl]-methyl-amine;
2-(2-Benzyl-6-p-tolyl-pyrimidin-4-yl)-ethylamine;
[2-(4-Fluoro-benzyl)-4-p-tolyl-pyrimidin-5-ylmethyl]-dimethyl-amine;
4-(4-Fluoro-phenyl)-2-phenyl-5,6,7,8-tetrahydro-pyrido[4,3-d]pyrimidine;
2-(3,3-Difluoro-cyclopentyl)-4-(4-fluoro-phenyl)-5,6,7,8-tetrahydro-pyrido[4,3-d]pyrimidine;
4-(4-Fluoro-phenyl)-2-(tetrahydro-furan-3-yl)-5,6,7,8-tetrahydro-pyrido[4,3-d]pyrimidine;
4-(4-Fluoro-phenyl)-2-(2-piperidin-1-yl-ethyl)-5,6,7,8-tetrahydro-pyrido[4,3-d]pyrimidine;
2-(1-Fluoro-1-methyl-ethyl)-4-(4-fluoro-phenyl)-5,6,7,8-tetrahydro-pyrido[4,3-d]pyrimidine;
3-(4-Fluoro-phenyl)-5-isopropyl-4,6,12-triaza-tricyclo[7.2.1.0^{2.7}]dodeca-2,4,6-triene;
7-(4-Fluoro-phenyl)-5-isopropyl-4,6,13-triaza-tricyclo[8.2.1.0^{3.8}]trideca-3,5,7-triene;
4-(4-Fluoro-phenyl)-2-(tetrahydro-pyran-4-yl)-5,6,7,8-tetrahydro-pyrido[4,3-d]pyrimidine;
4-(4-Fluoro-phenyl)-2-(tetrahydro-pyran-3-yl)-5,6,7,8-tetrahydro-pyrido[4,3-d]pyrimidine;
4-(4-Fluoro-phenyl)-2-(2-methoxy-ethyl)-5,6,7,8-tetrahydro-pyrido[4,3-d]pyrimidine;
2-[4-(4-Fluoro-phenyl)-5,6,7,8-tetrahydro-pyrido[4,3-d]pyrimidin-2-yl]-propan-2-ol;
4-(4-Fluoro-phenyl)-2-(1-methyl-1-phenyl-ethyl)-5,6,7,8-tetrahydro-pyrido[4,3-d]pyrimidine;
2-Cyclopent-3-enyl-4-(4-fluoro-phenyl)-5,6,7,8-tetrahydro-pyrido[4,3-d]pyrimidine;
3-[4-(4-Fluoro-phenyl)-5,6,7,8-tetrahydro-pyrido[4,3-d]pyrimidin-2-yl]-cyclohexanol;
4-(4-Fluoro-phenyl)-2-piperidin-4-yl-5,6,7,8-tetrahydro-pyrido[4,3-d]pyrimidine;
4-(4-Fluoro-phenyl)-2-(1-methyl-piperidin-4-yl)-5,6,7,8-tetrahydro-pyrido[4,3-d]pyrimidine;
[4-(4-Fluoro-phenyl)-5,6,7,8-tetrahydro-pyrido[4,3-d]pyrimidin-2-yl]-phenyl-methanol;
4-(4-Fluoro-phenyl)-2-(fluoro-phenyl-methyl)-5,6,7,8-tetrahydro-pyrido[4,3-d]pyrimidine;
2-(Difluoro-phenyl-methyl)-4-(4-fluoro-phenyl)-5,6,7,8-tetrahydro-pyrido[4,3-d]pyrimidine;
4-(4-Fluoro-phenyl)-2-phenyl-5,6,7,8-tetrahydro-pyrido[4,3-d]pyrimidine;
4-(4-Fluoro-phenyl)-2-(3-fluoro-phenyl)-5,6,7,8-tetrahydro-pyrido[4,3-d]pyrimidine;
4-(4-Fluoro-phenyl)-2-(4-methoxy-phenyl)-5,6,7,8-tetrahydro-pyrido[4,3-d]pyrimidine;
4-(4-Fluoro-phenyl)-2-o-tolyl-5,6,7,8-tetrahydro-pyrido[4,3-d]pyrimidine;
3-[4-(4-Fluoro-phenyl)-5,6,7,8-tetrahydro-pyrido[4,3-d]pyrimidin-2-yl]-benzonitrile;
4-(4-Fluoro-phenyl)-2-(2,2,2-trifluoro-1-trifluoromethyl-ethyl)-5,6,7,8-tetrahydro-pyrido[4,3-d]pyrimidine;
4-(4-Fluoro-phenyl)-2-(1-methyl-cyclopropyl)-5,6,7,8-tetrahydro-pyrido[4,3-d]pyrimidine;
2-[4-(4-Fluoro-phenyl)-5,6,7,8-tetrahydro-pyrido[4,3-d]pyrimidin-2-yl]-2-methyl-propionic acid;
2-[4-(4-Fluoro-phenyl)-5,6,7,8-tetrahydro-pyrido[4,3-d]pyrimidin-2-yl]-propionic acid;
2-(4-Fluoro-cyclohexyl)-4-(4-fluoro-phenyl)-5,6,7,8-tetrahydro-pyrido[4,3-d]pyrimidine;
2-(4,4-Difluoro-cyclohexyl)-4-(4-fluoro-phenyl)-5,6,7,8-tetrahydro-pyrido[4,3-d]pyrimidine;
4-(4-Fluoro-phenyl)-2-phenethyl-5,6,7,8-tetrahydro-pyrido[4,3-d]pyrimidine;
4-Furan-2-yl-2-isopropyl-5,6,7,8-tetrahydro-pyrido[4,3-d]pyrimidine;
2-Isopropyl-4-(5-methyl-furan-2-yl)-5,6,7,8-tetrahydro-pyrido[4,3-d]pyrimidine;
4-Furan-3-yl-2-isopropyl-5,6,7,8-tetrahydro-pyrido[4,3-d]pyrimidine;
4-(5-Fluoro-pyridin-2-yl)-2-isopropyl-5,6,7,8-tetrahydro-pyrido[4,3-d]pyrimidine;
2-Isopropyl-4-oxazol-2-yl-5,6,7,8-tetrahydro-pyrido[4,3-d]pyrimidine;
4-(4,5-Dimethyl-oxazol-2-yl)-2-isopropyl-5,6,7,8-tetrahydro-pyrido[4,3-d]pyrimidine;
2-Isopropyl-4-thiazol-2-yl-5,6,7,8-tetrahydro-pyrido[4,3-d]pyrimidine;
2-Isopropyl-4-(3H-[1,2,3]triazol-4-yl)-5,6,7,8-tetrahydro-pyrido[4,3-d]pyrimidine;
2-Isopropyl-4-(2H-pyrazol-3-yl)-5,6,7,8-tetrahydro-pyrido[4,3-d]pyrimidine;
2-Isopropyl-4-(1H-pyrazol-4-yl)-5,6,7,8-tetrahydro-pyrido[4,3-d]pyrimidine;
4-(4-Fluoro-phenyl)-2,6-diisopropyl-5,6,7,8-tetrahydro-pyrido[4,3-d]pyrimidine;
6-Ethyl-4-(4-fluoro-phenyl)-2-isopropyl-5,6,7,8-tetrahydro-pyrido[4,3-d]pyrimidine;
6-Cyclopropyl-4-(4-fluoro-phenyl)-2-isopropyl-5,6,7,8-tetrahydro-pyrido[4,3-d]pyrimidine;
6-Cyclobutyl-4-(4-fluoro-phenyl)-2-isopropyl-5,6,7,8-tetrahydro-pyrido[4,3-d]pyrimidine;
6-Cyclopentyl-4-(4-fluoro-phenyl)-2-isopropyl-5,6,7,8-tetrahydro-pyrido[4,3-d]pyrimidine;
6-Butyl-4-(4-fluoro-phenyl)-2-isopropyl-5,6,7,8-tetrahydro-pyrido[4,3-d]pyrimidine; and
4-(4-Fluoro-phenyl)-2-isopropyl-5,6,7,8-tetrahydro-pyrido[4,3-d]pyrimidine, citrate salt.

29. The compound of claim 1 wherein said pharmaceutically acceptable salt is an effective amino addition salt.

30. The compound of claim 1 wherein said pharmaceutically acceptable salt is selected from the group consisting of hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate.

31. A pharmaceutical composition comprising a pharmaceutically acceptable carrier and a therapeutically effective amount of at least one compound of Formulae (I) or (II): 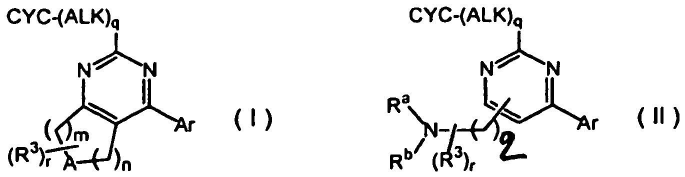 wherein
m is 1, 2, or 3;
n is 1, 2, or 3;
where when m and n are both present, m + n is greater than or equal to 2, and is less than or equal to 4;
R^{a} and R^{b} are independently -H, -C₁₋₇alkyl, or -C₃₋₇cycloalkyl, or R^{a} and R^{b} taken together with the nitrogen of attachment form piperidinyl, pyrrolidinyl, morpholinyl, thiomorpholinyl, or piperazinyl, where each R^{a} and R^{b} is optionally and independently substituted with -C₁₋₄alkyl;
q is 0 or 1;
A is >NR¹, >CHNR^{c}R^{d}, >CHOH, or -CH₂-, wherein
R¹ is selected from the group consisting of -H, -C₁₋₇alkyl, -C₃₋₇cycloalkyl, and benzyl, where each alkyl, cycloalkyl, or benzyl is optionally mono-, di-, or tri-substituted with R^{e};
R^{e} is selected from the group consisting of -C₁₋₄alkyl, -C₂₋₄alkenyl, -C₂₋₄alkynyl, -C₃₋₄cycloalkyl, halo, -CF₃, -OH, -OC₁₋₄alkyl, -OCF₃, -N(R^{f})R^{g} (wherein R^{f} and R^{g} are independently -H or -C₁₋₄alkyl, or R^{f} and R^{g} taken together with the nitrogen of attachment form piperidinyl, pyrrolidinyl, morpholinyl, thiomorpholinyl, or piperazinyl), -C(O)N(R^{f})R^{g}, -N(R^{h})C(O)R^{h}, -N(R^{h})SO₂C₁₋₇alkyl (wherein R^{h} is -H or -C₁₋₄alkyl, or two R^{h} in the same substituent taken together with the amide of attachment form an otherwise aliphatic 4- to 6-membered ring), -S(O)₀₋₂-C₁₋₄alkyl, -SO₂N(R^{f})R^{g}, -SCF₃, -C(O)C₁₋₄alkyl, -CN, -COOH, and -COOC₁₋₄alkyl;
R^{c} and R^{d} are independently selected from the group consisting of -H, -C₁₋₇alkyl, -C₃₋₇alkenyl, -C₃₋₇alkynyl, -C₃₋₇cycloalkyl, -C₁₋₇alkylC₃₋₇cycloalkyl, and -C₃₋₇cycloalkylC₁₋₇alkyl, or R^{c} and R^{d} taken together with the nitrogen of attachment form piperidinyl, pyrrolidinyl, morpholinyl, thiomorpholinyl, or piperazinyl, where each R^{e} and R^{d} is optionally and independently substituted with R^{e};
R³ is -C₁₋₄alkyl, -C₁₋₄alkenyl, or benzyl, each optionally substituted with -C₁₋₃alkyl, -OH. or halo, or two R³ substituents taken together form C₂₋₅alkylene optionally substituted with -C₁₋₃alkyl, -OH, or halo;
r is 0 or is an integer less than or equal to m + n + 1;
Ar is an aryl or heteroaryl ring selected from the group consisting of:
a) phenyl, optionally mono-, di-, or tri-substituted with Rⁱ or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH)₂-;
Rⁱ is selected from the group consisting of -C₁₋₇alkyl, -C₂₋₇alkenyl, -C₂₋₇alkynyl, -C₃₋₇cycloalkyl, halo, -CF₃, -OH, -OC₁₋₇alkyl, -OCF₃, -OC₃₋₇alkenyl, -OC₃₋₇alkynyl, -N(R^{j})R^{k} (wherein R^{j} and R^{k} are independently -H or -C₁₋₄alkyl). -C(O)N(R^{j})R^{k}, -N(R^{j})C(O)R^{k}, -N(R^{j})SO₂C₁₋₆alkyl, -S(O)₀₋₂-C₁₋₆alkyl, -SO₂N(R^{j})R^{k}, -SCF₃, -C(O)C₁₋₆alkyl, -NO₂, -CN, -COOH, and -COOC₁₋₇alkyl;
b) a monocyclic aromatic hydrocarbon group having five ring atoms, having a carbon atom which is the point of attachment, having one carbon atom replaced by >O, >S, >NH, or >N(C₁₋₄alkyl), having up to two additional carbon atoms optionally replaced by - N=, optionally mono- or di-substituted with Rⁱ;
c) a monocyclic aromatic hydrocarbon group having six ring atoms, having a carbon atom which is the point of attachment, having one or two carbon atoms replaced by - N=, optionally mono- or di-substituted with Rⁱ; and
d) phenyl or pyridyl, substituted with a substituent selected from the group consisting of phenyl, phenoxy, pyridyl, thiophenyl, oxazolyl, and tetrazolyl, where the resultant substituted moiety is optionally further mono-, di-, or tri-substituted with Rⁱ;
ALK is a branched or unbranched C₁₋₇alkylene, C₂₋₇alkenylene, C₂₋₇alkynylene, C₃₋₇cycloalkylene, or C₃₋₇cycloalkenylene, optionally mono-, di-, or tri-substituted with R^{m};
R^{m} is selected from the group consisting of halo, -CF₃, -OH, -OC₁₋₇alkyl,. -OC₃₋₇cycloalkyl, -OCF₃, -N(R^{p})R^{s} (wherein R^{p} and R^{s} are independently -H or -C₁₋₇alkyl), -C(O)N(R^{p})R^{s}, -N(R^{t})C(O)R^{t}, -N(R^{t})SO₂C₁₋₆alkyl (wherein R^{t} is -H or - C₁₋₇alkyl), -S(O)₀₋₂-C₁₋₆alkyl, -SO₂N(R^{p})R^{s}, -SCF₃, -CN, -NO₂, -C(O)C₁₋₇alkyl, -COOH, and -COOC₁₋₇alkyl;
CYC is -H or is a ring system selected from the group consisting of:
i) phenyl, optionally mono-, di-, or tri-substituted with R^{u} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-,
R^{u} is selected from the group consisting of -C₁₋₇alkyl, -C₃₋₇cycloalkyl, phenyl, benzyl, halo, -CF₃, -OH, -OC₁₋₇alkyl, -OC₃₋₇cycloalkyl, -Ophenyl, -Obenzyl, -OCF₃, -N(R^{v})R^{w} (wherein R^{v} and R^{w} are independently -H or -C₁₋₇alkyl, or R^{v} and R^{w} taken together with the nitrogen of attachment form piperidinyl, pyrrolidinyl, morpholinyl, thiomorpholinyl, or piperazinyl, where each R^{v} and R^{w} is optionally and independently substituted with -OH or -C₁₋₇alkyl), -C(O)N(R^{v})R^{w}, -N(R^{x})C(O)R^{x}, -N(R^{x})SO₂C₁₋₆alkyl (wherein R^{x} is -H or -C₁₋₇alkyl, or two R^{x} in the same substituent taken together with the amide of attachment form an otherwise aliphatic 4- to 6-membered ring), -N-(SO₂C₁₋₆alkyl), -S(O)₀₋₂-C₁₋₆alkyl, -SO₂N(R^{v})R^{w}, -SCF₃, -C(O)C₁₋₆alkyl, -NO₂, -CN, -COOH, and -COOC₁₋₇alkyl;
ii) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atoms optionally replaced by -N=, optionally mono- or di-substituted with R^{u};
iii) a monocyclic aromatic hydrocarbon group having six ring atoms, having a carbon atom which is the point of attachment, having one or two carbon atoms replaced by - N=, optionally mono- or di-substituted with R^{u}; and
iv) a non-aromatic heterocyclic ring having 4 to 8 members, said ring having 0, 1, or 2 non-adjacent heteroatom members selected from the group consisting of O, S, -N=, >NH, and >N(C₁₋₄alkyl), having 0, 1, or 2 double bonds, having 0, 1, or 2 carbon members which is a carbonyl, optionally having one carbon member which forms a bridge, having 0 to 5 substituents R^{u}, and where when q is 0, said ring has a carbon atom which is the point of attachment;
and enantiomers, diastereomers, hydrates, solvates and pharmaceutically acceptable salts, esters and amides thereof;
with the proviso that in Formula (I):
(a) when ALK is methylene, ethylene, propylene, or isopropylene, CYC is -H, Ar is phenyl or mono-substituted phenyl, m is 2, n is 1, and A is >NR¹, then R¹ is not -C₁₋₄alkyl or benzyl;
(b) when q is 0, CYC is phenyl, Ar is phenyl or 3-chlorophenyl, m is 2, and n is 1, then A is not unsubstituted -CH₂-; and
(c) when q is 0, CYC is 2-pyridyl, Ar is 2-pyridyl, m is 2, and n is 1, then A is not unsubstituted -CH₂-.

32. A compound of Formulae (I) or (II): 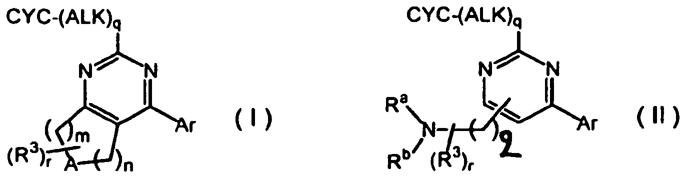 wherein
m is 1, 2, or 3;
n is 1, 2, or 3;
where when m and n are both present, m + n is greater than or equal to 2, and is less than or equal to 4;
R^{a} and R^{b} are independently -H, -C₁₋₇alkyl, or -C₃₋₇cycloalkyl, or R^{a} and R^{b} taken together with the nitrogen of attachment form piperidinyl, pyrrolidinyl, morpholinyl, thiomorpholinyl, or piperazinyl, where each R^{a} and R^{b} is optionally and independently substituted with -C₁₋₄alkyl;
q is 0 or 1;
A is >NR¹, >CHNR^{c}R^{d}, >CHOH, or -CH₂-, wherein
R¹ is selected from the group consisting of -H, -C₁₋₇alkyl, -C₃₋₇cycloalkyl, and benzyl, where each alkyl, cycloalkyl, or benzyl is optionally mono-, di-, or tri-substituted with R^{e}; R^{e} is selected from the group consisting of -C₁₋₄alkyl, -C₂₋₄alkenyl, -C₂₋₄alkynyl, -C₃₋cycloalkyl, halo, -CF₃, -OH, -OC₁₋₄alkyl, -OCF₃, -N(R^{f})R^{g} (wherein R^{f} and R^{g} are independently -H or -C₁₋₄alkyl, or R^{f} and R^{g} taken together with the nitrogen of attachment form piperidinyl, pyrrolidinyl, morpholinyl, thiomorpholinyl, or piperazinyl), -C(O)N(R^{f})R^{g}, -N(R^{h})C(O)R^{h}. -N(R^{h})SO₂C₁₋₇alkyl (wherein R^{h} is -H or -C₁₋₄alkyl, or two R^{h} in the same substituent taken together with the amide of attachment form an otherwise aliphatic 4- to 6-membered ring), -S(O)₀₋₂-C₁₋₄alkyl, -SO₂N(R^{f})R^{g}, -SCF₃, -C(O)C₁₋₄alkyl, -CN, -COOH, and -COOC₁₋₄alkyl;
R^{c} and R^{d} are independently selected from the group consisting of -H, -C₁₋₇alkyl, -C₃₋₇alkenyl, -C₃₋₇alkynyl, -C₃₋₇cycloalkyl, -C₁₋₇alkylC₃₋₇cycloalkyl, and -C₃₋₇cycloalkylC₁₋₇alkyl, or R^{c} and R^{d} taken together with the nitrogen of attachment form piperidinyl, pyrrolidinyl, morpholinyl, thiomorpholinyl, or piperazinyl, where each R^{c} and R^{d} is optionally and independently substituted with R^{e};
R³ is -C₁₋₄alkyl, -C₁₋₄alkenyl, or benzyl, each optionally substituted with -C₁₋₃alkyl, -OH, or halo, or two R³ substituents taken together form C₂₋₅alkylene optionally substituted with -C₁₋₃alkyl, -OH, or halo;
r is 0 or is an integer less than or equal to m + n + 1;
Ar is an aryl or heteroaryl ring selected from the group consisting of:
a) phenyl, optionally mono-, di-, or tri-substituted with Rⁱ or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
Rⁱ is selected from the group consisting of -C₁₋₇alkyl, -C₂₋₇alkenyl, -C₂₋₇alkynyl, -C₃₋₇cycloalkyl, halo, -CF₃, -OH, -OC₁₋₇alkyl, -OCF₃, -OC₃₋₇alkenyl, -OC₃₋₇alkynyl, -N(Rⁱ)R^{k} (wherein R^{j} and R^{k} are independently -H or -C₁₋₄alkyl), -C(O)N(Rⁱ)R^{k}, -N(R^{j})C(O)R^{k}, -N(R^{j})SO₂C₁₋₆alkyl, -S(O)₀₋₂-C₁₋₆alkyl, -SO₂N(R^{j})R^{k}, -SCF₃, -C(O)C₁₋₆alkyl, -NO₂, -CN, -COOH, and -COOC₁₋₇alkyl;
b) a monocyclic aromatic hydrocarbon group having five ring atoms, having a carbon atom which is the point of attachment, having one carbon atom replaced by >O, >S, >NH, or >N(C₁₋₄alkyl), having up to two additional carbon atoms optionally replaced by - N=, optionally mono- or di-substituted with Rⁱ;
c) a monocyclic aromatic hydrocarbon group having six ring atoms, having a carbon atom which is the point of attachment, having one or two carbon atoms replaced by - N=, optionally mono- or di-substituted with Rⁱ; and
d) phenyl or pyridyl, substituted with a substituent selected from the group consisting of phenyl, phenoxy, pyridyl, thiophenyl, oxazolyl, and tetrazolyl, where the resultant substituted moiety is optionally further mono-, di-, or tri-substituted with Rⁱ;
ALK is a branched or unbranched C₁₋₇alkylene, C₂₋₇alkenylene, C₂₋₇alkynylene, C₃₋₇cycloalkylene, or C₃₋₇cycloalkenylene, optionally mono-, di-, or tri-substituted with R^{m};
R^{m} is selected from the group consisting of halo, -CF₃, -OH, -OC₁₋₇alkyl, -OC₃₋₇cycloalkyl, -OCF₃, -N(R^{p})R^{s} (wherein R^{p} and R^{s} are independently -H or -C₁₋₇alkyl), -C(O)N(R^{p})R^{s}, -N(R^{t})C(O)R^{t}, -N(R^{t})SO₂C₁₋₈alkyl (wherein R^{t} is -H or - C₁₋₇alkyl), -S(O)₀₋₂C₁₋₆alkyl, -SO₂N(R^{p})R^{s}, -SCF₃, -CN, -NO₂, -C(O)C₁₋₇alkyl, -COOH, and -COOC₁₋₇alkyl;
CYC is -H or is a ring system selected from the group consisting of:
i) phenyl, optionally mono-, di-, or tri-substituted with R^{u} or di-substituted on adjacent carbons with -OC₁₋₄alkyleneO-, -(CH₂)₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
R^{u} is selected from the group consisting of -C₁₋₇alkyl, -C₃₋₇cycloalkyl, phenyl, benzyl, halo, -CF₃, -OH, -OC1-₇alkyl, -OC₃₋₇cycloalkyl, -Ophenyl, -Obenzyl, -OCF₃, -N(R^{v})R^{w} (wherein R^{v} and R^{w} are independently -H or C₁₋₇alkyl, or R^{v} and R^{w} taken together with the nitrogen of attachment form piperidinyl, pyrrolidinyl, morpholinyl, thiomorpholinyl, or piperazinyl, where each R^{v} and R^{w} is optionally and independently substituted with -OH or -C₁₋₇alkyl), -C(O)N(R^{v})R^{w}, -N(R^{x})C(O)R^{x}, -N(R^{x})SO₂C₁₋₈alkyl (wherein R^{x} is -H or -C₁₋₇alkyl, or two R^{x} in the same substituent taken together with the amide of attachment form an otherwise aliphatic 4- to 6-membered ring), -N-(SO₂C₁₋₆alkyl)₂, -S(O)₀-₂-C₁₋₆alkyl, -SO₂N(R^{v})R^{w}, -SCF₃, -C(O)C₁₋₈alkyl, -NO₂, -CN, -COOH, and -COOC₁₋₇alkyl_{;}
ii) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atoms optionally replaced by -N=, optionally mono- or di-substituted with R^{u};
iii) a monocyclic aromatic hydrocarbon group having six ring atoms, having a carbon atom which is the point of attachment, having one or two carbon atoms replaced by - N=, optionally mono- or di-substituted with R^{u}; and
iv) a non-aromatic heterocyclic ring having 4 to 8 members, said ring having 0, 1, or 2 non-adjacent heteroatom members selected from the group consisting of O, S, -N=, >NH, and >N(C₁₋₄alkyl), having 0, 1, or 2 double bonds, having 0, 1, or 2 carbon members which is a carbonyl, optionally having one carbon member which forms a bridge, having 0 to 5 substituents R^{u}, and where when q is 0, said ring has a carbon atom which is the point of attachment;
and enantiomers, diastereomers, hydrates, solvates and pharmaceutically acceptable salts, esters and amides thereof;
with the proviso that In Formula (I):
(a) when ALK is methylene, ethylene, propylene, or isopropylene, CYC is -H, Ar is phenyl or mono-substituted phenyl, m is 2, n is 1, and A is >NR¹, then R¹ is not -C₁₋₄alkyl or benzyl;
(b) when q is 0, CYC is phenyl, Ar is phenyl or 3-chlorophenyl, m is 2, and n is 1, then A is not unsubstituted -CH₂-; and
(c) when q is 0, CYC is 2-pyridyl, Ar is 2-pyridyl, m is 2, and n is 1, then A is not unsubstituted -CH₂-, for use in therapy.

33. The use of at least one compound of Formulae (I) or (II): 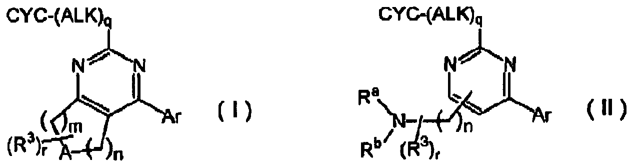 wherein
m is 1, 2, or 3;
n is 1, 2, or 3;
where when m and n are both present, m + n is greater than or equal to 2, and is less than or equal to 4;
R^{a} and R^{b} are independently -H, -C₁₋₇alkyl, or -C₃₋₇cycloalkyl, or R^{a} and R^{b} taken together with the nitrogen of attachment form piperidinyl, pyrrolidinyl, morpholinyl, thlomorphollnyl, or piperazinyl, where each R^{a} and R^{b} is optionally and independently substituted with -C₁₋₄alkyl;
q is 0 or 1;
A is >NR¹, >CHNR^{c}R^{d}, >CHOH, or -CH₂-, wherein
R¹ is selected from the group consisting of -H, -C₁₋₇alkyl, -C₃₋₇cycloalkyl, and benzyl, where each alkyl, cycloalkyl, or benzyl is optionally mono-, di-, or tri-substituted with R^{e};
R^{e} is selected from the group consisting of -C₁₋₄alkyl, -C₂₋₄alkenyl, -C₂₋₄alkynyl, -C₃₋₆cycloalkyl, halo, -CF₃, -OH, -OC₁₋₄alkyl, -OCF₃, -N(R^{f})R^{g} (wherein R^{f} and R^{g} are independently -H or -C₁₋₄alkyl, or R^{f} and R^{g} taken together with the nitrogen of attachment form piperidinyl, pyrrolidinyl, morpholinyl, thiomorpholinyl, or piperazinyl), -C(O)N(R^{f})R^{g}, -N(R^{h})C(O)R^{h}, -N(R^{h})SO₂C₁₋₇alkyl (wherein R^{h} is -H or -C₁₋₄alkyl, or two R^{h} in the same substituent taken together with the amide of attachment form an otherwise aliphatic 4- to 6-membered ring), -S(O)₀₋₂-C₁₋₄alkyl, -SO₂N(R^{f})R^{g}, -SCF₃, -C(O)C₁₋₄alkyl, -CN, -COOH, and -COOC₁₋₄alkyl;
R^{c} and R^{d} are independently selected from the group consisting of -H, -C₁₋₇alkyl, -C₁-₇alkenyl, -C₃₋₇alkynyl, -C₃₋₇cycloalkyl, -C₁₋₇alkylC₃₋₇cycloalkyl, and -C₃₋₇cycloalkylC₁₋₇alkyl, or R^{c} and R^{d} taken together with the nitrogen of attachment form piperidinyl, pyrrolidinyl, morpholinyl, thiomorpholinyl, or piperazinyl, where each R^{c} and R^{d} is optionally and independently substituted with R^{e}_{;}
R³ is -C₁₋₄alkyl, -C₁₋₄alkenyl, or benzyl, each optionally substituted with -C₁₋₃alkyl, -OH,
or halo, or two R³ substituents taken together form C₂₋₅alkylene optionally substituted with -C₁₋₃alkyl, -OH, or halo;
r is 0 or is an integer less than or equal to m + n + 1;
Ar is an aryl or heteroaryl ring selected from the group consisting of:
a) phenyl, optionally mono-, di-, or tri-substituted with Rⁱ or di-substituted on adjacent carbons with -OC₁₋₄alkyleneO-. -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
Rⁱ is selected from the group consisting of -C₁₋₇alkyl, -C₂₋₇alkenyl, -C₂₋₇,alkynyl, -C₃₋₇cycloalkyl, halo, -CF₃, -OH, -OC₁₋₇alkyl, -OCF₃, -OC₃₋₇alkenyl, -OC₃₋₇alkynyl, -N(Rⁱ)R^{k} (wherein R^{j} and R^{k} are independently -H or -C₁₋₄alkyl), -C(O)N(R^{j})R^{k}, -N(R^{j})C(O)R^{k}, -N(R^{j})SO₂C₁₋₆alkyl, -S(O)₀₋₂-C₁₋₆alkyl, -SO₂N(R^{j})R^{k}, -SCF₃, -C(O)C₁₋₆alkyl, -NO₂, -CN, -COOH, and -COOC₁₋₇alkyl;
b) a monocyclic aromatic hydrocarbon group having five ring atoms, having a carbon atom which is the point of attachment, having one carbon atom replaced by >O, >S, >NH, or >N(C₁₋₄alkyl), having up to two additional carbon atoms optionally replaced by -N=, optionally mono- or di-substituted with Rⁱ;
c) a monocyclic aromatic hydrocarbon group having six ring atoms, having a carbon atom which is the point of attachment, having one or two carbon atoms replaced by -N=, optionally mono- or di-substituted with Rⁱ; and
d) phenyl or pyridyl, substituted with a substituent selected from the group consisting of phenyl, phenoxy, pyridyl, thiophenyl, oxazolyl, and tetrazolyl, where the resultant substituted moiety is optionally further mono-, di-, or tri-substituted with Rⁱ_{;}
ALK is a branched or unbranched C₁₋₇alkylene, C₂₋₇alkenylene, C₂₋₇alkynylene, C₃₋₇cycloalkylene, or C₃₋₇cycloalkenylene, optionally mono-, di-, or tri-substituted with R^{m};
R^{m} is selected from the group consisting of halo, -CF₃, -OH, -OC₁₋₇alkyl, -OC₃₋₇cycloalkyl, -OCF₃, -N(R^{p})R^{s} (wherein R^{p} and R^{s} are independently -H or -C₁₋₇alkyl), -C(O)N(R^{p})R^{s}, -N(R^{t})C(O)R^{t}, -N(R^{t})SO₂C₁₋₆alkyl (wherein R^{t} is -H or - C₁₋₇alkyl), -S(O)₀₋₂-C₁₋₆alkyl, -SO₂N(R^{p})R^{s}, -SCF₃, -CN, -NO₂, -C(O)C₁₋₇alkyl, -COOH, and -COOC₁₋₇alkyl;
CYC is -H or is a ring system selected from the group consisting of:
i) phenyl, optionally mono-, di-, or tri-substituted with R^{u} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
R^{u} is selected from the group consisting of -C₁₋₇alkyl, -C₃₋₇cycloalkyl, phenyl, benzyl, halo, -CF₃, -OH, -OC₁₋₇alkyl, -OC₃₋₇cycloalkyl, -Ophenyl, -Obenzyl, -OCF₃, -N(R^{v})R^{w} (wherein R^{v} and R^{w} are independently -H or -C₁₋₇alkyl, or R^{v} and R^{w} taken together with the nitrogen of attachment form piperidinyl, pyrrolidinyl, morpholinyl, thiomorpholinyl, or piperazinyl, where each R^{v} and R^{w} is optionally and independently substituted with -OH or -C₁₋₇alkyl), -C(O)N(R^{v})R^{w}, -N(R^{x})C(O)R^{x}, -N(R^{x})SO₂C₁₋₆alkyl (wherein R^{x} is -H or -C₁₋₇alkyl, or two R^{x} in the same substituent taken together with the amide of attachment form an otherwise aliphatic 4- to 6-membered ring), -N-(SO₂C₁₋₆alkyl)₂, -S(O)₀₋₂C₁₋₆alkyl, -SO₂N(R^{v})R^{w}, -SCF₃, -C(O)C₁₋₆alkyl, -NO₂, -CN, -COOH, and -COOC₁₋₇alkyl;
ii) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atoms optionally replaced by -N=, optionally mono- or di-substituted with R^{u};
iii) a monocyclic aromatic hydrocarbon group having six ring atoms, having a carbon atom which is the point of attachment, having one or two carbon atoms replaced by -N=, optionally mono- or di-substituted with R^{u}; and
iv) a non-aromatic heterocyclic ring having 4 to 8 members, said ring having 0, 1, or 2 non-adjacent heteroatom members selected from the group consisting of O, S, -N=, >NH, and >N(C₁₋₄alkyl), having 0, 1, or 2 double bonds, having 0, 1, or 2 carbon members which is a carbonyl, optionally having one carbon member which forms a bridge, having 0 to 5 substituents R^{u}, and where when q is 0, said ring has a carbon atom which is the point of attachment;
and enantiomers, diastereomers, hydrates, solvates and pharmaceutically acceptable
salts, esters and amides thereof, for the manufacture of a medicament for the treatment or prevention of a CNS disorder selected from the group consisting of: sleep disorders, depression/anxiety, generalized anxiety disorder, schizophrenia, bipolar disorders, psychotic disorders, obsessive-compulsive disorder, mood disorders, post-traumatic stress and other stress-related disorders, migraine, pain, eating disorders, obesity, sexual dysfunction, metabolic disturbances, hormonal imbalance, alcohol abuse, addictive disorders, nausea, inflammation, centrally mediated hypertension, sleep/wake disturbances, jetlag, and circadian rhythm abnormalities in mammals;
for the treatment or prevention of a disease or condition selected from the group consisting of: hypotension, peripheral vascular disorders, cardiovascular shock, renal disorders, gastric motility, diarrhea, spastic colon, irritable bowel disorders, ischemias, septic shock, urinary incontinence, and other disorders related to the gastrointestinal and vascular systems in mammals;
for the treatment or prevention of an ocular disorder selected from the group consisting of: glaucoma, optic neuritis, diabetic retinopathy, retinal edema, and age-related macular degeneration in mammals;

34. The use of claim 33 wherein said CNS disorder is selected from the group consisting of: depression/anxiety, sleep disorders, and circadian rhythm abnormalities.

35. A compound of Formulae (I) or (II): 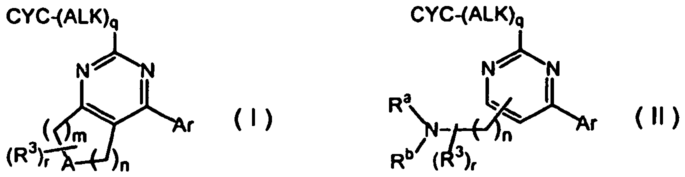 wherein
m is 1, 2, or 3;
n is 1, 2, or 3;
where when m and n are both present, m + n is greater than or equal to 2, and is less than or equal to 4;
R^{a} and R^{b} are independently -H, -C₁₋₇alkyl, or -C₃₋₇cycloalkyl, or R^{a} and R^{b} taken together with the nitrogen of attachment form piperidinyl, pyrrolidinyl, morpholinyl, thiomorpholinyl, or piperazinyl, where each R^{a} and R^{b} is optionally and independently substituted with -C₁₋₄alkyl;
q is 0 or 1;
A is >NR¹, >CHNR^{c}R^{d}, >CHOH, or -CH₂-, wherein
R¹ is selected from the group consisting of -H, -C₁₋₇alkyl, -C₃₋₇cycloalkyl, and benzyl, where each alkyl, cycloalkyl, or benzyl is optionally mono-, di-, or tri-substituted with R^{e}_{;}
R^{e} is selected from the group consisting of -C₁₋₄alkyl, -C₂₋₄alkenyl, -C₂₋₄alkynyl, C₃₋₆cycloalkyl, halo, -CF₃, -OH, -OC₁₋₄alkyl, -OCF₃, -N(R^{f})R^{g} (wherein R^{f} and R^{g} are independently -H or -C₁₋₄alkyl, or R^{f} and R^{g} taken together with the nitrogen of attachment form piperidinyl, pyrrolidinyl, morpholinyl, thiomorpholinyl, or piperazinyl), -C(O)N(R^{f})R^{g}, -N(R^{h})C(O)R^{h}, -N(R^{h})SO₂C₁₋₇alkyl (wherein R^{h} is -H or -C₁₋₄alkyl, or two R^{h} in the same substituent taken together with the amide of attachment form an otherwise aliphatic 4- to 6-membered ring), -S(O)₀₋₂-C₁₋₄alkyl, -SO₂N(R^{f})R^{g}, -SCF₃, -C(O)C₁₋₄alkyl, -CN, -COOH, and -COOC₁₋₄alkyl;
R^{c} and R^{d} are independently selected from the group consisting of -H, -C₁₋₇alkyl, -C₃₋₇alkenyl, -C₃₋₇alkynyl, -C₃₋₇cycloalkyl, -C₁₋₇alkylC₃₋₇cycloalkyl, and -C₃₋₇cycloalkylC₁₋₇alkyl, or R^{c} and R^{d} taken together with the nitrogen of attachment form piperidinyl, pyrrolidinyl, morpholinyl, thiomorpholinyl, or piperazinyl, where each R^{c} and R^{d} is optionally and independently substituted with R^{e}_{;}
R³ is -C₁₋₄alkyl, -C₁₋₄alkenyl, or benzyl, each optionally substituted with -C₁₋₃alkyl, -OH, or halo, or two R³ substituents taken together form C₂₋₅alkylene optionally substituted with -C₁₋₃alkyl, -OH, or halo;
r is 0 or is an integer less than or equal to m + n + 1;
Ar is an aryl or heteroaryl ring selected from the group consisting of:
a) phenyl, optionally mono-, di-, or tri-substituted with Rⁱ or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
Rⁱ is selected from the group consisting of -C₁₋₇alkyl, -C₂₋₇alkenyl, -C₂₋₇alkynyl, -C₃₋₇cycloalkyl, halo, -CF₃, -OH, -OC₁₋₇alkyl, -OCF₃, -OC₃₋₇alkenyl, -OC₃₋₇alkynyl, -N(R^{j})R^{k} (wherein R^{j} and R^{k} are independently -H or -C₁₋₄alkyl), -C(O)N(R^{j})R^{k}, -N(Rⁱ)C(O)R^{k}, -N(Rⁱ)SO₂C₁₋₆alkyl, -S(O)₀₋₂-C₁₋₆alkyl, -SO₂N(R^{j})R^{k}, -SCF₃, -C(O)C₁₋₆alkyl, -NO₂, -CN, -COOH, and -COOC₁₋₇alkyl;
b) a monocyclic aromatic hydrocarbon group having five ring atoms, having a carbon atom which is the point of attachment, having one carbon atom replaced by >O, >S, >NH, or >N(C₁₋₄alkyl), having up to two additional carbon atoms optionally replaced by -N=, optionally mono- or di-substituted with Rⁱ;
c) a monocyclic aromatic hydrocarbon group having six ring atoms, having a carbon atom which is the point of attachment, having one or two carbon atoms replaced by -N=, optionally mono- or di-substituted with Rⁱ; and
d) phenyl or pyridyl, substituted with a substituent selected from the group consisting of phenyl, phenoxy, pyridyl, thiophenyl, oxazolyl, and tetrazolyl, where the resultant substituted moiety is optionally further mono-, di-, or tri-substituted with Rⁱ;
ALK is a branched or unbranched C₁₋₇alkylene, C₂₋₇alkenylene, C₂₋₇alkynylene, C₃₋₇cycloalkylene, or C₃₋₇cycloalkenylene, optionally mono-, di-, or tri-substituted with R^{m}_{;}
R^{m} is selected from the group consisting of halo, -CF₃, -OH, -OC₁₋₇alkyl, -OC₃₋₇cycloalkyl, -OCF₃, -N(R^{p})R^{s} (wherein R^{p} and R^{s} are independently -H or -C₁₋₇alkyl), -C(O)N(R^{p})R^{s}, -N(R^{t})C(O)R^{t}, -N(R^{t})SO₂C₁₋₆alkyl (wherein R^{t} is -H or - C₁₋₇alkyl), -S(O)₀₋₂C₁₋₆alkyl, -SO₂N(R^{p})R^{s}. -SCF₃, -CN, -NO₂, -C(O)C₁₋₇alkyl, -COOH, and -COOC₁₋₇alkyl;
CYC is -H or is a ring system selected from the group consisting of:
i) phenyl, optionally mono-, di-, or tri-substituted with R^{u} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)-, or -(CH₂)₁₋₂N(C₁₋₄alkyl)(CH₂)-;
R^{u} is selected from the group consisting of -C₁₋₇alkyl, -C₃₋₇cycloalkyl, phenyl, benzyl, halo, -CF₃, -OH, -OC₁₋₇alkyl, -OC₃₋₇cycloalkyl, -Ophenyl, -Obenzyl, -OCF₃, -N(R^{v})R^{w} (wherein R^{v} and R^{w} are independently -H or -C₁₋₇alkyl, or R^{v} and R^{w} taken together with the nitrogen of attachment form piperidinyl, pyrrolidinyl, morpholinyl, thiomorpholinyl, or piperazinyl, where each R^{v} and R^{w} is optionally and independently substituted with -OH or -C₁₋₇alkyl), -C(O)N(R^{v})R^{w}, -N(R^{x})C(O)R^{x}, -N(R^{x})SO₂C₁₋₆alkyl (wherein R^{x} is -H or -C₁₋₇alkyl, or two R^{x} in the same substituent taken together with the amide of attachment form an otherwise aliphatic 4- to 6-membered ring), -N-(SO₂C₁₋₆alkyl)₂, -S(O)₀₋₂-C₁₋₆alkyl, -SO₂N(R^{v})R^{w}, -SCF₃, -C(O)C₁₋₆alkyl, -NO₂, -CN, -COOH, and -COOC₁₋₇alkyl;
ii) a monocyclic aromatic hydrocarbon group having five ring atoms, having a carbon atom which is the point of attachment, having one carbon atom replaced by >O, >S, >NH, or >N(C₁₋₄alkyl), having up to one additional carbon atoms optionally replaced by -N=, optionally mono- or di-substituted with R^{u};
iii) a monocyclic aromatic hydrocarbon group having six ring atoms, having a carbon atom which is the point of attachment, having one or two carbon atoms replaced by -N=, optionally mono- or di-substituted with R^{u}; and
iv) a non-aromatic heterocyclic ring having 4 to 8 members, said ring having 0, 1, or 2 non-adjacent heteroatom members selected from the group consisting of O, S, -N=, >NH, and >N(C₁₋₄alkyl), having 0, 1, or 2 double bonds, having 0, 1, or 2 carbon members which is a carbonyl, optionally having one carbon member which forms a bridge, having 0 to 5 substituents R^{u}, and where when q is 0, said ring has a carbon atom which is the point of attachment;
and enantiomers, diastereomers, hydrates, solvates and pharmaceutically acceptable salts, esters and amides thereof,
for use in the treatment or prevention of a CNS disorder selected from the group consisting of: sleep disorders, depression/anxiety, generalized anxiety disorder, schizophrenia, bipolar disorders, psychotic disorders, obsessive-compulsive disorder, mood disorders, post-traumatic stress and other stress-related disorders, migraine, pain, eating disorders, obesity, sexual dysfunction, metabolic disturbances, hormonal imbalance, alcohol abuse, addictive disorders, nausea, inflammation, centrally mediated hypertension, sleep/wake disturbances, jetlag, and circadian rhythm abnormalities in mammals;
for use in the treatment or prevention of a disease or condition selected from the group consisting of: hypotension, peripheral vascular disorders, cardiovascular shock, renal disorders, gastric motility, diarrhea, spastic colon, irritable bowel disorders, ischemias, septic shock, urinary incontinence, and other disorders related to the gastrointestinal and vascular systems in mammals;
for use In the treatment or prevention of an ocular disorder selected from the group consisting of: glaucoma, optic neuritis, diabetic retinopathy, retinal edema, and age-related macular degeneration in mammals.

36. A compound of Formulae (I) or (II) as defined in claim 31 isotopically-labelled to be detectable by PET or SPECT.

37. A method for studying serotonin-mediated disorders comprising the step of using an ¹⁸F-labeled or ¹¹C-labelled compound of Formulae (I) or (II) as defined In claim 31 as a positron emission tomography (PET) molecular probe, wherein said method is not a method for treatment of the human or animal body by surgery or therapy or a diagnostic method practised on the human or animal body.

38. A compound of claim 1 selected from the group consisting of {2-[2 tert-butyl-6-(4-fluoro phenyl)-pyrimidin-4-yl]-ethyl}-methyl-amine and {2-[2-tert-butyl-6-(4-fluorophenyl)-pyrimidin-4-yl]-ethyl}-dimethyl-amine.

39. A compound of claim 1 that is 4-(4-fluoro-phenyl)-2-isopropyl-5,6,7,8-tetrahydro-pyrido[4,3-d]pyrimidine or a pharmaceutically acceptable salt thereof.

## Patentansprüche

1. Verbindung der Formeln (I) oder (II): 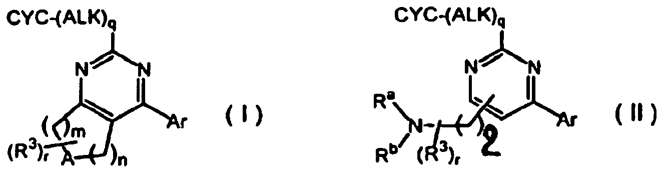 wobei
m 1, 2 oder 3 ist;
n 1, 2 oder 3 ist;
wenn m und n vorhanden sind, m + n größer oder gleich 2 ist und kleiner oder gleich 4 ist;
R^{a} und R^{b} unabhängig -H, -C₁₋₇-Alkyl oder -C₃-₇-Cycloalkyl sind oder R^{a} und R^{b} zusammen genommen mit dem Stickstoff, an den sie gebunden sind, Piperidinyl, Pyrrolidinyl, Morpholinyl, Thiomorpholinyl oder Piperazinyl bilden, wobei jedes R^{a} und R^{b} gegebenenfalls und unabhängig mit -C₁₋₄-Alkyl substituiert ist;
q 0 oder 1 ist;
A >NR¹, >CHNR^{c}R^{d}, >CHOH oder -CH₂- ist, wobei
R¹ ausgewählt ist aus der Gruppe bestehend aus -H, -C₁₋₇-Alkyl, -C₃₋₇-Cycloalkyl und Benzyl, wobei jedes Alkyl, Cycloalkyl oder Benzyl gegebenenfalls mit R^{e} mono-, di- oder trisubstituiert ist;
R^{e} ausgewählt ist aus der Gruppe bestehend aus -C₁₋₄-Alkyl, -C₂₋₄-Alkenyl, -C₂₋₄-Alkinyl, -C₃₋₆-Cycloalkyl, Halogen, -CF₃, -OH, -OC₁₋₄-Alkyl, -OCF₃, -N(R^{f})R^{g} (wobei R^{f} und R^{g} unabhängig -H oder -C₁₋₄-Alkyl sind oder R^{f} und R^{g} zusammen genommen mit dem Stickstoff, an den sie gebunden sind, Piperidinyl, Pyrrolidinyl, Morpholinyl, Thiomorpholinyl oder Piperazinyl bilden), -C(O)N(R^{f})R^{g}, -N(R^{h})C(O)R^{h}, -N(R^{h})SO₂C₁₋₇-Alkyl (wobei R^{h} -H oder -C₁_ ₄-Alkyl ist oder zwei R^{h} an dem gleichen Substituenten zusammen genommen mit dem Amid, an das sie gebunden sind, einen ansonsten aliphatischen 4- bis 6-gliedrigen Ring bilden), -S(O)₀₋₂-C₁₋₄-Alkyl, -SO₂N(R^{f})R^{g}-SCF₃, -C(O)C₁₋₉-Alkyl, -CN, -COOH und -COOC₁₋₉-Alkyl;
R^{c} und R^{d} unabhängig ausgewählt sind aus der Gruppe bestehend aus -H, -C₁₋₇-Alkyl, -C₃₋₇-Alkenyl, -C₃₋₇-Alkinyl, -C₃₋₇-Cycloalkyl, -C₁₋₇-Alkyl-C₃₋₇-cycloalkyl und -C₃₋₇-Cycloalkyl-C₁₋₇-alkyl, oder R^{c} und R^{d} zusammen genommen mit dem Stickstoff, an den sie gebunden sind, Piperidinyl, Pyrrolidinyl, Morpholinyl, Thiomorpholinyl oder Piperazinyl bilden, wobei jedes R^{c} und R^{d} gegebenenfalls und unabhängig mit R^{e} substituiert ist;
R³ -C₁₋₄-Alkyl, -C₁₋₄-Alkenyl oder Benzyl ist, jeweils gegebenenfalls substituiert mit -C₁₋₃-Alkyl, -OH oder Halogen, oder zwei R³-Substituenten zusammen genommen C₂₋₅-Alkylen bilden, gegebenenfalls substituiert mit -C₁₋₃-Alkyl, -OH oder Halogen;
r 0 ist oder eine ganze Zahl kleiner oder gleich m + n + 1 ist;
Ar ein Aryl- oder Heteroarylring ist, ausgewählt aus der Gruppe bestehend aus:
a) Phenyl, gegebenenfalls mono-, di- oder trisubstituiert mit R¹ oder an benachbarten Kohlenstoffen disubstituiert mit -OC₁₋₄-Alkylen-O-, -(CH₂)₂-₃NH-, -(CH₂)₁₋₂NH(CH₂)-, - (CH₂)₂₋₃N(C₁₋₄-Alkyl)- oder -(CH₂)₁₋₂N(C₁₋₄-Alkyl)(CH₂)-;
R¹ ausgewählt ist aus der Gruppe bestehend aus -C₁₋₇-Alkyl, -C₂₋₇-Alkenyl, -C₂₋₇-Alkinyl, -C₃₋₇-Cycloalkyl, Halogen, -CF₃, -OH, -OC₁₋₇-Alkyl, -OCF₃, -OC₃₋₇-Alkenyl, -OC₃₋₇-Alkinyl, -N (R^{j}) R^{k} (wobei R^{j} und R^{k} unabhängig -H oder -C₁₋₄-Alkyl sind), -C(O)N(R^{j}) R^{k}, -N(R^{j})C(O)R^{k},-N (R^{j}) SO₂C₁₋₆-Alkyl, -S(O)₀₋₂-C₁₋₆-Alkyl, -SO₂N(R^{j})R^{k}, -SCF₃, -C(O)C₁₋₆-Alkyl, -NO₂, -CN, -COOH und -COOC₁₋₇-Alkyl;
b) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu zwei zusätzliche Kohlenstoffatomen gegebenenfalls durch -N= ersetzt sind, gegebenenfalls mono- oder disubstituiert mit Rⁱ ;
c) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit Rⁱ; und
d) Phenyl oder Pyridyl, substituiert mit einem Substituenten, ausgewählt aus der Gruppe bestehend aus Phenyl, Phenoxy, Pyridyl, Thiophenyl, Oxazolyl und Tetrazolyl, wobei die erhaltene substituierte Einheit gegebenenfalls weiter mono-, di- oder trisubstituiert ist mit Rⁱ ;
ALK ein verzweigtes oder nichtverzweigtes C₁₋₇-Alkylen, C₂₋₇-Alkenylen, C₂₋₇-Alkinylen, C₃₋₇-Cycloalkylen oder C₃₋₇-Cycloalkenylen ist, gegebenenfalls mono-, di- oder trisubstituiert mit R^{m};
R^{m} ausgewählt ist aus der Gruppe bestehend aus Halogen, -CF₃, -OH, -OC₁₋₇-Alkyl, -OC₃₋₇-Cycloalkyl, -OCF₃, -N(R^{p})R^{s} (wobei R^{p} und R^{s} unabhängig -H oder -C₁₋₇-Alkyl sind), -C(O)N(R^{p})R^{s}, -N(R^{t})C(O)R^{t}, -N(R^{t})SO₂C₁₋₆-Alkyl (wobei R^{t} -H oder -C₁₋₇-Alkyl ist), -S(O)₀₋₂₋C₁-₆-Alkyl, -SO₂N(R^{p})R^{s}, -SCF₃, -CN, -NO₂, -C(O)C₁-₇-Alkyl, -COOH und -COOC₁₋₇-Alkyl;
CYC -H ist oder ein Ringsystem ist, ausgewählt aus der Gruppe bestehend aus:
i) Phenyl, gegebenenfalls mono-, di- oder trisubstituiert mit R^{u} oder an benachbarten Kohlenstoffen disubstituiert mit -OC₁₋₄-Alkylen-O-, - (CH₂)₂₋₃NH-, - (CH₂)₁₋₂NH(CH₂)-, - (CH₂)₂₋₃N(C₁₋₄-Alkyl) oder - (CH₂)₁₋₂N(C₁₋₄-Alkyl) (CH₂) -;
R^{u} ausgewählt ist aus der Gruppe bestehend aus -C₁₋₇-Alkyl, -C₃₋₇-Cycloalkyl, Phenyl, Benzyl, Halogen, -CF₃, -OH, -OC₁₋₇-Alkyl, -OC₃₋₇-Cycloalkyl, -OPhenyl, -OBenzyl, -OCF₃, -N(R^{v})R^{w} (wobei R^{v} und R^{w} unabhängig -H oder -C₁₋₇-Alkyl sind oder R^{v} und R^{w} zusammen genommen mit dem Stickstoff, an den sie gebunden sind, Piperidinyl, Pyrrolidinyl, Morpholinyl, Thiomorpholinyl oder Piperazinyl bilden, wobei jedes R^{v} und R^{w} gegebenenfalls und unabhängig mit -OH oder -C₁₋₇-Alkyl substituiert ist), -C(O)N(R^{v})R^{w}, -N(R^{x})C(O)R^{x}, -N(R^{x})SO₂C₁₋₆-Alkyl (wobei R^{x} -H oder -C₁₋₇-Alkyl ist oder zwei R^{x} an dem gleichen Substituenten zusammen genommen mit dem Amid, an das sie gebunden sind, einen ansonsten aliphatischen 4- bis 6-gliedrigen Ring bilden), -N-(SO₂C₁₋₆-Alkyl)₂, -S(O)₀₋₂-C₁₋₆-Alkyl, -SO₂N(R^{v})R^{w}, -SCF₃, -C(O)C₁₋₆-Alkyl, -NO₂, -CN, -COOH und -COOC₁₋₇-Alkyl;
ii) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu einem zusätzlichen Kohlenstoffatom gegebenenfalls durch -N= ersetzt ist, gegebenenfalls mono- oder disubstituiert mit R^{u};
iii) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit R^{u}; und
iv) einem nichtaromatischen heterocyclischen Ring mit 4 bis 8 Gliedern, wobei der Ring 0, 1 oder 2 nichtbenachbarte Heteroatom-Glieder aufweist, ausgewählt aus der Gruppe bestehend aus O, S, -N=, >NH und >N(C₁₋₉-Alkyl) , mit 0, 1 oder 2 Doppelbindungen, mit 0, 1 oder 2 Kohlenstoffgliedern, die ein Carbonyl sind, gegebenenfalls mit einem Kohlenstoffglied, das eine Brücke bildet, mit 0 bis 5 Substituenten R^{u}, und wobei, wenn q 0 ist, der Ring ein Kohlenstoffatom aufweist, das der Punkt der Befestigung ist;
und Enantiomere, Diastereomere, Hydrate, Solvate und pharmazeutisch verträgliche Salze, Ester und Amide davon;
mit der Maßgabe, dass in Formel (I):
(a) wenn ALK Methylen, Ethylen, Propylen oder Isopropylen ist, CYC -H ist, Ar Phenyl oder monosubstituiertes Phenyl ist, m 2 ist, n 1 ist und A >NR¹ ist, dann R¹ nicht -C₁₋₄-Alkyl oder Benzyl ist;
(b) wenn q 0 ist, CYC Phenyl ist, Ar Phenyl oder 3-Chlorphenyl ist, m 2 ist und n 1 ist, dann A nicht nichtsubstituiertes -CH₂- ist; und
(c) wenn q 0 ist, CYC 2-Pyridyl ist, Ar 2-Pyridyl ist, m 2 ist und n 1 ist, dann A nicht nichtsubstituiertes -CH₂- ist; wobei die Verbindung nicht 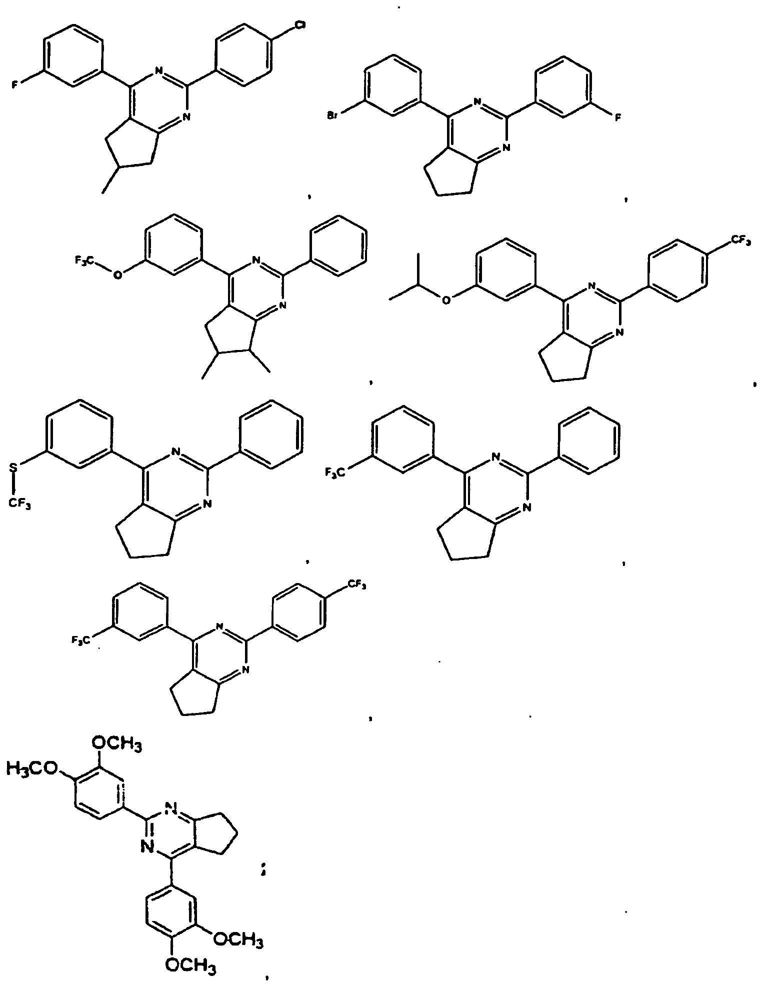 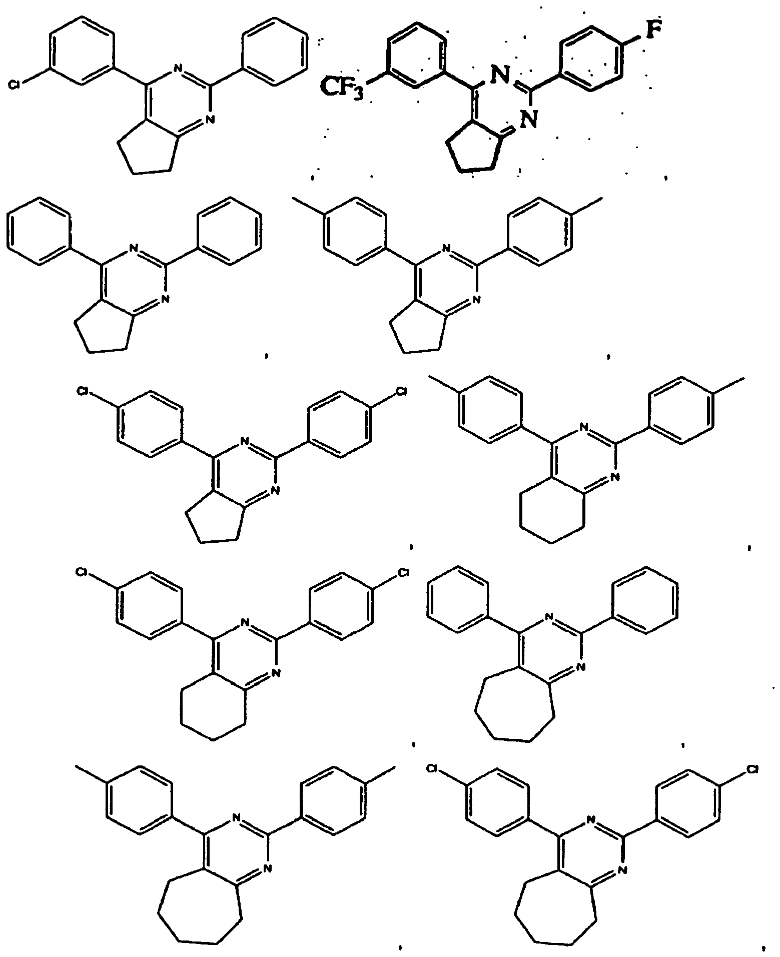 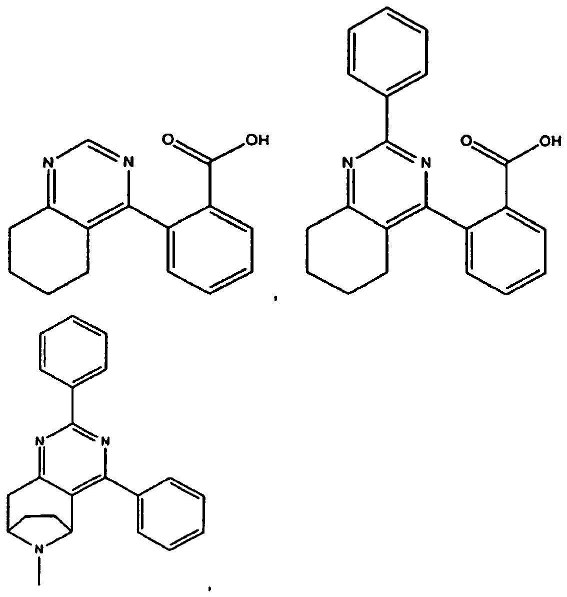
7-Benzyl-2-methyl-4-phenyl-5,8-dihydro-6H-pyrido[3,4-d]pyrimidin, 2-Methyl-4-phenyl-5,8-dihydro-6H-pyrido[3,4-d]pyrimidin oder 4-(2-Methyl-4-nitrophenyl)-6,7-dihydro-5H-cyclopentapyrimidin ist.

2. Verbindung gemäß Anspruch 1, wobei m 1 ist und n 1 ist.

3. Verbindung gemäß Anspruch 1, wobei m 1 ist und n 2 ist.

4. Verbindung gemäß Anspruch 1, wobei m 2 ist und n 1 ist.

5. Verbindung gemäß Anspruch 1, wobei m 2 ist und n 2 ist.

6. Verbindung gemäß Anspruch 1, wobei m 1 ist und n 3 ist.

7. Verbindung gemäß Anspruch 1, wobei m 3 ist und n 1 ist.

8. Verbindung gemäß Anspruch 1, wobei q 1 ist.

9. Verbindung gemäß Anspruch 1, wobei -N(R^{a})R^{b} Amino, Methylamino, Ethylamino, Isopropylamino, Dimethylamino, Diethylamino, Diisopropylamino, Cyclopropylamino, Cyclopentylamino, Piperidinyl, Pyrrolidinyl, Morpholinyl, Thiomorpholinyl oder Piperazinyl ist.

10. Verbindung gemäß Anspruch 1, wobei -N(R^{a})R^{b} Amino, Methylamino, Dimethylamino oder N-Methylpiperazinyl ist.

11. Verbindung gemäß Anspruch 1, wobei A >NR¹ ist.

12. Verbindung gemäß Anspruch 1, wobei R¹ ausgewählt ist aus der Gruppe bestehend aus Wasserstoff, Methyl, Ethyl, Isopropyl, Butyl, Hexyl, Cyclopropyl, Cyclobutyl, Cyclopentyl und Benzyl, jeweils gegebenenfalls mono-, di- oder trisubstituiert mit R^{e}.

13. Verbindung gemäß Anspruch 1, wobei R¹, gegebenenfalls R^{e}-substituiert, ausgewählt ist aus der Gruppe bestehend aus Wasserstoff, Methyl, Ethyl, Isopropyl und Benzyl.

14. Verbindung gemäß Anspruch 1, wobei R¹ Wasserstoff oder Methyl ist.

15. Verbindung gemäß Anspruch 1, wobei R³, gegebenenfalls substituiert, ausgewählt ist aus der Gruppe bestehend aus Methyl, Ethyl, Propyl, Isopropyl, Butyl, Methylen, Allyl und Benzyl.

16. Verbindung gemäß Anspruch 1, wobei zwei R³-Substituenten zusammen genommen Ethylen bilden.

17. Verbindung gemäß Anspruch 1, wobei R³ Methyl ist.

18. Verbindung gemäß Anspruch 1, wobei r 0, 1 oder 2 ist.

19. Verbindung gemäß Anspruch 1, wobei Ar, gegebenenfalls substituiert, ausgewählt ist aus der Gruppe bestehend aus:
a) Phenyl, 5-, 6-, 7-, 8-Benzo-1,4-dioxanyl, 4-, 5-, 6-, 7-Benzo-1,3-dioxolyl, 4-, 5-, 6-, 7-Indolinyl, 4-, 5-, 6-, 7-Isoindolinyl, 1,2,3,4-Tetrahydrochinolin-4, 5, 6 oder 7-yl, 1,2,3,4-Tetrahydroisochinolin-4, 5, 6 oder 7-yl,
b) Furanyl, Oxazolyl, Isoxazolyl, 1,2,3-Oxadiazolyl, 1,2,4-Oxadiazolyl, 1,2,5-Oxadiazolyl, 1,3,4-Oxadiazolyl, Thiophenyl, Thiazolyl, Isothiazolyl, Pyrrolyl, Imidazolyl, Pyrazolyl, 1,2,3-Triazolyl, 1,2,4-Triazolyl,
c) Pyridinyl, Pyridinyl-N-oxid, Pyrazinyl, Pyrimidinyl, Pyridazinyl, und
d) Biphenyl und 4-Tetrazolylphenyl.

20. Verbindung gemäß Anspruch 1, wobei Ar, gegebenenfalls substituiert, ausgewählt ist aus der Gruppe bestehend aus Phenyl, Pyridyl, Thiophen-2-yl und Thiophen-3-yl.

21. Verbindung gemäß Anspruch 1, wobei Ar ausgewählt ist aus der Gruppe bestehend aus Phenyl, 2-Methoxyphenyl, 3-Methoxyphenyl, 4-Methoxyphenyl, 2-Methylphenyl, 3-Methylphenyl, 4-Methylphenyl, 4-Ethylphenyl, 2-Chlorphenyl, 3-Chlorphenyl, 4-Chlorphenyl, 2-Fluorphenyl, 3-Fluorphenyl, 4-Fluorphenyl, 2-Bromphenyl, 3-Bromphenyl, 4-Bromphenyl, 2-Trifluormethylphenyl, 3-Trifluormethylphenyl, 4-Trifluormethylphenyl, 3-Trifluormethoxyphenyl, 4-Trifluormethoxyphenyl, 3-Cyanophenyl, 4-Cyanophenyl, 3-Acetylphenyl, 4-Acetylphenyl, 3,4-Difluorphenyl, 3,4-Dichlorphenyl, 2,3-Difluorphenyl, 2,3-Dichlorphenyl, 2,4-Difluorphenyl, 2,4-Dichlorphenyl, 3-Nitrophenyl, 4-Nitrophenyl, 3-Chlor-4-fluorphenyl, 3-Fluor-4-chlorphenyl, Benzo[1,3]dioxol-4 oder 5-yl, 3-Hydroxyphenyl, 4-Hydroxyphenyl, 4-Hydroxy-2-methylphenyl, 4-Hydroxy-3-fluorphenyl, 3,4-Dihydroxyphenyl, 4-Dimethylaminophenyl, 4-Carbamoylphenyl, 4-Fluor-3-methylphenyl, 2-Phenoxyphenyl, Furan-2-yl, Furan-3-yl, 5-Methylfuran-2-yl, Thiophen-2-yl, Thiophen-3-yl, 5-Chlorthiophen-2-yl, 5-Methylthiophen-2-yl, 5-Chlorthiophen-3-yl, 5-Methylthiophen-3-yl, Oxazol-2-yl, 4,5-Dimethyloxazol-2-yl, Thiazol-2-yl, 3H-[1,2,3]Triazol-4-yl, 2H-Pyrazol-3-yl, 1H-Pyrazol-4-yl, 4-Pyridyl, 5-Fluorpyridin-2-yl, 4'-Chlorbiphenyl und 4-Tetrazolylphenyl.

22. Verbindung gemäß Anspruch 1, wobei ALK, gegebenenfalls substituiert, ausgewählt ist aus der Gruppe bestehend aus Methylen, Ethylen, Propylen, Butylen, sec-Butylen, tert-Butylen, Pentylen, 1-Ethylpropylen, 2-Ethylpropylen, 2-Ethylbutylen, Isopropylen, But-3-enylen, Isobutylen, 3-Methylbutylen, Allylen, Prop-2-inylen, Cyclopropylen, Cyclobutylen, Cyclopentylen, Cyclohexylen und Cycloheptylen.

23. Verbindung gemäß Anspruch 1, wobei ALK ausgewählt ist aus der Gruppe bestehend aus Methylen, Hydroxymethylen, Fluormethylen, Difluormethylen, Trifluormethylmethylen, 2,2,2-Trifluor-1-trifluormethylethylen, Methoxycarbonylmethyl, Methylcarbamoylmethyl, Ethylen, 2-Dimethylaminoethylen, 2-Cyanoethylen, 2-Methoxyethylen, 1-Carboxyethylen, Propylen, 3-Methoxycarbonylpropylen, 3-Carboxypropylen, Isopropylen, 1-Fluor-1-methylethylen, 1-Hydroxy-1-methylethylen, 1-Carboxy-1-methylethylen, 1-Ethylpropylen, 2-Ethylpropylen, Butylen, tert-Butylen, sec-Butylen, Isobutylen, 4-Hydroxybutylen, 4-Methoxycarbonylbutylen, 4-Carboxybutylen, 2-Ethylbutylen, Isobutylen, 3-Methylbutylen, Prop-2-inylen, But-3-enylen, Pentylen, 5-Hydroxypentylen, Cyclopropylen, Cyclobutylen, Cyclopentylen, Cyclopentenylen, 3,3-Difluorcyclopentylen, 3-Hydroxycyclohexylen, 4-Fluorcyclohexylen, 4,4-Difluorcyclohexylen und 1-Methylcyclopropylen.

24. Verbindung gemäß Anspruch 1, wobei CYC, gegebenenfalls substituiert, Wasserstoff ist oder ein Ringsystem ist, ausgewählt aus der Gruppe bestehend aus:
i) Phenyl, 5-, 6-, 7-, 8-Benzo-1,4-dioxanyl, 4-, 5-, 6-, 7-Benzo-1,3-dioxolyl, 4-, 5-, 6-, 7-Indolinyl, 4-, 5-, 6-, 7-Isoindolinyl, 1,2,3,4-Tetrahydrochinolin-4, 5, 6 oder 7-yl, 1,2,3,4-Tetrahydroisochinolin-4, 5, 6 oder 7-yl,
ii) Furanyl, Oxazolyl, Isoxazolyl, 1,2,3-Oxadiazolyl, 1,2,4-Oxadiazolyl, 1,2,5-Oxadiazolyl, 1,3,4-Oxadiazolyl, Thiophenyl, Thiazolyl, Isothiazolyl, Pyrrolyl, Imidazolyl, Pyrazolyl, 1,2,3-Triazolyl, 1,2,4-Triazolyl,
iii) Pyridinyl, Pyridinyl-N-oxid, Pyrazinyl, Pyrimidinyl, Pyridazinyl, und
iv) Pyrrolinyl, Pyrrolidinyl, Pyrazolinyl, Piperidinyl, Homopiperidinyl, Azepanyl, Tetrahydrofuranyl, Tetrahydropyranyl, Piperazinyl, Morpholinyl, Thiomorpholinyl und Piperidinonyl.

25. Verbindung gemäß Anspruch 1, wobei CYC, gegebenenfalls substituiert, ausgewählt ist aus der Gruppe bestehend aus Wasserstoff, Phenyl, Thiophen-2-yl, Thiophen-3-yl, Furan-2-yl, Furan-3-yl, Pyridinyl, Piperidin-1, 2, 3 oder 4-yl, 2-Pyrrolin-2, 3, 4 oder 5-yl, 3-Pyrrolin-2 oder 3-yl, 2-Pyrazolin-3, 4 oder 5-yl, Tetrahydrofuran-3-yl, Tetrahydropyran-4-yl, Morpholin-2, 3 oder 4-yl, Thiomorpholin-2, 3 oder 4-yl, Piperazin-1, 2, 3 oder 4-yl, Pyrrolidin-1, 2 oder 3-yl und Homopiperidinyl.

26. Verbindung gemäß Anspruch 1, wobei CYC, gegebenenfalls substituiert, ausgewählt ist aus der Gruppe bestehend aus Wasserstoff, Phenyl, Pyridyl, Thiophen-2-yl, Thiophen-3-yl, Tetrahydropyranyl, Furan-2-yl, Furan-3-yl, Tetrahydrofuran-3-yl und Piperidinyl.

27. Verbindung gemäß Anspruch 1, wobei CYC ausgewählt ist aus der Gruppe bestehend aus Wasserstoff, Phenyl, 2-Methoxyphenyl, 3-Methoxyphenyl, 4-Methoxyphenyl, 2-Methylphenyl, 3-Methylphenyl, 4-Methylphenyl, 4-Ethylphenyl, 2-Chlorphenyl, 3-Chlorphenyl, 4-Chlorphenyl, 2-Fluorphenyl, 3-Fluorphenyl, 4-Fluorphenyl, 2-Bromphenyl, 3-Bromphenyl, 4-Bromphenyl, 2-Trifluormethylphenyl, 3-Trifluormethylphenyl, 4-Trifluormethylphenyl, 3-Trifluormethoxyphenyl, 4-Trifluormethoxyphenyl, 2-Cyanophenyl, 3-Cyanophenyl, 4-Cyanophenyl, 3-Acetylphenyl, 4-Acetylphenyl, 3,4-Difluorphenyl, 3,4-Dichlorphenyl, 2,3-Difluorphenyl, 2,3-Dichlorphenyl, 2,4-Difluorphenyl, 2,4-Dichlorphenyl, 2,6-Difluorphenyl, 2,6-Dichlorphenyl, 2,6-Dimethylphenyl, 2,4,6-Trifluorphenyl, 2,4,6-Trichlorphenyl, 3,4,5-Trimethoxyphenyl, 4-Fluor-3-methylphenyl, 3-Nitrophenyl, 4-Nitrophenyl, 4-Methyl-3-fluorphenyl, 3,4-Dimethylphenyl, 4-Methoxy-3-fluorphenyl, 4-Methoxy-2-methylphenyl, 3-Aminophenyl, 4-Aminophenyl, 4-Carbomethoxyphenyl, 3-Methansulfonylaminophenyl, 4-Methansulfonylaminophenyl, 3-Dimethansulfonylaminophenyl, 4-Dimethansulfonylaminophenyl, Thiophen-2-yl, Thiophen-3-yl, 5-Chlorthiophen-2-yl, Benzo[1,3]dioxol-4 oder 5-yl, Tetrahydrofuran-3-yl, Tetrahydropyran-2, 3 oder 4-yl, Furan-2-yl, Furan-3-yl, 5-Carboxyethylfuran-2-yl, Piperidinyl, 3,4-Bisbenzyloxyphenyl, 2-Hydroxyphenyl, 3-Hydroxyphenyl, 4-Hydroxyphenyl, 4-Hydroxy-2-methylphenyl, 4-Hydroxy-3-fluorphenyl, 3,4-Dihydroxyphenyl, 1-Piperidinyl, 4-Piperidinyl und 1-Methyl-4-piperidinyl.

28. Verbindung, ausgewählt aus der Gruppe bestehend aus:
2-tert-Butyl-4-(4-fluorphenyl)-5,6,7,8-tetrahydropyrido[4,3-d]pyrimidin;
2-Benzyl-4-(4-fluorphenyl)-5,6,7,8-tetrahydropyrido[4,3-d]pyrimidin;
2-sec-Butyl-4-(4-fluorphenyl)-5,6,7,8-tetrahydropyrido[4,3-d]pyrimidin-hydrochlorid;
2-sec-Butyl-4-p-tolyl-5,6,7,8-tetrahydropyrido[4,3-d]pyrimidin-hydrochlorid;
2-Cyclobutyl-4-(4-fluorphenyl)-5,6,7,8-tetrahydropyrido[4,3-d]pyrimidin-hydrochlorid;
2-Cyclobutyl-4-p-tolyl-5,6,7,8-tetrahydropyrido[4,3-d]pyrimidin;
2-Cyclopropyl-4-(4-fluorphenyl)-5,6,7,8-tetrahydropyrido[4,3-d]pyrimidin;
2-Benzyl-4-p-tolyl-5,6,7,8-tetrahydropyrido[4,3-d]pyrimidin;
2-Benzyl-4-(4-trifluormethylphenyl)-5,6,7,8-tetrahydropyrido[4,3-d]pyrimidin;
2-Benzyl-4-(3,4-difluorphenyl)-5,6,7,8-tetrahydropyrido[4,3-d]pyrimidin;
2-Benzyl-4-phenyl-5,6,7,8-tetrahydropyrido[4,3-d]pyrimidin;
2-Benzyl-4-(3-fluorphenyl)-5,6,7,8-tetrahydropyrido[4,3-d]pyrimidin;
2-(4-Fluorbenzyl)-4-(4-fluorphenyl)-5,6,7,8-tetrahydropyrido[4,3-d]pyrimidin;
2-(4-Fluorbenzyl)-4-(4-fluorphenyl)-6-methyl-5,6,7,8-tetrahydropyrido[4,3-d]pyrimidin;
4-[2-(4-Fluorbenzyl)-6-methyl-5,6,7,8-tetrahydropyrido[4,3-d]pyrimidin-4-yl]benzonitril;
4-[2-(4-Fluorbenzyl)-5,6,7,8-tetrahydropyrido[4,3-d]pyrimidin-4-yl]benzonitril;
2-Cyclopentyl-4-(4-fluorphenyl)-5,6,7,8-tetrahydropyrido[4,3-d]pyrimidin;
2-Cyclopentyl-4-p-tolyl-5,6,7,8-tetrahydropyrido[4,3-d]pyrimidin;
2-Cyclopentyl-4-(4-methoxyphenyl)-5,6,7,8-tetrahydropyrido[4,3-d]pyrimidin;
4-(2-Cyclopentyl-5,6,7,8-tetrahydropyrido[4,3-d]pyrimidin-4-yl)benzonitril;
4-(4-Fluorphenyl)-2-isopropyl-5,6,7,8-tetrahydropyrido[4,3-d]pyrimidin-hydrochlorid;
4-(4-Fluorphenyl)-2-isopropyl-6-methyl-5,6,7,8-tetrahydropyrido[4,3-d]pyrimidin;
4-(3,4-Dichlorphenyl)-2-isopropyl-5,6,7,8-tetrahydropyrido[4,3-d]pyrimidin-hydrochlorid;
4-(3,4-Difluorphenyl)-2-isopropyl-5,6,7,8-tetrahydropyrido[4,3-d]pyrimidin-hydrochlorid;
4-(3-Fluorphenyl)-2-isopropyl-5,6,7,8-tetrahydropyrido[4,3-d]pyrimidin;
4-(2-Fluorphenyl)-2-isopropyl-5,6,7,8-tetrahydropyrido[4,3-d]pyrimidin;
4-(2,4-Difluorphenyl)-2-isopropyl-5,6,7,8-tetrahydropyrido[4,3-d]pyrimidin;
2-Isopropyl-4-p-tolyl-5,6,7,8-tetrahydropyrido[4,3-d]pyrimidin;
4-(4-Chlorphenyl)-2-isopropyl-5,6,7,8-tetrahydropyrido[4,3-d]pyrimidin;
2-Isopropyl-4-(4-methoxyphenyl)-5,6,7,8-tetrahydropyrido[4,3-d]pyrimidin;
2-Isopropyl-4-phenyl-5,6,7,8-tetrahydropyrido[4,3-d]pyrimidin;
2-Isopropyl-4-(4-trifluormethylphenyl)-5,6,7,8-tetrahydropyrido[4,3-d]pyrimidin;
2-Isopropyl-4-(2-phenoxyphenyl)-5,6,7,8-tetrahydropyrido[4,3-d]pyrimidin;
2-Isobutyl-4-thiophen-3-yl-5,6,7,8-tetrahydropyrido[4,3-d]pyrimidin;
2-Isobutyl-4-thiophen-2-yl-5,6,7,8-tetrahydropyrido[4,3-d]pyrimidin;
2-Isobutyl-4-pyridin-4-yl-5,6,7,8-tetrahydropyrido[4,3-d]pyrimidin;
4-(4-Fluorphenyl)-2-isobutyl-5,6,7,8-tetrahydropyrido[4,3-d]pyrimidin;
2-Isobutyl-4-p-tolyl-5,6,7,8-tetrahydropyrido[4,3-d]pyrimidin;
4-(4-Fluor-3-methylphenyl)-2-isobutyl-5,6,7,8-tetrahydropyrido[4,3-d]pyrimidin;
4-(2-Isobutyl-5,6,7,8-tetrahydropyrido[4,3-d]pyrimidin-4-yl)-benzonitril;
2-Isobutyl-4-(4-methoxyphenyl)-5,6,7,8-tetrahydropyrido[4,3-d]pyrimidin;
2-sec-Butyl-4-(2-fluorphenyl)-5,6,7,8-tetrahydropyrido[4,3-d]pyrimidin-hydrochlorid;
2-sec-Butyl-4-(3-fluorphenyl)-5,6,7,8-tetrahydropyrido[4,3-d]pyrimidin;
2-sec-Butyl-4-(4-methoxyphenyl)-5,6,7,8-tetrahydropyrido[4,3-d]pyrimidin;
2-sec-Butyl-4-(4-trifluormethoxyphenyl)-5,6,7,8-tetrahydropyrido[4,3-d]pyrimidin;
2-Cyclopentyl-4-(4-fluorphenyl)-6,7,8,9-tetrahydro-5H-pyrimido[4,5-d]azepin;
2-Cyclopentyl-4-p-tolyl-6,7,8,9-tetrahydro-5H-pyrimido[4,5-d]azepin;
2-Cyclopentyl-4-(4-methoxyphenyl)-6,7,8,9-tetrahydro-5H-pyrimido[4,5-d]azepin;
4-(2-Cyclopentyl-6,7,8,9-tetrahydro-5H-pyrimido[4,5-d]azepin-4-yl)benzonitril;
4-(4-Fluorphenyl)-2-isopropyl-6,7,8,9-tetrahydro-5H-pyrimido[4,5-d]azepin-hydrochlorid;
4-(4-Chlorphenyl)-2-methyl-6,7,8,9-tetrahydro-5H-pyrimido[4,5-d]azepin;
2-Methyl-4-phenyl-6,7,8,9-tetrahydro-5H-pyrimido[4,5-d]azepin;
4-(3-Chlorphenyl)-2-methyl-6,7,8,9-tetrahydro-5H-pyrimido[4,5-d]azepin;
2-Benzyl-4-(4-fluorphenyl)-6,7,8,9-tetrahydro-5H-pyrimido[4,5-d]azepin;
2-Benzyl-4-p-tolyl-6,7,8,9-tetrahydro-5H-pyrimido[4,5-d]azepin;
2-Benzyl-4-(4-trifluormethylphenyl)-6,7,8,9-tetrahydro-5H-pyrimido[4,5-d]azepin;
2-(4-Fluorbenzyl)-4-(4-fluorphenyl)-6,7,8,9-tetrahydro-5H-pyrimido[4,5-d]azepin;
2-Cyclopentyl-4-(4-fluorphenyl)-7-methyl-6,7,8,9-tetrahydro-5H-pyrimido[4,5-d]azepin;
2-Cyclopentyl-7-methyl-4-p-tolyl-6,7,8,9-tetrahydro-5H-pyrimido[4,5-d]azepin;
2-Cyclopentyl-4-(4-methoxyphenyl)-7-methyl-6,7,8,9-tetrahydro-5H-pyrimido[4,5-d]azepin;
2-Benzyl-7-methyl-4-p-tolyl-6,7,8,9-tetrahydro-5H-pyrimido[4,5-d]azepin;
2-(4-Fluorbenzyl)-4-(4-fluorphenyl)-7-methyl-6,7,8,9-tetrahydro-5H-pyrimido[4,5-d]azepin;
2-(4-Fluorbenzyl)-4-(4-fluorphenyl)-7-methyl-9-methylen-6,7,8,9-tetrahydro-5H-pyrimido[4,5-d]azepin;
2-Benzyl-4-(4-fluorphenyl)-6,7,8,9-tetrahydro-5H-pyrimido[4,5-c]azepin-hydrochlorid;
4-(4-Fluorphenyl)-2-isopropyl-6,7,8,9-tetrahydro-5H-pyrimido[4,5-c]azepin-hydrochlorid;
2-Isopropyl-4-p-tolyl-6,7,8,9-tetrahydro-5H-pyrimido[4,5-c]azepin-hydrochlorid;
2-Isopropyl-4-(4-methoxyphenyl)-6,7,8,9-tetrahydro-5H-pyrimido[4,5-c]azepin;
2-Isopropyl-4-phenyl-6,7,8,9-tetrahydro-5H-pyrimido[4,5-c]azepin;
2-Benzyl-4-(4-fluorphenyl)-6,7,8,9-tetrahydro-5H-1,3,6-triazabenzocyclohepten-hydrochlorid;
2,7-Dibenzyl-4-(4-fluorphenyl)-5,6,7,8-tetrahydropyrido[3,4-d]pyrimidin-hydrochlorid;
2,7-Dibenzyl-4-p-tolyl-5,6,7,8-tetrahydropyrido[3,4-d]pyrimidin;
2,7-Dibenzyl-4-phenyl-5,6,7,8-tetrahydropyrido[3,4-d]pyrimidin;
2,7-Dibenzyl-4-(4-methoxyphenyl)-5,6,7,8-tetrahydropyrido[3,4-d]pyrimidin;
7-Benzyl-4-(4-fluorphenyl)-2-isopropyl-5,6,7,8-tetrahydropyrido[3,4-d]pyrimidin;
7-Benzyl-2-isopropyl-4-phenyl-5,6,7,8-tetrahydropyrido[3,4-d]pyrimidin;
2-Benzyl-4-(4-fluorphenyl)-5,6,7,8-tetrahydropyrido[3,4-d]pyrimidin-hydrochlorid;
2-Benzyl-4-p-tolyl-5,6,7,8-tetrahydropyrido[3,4-d]pyrimidin-hydrochlorid;
2-Benzyl-4-phenyl-5,6,7,8-tetrahydropyrido[3,4-d]pyrimidin;
2-Benzyl-4-(4-methoxyphenyl)-5,6,7,8-tetrahydropyrido[3,4-d]pyrimidin;
4-(4-Fluorphenyl)-2-isopropyl-5,6,7,8-tetrahydropyrido[3,4-d]pyrimidin-hydrochlorid;
2-Isopropyl-4-phenyl-5,6,7,8-tetrahydropyrido[3,4-d]pyrimidin;
2-Benzyl-4-(4-fluorphenyl)-7-methyl-5,6,7,8-tetrahydropyrido[3,4-d]pyrimidin-hydrochlorid;
2-Benzyl-4-(4-fluorphenyl)-7-isopropyl-5,6,7,8-tetrahydropyrido[3,4-d]pyrimidin;
4-(4-Fluorphenyl)-2-isopropyl-7-methyl-5,6,7,8-tetrahydropyrido[3,4-d]pyrimidin-hydrochlorid;
2-Isopropyl-7-methyl-4-phenyl-5,6,7,8-tetrahydropyrido[3,4-d]pyrimidin;
7-Benzyl-2-isopropyl-4-(5-methylthiophen-3-yl)-5,6,7,8-tetrahydropyrido[3,4-d]pyrimidin-Hydrochlorid;
7-Benzyl-2-isopropyl-4-thiophen-3-yl-5,6,7,8-tetrahydropyrido[3,4-d]pyrimidin-hydrochlorid;
2-Isopropyl-4-(5-methylthiophen-3-yl)-5,6,7,8-tetrahydropyrido[3,4-d]pyrimidin-hydrochlorid;
2-Isopropyl-4-thiophen-3-yl-5,6,7,8-tetrahydropyrido[3,4-d]pyrimidin-hydrochlorid;
2-Isopropyl-7-methyl-4-(5-methylthiophen-3-yl)-5,6,7,8-tetrahydropyrido[3,4-d]pyrimidin-hydrochlorid;
2-Isopropyl-7-methyl-4-thiophen-3-yl-5,6,7,8-tetrahydropyrido[3,4-d]pyrimidin-hydrochlorid;
6-Benzyl-4-(4-fluorphenyl)-2-isopropyl-8-methyl-5,6,7,8-tetrahydropyrido[4,3-d]pyrimidin;
6-Benzyl-4-(3-chlor-4-fluorphenyl)-2-isopropyl-8-methyl-5,6,7,8-tetrahydropyrido[4,3-d]pyrimidin;
6-Benzyl-2-isopropyl-8-methyl-4-p-tolyl-5,6,7,8-tetrahydropyrido[4,3-d]pyrimidin;
6-Benzyl-2-isopropyl-8-methyl-4-phenyl-5,6,7,8-tetrahydropyrido[4,3-d]pyrimidin;
6-Benzyl-2-isopropyl-8-methyl-4-(4-trifluormethylphenyl)-5,6,7,8-tetrahydropyrido[4,3-d]pyrimidin;
6-Benzyl-4-(4-chlorphenyl)-2-isopropyl-8-methyl-5,6,7,8-tetrahydropyrido[4,3-djpyrimidin;
6-Benzyl-2-isopropyl-8-methyl-4-thiophen-3-yl-5,6,7,8-tetrahydropyrido[4,3-d]pyrimidin;
6-Benzyl-4-(4'-chlorbiphenyl-4-yl)-2-isopropyl-8-methyl-5,6,7,8-tetrahydropyrido[4,3-d]pyrimidin;
4-(4-Fluorphenyl)-2-isopropyl-8-methyl-5,6,7,8-tetrahydropyrido[4,3-d]pyrimidin-hydrochlorid;
4-(3-Chlor-4-fluorphenyl)-2-isopropyl-8-methyl-5,6,7,8-tetrahydropyrido[4,3-d]pyrimidin-hydrochlorid;
2-Isopropyl-8-methyl-4-p-tolyl-5,6,7,8-tetrahydropyrido[4,3-d]pyrimidin-hydrochlorid;
2-Isopropyl-8-methyl-4-phenyl-5,6,7,8-tetrahydropyrido[4,3-d]pyrimidin;
2-Isopropyl-8-methyl-4-(4-trifluormethylphenyl)-5,6,7,8-tetrahydropyrido[4,3-d]pyrimidin;
2-Isopropyl-8-methyl-4-thiophen-3-yl-5,6,7,8-tetrahydropyrido[4,3-d]pyrimidin;
4-(4-Fluorphenyl)-2-isopropyl-6,7-dihydro-5H-pyrrolo[3,4-d]pyrimidin-hydrochlorid;
4-(4-Fluorphenyl)-2-isopropyl-7-pyrrolidin-1-yl-5,6,7,8-tetrahydrochinazolin;
[4-(4-Fluorphenyl)-2-isopropyl-5,6,7,8-tetrahydrochinazolin-7-yl]methylamin-hydrochlorid;
[4-(4-Fluorphenyl)-2-isopropyl-5,6,7,8-tetrahydrochinazolin-6-yl]methylamin-hydrochlorid;
4-(4-Fluorphenyl)-2-isopropyl-5,6,7,8-tetrahydrochinazolin-7-ol;
4-(4-Fluorphenyl)-2-isopropyl-5,6,7,8-tetrahydrochinazolin;
(2-Benzyl-6-p-tolylpyrimidin-4-ylmethyl)dimethylamin;
2-Benzyl-4-(4-methylpiperazin-1-ylmethyl)-6-p-tolylpyrimidin;
[6-(4-Fluorphenyl)-2-isopropylpyrimidin-4-ylmethyl]methylamin;
2-(2-Benzyl-6-p-tolylpyrimidin-4-yl)ethylamin;
[2-(4-Fluorbenzyl)-4-p-tolylpyrimidin-5-ylmethyl]dimethylamin;
4-(4-Fluorphenyl)-2-phenyl-5,6,7,8-tetrahydropyrido[4,3-d]pyrimidin;
2-(3,3-Difluorcyclopentyl)-4-(4-fluorphenyl)-5,6,7,8-tetrahydropyrido[4,3-d]pyrimidin;
4-(4-Fluorphenyl)-2-(tetrahydrofuran-3-yl)-5,6,7,8-tetrahydropyrido[4,3-d]pyrimidin;
4-(4-Fluorphenyl)-2-(2-piperidin-1-ylethyl)-5,6,7,8-tetrahydropyrido[4,3-d]pyrimidin;
2-(1-Fluor-1-methylethyl)-4-(4-fluorphenyl)-5,6,7,8-tetrahydropyrido[4,3-d]pyrimidin;
3-(4-Fluorphenyl)-5-isopropyl-4,6,12-triaza-tricyclo[7.2.1.0^{2.7}]dodeca-2,4,6-trien;
7-(4-Fluorphenyl)-5-isopropyl-4,6,13-triaza-tricyclo[8.2.1.0^{3.8}]trideca-3,5,7-trien;
4-(4-Fluorphenyl)-2-(tetrahydropyran-4-yl)-5,6,7,8-tetrahydropyrido[4,3-d]pyrimidin;
4-(4-Fluorphenyl)-2-(tetrahydropyran-3-yl)-5,6,7,8-tetrahydropyrido[4,3-d]pyrimidin;
4-(4-Fluorphenyl)-2-(2-methoxyethyl)-5,6,7,8-tetrahydropyrido[4,3-d]pyrimidin;
2-[4-(4-Fluorphenyl)-5,6,7,8-tetrahydropyrido[4,3-d]pyrimidin-2-yl]propan-2-ol;
4-(4-Fluorphenyl)-2-(1-methyl-1-phenylethyl)-5,6,7,8-tetrahydropyrido[4,3-d]pyrimidin;
2-Cyclopent-3-enyl-4-(4-fluorphenyl)-5,6,7,8-tetrahydropyrido[4,3-d]pyrimidin;
3-[4-(4-Fluorphenyl)-5,6,7,8-tetrahydropyrido[4,3-d]pyrimidin-2-yl]cyclohexanol;
4-(4-Fluorphenyl)-2-piperidin-4-yl-5,6,7,8-tetrahydropyrido[4,3-d]pyrimidin;
4-(4-Fluorphenyl)-2-(1-methylpiperidin-4-yl)-5,6,7,8-tetrahydropyrido[4,3-d]pyrimidin;
[4-(4-Fluorphenyl)-5,6,7,8-tetrahydropyrido[4,3-d]pyrimidin-2-yl]phenylmethanol;
4-(4-Fluorphenyl)-2-(fluorphenylmethyl)-5,6,7,8-tetrahydropyrido[4,3-d]pyrimidin;
2-(Difluorphenylmethyl)-4-(4-fluorphenyl)-5,6,7,8-tetrahydropyrido[4,3-d]pyrimidin;
4-(4-Fluorphenyl)-2-phenyl-5,6,7,8-tetrahydropyrido[4,3-d]pyrimidin;
4-(4-Fluorphenyl)-2-(3-fluorphenyl)-5,6,7,8-tetrahydropyrido[4,3-d]pyrimidin;
4-(4-Fluorphenyl)-2-(4-methoxyphenyl)-5,6,7,8-tetrahydropyrido[4,3-d]pyrimidin;
4-(4-Fluorphenyl)-2-o-tolyl-5,6,7,8-tetrahydropyrido[4,3-d]pyrimidin;
3-[4-(4-Fluorphenyl)-5,6,7,8-tetrahydropyrido[4,3-d]pyrimidin-2-yl]benzonitril;
4-(4-Fluorphenyl)-2-(2,2,2-trifluor-1-trifluormethylethyl)-5,6,7,8-tetrahydropyrido[4,3-d]pyrimidin;
4-(4-Fluorphenyl)-2-(1-methylcyclopropyl)-5,6,7,8-tetrahydropyrido[4,3-d]pyrimidin;
2-[4-(4-Fluorphenyl)-5,6,7,8-tetrahydropyrido[4,3-d]pyrimidin-2-yl]-2-methylpropionsäure;
2-[4-(4-Fluorphenyl)-5,6,7,8-tetrahydropyrido[4,3-d]pyrimidin-2-yl]propionsäure;
2-(4-Fluorcyclohexyl)-4-(4-fluorphenyl)-5,6,7,8-tetrahydropyrido[4,3-d]pyrimidin;
2-(4,4-Difluorcyclohexyl)-4-(4-fluorphenyl)-5,6,7,8-tetrahydropyrido[4,3-d]pyrimidin;
4-(4-Fluorphenyl)-2-phenethyl-5,6,7,8-tetrahydropyrido[4,3-d]pyrimidin;
4-Furan-2-yl-2-isopropyl-5,6,7,8-tetrahydropyrido[4,3-d]pyrimidin;
2-Isopropyl-4-(5-methylfuran-2-yl)-5,6,7,8-tetrahydropyrido[4,3-d]pyrimidin;
4-Furan-3-yl-2-isopropyl-5,6,7,8-tetrahydropyrido[4,3-d]pyrimidin;
4-(5-Fluorpyridin-2-yl)-2-isopropyl-5,6,7,8-tetrahydropyrido[4,3-d]pyrimidin;
2-Isopropyl-4-oxazol-2-yl-5,6,7,8-tetrahydropyrido[4,3-d]pyrimidin;
4-(4,5-Dimethyloxazol-2-yl)-2-isopropyl-5,6,7,8-tetrahydropyrido[4,3-d]pyrimidin;
2-Isopropyl-4-thiazol-2-yl-5,6,7,8-tetrahydropyrido[4,3-d]pyrimidin;
2-Isopropyl-4-(3H-[1,2,3]triazol-4-yl)-5,6,7,8-tetrahydropyrido[4,3-d]pyrimidin;
2-Isopropyl-4-(2H-pyrazol-3-yl)-5,6,7,8-tetrahydropyrido[4,3-d]pyrimidin;
2-Isopropyl-4-(1H-pyrazol-4-yl)-5,6,7,8-tetrahydropyrido[4,3-d]pyrimidin;
4-(4-Fluorphenyl)-2,6-diisopropyl-5,6,7,8-tetrahydropyrido[4,3-d]pyrimidin;
6-Ethyl-4-(4-fluorphenyl)-2-isopropyl-5,6,7,8-tetrahydropyrido[4,3-d]pyrimidin;
6-Cyclopropyl-4-(4-fluorphenyl)-2-isopropyl-5,6,7,8-tetrahydropyrido[4,3-d]pyrimidin;
6-Cyclobutyl-4-(4-fluorphenyl)-2-isopropyl-5,6,7,8-tetrahydropyrido[4,3-d]pyrimidin;
6-Cyclopentyl-4-(4-fluorphenyl)-2-isopropyl-5,6,7,8-tetrahydropyrido[4,3-d]pyrimidin;
6-Butyl-4-(4-fluorphenyl)-2-isopropyl-5,6,7,8-tetrahydropyrido[4,3-d]pyrimidin; und
4-(4-Fluorphenyl)-2-isopropyl-5,6,7,8-tetrahydropyrido[4,3-d]pyrimidin-Citratsalz.

29. Verbindung gemäß Anspruch 1, wobei das pharmazeutisch verträgliche Salz ein wirksames Aminoadditionssalz ist.

30. Verbindung gemäß Anspruch 1, wobei das pharmazeutisch verträgliche Salz ausgewählt ist aus der Gruppe bestehend aus Hydrobromid, Hydrochlorid, Sulfat, Bisulfat, Nitrat, Acetat, Oxalat, Valerat, Oleat, Palmitat, Stearat, Laurat, Borat, Benzoat, Lactat, Phosphat, Tosylat, Citrat, Maleat, Fumarat, Succinat, Tartrat, Naphthylat, Mesylat, Glucoheptonat, Lactiobionat und Laurylsulfonat.

31. Pharmazeutische Zusammensetzung, umfassend einen pharmazeutisch verträglichen Träger und eine therapeutisch wirksame Menge von wenigstens einer Verbindung der Formeln (I) oder (II): 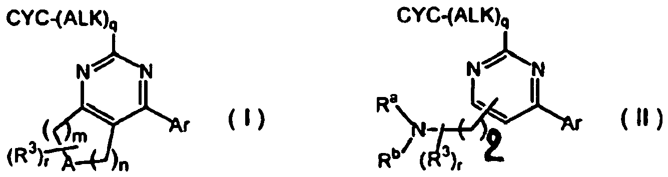 wobei
m 1, 2 oder 3 ist;
n 1, 2 oder 3 ist;
wobei wenn m und n beide vorhanden sind, m + n größer oder gleich 2 ist und kleiner oder gleich 4 ist;
R^{a} und R^{b} unabhängig -H, -C₁₋₇-Alkyl oder -C₃₋₇-Cycloalkyl sind oder R^{a} und R^{b} zusammen genommen mit dem Stickstoff, an den sie gebunden sind, Piperidinyl, Pyrrolidinyl, Morpholinyl, Thiomorpholinyl oder Piperazinyl bilden, wobei jedes R^{a} und R^{b} gegebenenfalls und unabhängig mit -C₁₋₄-Alkyl substituiert ist;
q 0 oder 1 ist;
A >NR¹, >CHNR^{c}R^{d}, >CHOH oder -CH₂- ist, wobei
R¹ ausgewählt ist aus der Gruppe bestehend aus -H, -C₁₋₇-Alkyl, -C₃₋₇-Cycloalkyl und Benzyl, wobei jedes Alkyl, Cycloalkyl oder Benzyl gegebenenfalls mit R^{e} mono-, di- oder trisubstituiert ist;
R^{e} ausgewählt ist aus der Gruppe bestehend aus -C₁-₄-Alkyl, -C₂₋₄-Alkenyl, -C₂₋₄-Alkinyl, -C₃₋₆-Cycloalkyl, Halogen, -CF₃, -OH, -OC₁₋₄-Alkyl, -OCF₃, -N(R^{f})R^{g} (wobei R^{f} und R^{g} unabhängig -H oder -C₁₋₄-Alkyl sind oder R^{f} und R^{g} zusammen genommen mit dem Stickstoff, an den sie gebunden sind, Piperidinyl, Pyrrolidinyl, Morpholinyl, Thiomorpholinyl oder Piperazinyl bilden), -C(O)N(R^{f})R^{g}, -N(R^{h})C(O)R^{h}, -N(R^{h})SO₂C₁₋₇-Alkyl (wobei R^{h} -H oder -C₁₋₄-Alkyl ist oder zwei R^{h} an dem gleichen Substituenten zusammen genommen mit dem Amid, an das sie gebunden sind, einen ansonsten aliphatischen 4- bis 6-gliedrigen Ring bilden), -S(O)₀₋₂-C₁₋₄-Alkyl, -SO₂N(R^{f})R^{g}, -SCF₃, -C(O)C₁₋₄-Alkyl, -CN, -COOH und -COOC₁₋₄-Alkyl ;
R^{c} und R^{d} unabhängig ausgewählt sind aus der Gruppe bestehend aus -H, -C₁₋₇-Alkyl, -C₃₋₇-Alkenyl, -C₃₋₇-Alkinyl, -C₃₋₇-Cycloalkyl, -C₁₋₇-Alkyl-C₃₋₇-cycloalkyl und -C₃₋₇-Cycloalkyl-C₁₋₇-alkyl, oder R^{c} und R^{d} zusammen genommen mit dem Stickstoff, an den sie gebunden sind, Piperidinyl, Pyrrolidinyl, Morpholinyl, Thiomorpholinyl oder Piperazinyl bilden, wobei jedes R^{c} und R^{d} gegebenenfalls und unabhängig mit R^{e} substituiert ist;
R³ -C₁₋₄-Alkyl, -C₁₋₄-Alkenyl oder Benzyl ist, jeweils gegebenenfalls substituiert mit -C₁₋₃-Alkyl, -OH oder Halogen, oder zwei R³-Substituenten zusammen genommen C₂₋₅-Alkylen bilden, gegebenenfalls substituiert mit -C₁₋₃-Alkyl, -OH oder Halogen;
r 0 ist oder eine ganze Zahl kleiner oder gleich m + n + 1 ist;
Ar ein Aryl- oder Heteroarylring ist, ausgewählt aus der Gruppe bestehend aus:
a) Phenyl, gegebenenfalls mono-, di- oder trisubstituiert mit Rⁱ oder an benachbarten Kohlenstoffen disubstituiert mit -OC₁₋₄-Alkylen-O-, -(CH₂)₂-₃NH-, -(CH₂)₁₋₂NH(CH₂)-, -(CH₂)₂-₃N (C₁₋₄-Alkyl)-oder -(CH₂)₁₋₂N(C₁₋₄-Alkyl)(CH₂)-;
R¹ ausgewählt ist aus der Gruppe bestehend aus -C₁-₇-Alkyl, -C₂₋₇-Alkenyl, -C₂₋₇-Alkinyl, -C₃₋₇-Cycloalkyl, Halogen, -CF₃, -OH, -OC₁₋₇-Alkyl, -OCF₃, -OC₃₋₇-Alkenyl, -OC₃₋₇-Alkinyl, -N(R^{j})R^{k} (wobei R^{j} und R^{k} unabhängig -H oder -C₁₋₄-Alkyl sind), -C(O)N(R^{j})R^{k}, -N(R^{j})C(O)R^{k}, -N(R^{j})SO₂C₁₋₆-Alkyl, -S(O)₀₋₂-C₁₋₆-Alkyl, -SO₂N(R^{j})R^{k}, -SCF₃, -C(O)C₁₋₆-Alkyl, -NO₂, -CN, -COOH und -COOC₁₋₇-Alkyl;
b) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu zwei zusätzliche Kohlenstoffatome gegebenenfalls durch -N= ersetzt sind, gegebenenfalls mono- oder disubstituiert mit Rⁱ;
c) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit Rⁱ; und
d) Phenyl oder Pyridyl, substituiert mit einem Substituenten, ausgewählt aus der Gruppe bestehend aus Phenyl, Phenoxy, Pyridyl, Thiophenyl, Oxazolyl und Tetrazolyl, wobei die erhaltene substituierte Einheit gegebenenfalls weiter mono-, di- oder trisubstituiert ist mit Rⁱ;
ALK ein verzweigtes oder nichtverzweigtes C₁₋₇-Alkylen, C₂₋₇-Alkenylen, C₂₋₇-Alkinylen, C₃₋₇-Cycloalkylen oder C₃₋₇-Cycloalkenylen ist, gegebenenfalls mono-, di- oder trisubstituiert mit R^{m};
R^{m} ausgewählt ist aus der Gruppe bestehend aus Halogen, -CF₃, -OH, -OC₁₋₇-Alkyl, -OC₃₋₇-Cycloalkyl, -OCF₃, -N(R^{p})R^{s} (wobei R^{p} und R^{s} unabhängig -H oder -C₁₋₇-Alkyl sind), -C(O)N(R^{p})R^{s}, -N(R^{t})C(O)R^{t}, -N(R^{t})SO₂C₁₋₆-Alkyl (wobei R^{t} -H oder -C₁₋₇-Alkyl ist), -S(0)₀₋₂-C₁₋₆-Alkyl, -SO₂N(R^{p})R^{s}, -SCF₃, -CN, -NO₂, -C(O)C₁₋₇-Alkyl, -COOH und -COOC₁₋₇-Alkyl;
CYC -H ist oder ein Ringsystem ist, ausgewählt aus der Gruppe bestehend aus:
i) Phenyl, gegebenenfalls mono-, di- oder trisubstituiert mit R^{u} oder an benachbarten Kohlenstoffen disubstituiert mit -OC₁₋₄-Alkylen-O-, -(CH₂)₂₋₃NH-, -(CH₂)₁₋₂NH(CH₂)-, -(CH₂)₂₋₃N(C₁₋₄-Alkyl)-oder -(CH₂)₁₋₂N(C₁₋₄-Alkyl)(CH₂)-;
R^{u} ausgewählt ist aus der Gruppe bestehend aus -C₁-₇-Alkyl), -C₃₋₇-Cycloalkyl, Phenyl, Benzyl, Halogen, -CF₃, -OH, -OC₁₋₇-Alkyl, -OC₃₋₇-Cycloalkyl, -OPhenyl, -OBenzyl, -OCF₃, -N (R^{v}) R^{w} (wobei R^{v} und R^{w} unabhängig -H oder -C₁₋₇-Alkyl sind oder R^{v} und R^{w} zusammen genommen mit dem Stickstoff, an den sie gebunden sind, Piperidinyl, Pyrrolidinyl, Morpholinyl, Thiomorpholinyl oder Piperazinyl bilden, wobei jedes R^{v} und R^{w} gegebenenfalls und unabhängig mit -OH oder -C₁₋₇-Alkyl substituiert ist), -C(O)N(R^{v})R^{w}, -N(R^{x})C(O)R^{x}, -N(R^{x})SO₂C₁₋₆-Alkyl (wobei R^{x} -H oder -C₁₋₇-Alkyl ist oder zwei R^{x} an dem gleichen Substituenten zusammen genommen mit dem Amid, an das sie gebunden sind, einen ansonsten aliphatischen 4- bis 6-gliedrigen Ring bilden), -N- (SO₂C₁₋₆-Alkyl)₂, -S (O)₀₋₂-C₁₋₆-Alkyl, -SO₂N(R^{v})R^{w}, -SCF₃, -C(O)C₁₋₆-Alkyl, -NO₂, -CN, -COOH und -COOC₁₋₇-Alkyl;
ii) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu einem zusätzlichen Kohlenstoffatom gegebenenfalls durch -N= ersetzt ist, gegebenenfalls mono- oder disubstituiert mit R^{u};
iii) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit R^{u}; und
iv) einem nichtaromatischen heterocyclischen Ring mit 4 bis 8 Gliedern, wobei der Ring 0, 1 oder 2 nichtbenachbarte Heteroatom-Glieder aufweist, ausgewählt aus der Gruppe bestehend aus O, S, -N=, >NH und >N (C₁₋₄-Alkyl) mit 0, 1 oder 2 Doppelbindungen, mit 0, 1 oder 2 Kohlenstoffgliedern, die ein Carbonyl sind, gegebenenfalls mit einem Kohlenstoffglied, das eine Brücke bildet, mit 0 bis 5 Substituenten R^{u}, und wobei, wenn q 0 ist, der Ring ein Kohlenstoffatom aufweist, das der Punkt der Befestigung ist;
und Enantiomere, Diastereomere, Hydrate, Solvate und pharmazeutisch verträgliche Salze, Ester und Amide davon;
mit der Maßgabe, dass in Formel (I):
(a) wenn ALK Methylen, Ethylen, Propylen oder Isopropylen ist, CYC -H ist, Ar Phenyl oder monosubstituiertes Phenyl ist, m 2 ist, n 1 ist und A >NR¹ ist, dann R¹ nicht -C₁₋₄-Alkyl oder Benzyl ist;
(b) wenn q 0 ist, CYC Phenyl ist, Ar Phenyl oder 3-Chlorphenyl ist, m 2 ist und n 1 ist, dann A nicht nichtsubstituiertes -CH₂- ist; und
(c) wenn q 0 ist, CYC 2-Pyridyl ist, Ar 2-Pyridyl ist, m 2 ist und n 1 ist, dann A nicht nichtsubstituiertes -CH₂- ist.

32. Verbindung der Formeln (I) oder (II): 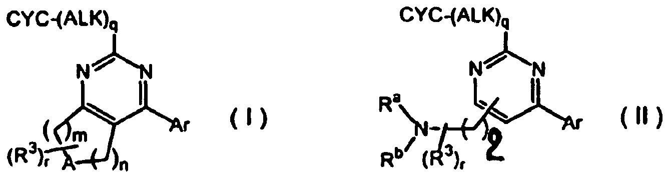 wobei
m 1, 2 oder 3 ist;
n 1, 2 oder 3 ist;
wobei wenn m und n beide vorhanden sind, m + n größer oder gleich 2 ist und kleiner oder gleich 4 ist;
R^{a} und R^{b} unabhängig -H, -C₁₋₇-Alkyl oder -C₃₋₇-Cycloalkyl sind oder R^{a} und R^{b} zusammen genommen mit dem Stickstoff, an den sie gebunden sind, Piperidinyl, Pyrrolidinyl, Morpholinyl, Thiomorpholinyl oder Piperazinyl bilden, wobei jedes R^{a} und R^{b} gegebenenfalls und unabhängig mit -C₁₋₄-Alkyl substituiert ist;
q 0 oder 1 ist;
A >NR¹, >CHNR^{c}R^{d}, >CHOH oder -CH₂- ist, wobei
R¹ ausgewählt ist aus der Gruppe bestehend aus -H, -C₁₋₇-Alkyl, -C₃₋₇-Cycloalkyl und Benzyl, wobei jedes Alkyl, Cycloalkyl oder Benzyl gegebenenfalls mit R^{e} mono-, di- oder trisubstituiert ist;
R^{e} ausgewählt ist aus der Gruppe bestehend aus -C₁₋₄-Alkyl, -C₂₋₄-Alkenyl, -C₂₋₄-Alkinyl, -C₃₋₆-Cycloalkyl, Halogen, -CF₃, -OH, -OC₁₋₄-Alkyl, -OCF₃, -N(R^{f})R^{g} (wobei R^{f} und R^{g} unabhängig -H oder -C₁₋₉-Alkyl sind oder R^{f} und R^{g} zusammen genommen mit dem Stickstoff, an den sie gebunden sind, Piperidinyl, Pyrrolidinyl, Morpholinyl, Thiomorpholinyl oder Piperazinyl bilden), -C(O)N(R^{f})R^{g}, -N(R^{h})C(O)R^{h}, -N(R^{h})SO₂C₁₋₇-Alkyl (wobei R^{h} -H oder -C₁₋₄-Alkyl ist oder zwei R^{h} an dem gleichen Substituenten zusammen genommen mit dem Amid, an das sie gebunden sind, einen ansonsten aliphatischen 4- bis 6-gliedrigen Ring bilden), -S (O)₀₋₂-C₁₋₄-Alkyl, -SO₂N (R^{f}) R^{g}, -SCF₃, -C(O)C₁₋₄-Alkyl, -CN, -COOH und -COOC₁₋₄-Alkyl;
R^{c} und R^{d} unabhängig ausgewählt sind aus der Gruppe bestehend aus -H, -C₁₋₇-Alkyl, -C₃₋₇-Alkenyl, -C₃₋₇-Alkinyl, -C₃₋₇-Cycloalkyl, -C₁₋₇-Alkyl-C₃₋₇-cycloalkyl und -C₃₋₇-Cycloalkyl-C₁₋₇-alkyl, oder R^{c} und R^{d} zusammen genommen mit dem Stickstoff, an den sie gebunden sind, Piperidinyl, Pyrrolidinyl, Morpholinyl, Thiomorpholinyl oder Piperazinyl bilden, wobei jedes R^{c} und R^{d} gegebenenfalls und unabhängig mit R^{e} substituiert ist;
R³ -C₁₋₄-Alkyl, -C₁₋₄-Alkenyl oder Benzyl ist, jeweils gegebenenfalls substituiert mit -C₁₋₃-Alkyl, -OH oder Halogen, oder zwei R³-Substituenten zusammen genommen C₂₋₅-Alkylen bilden, gegebenenfalls substituiert mit -C₁₋₃-Alkyl, -OH oder Halogen;
r 0 ist oder eine ganze Zahl kleiner oder gleich m + n + 1 ist;
Ar ein Aryl- oder Heteroarylring ist, ausgewählt aus der Gruppe bestehend aus:
a) Phenyl, gegebenenfalls mono-, di- oder trisubstituiert mit R¹ oder an benachbarten Kohlenstoffen disubstituiert mit -OC₁₋₉-Alkylen-O-, -(CH₂)₂-₃NH-, -(CH₂)₁₋₂NH(CH₂)-, -(CH₂)₂₋₃N(C₁₋₄-Alkyl)-oder -(CH₂)₁₋₂N(C₁₋₉-Alkyl)(CH₂)-;
R¹ ausgewählt ist aus der Gruppe bestehend aus -C₁₋₇-Alkyl, -C₂₋₇-Alkenyl, -C₂₋₇-Alkinyl, -C₃₋₇-Cycloalkyl, Halogen, -CF₃, -OH, -OC₁₋₇-Alkyl, -OCF₃, -OC₃₋₇-Alkenyl, -OC₃₋₇-Alkinyl, -N(R^{j})R^{k} (wobei R^{j} und R^{k} unabhängig -H oder C₁₋₄-Alkyl sind), -C(O)N(R^{j})R^{k}, -N(R^{j})C(O)R^{k} -N(R^{j})SO₂C₁₋₆-Alkyl, -S(O)₀₋₂C₁₋₆-Alkyl, -SO₂N(R^{j})R^{k}, -SCF₃, -C(O)C₁₋₆-Alkyl, -NO₂, -CN, -COOH und -COOC₁₋₇-Alkyl;
b) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N (C₁₋₄-Alkyl) ersetzt ist, wobei bis zu zwei zusätzliche Kohlenstoffatome gegebenenfalls durch -N= ersetzt sind, gegebenenfalls mono- oder disubstituiert mit Rⁱ ;
c) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit R¹; und
d) Phenyl oder Pyridyl, substituiert mit einem Substituenten, ausgewählt aus der Gruppe bestehend aus Phenyl, Phenoxy, Pyridyl, Thiophenyl, Oxazolyl und Tetrazolyl, wobei die erhaltene substituierte Einheit gegebenenfalls weiter mono-, di- oder trisubstituiert ist mit Rⁱ ;
ALK ein verzweigtes oder nichtverzweigtes C₁₋₇-Alkylen, C₂₋₇-Alkenylen, C₂₋₇-Alkinylen, C₃₋₇-Cycloalkylen oder C₃₋₇-Cycloalkenylen ist, gegebenenfalls mono-, di- oder trisubstituiert mit R^{m};
R^{m} ausgewählt ist aus der Gruppe bestehend aus Halogen, -CF₃, -OH, -OC₁₋₇-Alkyl, -OC₃₋₇-Cycloalkyl, -OCF₃, -N(R^{p})R^{s} (wobei R^{p} und R^{s} unabhängig -H oder -C₁₋₇-Alkyl sind), -C(O)N(R^{p})R^{s}, -N(R^{t})C(O)R^{t}, -N (R^{t}) SO₂C₁₋₆-Alkyl (wobei R^{t} -H oder -C₁₋₇-Alkyl ist), -S (O)₀₋₂-C₁₋₆-Alkyl, -SO₂N (R^{p}) R^{s}, -SCF₃, -CN, -NO₂, -C(O)C₁₋₇-Alkyl, -COOH und -COOC₁₋₇-Alkyl;
CYC -H ist oder ein Ringsystem ist, ausgewählt aus der Gruppe bestehend aus:
i) Phenyl, gegebenenfalls mono-, di- oder trisubstituiert mit R^{u} oder an benachbarten Kohlenstoffen disubstituiert mit -OC₁₋₄-Alkylen-O-, -(CH₂)₂-₃NH-, -(CH₂)₁₋₂NH(CH₂)-, -(CH₂)₂₋₃N(C₁₋₄-Alkyl)- oder -(CH₂)₁₋₂N(C₁₋₄-Alkyl) (CH₂)-;
R^{u} ausgewählt ist aus der Gruppe bestehend aus -C₁-₇-Alkyl, -C₃₋₇-Cycloalkyl, Phenyl, Benzyl, Halogen, -CF₃, -OH, -OC₁₋₇-Alkyl, -OC₃₋₇-Cycloalkyl, -OPhenyl, -OBenzyl, -OCF₃, -N (R^{v}) R^{w} (wobei R^{v} und R^{w} unabhängig -H oder -C₁₋₇-Alkyl sind oder R^{v} und R^{w} zusammen genommen mit dem Stickstoff, an den sie gebunden sind, Piperidinyl, Pyrrolidinyl, Morpholinyl, Thiomorpholinyl oder Piperazinyl bilden, wobei jedes R^{v} und R^{w} gegebenenfalls und unabhängig mit -OH oder -C₁₋₇-Alkyl substituiert ist), -C(O)N(R^{v})R^{w}, -N(R^{x})C(O)R^{x}, -N(R^{x})SO₂C₁₋₆-Alkyl (wobei R^{x} -H oder -C₁₋₇-Alkyl ist oder zwei R^{x} an dem gleichen Substituenten zusammen genommen mit dem Amid, an das sie gebunden sind, einen ansonsten aliphatischen 4- bis 6-gliedrigen Ring bilden), -N-(SO₂C₁₋₆-Alkyl)₂, -S(O)₀₋₂-C₁₋₆-Alkyl, -SO₂N(R^{v})R^{w}, -SCF₃, -C(O)C₁₋₆-Alkyl, -NO₂, -CN, -COOH und -COOC₁₋₇-Alkyl;
ii) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu einem zusätzlichen Kohlenstoffatom gegebenenfalls durch -N= ersetzt ist, gegebenenfalls mono- oder disubstituiert mit R^{u};
iii) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit R^{u}; und
iv) einem nichtaromatischen heterocyclischen Ring mit 4 bis 8 Gliedern, wobei der Ring 0, 1 oder 2 nichtbenachbarte Heteroatom-Glieder aufweist, ausgewählt aus der Gruppe bestehend aus O, S, -N=, >NH und >N(C₁₋₄-Alkyl), mit 0, 1 oder 2 Doppelbindungen, mit 0, 1 oder 2 Kohlenstoffgliedern, die ein Carbonyl sind, gegebenenfalls mit einem Kohlenstoffglied, das eine Brücke bildet, mit 0 bis 5 Substituenten R^{u}, und wobei, wenn q 0 ist, der Ring ein Kohlenstoffatom aufweist, das der Punkt der Befestigung ist;
und Enantiomere, Diastereomere, Hydrate, Solvate und pharmazeutisch verträgliche Salze, Ester und Amide davon;
mit der Maßgabe, dass in Formel (I):
(a) wenn ALK Methylen, Ethylen, Propylen oder Isopropylen ist, CYC -H ist, Ar Phenyl oder monosubstituiertes Phenyl ist, m 2 ist, n 1 ist und A >NR¹ ist, dann R¹ nicht -C₁₋₄-Alkyl oder Benzyl ist;
(b) wenn q 0 ist, CYC Phenyl ist, Ar Phenyl oder 3-Chlorphenyl ist, m 2 ist und n 1 ist, dann A nicht nichtsubstituiertes -CH₂- ist; und
(c) wenn q 0 ist, CYC 2-Pyridyl ist, Ar 2-Pyridyl ist, m 2 ist und n 1 ist, dann A nicht nichtsubstituiertes -CH₂- ist,
zur Verwendung bei der Therapie.

33. Verwendung von wenigstens einer Verbindung der Formeln (I) oder (II): 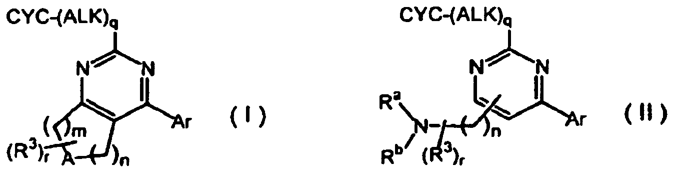 wobei
m 1, 2 oder 3 ist;
n 1, 2 oder 3 ist;
wobei wenn m und n beide vorhanden sind, m + n größer oder gleich 2 ist und kleiner oder gleich 4 ist;
R^{a} und R^{b} unabhängig -H, -C₁₋₇-Alkyl oder -C₃₋₇-Cycloalkyl sind oder R^{a} und R^{b} zusammen genommen mit dem Stickstoff, an den sie gebunden sind, Piperidinyl, Pyrrolidinyl, Morpholinyl, Thiomorpholinyl oder Piperazinyl bilden, wobei jedes R^{a} und R^{b} gegebenenfalls und unabhängig mit -C₁₋₄-Alkyl substituiert ist;
q 0 oder 1 ist;
A >NR¹, >CHNR^{c}R^{d}, >CHOH oder CH₂- ist, wobei
R¹ ausgewählt ist aus der Gruppe bestehend aus -H, -C₁₋₇-Alkyl, -C₃₋₇-Cycloalkyl und Benzyl, wobei jedes Alkyl, Cycloalkyl oder Benzyl gegebenenfalls mit R^{e} mono-, di- oder trisubstituiert ist;
R^{e} ausgewählt ist aus der Gruppe bestehend aus -C₁₋₉-Alkyl, -C₂₋₄-Alkenyl, -C₂₋₉-Alkinyl, -C₃₋₆-Cycloalkyl, Halogen, -CF₃, -OH, -OC₁₋₄-Alkyl, -OCF₃, -N(R^{f})R^{g} (wobei R^{f} und R^{g} unabhängig -H oder -C₁₋₄-Alkyl sind oder R^{f} und R^{g} zusammen genommen mit dem Stickstoff, an den sie gebunden sind, Piperidinyl, Pyrrolidinyl, Morpholinyl, Thiomorpholinyl oder Piperazinyl bilden), -C(O)N(R^{f})R^{g}, -N(R^{h})C(O)R^{h}, -N(R^{h})SO₂C₁₋₇-Alkyl (wobei R^{h} -H oder -C₁-₄-Alkyl ist oder zwei R^{h} an dem gleichen Substituenten zusammen genommen mit dem Amid, an das sie gebunden sind, einen ansonsten aliphatischen 4- bis 6-gliedrigen Ring bilden), -S (O)₀₋₂-C₁₋₄-Alkyl, -SO₂N(R^{f})R^{g}, -SCF₃, -C(O)C₁₋₄-Alkyl, -CN, -COOH und -COOC₁₋₄-Alkyl;
R^{c} und R^{d} unabhängig ausgewählt sind aus der Gruppe bestehend aus -H, -C₁₋₇-Alkyl, -C₃₋₇-Alkenyl, -C₃₋₇-Alkinyl, -C₃₋₇-Cycloalkyl, -C₁₋₇-Alkyl-C₃₋₇-cycloalkyl und -C₃₋₇-Cycloalkyl-C₁₋₇-alkyl, oder R^{c} und R^{d} zusammen genommen mit dem Stickstoff, an den sie gebunden sind, Piperidinyl, Pyrrolidinyl, Morpholinyl, Thiomorpholinyl oder Piperazinyl bilden, wobei jedes R^{c} und R^{d} gegebenenfalls und unabhängig mit R^{e} substituiert ist;
R³ -C₁₋₄-Alkyl, -C₁₋₄-Alkenyl oder Benzyl ist, jeweils gegebenenfalls substituiert mit -C₁₋₃-Alkyl, -OH oder Halogen, oder zwei R³-Substituenten zusammen genommen C₂₋₅-Alkylen bilden, gegebenenfalls substituiert mit -C₁₋₃-Alkyl, -OH oder Halogen;
r 0 ist oder eine ganze Zahl kleiner oder gleich m + n + 1 ist;
Ar ein Aryl- oder Heteroarylring ist, ausgewählt aus der Gruppe bestehend aus:
a) Phenyl, gegebenenfalls mono-, di- oder trisubstituiert mit R¹ oder an benachbarten Kohlenstoffen disubstituiert mit -OC₁₋₄-Alkylen-O-, -(CH₂)₂₋₃NH-, -(CH₂)₁₋₂NH(CH₂)-, - (CH₂)₂₋₃N(C₁₋₄-Alkyl)- oder -(CH₂)₁₋₂N(C₁₋₄-Alkyl)(CH₂)-;
Rⁱ ausgewählt ist aus der Gruppe bestehend aus -C₁₋₇-Alkyl, -C₂₋₇-Alkenyl, -C₂₋₇-Alkinyl, -C₃₋₇-Cycloalkyl, Halogen, -CF₃, -OH, -OC₁₋₇-Alkyl, -OCF₃, -OC₃₋₇-Alkenyl, -OC₃₋₇-Alkinyl, -N (R^{j}) R^{k} (wobei Rⁱ und R^{k} unabhängig -H oder -C₁₋₄-Alkyl sind), -C(O)N(R^{j})R^{k}, -N(R^{j})C(O)R^{k}, -N(R^{j})SO₂C₁₋₆-Alkyl, -S(O)₀₋₂-C₁₋₆-Alkyl, -SO₂N(R^{j}) R^{k}, -SCF₃, -C(O)C₁₋₆-Alkyl, -NO₂, -CN, -COOH und -COOC₁₋₇-Alkyl;
b) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu zwei zusätzliche Kohlenstoffatome gegebenenfalls durch -N= ersetzt sind, gegebenenfalls mono- oder disubstituiert mit Rⁱ;
c) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit Rⁱ; und
d) Phenyl oder Pyridyl, substituiert mit einem Substituenten, ausgewählt aus der Gruppe bestehend aus Phenyl, Phenoxy, Pyridyl, Thiophenyl, Oxazolyl und Tetrazolyl, wobei die erhaltene substituierte Einheit gegebenenfalls weiter mono-, di- oder trisubstituiert ist mit Rⁱ;
ALK ein verzweigtes oder nichtverzweigtes C₁₋₇-Alkylen, C₂₋₇-Alkenylen, C₂₋₇-Alkinylen, C₃₋₇-Cycloalkylen oder C₃₋₇-Cycloalkenylen ist, gegebenenfalls mono-, di- oder trisubstituiert mit R^{m};
R^{m} ausgewählt ist aus der Gruppe bestehend aus Halogen, -CF₃, -OH, -OC₁₋₇-Alkyl, -OC₃₋₇-Cycloalkyl, -OCF₃, -N(R^{p})R^{s} (wobei R^{p} und R^{s} unabhängig -H oder -C₁₋₇-Alkyl sind), -C(O)N(R^{p}) R^{s}, -N (R^{t}) C (O) R^{t}, -N (R^{t}) SO₂C₁₋₆-Alkyl (wobei R^{t} -H oder -C₁₋₇-Alkyl ist), -S(O)₀₋₂-C₁₋₆-Alkyl, -SO₂N(R^{p})R^{s}, -SCF₃, -CN, -NO₂, -C(O)C₁₋₇-Alkyl, -COOH und -COOC₁₋₇-Alkyl;
CYC -H ist oder ein Ringsystem ist, ausgewählt aus der Gruppe bestehend aus:
i) Phenyl, gegebenenfalls mono-, di- oder trisubstituiert mit R^{u} oder an benachbarten Kohlenstoffen disubstituiert mit -OC₁₋₄-Alkylen-O-, -(CH₂)₂₋₃NH-, - (CH₂)₁₋₂NH (CH₂)-, - (CH₂)₂₋₃N(C₁₋₄-Alkyl)- oder -(CH₂)₁₋₂N(C₁₋₄-Alkyl)(CH₂)-;
R^{u} ausgewählt ist aus der Gruppe bestehend aus -C₁₋₇-Alkyl, -C₃₋₇-Cycloalkyl, Phenyl, Benzyl, Halogen, -CF₃, -OH, -OC₁₋₇-Alkyl, -OC₃₋₇-Cycloalkyl, -OPhenyl, -OBenzyl, -OCF₃, -N(R^{v}) R^{w} (wobei R^{v} und R^{w} unabhängig -H oder -C₁₋₇-Alkyl sind oder R^{v} und R^{w} zusammen genommen mit dem Stickstoff, an den sie gebunden sind, Piperidinyl, Pyrrolidinyl, Morpholinyl, Thiomorpholinyl oder Piperazinyl bilden, wobei jedes R^{v} und R^{w} gegebenenfalls und unabhängig mit -OH oder -C₁₋₇-Alkyl substituiert ist), -C (O)N (R^{v}) R^{w}, -N(R^{x})C(O)R^{x}, -N(R^{x})SO₂C₁₋₆-Alkyl (wobei R^{x} -H oder -C₁₋₇-Alkyl ist oder zwei R^{x} an dem gleichen Substituenten zusammen genommen mit dem Amid, an das sie gebunden sind, einen ansonsten aliphatischen 4- bis 6-gliedrigen Ring bilden), -N-(SO₂C₁₋₆-Alkyl)₂, -S(O)₀₋₂-C₁₋₆-Alkyl, -SO₂N(R^{v})R^{w}, -SCF₃, -C(O)C₁₋₆-Alkyl, -NO₂, -CN, -COOH und -COOC₁₋₇-Alkyl;
ii) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu einem zusätzlichen Kohlenstoffatom gegebenenfalls durch -N= ersetzt ist, gegebenenfalls mono- oder disubstituiert mit R^{u};
iii) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit R^{u}; und
iv) einem nichtaromatischen heterocyclischen Ring mit 4 bis 8 Gliedern, wobei der Ring 0, 1 oder 2 nichtbenachbarte Heteroatom-Glieder aufweist, ausgewählt aus der Gruppe bestehend aus O, S, -N=, >NH und >N(C₁₋₄-Alkyl), mit 0, 1 oder 2 Doppelbindungen, mit 0, 1 oder 2 Kohlenstoffgliedern, die ein Carbonyl sind, gegebenenfalls mit einem Kohlenstoffglied, das eine Brücke bildet, mit 0 bis 5 Substituenten R^{u}, und wobei, wenn q 0 ist, der Ring ein Kohlenstoffatom aufweist, das der Punkt der Befestigung ist;
und Enantiomere, Diastereomere, Hydrate, Solvate und pharmazeutisch verträgliche Salze, Ester und Amide davon,
für die Herstellung eines Medikaments
für die Behandlung oder Vorbeugung einer ZNS-Störung, ausgewählt aus der Gruppe bestehend aus: Schlafstörungen, Depression/Angstzuständen, generalisierter Angststörung, Schizophrenie, bipolaren Störungen, psychotischen Störungen, Zwangsstörung, Gemütsstörungen, posttraumatischem Stress und anderen stressbedingten Störungen, Migräne, Schmerz, Essstörungen, Adipositas, sexueller Dysfunktion, metabolischen Störungen, hormonellem Ungleichgewicht, Alkoholmissbrauch, Suchtstörungen, Übelkeit, Entzündung, zentral vermitteltem Bluthochdruck, Schlaf/Wach-Störungen, Jetlag und Anomalien des zirkadianen Rhythmus, bei Säugern;
für die Behandlung oder Vorbeugung einer Erkrankung oder eines Zustands, ausgewählt aus der Gruppe bestehend aus: Hypotonie, peripheren Gefäßstörungen, kardiovaskulärem Schock, Nierenstörungen, Magenmotilität, Diarrhoe, spastischem Kolon, Reizdarmstörungen, Ischämien, septischem Schock, Harninkontinenz und anderen Störungen, die mit dem Gastrointestinal- und dem Gefäßsystem in Verbindung stehen, bei Säugern;
für die Behandlung oder Vorbeugung einer Augenstörung, ausgewählt aus der Gruppe bestehend aus: Glaukom, optischer Neuritis, diabetischer Retinopathie, Retinalödem und altersbedingter Makulardegeneration, bei Säugern.

34. Verwendung gemäß Anspruch 33, wobei die ZNS-Störung ausgewählt ist aus der Gruppe bestehend aus: Depression/Angstzuständen, Schlafstörungen und Anomalien des zirkadianen Rhythmus.

35. Verbindung der Formeln (I) oder (II): 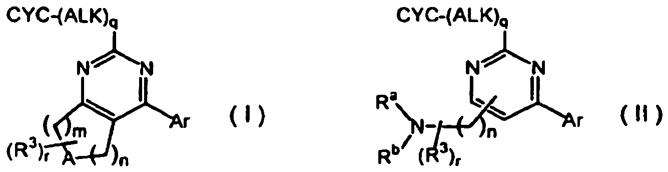 wobei
m 1, 2 oder 3 ist;
n 1, 2 oder 3 ist;
wobei wenn m und n beide vorhanden sind, m + n größer oder gleich 2 ist und kleiner oder gleich 4 ist;
R^{a} und R^{b} unabhängig -H, -C₁₋₇-Alkyl oder -C₃₋₇-Cycloalkyl sind oder R^{a} und R^{b} zusammen genommen mit dem Stickstoff, an den sie gebunden sind, Piperidinyl, Pyrrolidinyl, Morpholinyl, Thiomorpholinyl oder Piperazinyl bilden, wobei jedes R^{a} und R^{b} gegebenenfalls und unabhängig mit -C₁₋₄-Alkyl substituiert ist;
q 0 oder 1 ist;
A >NR¹, >CHNR^{c}R^{d}, >CHOH oder -CH₂- ist, wobei
R¹ ausgewählt ist aus der Gruppe bestehend aus -H, -C₁₋₇-Alkyl, -C₃₋₇-Cycloalkyl und Benzyl, wobei jedes Alkyl, Cycloalkyl oder Benzyl gegebenenfalls mit R^{e} mono-, di- oder trisubstituiert ist;
R^{e} ausgewählt ist aus der Gruppe bestehend aus -C₁₋₉-Alkyl, -C₂₋₄-Alkenyl, -C₂₋₄-Alkinyl, -C₃₋₆-Cycloalkyl, Halogen, -CF₃, -OH, -OC₁₋₄-Alkyl, -OCF₃, -N(R^{f})R^{g} (wobei R^{f} und R^{g} unabhängig -H oder -C₁₋₄-Alkyl sind oder R^{f} und R^{g} zusammen genommen mit dem Stickstoff, an den sie gebunden sind, Piperidinyl, Pyrrolidinyl, Morpholinyl, Thiomorpholinyl oder Piperazinyl bilden), -C(O)N(R^{f})R^{g}, -N(R^{h})C(O)R^{h}, -N(R^{h})SO₂C₁₋₇-Alkyl (wobei R^{h} -H oder -C₁₋₄-Alkyl ist oder zwei R^{h} an dem gleichen Substituenten zusammen genommen mit dem Amid, an das sie gebunden sind, einen ansonsten aliphatischen 4- bis 6-gliedrigen Ring bilden), -S(O)₀₋₂-C₁₋₄ Alkyl, -SO₂N(R^{f})R^{g}, -SCF₃, -C(O)C₁₋₄-Alkyl, -CN, -COOH und -COOC₁₋₄-Alkyl;
R^{c} und R^{d} unabhängig ausgewählt sind aus der Gruppe bestehend aus -H, -C₁₋₇-Alkyl, -C₃₋₇-Alkenyl, -C₃₋₇-Alkinyl, -C₃₋₇-Cycloalkyl, -C₁₋₇-Alkyl-C₃₋₇-cycloalkyl und -C₃₋₇-Cycloalkyl-C₁₋₇-alkyl, oder R^{c} und R^{d} zusammen genommen mit dem Stickstoff, an den sie gebunden sind, Piperidinyl, Pyrrolidinyl, Morpholinyl, Thiomorpholinyl oder Piperazinyl bilden, wobei jedes R^{c} und R^{d} gegebenenfalls und unabhängig mit R^{e} substituiert ist;
R³ -C₁₋₄-Alkyl, -C₁₋₉-Alkenyl oder Benzyl ist, jeweils gegebenenfalls substituiert mit -C₁₋₃-Alkyl, -OH oder Halogen, oder zwei R³-Substituenten zusammen genommen C₂₋₅-Alkylen bilden, gegebenenfalls substituiert mit -C₁₋₃-Alkyl, -OH oder Halogen;
r 0 ist oder eine ganze Zahl kleiner oder gleich m + n + 1 ist;
Ar ein Aryl- oder Heteroarylring ist, ausgewählt aus der Gruppe bestehend aus:
a) Phenyl, gegebenenfalls mono-, di- oder trisubstituiert mit Rⁱ oder an benachbarten Kohlenstoffen disubstituiert mit -OC₁₋₄-Alkylen-O-, -(CH₂)₂₋₃NH-, -(CH₂)₁₋₂NH (CH₂)-, -(CH₂)₂₋₃N (C₁₋₄-Alkyl) oder -(CH₂)₁₋₂N (C₁₋₉-Alkyl)(CH₂)
Rⁱ ausgewählt ist aus der Gruppe bestehend aus -C₁₋₇-Alkyl, -C₂₋₇-Alkenyl, -C₂₋₇-Alkinyl, -C₃₋₇-Cycloalkyl, Halogen, -CF₃, -OH, -OC₁₋₇-Alkyl, -OCF₃, -OC₃₋₇-Alkenyl, -OC₃₋₇-Alkinyl, -N (R^{j}) R^{k} (wobei R^{j} und R^{k} unabhängig -H oder -C₁₋₄-Alkyl sind), -C(O)N(Rⁱ)R^{k}, -N(R^{j})C(O)R^{k}, -N (R^{j})SO₂C₁₋₆-Alkyl, -S (O)₀₋₂-C₁₋₆-Alkyl, -SO₂N (R^{j}) R^{k}, -SCF₃, -C(O)C₁₋₆-Alkyl, -NO₂, -CN, -COOH und -COOC₁₋₇-Alkyl;
b) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₄-Alkyl) ersetzt ist, wobei bis zu zwei zusätzliche Kohlenstoffatome gegebenenfalls durch -N= ersetzt sind, gegebenenfalls mono- oder disubstituiert mit Rⁱ;
c) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit Rⁱ; und
d) Phenyl oder Pyridyl, substituiert mit einem Substituenten, ausgewählt aus der Gruppe bestehend aus Phenyl, Phenoxy, Pyridyl, Thiophenyl, Oxazolyl und Tetrazolyl, wobei die erhaltene substituierte Einheit gegebenenfalls weiter mono-, di- oder trisubstituiert ist mit Rⁱ;
ALK ein verzweigtes oder nichtverzweigtes C₁₋₇-Alkylen, C₂₋₇-Alkenylen, C₂₋₇-Alkinylen, C₃₋₇-Cycloalkylen oder C₃₋₇-Cycloalkenylen ist, gegebenenfalls mono-, di- oder trisubstituiert mit R^{m};
R^{m} ausgewählt ist aus der Gruppe bestehend aus Halogen, -CF₃, -OH, -OC₁₋₇-Alkyl, -OC₃₋₇-Cycloalkyl, -OCF₃, -N(R^{p})R^{s} (wobei R^{p} und R^{s} unabhängig -H oder -C₁₋₇-Alkyl sind), -C (O)N (R^{p}) R^{s}, -N(R^{t}) C (O) R^{t}, -N (R^{t}) SO₂C₁₋₆-Alkyl (wobei R^{t} -H oder -C₁₋₇-Alkyl ist), -S(O)₀₋₂-C₁₋₆-Alkyl, -SO₂N(R^{p})R^{s}, -SCF₃, -CN, -NO₂, -C(O)C₁-₇-Alkyl, -COOH und -COOC₁₋₇-Alkyl;
CYC -H ist oder ein Ringsystem ist, ausgewählt aus der Gruppe bestehend aus:
i) Phenyl, gegebenenfalls mono-, di- oder trisubstituiert mit R^{u} oder an benachbarten Kohlenstoffen disubstituiert mit -OC₁₋₄-Alkylen-O-, -(CH₂)₂₋₃NH-, - (CH₂)₁₋₂NH (CH₂)-, - (CH₂)₂₋₃N (C₁₋₄-Alkyl) oder -(CH₂)₁₋₂N (C₁₋₄-Alkyl) (CH₂)-;
R^{u} ausgewählt ist aus der Gruppe bestehend aus -C₁₋₇-Alkyl, -C₃₋₇-Cycloalkyl, Phenyl, Benzyl, Halogen, -CF₃, -OH, -OC₁₋₇-Alkyl, -OC₃₋₇-Cycloalkyl, -OPhenyl, -OBenzyl, -OCF₃, -N (R^{v}) R^{w} (wobei R^{v} und R^{w} unabhängig -H oder -C₁₋₇-Alkyl sind oder R^{v} und R^{w} zusammen genommen mit dem Stickstoff, an den sie gebunden sind, Piperidinyl, Pyrrolidinyl, Morpholinyl, Thiomorpholinyl oder Piperazinyl bilden, wobei jedes R^{v} und R^{w} gegebenenfalls und unabhängig mit -OH oder -C₁₋₇-Alkyl substituiert ist), -C(O)N(R^{v})R^{w}, -N(R^{x})C(O)R^{x}, -N(R^{x})SO₂C₁₋₆-Alkyl (wobei R^{x} -H oder -C₁₋₇-Alkyl ist oder zwei R^{x} an dem gleichen Substituenten zusammen genommen mit dem Amid, an das sie gebunden sind, einen ansonsten aliphatischen 4- bis 6-gliedrigen Ring bilden), -N-(SO₂C₁₋₆-Alkyl)₂, -S(O)₀₋₂-C₁₋₆-Alkyl, -SO₂N(R^{v})R^{w}, -SCF₃, -C(O)C₁₋₆-Alkyl, -NO₂, -CN, -COOH und -COOC₁₋₇-Alkyl;
ii) einer monocyclischen aromatischen Kohlenwasserstoffgruppe mit fünf Ringatomen, wobei ein Kohlenstoffatom der Punkt der Befestigung ist, wobei ein Kohlenstoffatom durch >O, >S, >NH oder >N(C₁₋₉-Alkyl) ersetzt ist, wobei bis zu einem zusätzlichen Kohlenstoffatom gegebenenfalls durch -N= ersetzt ist, gegebenenfalls mono- oder disubstituiert mit R^{u};
iii) einer monocyclischen aromatischen Kohlenwasserstoffgruppe mit sechs Ringatomen, wobei ein Kohlenstoffatom der Punkt der Befestigung ist, wobei ein oder zwei Kohlenstoffatome durch -N= ersetzt sind, gegebenenfalls mono- oder disubstituiert mit R^{u}; und
iv) einem nichtaromatischen heterocyclischen Ring mit 4 bis 8 Gliedern, wobei der Ring 0, 1 oder 2 nichtbenachbarte Heteroatom-Glieder aufweist, ausgewählt aus der Gruppe bestehend aus O, S, -N=, >NH und >N(C₁₋₉-Alkyl), mit 0, 1 oder 2 Doppelbindungen, mit 0, 1 oder 2 Kohlenstoffgliedern, die ein Carbonyl sind, gegebenenfalls mit einem Kohlenstoffglied, das eine Brücke bildet, mit 0 bis 5 Substituenten R^{u}, und wobei, wenn q 0 ist, der Ring ein Kohlenstoffatom aufweist, das der Punkt der Befestigung ist;
und Enantiomere, Diastereomere, Hydrate, Solvate und pharmazeutisch verträgliche Salze, Ester und Amide davon,
für die Verwendung bei der Behandlung oder Vorbeugung einer ZNS-Störung, ausgewählt aus der Gruppe bestehend aus: Schlafstörungen, Depression/Angstzuständen, generalisierter Angststörung, Schizophrenie, bipolaren Störungen, psychotischen Störungen, Zwangsstörung, Gemütsstörungen, posttraumatischem Stress und anderen stressbedingten Störungen, Migräne, Schmerz, Essstörungen, Adipositas, sexueller Dysfunktion, metabolischen Störungen, hormonellem Ungleichgewicht, Alkoholmissbrauch, Suchtstörungen, Übelkeit, Entzündung, zentral vermitteltem Bluthochdruck, Schlaf/Wach-Störungen, Jetlag und Anomalien des zirkadianen Rhythmus, bei Säugern;
für die Verwendung bei der Behandlung oder Vorbeugung einer Erkrankung oder eines Zustands, ausgewählt aus der Gruppe bestehend aus: Hypotonie, peripheren Gefäßstörungen, kardiovaskulärem Schock, Nierenstörungen, Magenmotilität, Diarrhoe, spastischem Kolon, Reizdarmstörungen, Ischämien, septischem Schock, Harninkontinenz und anderen Störungen, die mit dem Gastrointestinal- und dem Gefäßsystem in Verbindung stehen, bei Säugern;
für die Verwendung bei der Behandlung oder Vorbeugung einer Augenstörung, ausgewählt aus der Gruppe bestehend aus: Glaukom, optischer Neuritis, diabetischer Retinopathie, Retinalödem und altersbedingter Makulardegeneration, bei Säugern.

36. Verbindung der Formeln (I) oder (II) gemäß Anspruch 31, die isotopenmarkiert ist, um durch PET oder SPECT nachweisbar zu sein.

37. Verfahren zum Untersuchen von Serotoninvermittelten Störungen, umfassend den Schritt der Verwendung einer ¹⁸F-markierten oder ¹¹C-markierten Verbindung der Formeln (I) oder (II) gemäß Anspruch 31 als molekulare Sonde für die Positronenemissionstomographie (PET), wobei das Verfahren kein Verfahren für die Behandlung des menschlichen Körpers oder Tierkörpers durch Chirurgie oder Therapie oder ein diagnostisches Verfahren ist, das am menschlichen Körper oder Tierkörper ausgeführt wird.

38. Verbindung gemäß Anspruch 1, ausgewählt aus der Gruppe bestehend aus {2-[2-tert-Butyl-6-(4-fluorphenyl)pyrimidin-4-yl]ethyl}methylamin und {2-[2-tert-Butyl-6-(4-fluorphenyl)pyrimidin-4-yl]ethyl}dimethylamin.

39. Verbindung gemäß Anspruch 1, die 4-(4-Fluorphenyl)-2-isopropyl-5,6,7,8-tetrahydropyrido[4,3-d]pyrimidin oder ein pharmazeutisch verträgliches Salz davon ist.

## Revendications

1. Composé de Formule (I) ou (II) : 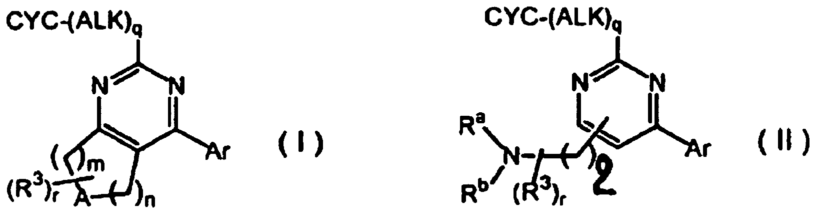 dans laquelle
m vaut 1, 2 ou 3 ;
n vaut 1, 2 ou 3 ;
lorsque m et n sont présents, m + n est supérieur ou égal à 2, et est inférieur ou égal à 4 ;
R^{a} et R^{b} sont indépendamment -H, -C₁₋₇alkyle, ou -C₃₋₇-cycloalkyle, ou R^{a} et R^{b}, pris conjointement avec l'azote auquel ils sont liés, forment pipéridinyle, pyrrolidinyle, morpholinyle, thiomorpholinyle ou pipérazinyle, où chaque R^{a} et R^{b} est éventuellement et indépendamment substitué par -C₁₋₄alkyle ;
q vaut 0 ou 1 ;
A représente >NR¹, >CHNR^{c}R^{d}, >CHOH, ou -CH₂-, où R¹ est sélectionné dans le groupe constitué par -H, -C₁-₇alkyle, -C₃₋₇cycloalkyle et benzyle, où chaque alkyle, cycloalkyle ou benzyle est éventuellement mono-, di- ou trisubstitué par R^{e} ;
R^{e} est sélectionné dans le groupe constitué par -C₁-₄alkyle, -C₂₋₄alcényle, -C₂₋₄alcynyle, -C₃₋₆cycloalkyle, halogéno, -CF₃, -OH, -OC₁₋₄alkyle, -OCF₃, -N(R^{f})R^{g} (où R^{f} et R^{g} sont indépendamment -H ou -C₁₋₉alkyle, ou R^{f} et R^{g}, pris conjointement avec l'azote auquel ils sont liés, forment pipéridinyle, pyrrolidinyle, morpholinyle, thiomorpholinyle ou pipérazinyle), -C(O)N(R^{f})R^{g}, -N(R^{h})C(O)R^{h}, -N(R^{h})SO₂C₁₋₇alkyle (où R^{h} est -H ou -C₁₋₄alkyle, ou deux R^{h} dans le même substituant, pris conjointement avec l'amide auquel ils sont liés, forment un cycle de 4 à 6 chaînons par ailleurs aliphatique), -S (O)₀₋₂-C₁₋₄alkyle, -SO₂N (R^{f}) R^{g}, -SCF₃, -C(O)C₁₋₉alkyle, -CN, -COOH et -COOC₁₋₄alkyle ;
R^{c} et R^{d} sont sélectionnés indépendamment dans le groupe constitué par -H, -C₁₋₇alkyle, -C₃₋₇alcényle, -C₃₋₇alcynyle, -C₃₋₇cycloalkyle, -C₁₋₇alkylC₃₋₇cycloalkyle et - C₃₋₇cycloalkylC₁₋₇alkyle, ou R^{c} et R^{d}, pris conjointement avec l'azote auquel ils sont liés, forment pipéridinyle, pyrrolidinyle, morpholinyle, thiomorpholinyle ou pipérazinyle, où chaque R^{c} et R^{d} est éventuellement et indépendamment substitué par R^{e} ;
R³ est -C₁₋₄alkyle, -C₁₋₉alcényle ou benzyle, chacun éventuellement substitué par -C₁₋₃alkyle, -OH ou halogéno, ou deux substituants R³ pris conjointement forment C₂₋₅alkylène éventuellement substitué par -C₁₋₃alkyle, -OH ou halogéno ;
r vaut 0 ou est un entier inférieur ou égal à m + n + 1 ;
Ar est un cycle aryle ou hétéroaryle sélectionné dans le groupe constitué par :
a) phényle, éventuellement mono-, di- ou trisubstitué par Rⁱ ou disubstitué sur des carbones adjacents par -OC₁₋₄alkylèneO-, -(CH₂)₂₋₃NH-, -(CH₂)₁₋₂NH (CH₂) -, - (CH₂)₂₋₃N (C₁₋₄alkyl) -, ou - (CH₂)₁₋₂N(C₁₋₉alkyl)(CH₂)- ;
Rⁱ est sélectionné dans le groupe constitué par -C₁-₇alkyle, -C₂₋₇alcényle, -C₂₋₇alcynyle, -C₃₋₇cycloalkyle, halogéno, -CF₃, -OH, -OC₁₋₇alkyle, -OCF₃, -OC₃₋₇alcényle, -OC₃₋₇alcynyle, -N(R^{j})R^{k} (où Rⁱ et R^{k} sont indépendamment -H ou -C₁₋₉alkyle), -C(O)N(R^{j})R^{k}, -N(R^{j})C(O)R^{k}, -N(R^{j})SO₂C₁₋₆alkyle, -S(O)₀₋₂-C₁₋₆alkyle, -SO₂N(R^{j})R^{k}, -SCF₃, -C(O)C₁₋₆alkyle, -NO₂, -CN, -COOH et -COOC₁₋₇alkyle ;
b) un groupement hydrocarboné aromatique monocyclique à cinq atomes de cycle, ayant un atome de carbone comme point de liaison, ayant un atome de carbone remplacé par >O, >S, >NH ou >N(C₁₋₄alkyl), ayant jusqu'à deux atomes de carbone supplémentaires éventuellement remplacés par -N=, éventuellement mono-ou disubstitué par Rⁱ ;
c) un groupement hydrocarboné aromatique monocyclique à six atomes de cycle, ayant un atome de carbone comme point de liaison, ayant un ou deux atomes de carbone remplacés par -N=, éventuellement mono- ou disubstitué par Rⁱ ; et
d) phényle ou pyridyle, substitué par un substituant sélectionné dans le groupe constitué par phényle, phénoxy, pyridyle, thiophényle, oxazolyle et tétrazolyle, où le motif substitué résultant est éventuellement en outre mono-, di- ou trisubstitué par Rⁱ ;
ALK est C₁₋₇alkylène, C₂₋₇alcénylène, C₂₋₇alcynylène, C₃₋₇cycloalkylène ou C₃₋₇cycloalcénylène ramifié ou non ramifié, éventuellement mono-, di- ou trisubstitué par R^{m} ;
R^{m} est sélectionné dans le groupe constitué par halogéno, -CF₃, -OH, -OC₁₋₇alkyle, -OC₃₋₇cycloalkyle, -OCF₃, -N(R^{p})R^{s} (où R^{p} et R^{s} sont indépendamment -H ou -C₁₋₇alkyle), -C(O)N(R^{p})R^{s}, -N(R^{t})C(O)R^{t}, -N(R^{t})SO₂-C₁₋₆alkyle (où R^{t} est -H ou -C₁₋₇alkyle), -S(O)₀₋₂-C₁₋₆alkyle, -SO₂N(R^{p})R^{s}, -SCF₃, -CN, -NO₂, -C(O)C₁₋₇alkyle, -COOH et -COOC₁₋₇alkyle ;
CYC est -H ou est un noyau sélectionné dans le groupe constitué par :
i) phényle, éventuellement mono-, di- ou trisubstitué par R^{u} ou disubstitué sur des carbones adjacents par -OC₁₋₄alkylèneO-, -(CH₂)₂₋₃NH-, -(CH₂)₁₋₂NH(CH₂)-, -(CH₂)₂₋₃N(C₁₋₄alkyl)-, ou -(CH₂)₁₋₂N(C₁-₄alkyl)(CH₂)- ; R^{u} est sélectionné dans le groupe constitué par -C₁-₇alkyle, -C₃₋₇cycloalkyle, phényle, benzyle, halogéno, -CF₃, -OH, -OC₁₋₇alkyle, -OC₃₋₇cycloalkyle, -Ophényle, -Obenzyle, -OCF₃, -N(R^{v})R^{w} (où R^{v} et R^{w} sont indépendamment -H ou -C₁₋₇alkyle, ou R^{v} et R^{w}, pris conjointement avec l'azote auquel ils sont liés, forment pipéridinyle, pyrrolidinyle, morpholinyle, thiomorpholinyle ou pipérazinyle, où chaque R^{v} et R^{w} est éventuellement et indépendamment substitué par -OH ou -C₁₋₇alkyle), -C(O)N(R^{v})R^{w}, -N(R^{x})C(O)R^{x}, -N(R^{x})SO₂C₁-₆alkyle (où R^{x} est -H ou -C₁₋₇alkyle, ou deux R^{x} dans le même substituant, pris conjointement avec l'amide auquel ils sont liés, forment un cycle de 4 à 6 chaînons par ailleurs aliphatique), -N-(SO₂C₁₋₆alkyl)₂, -S(O)₀₋₂-C₁₋₆alkyle, -SO₂N(R^{v})R^{w}, -SCF₃, -C(O)C₁₋₆alkyle, -NO₂, -CN, -COOH et -COOC₁₋₇alkyle ;
ii) un groupement hydrocarboné aromatique monocyclique à cinq atomes de cycle, ayant un atome de carbone comme point de liaison, ayant un atome de carbone remplacé par >O, >S, >NH ou >N(C₁₋₄alkyl), ayant jusqu'à un atome de carbone supplémentaire éventuellement remplacé par -N=, éventuellement mono-ou disubstitué par R^{u} ;
iii) un groupement hydrocarboné aromatique monocyclique à six atomes de cycle, ayant un atome de carbone comme point de liaison, ayant un ou deux atomes de carbone remplacés par -N=, éventuellement mono- ou disubstitué par R^{u} ; et
iv) un cycle hétérocyclique non aromatique de 4 à 8 chaînons, ledit cycle ayant 0, 1 ou 2 chaînons d'hétéroatomes non adjacents sélectionnés dans le groupe constitué par O, S, -N=, >NH et >N(C₁₋₄alkyl), ayant 0, 1 ou 2 doubles liaisons, ayant 0, 1 ou 2 chaînons de carbone s'agissant de carbonyle, éventuellement ayant un chaînon de carbone qui forme un pont, ayant 0 à 5 substituants R^{u}, et où, quand q vaut 0, ledit cycle comporte un atome de carbone comme point de liaison ;
ainsi que les énantiomères, diastéréoisomères, hydrates, solvates et sels pharmaceutiquement acceptables, esters et amides de celui-ci ;
à condition que dans la Formule (I) :
(a) lorsque ALK est méthylène, éthylène, propylène ou isopropylène, CYC est -H, Ar est phényle ou phényle monosubstitué, m vaut 2, n vaut 1 et A est >NR¹, alors R¹ n'est pas -C₁₋₄alkyle ou benzyle ;
(b) lorsque q vaut 0, CYC est phényle, Ar est phényle ou 3-chlorophényle, m vaut 2 et n vaut 1, alors A n'est pas -CH₂- non substitué ; et
(c) lorsque q vaut 0, CYC est 2-pyridyle, Ar est 2-pyridyle, m vaut 2 et n vaut 1, alors A n'est pas -CH₂-non substitué ; où ledit composé ne représente pas : 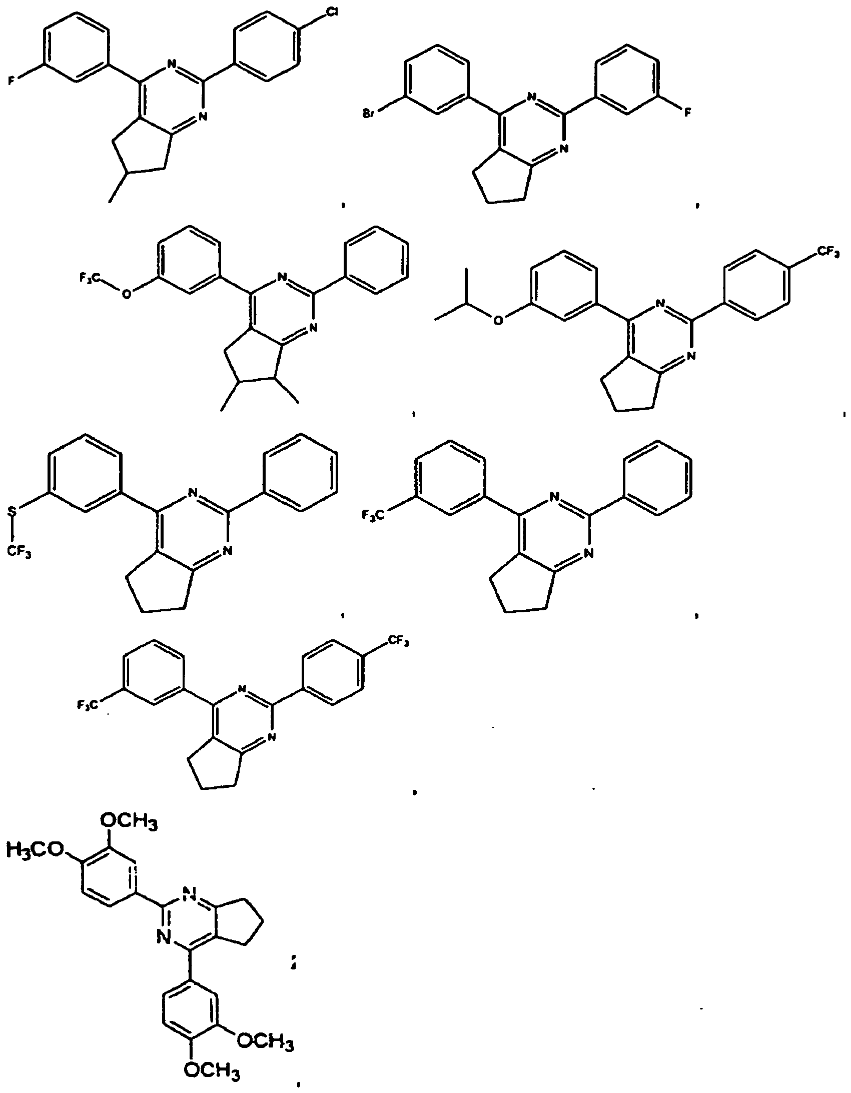 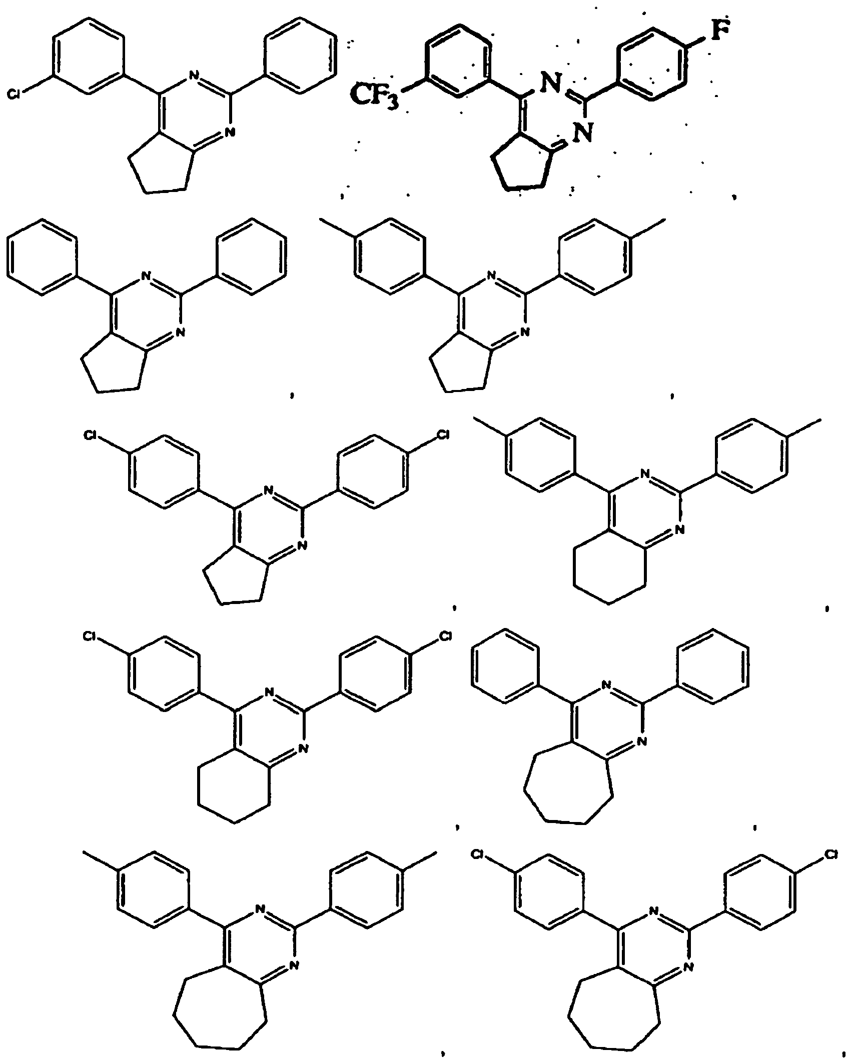 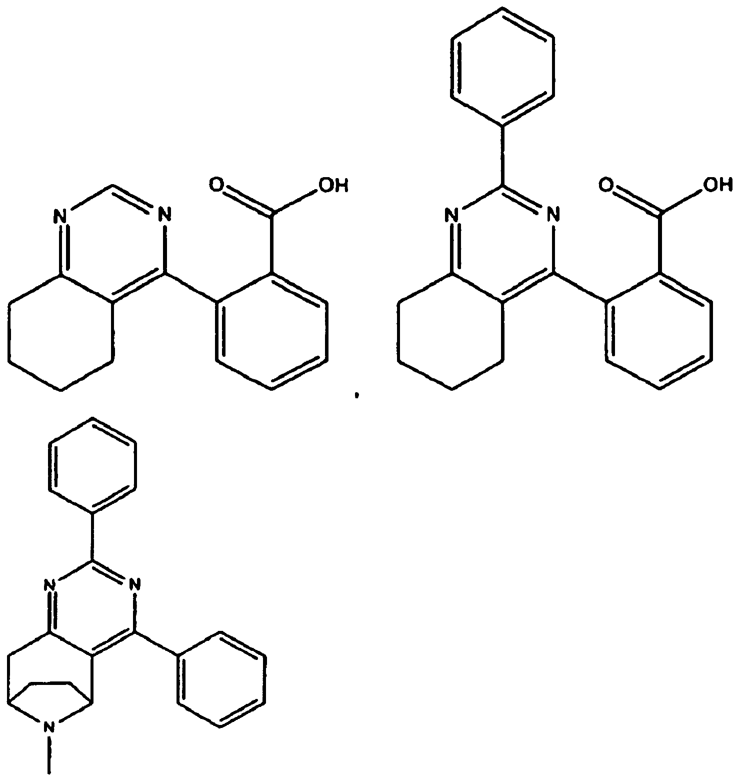
la 7-benzyl-2-méthyl-4-phényl-5,8-dihydro-6H-pyrido[3,4-d]pyrimidine,
la 2-méthyl-4-phényl-5,8-dihydro-6H-pyrido[3,4-d)pyrimidine, ou
la 4-(2-méthyl-4-nitrophényl)-6,7-dihydro-5H-cyclopentapyrimidine.

2. Composé selon la revendication 1, dans lequel m vaut 1 et n vaut 1.

3. Composé selon la revendication 1, dans lequel m vaut 1 et n vaut 2.

4. Composé selon la revendication 1, dans lequel m vaut 2 et n vaut 1.

5. Composé selon la revendication 1, dans lequel m vaut 2 et n vaut 2.

6. Composé selon la revendication 1, dans lequel m vaut 1 et n vaut 3.

7. Composé selon la revendication 1, dans lequel m vaut 3 et n vaut 1.

8. Composé selon la revendication 1, dans lequel q vaut 1.

9. Composé selon la revendication 1, dans lequel -N(R^{a})R^{b} est amino, méthylamino, éthylamino, isopropylamino, diméthylamino, diéthylamino, diisopropylamino, cyclopropylamino, cyclopentylamino, pipéridinyle, pyrrolidinyle, morpholinyle, thiomorpholinyle ou pipérazinyle.

10. Composé selon la revendication 1, dans lequel -N(R^{a})R^{b} est amino, méthylamino, diméthylamino ou N-méthylpipérazinyle.

11. Composé selon la revendication 1, dans lequel A représente >NR¹.

12. Composé selon la revendication 1, dans lequel R¹ est sélectionné dans le groupe constitué par hydrogène, méthyle, éthyle, isopropyle, butyle, hexyle, cyclopropyle, cyclobutyle, cyclopentyle et benzyle, chacun éventuellement mono-, di- ou trisubstitué par R^{e}.

13. Composé selon la revendication 1, dans lequel R¹, éventuellement R^{e} substitué, est sélectionné dans le groupe constitué par hydrogène, méthyle, éthyle, isopropyle et benzyle.

14. Composé selon la revendication 1, dans lequel R¹ est hydrogène ou méthyle.

15. Composé selon la revendication 1, dans lequel R³ éventuellement substitué, est sélectionné dans le groupe constitué par méthyle, éthyle, propyle, isopropyle, butyle, méthylène, allyle et benzyle.

16. Composé selon la revendication 1, dans lequel deux substituants R³ pris conjointement forment éthylène.

17. Composé selon la revendication 1, dans lequel R³ est méthyle.

18. Composé selon la revendication 1, dans lequel r vaut 0, 1 ou 2.

19. Composé selon la revendication 1, dans lequel Ar, éventuellement substitué, est sélectionné dans le groupe constitué par :
a) phényle, 5-, 6-, 7-, 8-benzo-1,4-dioxanyle, 4-, 5-, 6-, 7-benzo-1,3-dioxolyle, 4-, 5-, 6-, 7-indolinyle, 4-, 5-, 6-, 7-isoindolinyle, 1,2,3,4-tétrahydroquinoléin-4, 5, 6 ou 7-yle, 1,2,3,4-tétrahydroisoquinoléin-4, 5, 6 ou 7-yle,
b) furanyle, oxazolyle, isoxazolyle, 1,2,3-oxadiazolyle, 1,2,4-oxadiazolyle, 1,2,5-oxadiazolyle, 1,3,4-oxadiazolyle, thiophényle, thiazolyle, isothiazolyle, pyrrolyle, imidazolyle, pyrazolyle, 1,2,3-triazolyle, 1,2,4-triazolyle,
c) pyridinyle, pyridinyl-N-oxyde, pyrazinyle, pyrimidinyle, pyridazinyle, et
d) biphényle et 4-tétrazolylphényle.

20. Composé selon la revendication 1, dans lequel Ar, éventuellement substitué, est sélectionné dans le groupe constitué par phényle, pyridyle, thiophén-2-yle et thiophén-3-yle.

21. Composé selon la revendication 1, dans lequel Ar est sélectionné dans le groupe constitué par phényle, 2-méthoxyphényle, 3-méthoxyphényle, 4-méthoxyphényle, 2-méthylphényle, 3-méthylphényle, 4-méthylphényle, 4-éthylphényle, 2-chlorophényle, 3-chlorophényle, 4-chlorophényle, 2-fluorophényle, 3-fluorophényle, 4-fluorophényle, 2-bromophényle, 3-bromophényle, 4-bromophényle, 2-trifluorométhylphényle, 3-trifluorométhylphényle, 4-trifluorométhylphényle, 3-trifluorométhoxyphényle, 4-trifluorométhoxyphényle, 3-cyanophényle, 4-cyanophényle, 3-acétylphényle, 4-acétylphényle, 3,4-difluorophényle, 3,4-dichlorophényle, 2,3-difluorophényle, 2,3-dichlorophényle, 2,4-difluorophényle, 2,4-dichlorophényle, 3-nitrophényle, 4-nitrophényle, 3-chloro-4-fluorophényle, 3-fluoro-4-chlorophényle, benzo[1,3]dioxol-4 ou 5-yle, 3-hydroxyphényle, 4-hydroxyphényle, 4-hydroxy-2-méthylphényle, 4-hydroxy-3-fluorophényle, 3,4-dihydroxy-phényle, 4-diméthylaminophényle, 4-carbamoylphényle, 4-fluoro-3-méthylphényle, 2-phénoxyphényle, furan-2-yle, furan-3-yle, 5-méthylfuran-2-yle, thiophén-2-yle, thiophén-3-yle, 5-chlorothiophén-2-yle, 5-méthylthio-phén-2-yle, 5-chlorothiophén-3-yle, 5-méthylthiophén-3-yle, oxazol-2-yle, 4,5-diméthyloxazol-2-yle, thiazol-2-yle, 3H-[1,2,3]triazol-4-yle, 2H-pyrazol-3-yle, 1H-pyrazol-4-yle, 4-pyridyle, 5-fluoropyridin-2-yle, 4'-chlorobiphényle et 4-tétrazolylphényle.

22. Composé selon la revendication 1, dans lequel ALK, éventuellement substitué, est sélectionné dans le groupe constitué par méthylène, éthylène, propylène, butylène, sec-butylène, tertio-butylène, pentylène, 1-éthylpropylène, 2-éthylpropylène, 2-éthylbutylène, isopropylène, but-3-énylène, isobutylène, 3-méthylbutylène, allylène, prop-2-ynylène, cyclopropylène, cyclobutylène, cyclopentylène, cyclohexylène et cycloheptylène.

23. Composé selon la revendication 1, dans lequel ALK est sélectionné dans le groupe constitué par méthylène, hydroxyméthylène, fluorométhylène, difluorométhylène, trifluorométhylméthylène, 2,2,2-trifluoro-1-trifluoro-méthyléthylène, méthoxycarbonylméthyle, méthyl-carbamoylméthyle, éthylène, 2-diméthylaminoéthylène, 2-cyanoéthylène, 2-méthoxyéthylène, 1-carboxyéthylène, propylène, 3-méthoxycarbonylpropylène, 3-carboxy-propylène, isopropylène, 1-fluoro-1-méthyléthylène, 1-hydroxy-1-méthyléthylène, 1-carboxy-1-méthyléthylène, 1-éthylpropylène, 2-éthylpropylène, butylène, tertio-butylène, sec-butylène, isobutylène, 4-hydroxybutylène, 4-méthoxycarbonylbutylène, 4-carboxybutylène, 2-éthylbutylène, isobutylène, 3-méthylbutylène, prop-2-ynylène, but-3-énylène, pentylène, 5-hydroxypentylène, cyclopropylène, cyclobutylène, cyclopentylène, cyclopenténylène, 3,3-difluorocyclopentylène, 3-hydroxycyclohexylène, 4-fluorocyclohexylène, 4,4-difluorocyclohexylène et 1-méthylcyclopropylène.

24. Composé selon la revendication 1, dans lequel CYC, éventuellement substitué, est hydrogène ou est un noyau sélectionné dans le groupe constitué par :
i) phényle, 5-, 6-, 7-, 8-benzo-1,4-dioxanyle, 4-, 5-, 6-, 7-benzo-1,3-dioxolyle, 4-, 5-, 6-, 7-indolinyle, 4-, 5-, 6-, 7-isoindolinyle, 1,2,3,4-tétrahydroquinoléin-4, 5, 6 ou 7-yle, 1,2,3,4-tétrahydroisoquinoléin-4, 5, 6 ou 7-yle,
ii) furanyle, oxazolyle, isoxazolyle, 1,2,3-oxadiazolyle, 1,2,4-oxadiazolyle, 1,2,5-oxadiazolyle, 1,3,4-oxadiazolyle, thiophényle, thiazolyle, isothiazolyle, pyrrolyle, imidazolyle, pyrazolyle, 1,2,3-triazolyle, 1,2,4-triazolyle,
iii) pyridinyle, pyridinyl-N-oxyde, pyrazinyle, pyrimidinyle, pyridazinyle, et
iv) pyrrolinyle, pyrrolidinyle, pyrazolinyle, pipéridinyle, homopipéridinyle, azépanyle, tétrahydrofuranyle, tétrahydropyranyle, pipérazinyle, morpholinyle, thiomorpholinyle et pipéridinonyle.

25. Composé selon la revendication 1, dans lequel CYC, éventuellement substitué, est sélectionné dans le groupe constitué par hydrogène, phényle, thiophén-2-yle, thiophén-3-yle, furan-2-yle, furan-3-yle, pyridinyle, pipéridin-1, 2, 3 ou 4-yle, 2-pyrrolin-2, 3, 4, ou 5-yle, 3-pyrrolin-2 ou 3- yle, 2-pyrazolin-3, 4 ou 5-yle, tétrahydrofuran-3-yle, tétrahydropyran-4-yle, morpholin-2, 3, ou 4-yle, thiomorpholin-2, 3, ou 4-yle, pipérazin-1, 2, 3, ou 4-yle, pyrrolidin-1, 2 ou 3-yle et homopipéridinyle.

26. Composé selon la revendication 1, dans lequel CYC, éventuellement substitué, est sélectionné dans le groupe constitué par hydrogène, phényle, pyridyle, thiophén-2-yle, thiophén-3-yle, tétrahydropyranyle, furan-2-yle, furan-3-yle, tétrahydrofuran-3-yle et pipéridinyle.

27. Composé selon la revendication 1, dans lequel CYC est sélectionné dans le groupe constitué par hydrogène, phényle, 2-méthoxyphényle, 3-méthoxyphényle, 4-méthoxyphényle, 2-méthylphényle, 3-méthylphényle, 4-méthylphényle, 4-éthylphényle, 2-chlorophényle, 3-chlorophényle, 4-chlorophényle, 2-fluorophényle, 3-fluorophényle, 4-fluorophényle, 2-bromophényle, 3-bromophényle, 4-bromophényle, 2-trifluorométhylphényle, 3-trifluorométhylphényle, 4-trifluorométhylphényle, 3-trifluorométhoxyphényle, 4-trifluorométhoxyphényle, 2-cyanophényle, 3-cyanophényle, 4-cyanophényle, 3-acétylphényle, 4-acétylphényle, 3,4-difluorophényle, 3,4-dichlorophényle, 2,3-difluorophényle, 2,3-dichlorophényle, 2,4-difluorophényle, 2,4-dichlorophényle, 2,6-difluorophényle, 2,6-dichlorophényle, 2,6-diméthylphényle, 2,4,6-trifluorophényle, 2,4,6-trichloro-phényle, 3,4,5-triméthoxyphényle, 4-fluoro-3-méthylphényle, 3-nitrophényle, 4-nitrophényle, 4-méthyl-3-fluorophényle, 3,4-diméthylphényle, 4-méthoxy-3-fluorophényle, 4-méthoxy-2-méthylphényle, 3-aminophényle, 4-aminophényle, 4-carbométhoxyphényle, 3-méthanesulfonylaminophényle, 4-méthanesulfonylamino-phényle, 3-diméthanesulfonylaminophényle, 4-diméthanesulfonylaminophényle, thiophén-2-yle, thiophén-3-yle, 5-chlorothiophén-2-yle, benzo[1,3]dioxol-4 ou 5-yle, tétrahydrofuran-3-yle, tétrahydropyran-2,3 ou 4-yle, furan-2-yle, furan-3-yle, 5-carboxyéthylfuran-2-yle, pipéridinyle, 3,4-bisbenzyloxyphényle, 2-hydroxyphényle, 3-hydroxyphényle, 4-hydroxyphényle, 4-hydroxy-2-méthylphényle, 4-hydroxy-3-fluorophényle, 3,4-dihydroxyphényle, 1-pipéridinyle, 4-pipéridinyle et 1-méthyl-4-pipéridinyle.

28. Composé sélectionné dans le groupe constitué par :
la 2-tertio-butyl-4-(4-fluorophényl)-5,6,7,8-tétra-hydropyrido[4,3-d]pyrimidine ;
la 2-benzyl-4-(4-fluorophényl)-5,6,7,8-tétrahydro-pyrido[4,3-d]pyrimidine ;
le chlorhydrate de 2-sec-butyl-4-(4-fluorophényl)-5,6,7,8-tétrahydropyrido[4,3-d]pyrimidine;
le chlorhydrate de 2-sec-butyl-4-p-tolyl-5,6,7,8-tétrahydropyrido[4,3-d]pyrimidine ;
le chlorhydrate de 2-cyclobutyl-4-(4-fluorophényl)-5,6,7,8-tétrahydropyrido[4,3-d]pyrimidine ;
la 2-cyclobutyl-4-p-tolyl-5,6,7,8-tétrahydropyrido[4,3-d]pyrimidine ;
la 2-cyclopropyl-4-(4-fluorophényl)-5,6,7,8-tétra-hydropyrido[4,3-d]pyrimidine ;
la 2-benzyl-4-p-tolyl-5,6,7,8-tétrahydropyrido[4,3-d]-pyrimidine ;
la 2-benzyl-4-(4-trifluorométhylphényl)-5,6,7,8-tétra-hydropyrido[4,3-d]pyrimidine ;
la 2-benzyl-4-(3,4-difluorophényl)-5,6,7,8-tétra-hydropyrido[4,3-d]pyrimidine ;
la 2-benzyl-4-phényl-5,6,7,8-tétrahydropyrido[4,3-d]-pyrimidine ;
la 2-benzyl-4-(3-fluorophényl)-5,6,7,8-tétrahydro-pyrido[4,3-d]pyrimidine ;
la 2-(4-fluorobenzyl)-4-(4-fluorophényl)-5,6,7,8-tétra-hydropyrido[4,3-d]pyrimidine ;
la 2-(4-fluorobenzyl)-4-(4-fluorophényl)-6-méthyl-5,6,7,8-tétrahydropyrido[4,3-d]pyrimidine ;
la 4-[2-(4-fluorobenzyl)-6-méthyl-5,6,7,8-tétra-hydropyrido[4,3-d]pyrimidin-4-yl]benzonitrile ;
le 4-[2-(4-fluorobenzyl)-5,6,7,8-tétrahydropyrido[4,3-d]pyrimidin-4-yl]benzonitrile ;
la 2-cyclopentyl-4-(4-fluorophényl)-5,6,7,8-tétrahydropyrido[4,3-d]pyrimidine ;
la 2-cyclopentyl-4-p-tolyl-5,6,7,8-tétrahydropyrido-[4,3-d]pyrimidine ;
la 2-cyclopentyl-4-(4-méthoxyphényl)-5,6,7,8-tétra-hydropyrido[4,3-d]pyrimidine ;
la 4-(2-cyclopentyl-5,6,7,8-tétrahydropyrido[4,3-d]-pyrimidin-4-yl)benzonitrile ;
le chlorhydrate de 4-(4-fluorophényl)-2-isopropyl-5,6,7,8-tétrahydropyrido[4,3-d]pyrimidine ;
la 4-(4-fluorophényl)-2-isopropyl-6-méthyl-5,6,7,8-tétrahydropyrido[4,3-d]pyrimidine ;
le chlorhydrate de 4-(3,4-dichlorophényl)-2-isopropyl-5,6,7,8-tétrahydropyrido[4,3-d]pyrimidine ;
le chlorhydrate de 4-(3,4-difluorophényl)-2-isopropyl-5,6,7,8-tétrahydropyrido[4,3-d]pyrimidine ;
la 4-(3-fluorophényl)-2-isopropyl-5,6,7,8-tétrahydro-pyrido[4,3-d]pyrimidine ;
la 4-(2-fluorophényl)-2-isopropyl-5,6,7,8-tétrahydro-pyrido[4,3-d]pyrimidine ;
la 4-(2,4-difluorophényl)-2-isopropyl-5,6,7,8-tétra-hydropyrido[4,3-d]pyrimidine ;
la 2-isopropyl-4-p-tolyl-5,6,7,8-tétrahydropyrido[4,3-d]pyrimidine ;
la 4-(4-chlorophényl)-2-isopropyl-5,6,7,8-tétra-hydropyrido[4,3-d]pyrimidine ;
la 2-isopropyl-4-(4-méthoxyphényl)-5,6,7,8-tétra-hydropyrido[4,3-d]pyrimidine ;
la 2-isopropyl-4-phényl-5,6,7,8-tétrahydropyrido[4,3-d]pyrimidine ;
la 2-isopropyl-4-(4-trifluorométhylphényl)-5,6,7,8-tétrahydropyrido[4,3-d]pyrimidine ;
la 2-isopropyl-4-(2-phénoxyphényl)-5,6,7,8-tétra-hydropyrido[4,3-d]pyrimidine ;
la 2-isobutyl-4-thiophén-3-yl-5,6,7,8-tétra-hydropyrido[4,3-d]pyrimidine ;
la 2-isobutyl-4-thiophén-2-yl-5,6,7,8-tétra-hydropyrido[4,3-d]pyrimidine ;
la 2-isobutyl-4-pyridin-4-yl-5,6,7,8-tétra-hydropyrido[4,3-d]pyrimidine ;
la 4-(4-fluorophényl)-2-isobutyl-5,6,7,8-tétra-hydropyrido[4,3-d]pyrimidine ;
la 2-isobutyl-4-p-tolyl-5,6,7,8-tétrahydropyrido[4,3-d]pyrimidine ;
la 4-(4-fluoro-3-méthylphényl)-2-isobutyl-5,6,7,8-tétrahydropyrido[4,3-djpyrimidine ;
le 4-(2-isobutyl-5,6,7,8-tétrahydropyrido[4,3-d]-pyrimidin-4-yl)benzonitrile ;
la 2-isobutyl-4-(4-méthoxyphényl)-5,6,7,8-tétrahydro-pyrido[4,3-d]pyrimidine ;
le chlorhydrate de 2-sec-butyl-4-(2-fluorophényl)-5,6,7,8-tétrahydropyrido[4,3-d]pyrimidine ;
la 2-sec-butyl-4-(3-fluorophényl)-5,6,7,8-tétra-hydropyrido[4,3-d]pyrimidine ;
la 2-sec-butyl-4-(4-méthoxyphényl)-5,6,7,8-tétra-hydropyrido[4,3-d]pyrimidine ;
la 2-sec-butyl-4-(4-trifluorométhoxyphényl)-5,6,7,8-tétrahydropyrido[4,3-d]pyrimidine ;
la 2-cyclopentyl-4-(4-fluorophényl)-6,7,8,9-tétrahydro-5H-pyrimido[4,5-d]azépine ;
la 2-cyclopentyl-4-p-tolyl-6,7,8,9-tétrahydro-5H-pyrimido[4,5-d]azépine ;
la 2-cyclopentyl-4-(4-méthoxyphényl)-6,7,8,9-tétra-hydro-5H-pyrimido[4,5-d]azépine ;
le 4-(2-cyclopentyl-6,7,8,9-tétrahydro-5H-pyrimido[4,5-d]azépin-4-yl)benzonitrile ;
le chlorhydrate de 4-(4-fluorophényl)-2-isopropyl-6,7,8,9-tétrahydro-5H-pyrimido[4,5-d]azépine ;
la 4-(4-chlorophényl)-2-méthyl-6,7,8,9-tétrahydro-5H-pyrimido[4,5-d]azépine ;
la 2-méthyl-4-phényl-6,7,8,9-tétrahydro-5H-pyrimido-[4,5-d]azépine ;
la 4-(3-chlorophényl)-2-méthyl-6,7,8,9-tétrahydro-5H-pyrimido[4,5-d]azépine ;
la 2-benzyl-4-(4-fluorophényl)-6,7,8,9-tétrahydro-5H-pyrimido[4,5-d]azépine ;
la 2-benzyl-4-p-tolyl-6,7,8,9-tétrahydro-5H-pyrimido-[4,5-d]azépine ;
la 2-benzyl-4-(4-trifluorométhylphényl)-6,7,8,9-tétra-hydro-5H-pyrimido[4,5-d]azépine ;
la 2-(4-fluorobenzyl)-4-(4-fluorophényl)-6,7,8,9-tétrahydro-5H-pyrimido[4,5-d]azépine ;
la 2-cyclopentyl-4-(4-fluorophényl)-7-méthyl-6,7,8,9-tétrahydro-5H-pyrimido[4,5-d]azépine ;
la 2-cyclopentyl-7-méthyl-4-p-tolyl-6,7,8,9-tétrahydro-5H-pyrimido[4,5-d]azépine ;
la 2-cyclopentyl-4-(4-méthoxyphényl)-7-méthyl-6,7,8,9-tétrahydro-5H-pyrimido[4,5-d]azépine ;
la 2-benzyl-7-méthyl-4-p-tolyl-6,7,8,9-tétrahydro-5H-pyrimido[4,5-d]azépine ;
la 2-(4-fluorobenzyl)-4-(4-fluorophényl)-7-méthyl-6,7,8,9-tétrahydro-5H-pyrimido[4,5-d]azépine ;
la 2-(4-fluorobenzyl)-4-(4-fluorophényl)-7-méthyl-9-méthylène-6,7,8,9-tétrahydro-5H-pyrimido[4,5-d]-azépine ;
le chlorhydrate de 2-benzyl-4-(4-fluorophényl)-6,7,8,9-tétrahydro-5H-pyrimido[4,5-c]azépine ;
le chlorhydrate de 4-(4-fluorophényl)-2-isopropyl-6,7,8,9-tétrahydro-5H-pyrimido[4,5-c]azépine ;
le chlorhydrate de 2-isopropyl-4-p-tolyl-6,7,8,9-tétrahydro-5H-pyrimido[4,5-c]azépine ;
la 2-isopropyl-4-(4-méthoxyphényl)-6,7,8,9-tétrahydro-5H-pyrimido[4,5-c]azépine ;
la 2-isopropyl-4-phényl-6,7,8,9-tétrahydro-5H-pyrimido-[4,5-c]azépine ;
le chlorhydrate de 2-benzyl-4-(4-fluorophényl)-6,7,8,9-tétrahydro-5H-1,3,6-triazabenzocycloheptène ;
le chlorhydrate de 2,7-dibenzyl-4-(4-fluorophényl)-5,6,7,8-tétrahydropyrido[3,4-d]pyrimidine ;
la 2,7-dibenzyl-4-p-tolyl-5,6,7,8-tétrahydropyrido[3,4-d]pyrimidine ;
la 2,7-dibenzyl-4-phényl-5,6,7,8-tétrahydropyrido[3,4-d]pyrimidine ;
la 2,7-dibenzyl-4-(4-méthoxyphényl)-5,6,7,8-tétrahydro-pyrido[3,4-d]pyrimidine ;
la 7-benzyl-4-(4-fluorophényl)-2-isopropyl-5,6,7,8-tétrahydropyrido[3,4-d]pyrimidine ;
la 7-benzyl-2-isopropyl-4-phényl-5,6,7,8-tétrahydro-pyrido[3,4-d]pyrimidine ;
le chlorhydrate de 2-benzyl-4-(4-fluorophényl)-5,6,7,8-tétrahydropyrido[3,4-d]pyrimidine ;
le chlorhydrate de 2-benzyl-4-p-tolyl-5,6,7,8-tétrahydropyrido[3,4-d]pyrimidine ;
la 2-benzyl-4-phényl-5,6,7,8-tétrahydropyrido[3,4-d]-pyrimidine ;
la 2-benzyl-4-(4-méthoxyphényl)-5,6,7,8-tétrahydro-pyrido[3,4-d]pyrimidine ;
le chlorhydrate de 4-(4-fluorophényl)-2-isopropyl-5,6,7,8-tétrahydropyrido[3,4-d]pyrimidine ;
la 2-isopropyl-4-phényl-5,6,7,8-tétrahydropyrido[3,4-d]pyrimidine ;
le chlorhydrate de 2-benzyl-4-(4-fluorophényl)-7-méthyl-5,6,7,8-tétrahydropyrido[3,4-d]pyrimidine ;
la 2-benzyl-4-(4-fluorophényl)-7-isopropyl-5,6,7,8-tétrahydropyrido[3,4-d]pyrimidine ;
le chlorhydrate de 4-(4-fluorophényl)-2-isopropyl-7-méthyl-5,6,7,8-tétrahydropyrido[3,4-d]pyrimidine ;
la 2-isopropyl-7-méthyl-4-phényl-5,6,7,8-tétrahydro-pyrido[3,4-d]pyrimidine ;
le chlorhydrate de 7-benzyl-2-isopropyl-4-(5-méthylthiophén-3-yl)-5,6,7,8-tétrahydropyrido[3,4-d]-pyrimidine ;
le chlorhydrate de 7-benzyl-2-isopropyl-4-thiophén-3-yl-5,6,7,8-tétrahydropyrido[3,4-d]pyrimidine ;
le chlorhydrate de 2-isopropyl-4-(5-méthylthiophén-3-yl)-5,6,7,8-tétrahydropyrido[3,4-d]pyrimidine ;
le chlorhydrate de 2-isopropyl-4-thiophén-3-yl-5,6,7,8-tétrahydropyrido[3,4-d]pyrimidine ;
le chlorhydrate de 2-isopropyl-7-méthyl-4-(5-méthylthiophén-3-yl)-5,6,7,8-tétrahydropyrido[3,4-d]-pyrimidine ;
le chlorhydrate de 2-isopropyl-7-méthyl-4-thiophén-3-yl-5,6,7,8-tétrahydropyrido[3,4-d]pyrimidine ;
la 6-benzyl-4-(4-fluorophényl)-2-isopropyl-8-méthyl-5,6,7,8-tétrahydropyrido[4,3-d]pyrimidine ;
la 6-benzyl-4-(3-chloro-4-fluorophényl)-2-isopropyl-8-méthyl-5,6,7,8-tétrahydropyrido[4,3-d]pyrimidine ;
la 6-benzyl-2-isopropyl-8-méthyl-4-p-tolyl-5,6,7,8-tétrahydropyrido[4,3-d]pyrimidine ;
la 6-benzyl-2-isopropyl-8-méthyl-4-phényl-5,6,7,8-tétrahydropyrido[4,3-d]pyrimidine ;
la 6-benzyl-2-isopropyl-8-méthyl-4-(4-trifluorométhyl-phényl)-5,6,7,8-tétrahydropyrido[4,3-d]pyrimidine ;
la 6-benzyl-4-(4-chlorophényl)-2-isopropyl-8-méthyl-5,6,7,8-tétrahydropyrido[4,3-djpyrimidine ;
la 6-benzyl-2-isopropyl-8-méthyl-4-thiophén-3-yl-5,6,7,8-tétrahydropyrido[4,3-d]pyrimidine ;
la 6-benzyl-4-(4'-chlorobiphényl-4-yl)-2-isopropyl-8-méthyl-5,6,7,8-tétrahydropyrido[4,3-d]pyrimidine ;
le chlorhydrate de 4-(4-fluorophényl)-2-isopropyl-8-méthyl-5,6,7,8-tétrahydropyrido[4,3-d]pyrimidine ;
le chlorhydrate de 4-(3-chloro-4-fluorophényl)-2-isopropyl-8-méthyl-5,6,7,8-tétrahydropyrido[4,3-d]-pyrimidine ;
le chlorhydrate de 2-isopropyl-8-méthyl-4-p-tolyl-5,6,7,8-tétrahydropyrido[4,3-d]pyrimidine ;
la 2-isopropyl-8-méthyl-4-phényl-5,6,7,8-tétrahydro-pyrido[4,3-d]pyrimidine ;
la 2-isopropyl-8-méthyl-4-(4-trifluorométhylphényl)-5,6,7,8-tétrahydropyrido[4,3-d]pyrimidine ;
la 2-isopropyl-8-méthyl-4-thiophén-3-yl-5,6,7,8-tétrahydropyrido[4,3-d]pyrimidine ;
le chlorhydrate de 4-(4-fluorophényl)-2-isopropyl-6,7-dihydro-5H-pyrrolo[3,4-d]pyrimidine ;
la 4-(4-fluorophényl)-2-isopropyl-7-pyrrolidin-1-yl-5,6,7,8-tétrahydroquinazoline ;
le chlorhydrate de [4-(4-fluorophényl)-2-isopropyl-5,6,7,8-tétrahydroquinazolin-7-yl]méthylamine ;
le chlorhydrate de [4-(4-fluorophényl)-2-isopropyl-5,6,7,8-tétrahydroquinazolin-6-yl]méthylamine ;
le 4-(4-fluorophényl)-2-isopropyl-5,6,7,8-tétrahydro-quinazolin-7-ol ;
la 4-(4-fluorophényl)-2-isopropyl-5,6,7,8-tétrahydro-quinazoline ;
la (2-benzyl-6-p-tolylpyrimidin-4-ylméthyl)-diméthylamine ;
la 2-benzyl-4-(4-méthylpipérazin-1-ylméthyl)-6-p-tolylpyrimidine ;
la [6-(4-fluorophényl)-2-isopropylpyrimidin-4-ylméthyl]méthylamine ;
la 2-(2-benzyl-6-p-tolylpyrimidin-4-yl)éthylamine ;
la [2-(4-fluorobenzyl)-4-p-tolylpyrimidin-5-ylméthyl]-diméthylamine ;
la 4-(4-fluorophényl)-2-phényl-5,6,7,8-tétrahydro-pyrido[4,3-d]pyrimidine ;
la 2-(3,3-difluorocyclopentyl)-4-(4-fluorophényl)-5,6,7,8-tétrahydropyrido[4,3-d]pyrimidine ;
la 4-(4-fluorophényl)-2-(tétrahydrofuran-3-yl)-5,6,7,8-tétrahydropyrido[4,3-d]pyrimidine ;
la 4-(4-fluorophényl)-2-(2-pipéridin-1-yléthyl)-5,6,7,8-tétrahydropyrido[4,3-d]pyrimidine ;
la 2-(1-fluoro-1-méthyléthyl)-4-(4-fluorophényl)-5,6,7,8-tétrahydropyrido[4,3-d]pyrimidine ;
le 3-(4-fluorophényl)-5-isopropyl-4,6,12-triaza-tricyclo[7.2.1.0^{2,7}]dodéca-2,4,6-triène ;
le 7-(4-fluorophényl)-5-isopropyl-4,6,13-triaza-tricyclo[8.2.1.0^{3,8}]tridéca-3, 5, 7-triène ;
la 4-(4-fluorophényl)-2-(tétrahydropyran-4-yl)-5,6,7,8-tétrahydropyrido[4,3-d]pyrimidine ;
la 4-(4-fluorophényl)-2-(tétrahydropyran-3-yl)-5,6,7,8-tétrahydropyrido[4,3-d]pyrimidine ;
la 4-(4-fluorophényl)-2-(2-méthoxyéthyl)-5,6,7,8-tétrahydropyrido[4,3-d]pyrimidine ;
le 2-[4-(4-fluorophényl)-5,6,7,8-tétrahydropyrido[4,3-d]pyrimidin-2-yl]propan-2-ol ;
la 4-(4-fluorophényl)-2-(1-méthyl-1-phényléthyl)-5,6,7,8-tétrahydropyrido[4,3-d]pyrimidine ;
la 2-cyclopent-3-ényl-4-(4-fluorophényl)-5,6,7,8-tétrahydropyrido[4,3-d]pyrimidine ;
le 3-[4-(4-fluorophényl)-5,6,7,8-tétrahydropyrido[4,3-d]pyrimidin-2-yl]cyclohexanol ;
la 4-(4-fluorophényl)-2-pipéridin-4-yl-5,6,7,8-tétrahydropyrido[4,3-d]pyrimidine ;
la 4-(4-fluorophényl)-2-(1-méthylpipéridin-4-yl)-5,6,7,8-tétrahydropyrido[4,3-d]pyrimidine ;
le [4-(4-fluorophényl)-5,6,7,8-tétrahydropyrido[4,3-d]-pyrimidin-2-yl]phénylméthanol ;
la 4-(4-fluorophényl)-2-(fluorophénylméthyl)-5,6,7,8-tétrahydropyrido[4,3-d]pyrimidine ;
la 2-(difluorophénylméthyl)-4-(4-fluorophényl)-5,6,7,8-tétrahydropyrido[4,3-d]pyrimidine ;
la 4-(4-fluorophényl)-2-phényl-5,6,7,8-tétrahydro-pyrido[4,3-d]pyrimidine ;
la 4-(4-fluorophényl)-2-(3-fluorophényl)-5,6,7,8-tétrahydropyrido[4,3-d]pyrimidine ;
la 4-(4-fluorophényl)-2-(4-méthoxyphényl)-5,6,7,8-tétrahydropyrido[4,3-d]pyrimidine ;
la 4-(4-fluorophényl)-2-o-tolyl-5,6,7,8-tétrahydro-pyrido[4,3-d]pyrimidine ;
le 3-[4-(4-fluorophényl)-5,6,7,8-tétrahydropyrido[4,3-d]pyrimidin-2-yl]benzonitrile ;
la 4-(4-fluorophényl)-2-(2,2,2-trifluoro-1-trifluoro-méthyléthyl)-5,6,7,8-tétrahydropyrido[4,3-d]-pyrimidine ;
la 4-(4-fluorophényl)-2-(1-méthylcyclopropyl)-5,6,7,8-tétrahydropyrido[4,3-d]pyrimidine ;
l'acide 2-[4-(4-fluorophényl)-5,6,7,8-tétrahydro-pyrido[4,3-d]pyrimidin-2-yl]-2-méthylpropionique ;
l'acide 2-[4-(4-fluorophényl)-5,6,7,8-tétrahydro-pyrido[4,3-d]pyrimidin-2-yl]propionique ;
la 2-(4-fluorocyclohexyl)-4-(4-fluorophényl)-5,6,7,8-tétrahydropyrido[4,3-d]pyrimidine ;
la 2-(4,4-difluorocyclohexyl)-4-(4-fluorophényl)-5,6,7,8-tétrahydropyrido[4,3-d]pyrimidine ;
la 4-(4-fluorophényl)-2-phénéthyl-5,6,7,8-tétrahydro-pyrido[4,3-d]pyrimidine ;
la 4-furan-2-yl-2-isopropyl-5,6,7,8-tétrahydropyrido-[4,3-d]pyrimidine ;
la 2-isopropyl-4-(5-méthylfuran-2-yl)-5,6,7,8-tétrahydropyrido[4,3-d]pyrimidine ;
la 4-furan-3-yl-2-isopropyl-5,6,7,8-tétrahydropyrido-[4,3-d]pyrimidine ;
la 4-(5-fluoropyridin-2-yl)-2-isopropyl-5,6,7,8-tétrahydropyrido[4,3-d]pyrimidine ;
la 2-isopropyl-4-oxazol-2-yl-5,6,7,8-tétrahydropyrido-[4,3-d]pyrimidine ;
la 4-(4,5-diméthyloxazol-2-yl)-2-isopropyl-5,6,7,8-tétrahydropyrido[4,3-djpyrimidine ;
la 2-isopropyl-4-thiazol-2-yl-5,6,7,8-tétrahydropyrido-[4,3-d]pyrimidine ;
la 2-isopropyl-4-(3H-[1,2,3]triazol-4-yl)-5,6,7,8-tétrahydropyrido[4,3-d]pyrimidine ;
la 2-isopropyl-4-(2H-pyrazol-3-yl)-5,6,7,8-tétrahydro-pyrido[4,3-d]pyrimidine ;
la 2-isopropyl-4-(1H-pyrazol-4-yl)-5,6,7,8-tétrahydro-pyrido[4,3-d]pyrimidine ;
la 4-(4-fluorophényl)-2,6-diisopropyl-5,6,7,8-tétrahydropyrido[4,3-d]pyrimidine ;
la 6-éthyl-4-(4-fluorophényl)-2-isopropyl-5,6,7,8-tétrahydropyrido[4,3-d]pyrimidine ;
la 6-cyclopropyl-4-(4-fluorophényl)-2-isopropyl-5,6,7,8-tétrahydropyrido[4,3-d]pyrimidine ;
la 6-cyclobutyl-4-(4-fluorophényl)-2-isopropyl-5,6,7,8-tétrahydropyrido[4,3-d]pyrimidine ;
la 6-cyclopentyl-4-(4-fluorophényl)-2-isopropyl-5,6,7,8-tétrahydropyrido[4,3-d]pyrimidine ;
la 6-butyl-4-(4-fluorophényl)-2-isopropyl-5,6,7,8-tétrahydropyrido[4,3-d]pyrimidine ; et
la 4-(4-fluorophényl)-2-isopropyl-5,6,7,8-tétrahydro-pyrido[4,3-d]pyrimidine, sel de citrate.

29. Composé selon la revendication 1, dans lequel ledit sel pharmaceutiquement acceptable est un sel d'addition d'amino efficace.

30. Composé selon la revendication 1, dans lequel ledit sel pharmaceutiquement acceptable est sélectionné dans le groupe constitué par le bromhydrate, chlorhydrate, sulfate, bisulfate, nitrate, acétate, oxalate, valérate, oléate, palmitate, stéarate, laurate, borate, benzoate, lactate, phosphate, tosylate, citrate, maléate, fumarate, succinate, tartrate, naphtylate, mésylate, glucoheptonate, lactobionate et laurylsulfonate.

31. Composition pharmaceutique comprenant un véhicule pharmaceutiquement acceptable et une quantité thérapeutiquement efficace d'au moins un composé de Formule (I) ou (II) : 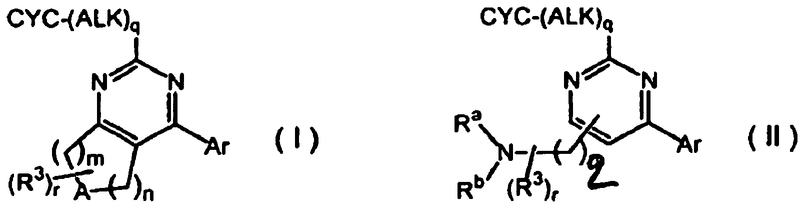 dans laquelle
m vaut 1, 2 ou 3 ;
n vaut 1, 2 ou 3 ;
où lorsque m et n sont tous deux présents, m + n est supérieur ou égal à 2, et est inférieur ou égal à 4 ;
R^{a} et R^{b} sont indépendamment -H, - C₁₋₇alkyle, ou -C₃₋₇cycloalkyle, ou R^{a} et R^{b}, pris conjointement avec l'azote auquel ils sont liés, forment pipéridinyle, pyrrolidinyle, morpholinyle, thiomorpholinyle ou pipérazinyle, où chaque R^{a} et R^{b} est éventuellement et indépendamment substitué par -C₁₋₄alkyle ;
q vaut 0 ou 1 ;
A représente >NR¹, >CHNR^{c}R^{d}, >CHOH, ou -CH₂-, où
R¹ est sélectionné dans le groupe constitué par -H, -C₁-₇alkyle, -C₃₋₇cycloalkyle et benzyle, où chaque alkyle, cycloalkyle ou benzyle est éventuellement mono-, di- ou trisubstitué par R^{e} ;
R^{e} est sélectionné dans le groupe constitué par -C₁-₄alkyle, -C₂₋₄alcényle, -C₂₋₄alcynyle, -C₃₋₆cycloal kyle, halogéno, -CF₃, -OH, -OC₁₋₄alkyle, -OCF₃, -N(R^{f})R^{g} (où R^{f} et R^{g} sont indépendamment -H ou -C₁₋₄alkyle, ou R^{f} et R^{g}, pris conjointement avec l'azote auquel ils sont liés, forment pipéridinyle, pyrrolidinyle, morpholinyle, thiomorpholinyle ou pipérazinyle), -C(O)N(R^{f})R^{g}, -N(R^{h})C(O)R^{h}, -N(R^{h})SO₂C₁₋₇alkyle (où R^{h} est -H ou -C₁-₄alkyle, ou deux R^{h} dans le même substituant, pris conjointement avec l'amide auquel ils sont liés, forment un cycle de 4 à 6 chaînons par ailleurs aliphatique), -S (O)₀₋₂-C₁₋₄alkyle, -SO₂N(R^{f})R^{g}, -SCF₃, -C(O)C₁₋₄alkyle, -CN, -COOH et -COOC₁₋₄alkyle ;
R^{c} et R^{d} sont sélectionnés indépendamment dans le groupe constitué par -H, -C₁₋₇alkyle, -C₃₋₇alcényle, -C₃₋₇alcynyle, -C₃₋₇cycloalkyle, -C₁₋₇alkylC₃₋₇cycloalkyle et -C₃₋₇cycloalkylC₁₋₇alkyle, ou R^{c} et R^{d}, pris conjointement avec l'azote auquel ils sont liés, forment pipéridinyle, pyrrolidinyle, morpholinyle, thiomorpholinyle ou pipérazinyle, où chaque R^{c} et R^{d} est éventuellement et indépendamment substitué par R^{e} ;
R³ est -C₁₋₄alkyle, -C₁₋₄alcényle ou benzyle, chacun éventuellement substitué par -C₁₋₃alkyle, -OH ou halogéno, ou deux substituants R³ pris conjointement forment C₂₋₅alkylène éventuellement substitué par -C₁-₃alkyle, -OH ou halogéno ;
r vaut 0 ou est un entier inférieur ou égal à m + n + 1 ;
Ar est un cycle aryle ou hétéroaryle sélectionné dans le groupe constitué par :
a) phényle, éventuellement mono-, di- ou trisubstitué par R¹ ou disubstitué sur des carbones adjacents par -OC₁₋₄alkylèneO-, -(CH₂)₂₋₃NH-, -(CH₂)₁-₂NH(CH₂)-, - (CH₂)₂₋₃N(C₁₋₄alkyl)-, ou -(CH₂)₁₋₂N(C₁-₄alkyl)(CH₂)- ;
Rⁱ est sélectionné dans le groupe constitué par -C₁₋₇alkyle, -C₂₋₇alcényle, -C₂₋₇alcynyle, -C₃₋₇cycloalkyle, halogéno, -CF₃, -OH, -OC₁₋₇alkyle, -OCF₃, -OC₃₋₇alcényle, -OC₃₋₇alcynyle, -N(R^{j})R^{k} (où R^{j} et R^{k} sont indépendamment -H ou -C₁₋₄alkyle), -C(O)N(R^{j})R^{k}, -N(R^{j})C(O)R^{k}, -N(R^{j})SO₂C₁₋₆alkyle, -S(O)₀₋₂-C₁₋₆alkyle, -SO₂N(R^{j})R^{k}, -SCF₃, -C(O)C₁₋₆alkyle, -NO₂, -CN, -COOH et -COOC₁₋₇alkyle ;
b) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à deux atomes de carbone supplémentaires éventuellement remplacés par -N=, éventuellement mono-ou disubstitué par Rⁱ ;
c) un groupement hydrocarboné aromatique monocyclique à six atomes de cycle, ayant un atome de carbone comme point de liaison, ayant un ou deux atomes de carbone remplacés par -N=, éventuellement mono- ou disubstitué par Rⁱ ; et
d) phényle ou pyridyle, substitué par un substituant sélectionné dans le groupe constitué par phényle, phénoxy, pyridyle, thiophényle, oxazolyle et tétrazolyle, où le motif substitué résultant est éventuellement en outre mono-, di- ou trisubstitué par Rⁱ ;
ALK est C₁₋₇alkylène, C₂₋₇alcénylène, C₂₋₇alcynylène, C₃₋₇cycloalkylène, ou C₃₋₇cycloalcénylène ramifié ou non ramifié, éventuellement mono-, di- ou trisubstitué par R^{m} ;
R^{m} est sélectionné dans le groupe constitué par halogéno, -CF₃, -OH, -OC₁₋₇alkyle, -OC₃₋₇cycloalkyle, -OCF₃, -N(R^{p})R^{s} (où R^{p} et R^{s} sont indépendamment -H ou -C₁₋₇alkyle), -C (O) N (R^{p}) R^{s}, -N (R^{t})C(O) R^{t}, -N(R^{t})SO₂C₁₋₆alkyle (où R^{t} est -H ou -C₁₋₇alkyle), -S(O)₀₋₂-C₁₋₆alkyle, -SO₂N(R^{p})R^{s}, -SCF₃, -CN, -NO₂, -C(O)C₁₋₇alkyle, -COOH et -COOC₁₋₇alkyle ;
CYC est -H ou est un noyau sélectionné dans le groupe constitué par :
i) phényle, éventuellement mono-, di- ou trisubstitué par R^{u} ou disubstitué sur des carbones adjacents par -OC₁₋₄alkylèneO-, -(CH₂)₂₋₃NH-, -(CH₂)₁-₂NH (CH₂) -, - (CH₂)₂₋₃N(C₁₋₄alkyl) -, ou - (CH₂)₁₋₂N(C₁₋₄alkyl) (CH₂) - ;
R^{u} est sélectionné dans le groupe constitué par -C₁₋₇alkyle, -C₃₋₇cycloalkyle, phényle, benzyle, halogéno, -CF₃, -OH, -OC₁₋₇alkyle, -OC₃₋₇cycloalkyle, -Ophényle, -Obenzyle, -OCF₃, -N (R^{v}) R^{w} (où R^{v} et R^{w} sont indépendamment -H ou -C₁₋₇alkyle, ou R^{v} et R^{w}, pris conjointement avec l'azote auquel ils sont liés, forment pipéridinyle, pyrrolidinyle, morpholinyle, thiomorpholinyle ou pipérazinyle, où chaque R^{v} et R^{w} est éventuellement et indépendamment substitué par -OH ou -C₁₋₇alkyle), -C(O)N(R^{v})R^{w}, -N(R^{x})C(O)R^{x}, -N(R^{x})SO₂C₁₋₆alkyle (où R^{x} est -H ou -C₁₋₇alkyle, ou deux R^{x} dans le même substituant, pris conjointement avec l'amide auquel ils sont liés, forment un cycle de 4 à 6 chaînons par ailleurs aliphatique), -N-(SO₂C₁₋₆alkyle)₂, -S (O)₀₋₂-C₁₋₆alkyle, -SO₂N (R^{v}) R^{w}, -SCF₃, -C (O) C₁₋₆alkyle, -NO₂, -CN, -COOH et -COOC₁₋₇alkyle ;
ii) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à un atome de carbone supplémentaire éventuellement remplacé par -N=, éventuellement mono-ou disubstitué par R^{u} ;
iii) un groupement hydrocarboné aromatique monocyclique à six atomes de cycle, ayant un atome de carbone comme point de liaison, ayant un ou deux atomes de carbone remplacés par -N=, éventuellement mono- ou disubstitué par R^{u} ; et
iv) un cycle hétérocyclique non aromatique de 4 à 8 chaînons, ledit cycle ayant 0, 1 ou 2 chaînons d'hétéroatomes non adjacents sélectionnés dans le groupe constitué par O, S, -N=, >NH et >N(C₁₋₄alkyl), ayant 0, 1 ou 2 doubles liaisons, ayant 0, 1 ou 2 chaînons de carbone s'agissant de carbonyle, éventuellement ayant un chaînon de carbone qui forme un pont, ayant 0 à 5 substituants R^{u}, et où, quand q représente 0, ledit cycle comporte un atome de carbone comme point de liaison ;
ainsi que les énantiomères, diastéréoisomères, hydrates, solvates et sels pharmaceutiquement acceptables, esters et amides de celui-ci ;
à condition que dans la Formule (I) :
(a) lorsque ALK est méthylène, éthylène, propylène, ou isopropylène, CYC est -H, Ar est phényle ou phényle monosubstitué, m vaut 2, n vaut 1 et A représente >NR¹, alors R¹ n'est pas -C₁₋₄alkyle ou benzyle ;
(b) lorsque q vaut 0, CYC est phényle, Ar est phényle ou 3-chlorophényle, m vaut 2 et n vaut 1, alors A n'est pas -CH₂- non substitué ; et
(c) lorsque q vaut 0, CYC est 2-pyridyle, Ar est 2-pyridyle, m vaut 2 et n vaut 1, alors A n'est pas -CH₂- non substitué.

32. Composé de Formule (I) ou (II) : 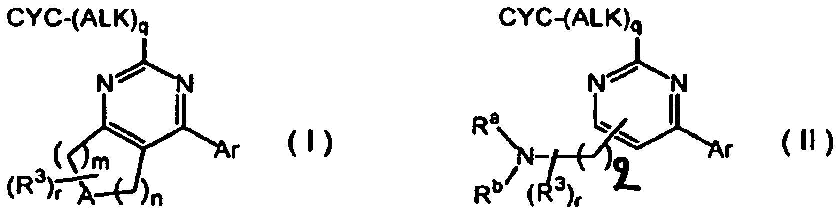 dans laquelle
m vaut 1, 2 ou 3 ;
n vaut 1, 2 ou 3 ;
où lorsque m et n sont tous deux présents, m + n est supérieur ou égal à 2, et est inférieur ou égal à 4 ;
R^{a} et R^{b} sont indépendamment -H, - C₁₋₇alkyle, ou -C₃₋₇cycloalkyle, ou R^{a} et R^{b}, pris conjointement avec l'azote auquel ils sont liés, forment pipéridinyle, pyrrolidinyle, morpholinyle, thiomorpholinyle ou pipérazinyle, où chaque R^{a} et R^{b} est éventuellement et indépendamment substitué par -C₁₋₄alkyle ;
q vaut 0 ou 1 ;
A représente >NR¹, >CHNR^{c}R^{d}, >CHOH, ou -CH₂-, où R¹ est sélectionné dans le groupe constitué par -H, -C₁-₇alkyle, -C₃₋₇cycloalkyle et benzyle, où chaque alkyle, cycloalkyle ou benzyle est éventuellement mono-, di- ou trisubstitué par R^{e} ;
R^{e} est sélectionné dans le groupe constitué par -C₁₋₄alkyle, -C₂₋₄alcényle, -C₂₋₄alcynyle, -C₃₋₆cycloalkyle, halogéno, -CF₃, -OH, -OC₁₋₄alkyle, -OCF₃, -N(R^{f})R^{g} (où R^{f} et R^{g} sont indépendamment -H ou -C₁₋₄alkyle, ou R^{f} et R^{g}, pris conjointement avec l'azote auquel ils sont liés, forment pipéridinyle, pyrrolidinyle, morpholinyle, thiomorpholinyle ou pipérazinyle), -C(O)N(R^{f})R^{g}, -N(R^{h}) C(O)R^{h}, -N(R^{h})SO₂C₁₋₇alkyle (où R^{h} est -H ou -C₁₋₄alkyle, ou deux R^{h} dans le même substituant, pris conjointement avec l'amide auquel ils sont liés, forment un cycle de 4 à 6 chaînons par ailleurs aliphatique), -S (O)₀₋₂-C₁₋₄alkyle, -SO₂N (R^{f}) R^{g}, -SCF₃, -C(O)C₁₋₄alkyle, -CN, -COOH et -COOC₁₋₄alkyle ;
R^{c} et R^{d} sont sélectionnés indépendamment dans le groupe constitué par -H, -C₁₋₇alkyle, -C₃₋₇alcényle, -C₃₋₇alcynyle, -C₃₋₇cycloalkyle, -C₁₋₇alkylC₃₋₇cycloalkyle et -C₃₋₇cycloalkylC₁₋₇alkyle, ou R^{c} et R^{d}, pris conjointement avec l'azote auquel ils sont liés, forment pipéridinyle, pyrrolidinyle, morpholinyle, thiomorpholinyle ou pipérazinyle, où chaque R^{c} et R^{d} est éventuellement et indépendamment substitué par R^{e} ;
R³ est -C₁₋₄alkyle, -C₁₋₄alcényle ou benzyle, chacun éventuellement substitué par -C₁₋₃alkyle, -OH ou halogéno, ou deux substituants R³ pris conjointement forment C₂₋₅alkylène éventuellement substitué par -C₁₋₃alkyle, -OH ou halogéno ;
r vaut 0 ou est un entier inférieur ou égal à m + n + 1 ;
Ar est un cycle aryle ou hétéroaryle sélectionné dans le groupe constitué par :
a) phényle, éventuellement mono-, di- ou trisubstitué par Rⁱ ou disubstitué sur des carbones adjacents par -OC₁₋₄alkylèneO-, -(CH₂)₂₋₃NH-, -(CH₂)₁-₂NH(CH₂)-, - (CH₂)₂₋₃N (C₁₋₄alkyl) -, ou - (CH₂)₁₋₂N (C₁₋₄alkyl) (CH₂)- ;
Rⁱ est sélectionné dans le groupe constitué par -C₁₋₇alkyle, -C₂₋₇alcényle, -C₂₋₇alcynyle, -C₃₋₇cycloalkyle, halogéno, -CF₃, -OH, -OC₁₋₇alkyle, -OCF₃, -OC₃₋₇alcényle, -OC₃₋₇alcynyle, -N(R^{j})R^{k} (où R^{j} et R^{k} sont indépendamment -H ou -C₁₋₄alkyle), -C(O)N(R^{j})R^{k}, -N(R^{j})C(O)R^{k}, -N (R^{j}) SO₂C₁₋₆alkyle, -S (O)₀₋₂-C₁₋₆alkyle, -SO₂N(R^{j})R^{k}, -SCF₃, -C(O)C₁₋₆alkyle, -NO₂, -CN, -COOH et -COOC₁₋₇alkyle ;
b) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à deux atomes de carbone supplémentaires éventuellement remplacés par -N=, éventuellement mono-ou disubstitué par Rⁱ ;
c) un groupement hydrocarboné aromatique monocyclique à six atomes de cycle, ayant un atome de carbone comme point de liaison, ayant un ou deux atomes de carbone remplacés par -N=, éventuellement mono- ou disubstitué par Rⁱ ; et
d) phényle ou pyridyle, substitué par un substituant sélectionné dans le groupe constitué par phényle, phénoxy, pyridyle, thiophényle, oxazolyle et tétrazolyle, où le motif substitué résultant est éventuellement en outre mono-, di- ou trisubstitué par Rⁱ ;
ALK est C₁₋₇alkylène, C₂₋₇alcénylène, C₂₋₇alcynylène, C₃₋₇cycloalkylène, ou C₃₋₇cycloalcénylène ramifié ou non ramifié, éventuellement mono-, di- ou trisubstitué par R^{m} ;
R^{m} est sélectionné dans le groupe constitué par halogéno, -CF₃, -OH, -OC₁₋₇alkyle, -OC₃₋₇cycloalkyle, -OCF₃, -N(R^{p})R^{s} (où R^{p} et R^{s} sont indépendamment -H ou -C₁₋₇alkyle), -C(O)N(R^{p})R^{s}, -N(R^{t})C(O)R^{t}, -N(R^{t})SO₂C_{1- 6}alkyle (où R^{t} est -H ou -C₁₋₇alkyle), -S(O)₀₋₂-C₁₋₆alkyle, -SO₂N(R^{p})R^{s}, -SCF₃, -CN, -NO₂, -C(O)C₁₋₇alkyle, -COOH et -COOC₁₋₇alkyle ;
CYC est -H ou est un noyau sélectionné dans le groupe constitué par :
i) phényle, éventuellement mono-, di- ou trisubstitué par R^{u} ou disubstitué sur des carbones adjacents par -OC₁₋₄alkylèneO-, -(CH₂)₂₋₃NH-, -(CH₂)₁₋₂NH(CH₂)-, -(CH2)₂₋₃N(C₁₋₄alkyl)-, ou -(CH₂)₁₋₂N(C₁₋₄alkyl) (CH₂) - ;
R^{u} est sélectionné dans le groupe constitué par -C₁₋₇alkyle, -C₃₋₇cycloalkyle, phényle, benzyle, halogéno, -CF₃, -OH, -OC₁₋₇alkyle, -OC₃₋₇cycloalkyle, -Ophényle, -Obenzyle, -OCF₃, -N (R^{v}) R^{w} (où R^{v} et R^{w} sont indépendamment -H ou -C₁₋₇alkyle, ou R^{v} et R^{w}, pris conjointement avec l'azote auquel ils sont liés, forment pipéridinyle, pyrrolidinyle, morpholinyle, thiomorpholinyle ou pipérazinyle, où chaque R^{v} et R^{w} est éventuellement et indépendamment substitué par -OH ou -C₁₋₇alkyle), -C(O)N(R^{v})R^{w}, -N(R^{x})C(O)R^{x}, -N(R^{x})SO₂C₁₋₆alkyle (où R^{x} est -H ou -C₁₋₇alkyle, ou deux R^{x} dans le même substituant, pris conjointement avec l'amide auquel ils sont liés, forment un cycle de 4 à 6 chaînons par ailleurs aliphatique), -N-(SO₂C₁₋₆alkyle)₂, -S(O)₀₋₂-C₁₋₆alkyle, -SO₂N(R^{v})R^{w}, -SCF₃, -C(O)C₁₋₆alkyle, -NO_{2,} -CN, -COOH et -COOC₁₋₇alkyle ;
ii) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à un atome de carbone supplémentaire éventuellement remplacé par -N=, éventuellement mono-ou disubstitué par R^{u} ;
iii) un groupement hydrocarboné aromatique monocyclique à six atomes de cycle, ayant un atome de carbone comme point de liaison, ayant un ou deux atomes de carbone remplacés par -N=, éventuellement mono- ou disubstitué par R^{u} ; et
iv) un cycle hétérocyclique non aromatique de 4 à 8 chaînons, ledit cycle ayant 0, 1 ou 2 chaînons d'hétéroatomes non adjacents sélectionnés dans le groupe constitué par O, S, -N=, >NH et >N(C₁₋₄alkyl), ayant 0, 1 ou 2 doubles liaisons, ayant 0, 1 ou 2 chaînons de carbone s'agissant de carbonyle, éventuellement ayant un chaînon de carbone qui forme un pont, ayant 0 à 5 substituants R^{u}, et où, quand q représente 0, ledit cycle comporte un atome de carbone comme point de liaison ;
ainsi que les énantiomères, diastéréoisomères, hydrates, solvates et sels pharmaceutiquement acceptables, esters et amides de celui-ci ;
à condition que dans la Formule (I) :
(a) lorsque ALK est méthylène, éthylène, propylène, ou isopropylène, CYC est -H, Ar est phényle ou phényle monosubstitué, m vaut 2, n vaut 1 et A représente >NR¹, alors R¹ n' est pas -C₁₋₄alkyle ou benzyle ;
(b) lorsque q vaut 0, CYC est phényle, Ar est phényle ou 3-chlorophényle, m vaut 2 et n vaut 1, alors A n'est pas -CH₂- non substitué ; et
(c) lorsque q vaut 0, CYC est 2-pyridyle, Ar est 2-pyridyle, m vaut 2 et n vaut 1, alors A n'est pas -CH₂- non substitué, pour une utilisation en thérapie.

33. Utilisation d'au moins un composé de Formule (I) ou (II) : 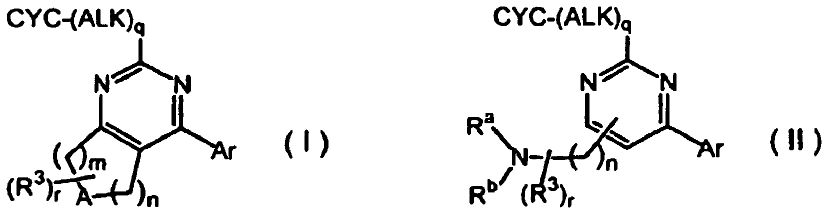 dans laquelle
m vaut 1, 2 ou 3 ;
n vaut 1, 2 ou 3 ;
où lorsque m et n sont tous deux présents, m + n est supérieur ou égal à 2, et est inférieur ou égal à 4 ;
R^{a} et R^{b} sont indépendamment -H, - C₁₋₇alkyle, ou -C₃₋₇cycloalkyle, ou R^{a} et R^{b}, pris conjointement avec l'azote auquel ils sont liés, forment pipéridinyle, pyrrolidinyle, morpholinyle, thiomorpholinyle ou pipérazinyle, où chaque R^{a} et R^{b} est éventuellement et indépendamment substitué par -C₁₋₄alkyle ;
q vaut 0 ou 1 ;
A représente >NR¹, >CHNR^{c}R^{d}, >CHOH, ou -CH₂-, où R¹ est sélectionné dans le groupe constitué par -H, -C₁₋₇alkyle, -C₃₋₇cycloalkyle et benzyle, où chaque alkyle, cycloalkyle ou benzyle est éventuellement mono-, di- ou trisubstitué par R^{e} ;
R^{e} est sélectionné dans le groupe constitué par -C₁₋₄alkyle, -C₂₋₄alcényle, -C₂₋₄alcynyle, -C₃₋₆cycloalkyle, halogéno, -CF₃, -OH, -OC₁₋₄alkyle, -OCF₃, -N(R^{f})R^{g} (où R^{f} et R^{g} sont indépendamment -H ou -C₁₋₄alkyle, ou R^{f} et R^{g}, pris conjointement avec l'azote auquel ils sont liés, forment pipéridinyle, pyrrolidinyle, morpholinyle, thiomorpholinyle ou pipérazinyle), -C(O)N(R^{f})R^{g}, -N(R^{h})C(O)R^{h}, -N(R^{h})SO₂C₁₋₇alkyle (où R^{h} est -H ou -C₁₋₄alkyle, ou deux R^{h} dans le même substituant, pris conjointement avec l'amide auquel ils sont liés, forment un cycle de 4 à 6 chaînons par ailleurs aliphatique), -S (O)₀₋₂-C₁₋₄alkyle, -SO₂N (R^{f}) R^{g}, -SCF₃, -C(O)C₁₋₄alkyle, -CN, -COOH et -COOC₁₋₄alkyle ;
R^{c} et R^{d} sont sélectionnés indépendamment dans le groupe constitué par -H, -C₁₋₇alkyle, -C₃₋₇alcényle, -C₃₋₇alcynyle, -C₃₋₇cycloalkyle, -C₁₋₇alkylC₃₋₇cycloalkyle et -C₃₋₇cycloalkylC₁₋₇alkyle, ou R^{c} et R^{d}, pris conjointement avec l'azote auquel ils sont liés, forment pipéridinyle, pyrrolidinyle, morpholinyle, thiomorpholinyle ou pipérazinyle, où chaque R^{c} et R^{d} est éventuellement et indépendamment substitué par R^{e} ;
R³ est -C₁₋₄alkyle, -C₁₋₄alcényle ou benzyle, chacun éventuellement substitué par -C₁₋₃alkyle, -OH ou halogéno, ou deux substituants R³ pris conjointement forment C₂₋₅alkylène éventuellement substitué par -C₁₋₃alkyle, -OH ou halogéno ;
r vaut 0 ou est un entier inférieur ou égal à m + n + 1 ;
Ar est un cycle aryle ou hétéroaryle sélectionné dans le groupe constitué par :
a) phényle, éventuellement mono-, di- ou trisubstitué par Rⁱ ou disubstitué sur des carbones adjacents par -OC₁₋₄alkylèneO-, -(CH₂)₂₋₃NH-, -(CH₂)₁₋₂NH (CH₂) -, -(CH₂)₂₋₃N(C₁₋₄alkyl)-, ou -(CH₂)₁₋₂N(C₁₋₄alkyl) (CH₂) - ;
Rⁱ est sélectionné dans le groupe constitué par -C₁₋₇alkyle, -C₂₋₇alcényle, -C₂₋₇alcynyle, -C₃₋₇cycloalkyle, halogéno, -CF₃, -OH, -OC₁₋₇alkyle, -OCF₃, -OC₃₋₇alcényle, -OC₃₋₇alcynyle, -N(R^{j})R^{k} (où R^{j} et R^{k} sont indépendamment -H ou -C₁₋₄alkyle), -C(O)N(R^{j})R^{k}, -N(R^{j})C(O)R^{k}, -N(R^{j})SO₂C₁₋₆alkyle, -S(O)₀₋₂-C₁₋₆alkyle, -SO₂N(R^{j})R^{k}, -SCF₃, -C(O)C₁₋₆alkyle, -NO₂, -CN, -COOH et -COOC₁₋₇alkyle ;
b) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à deux atomes de carbone supplémentaires éventuellement remplacés par -N=, éventuellement mono-ou disubstitué par Rⁱ ;
c) un groupement hydrocarboné aromatique monocyclique à six atomes de cycle, ayant un atome de carbone comme point de liaison, ayant un ou deux atomes de carbone remplacés par -N=, éventuellement mono- ou disubstitué par Rⁱ ; et
d) phényle ou pyridyle, substitué par un substituant sélectionné dans le groupe constitué par phényle, phénoxy, pyridyle, thiophényle, oxazolyle et tétrazolyle, où le motif substitué résultant est éventuellement en outre mono-, di- ou trisubstitué par Rᵢ ;
ALK est C₁₋₇alkylène, C₂₋₇alcénylène, C₂₋₇alcynylène, C₃₋₇cycloalkylène, ou C₃₋₇cycloalcénylène ramifié ou non ramifié, éventuellement mono-, di- ou trisubstitué par R^{m} ;
R^{m} est sélectionné dans le groupe constitué par halogéno, -CF₃, -OH, -OC₁₋₇alkyle, -OC₃₋₇cycloalkyle, -OCF₃, -N(R^{p})R^{s} (où R^{p} et R^{s} sont indépendamment -H ou -C₁₋₇alkyle), -C(O)N(R^{p})R^{s}, -N(R^{t})C(O)R^{t}, -N(R^{t})SO₂C₁₋₆alkyle (où R^{t} est -H ou -C₁₋₇alkyle), -S(O)₀₋₂-C₁₋₆alkyle, -SO₂N(R^{p})R^{s}, -SCF₃, -CN, -NO₂, -C(O)C₁₋₇alkyle, -COOH et -COOC₁₋₇alkyle ;
CYC est -H ou est un noyau sélectionné dans le groupe constitué par :
i) phényle, éventuellement mono-, di- ou trisubstitué par R^{u} ou disubstitué sur des carbones adjacents par -OC₁₋₄alkylèneO-, -(CH₂)₂₋₃NH-, -(CH₂)₁₋₂NH(CH₂)-, -(CH₂)₂₋₃N (C₁₋₄alkyl)-, ou -(CH₂)₁₋₂N(C₁₋₄alkyl)(CH₂)- ;
R^{u} est sélectionné dans le groupe constitué par -C₁₋₇alkyle, -C₃₋₇cycloalkyle, phényle, benzyle, halogéno, -CF₃, -OH, -OC₁₋₇alkyle, -OC₃₋₇cycloalkyle, -Ophényle, -Obenzyle, -OCF₃, -N(R^{v})R^{w} (où R^{v} et R^{w} sont indépendamment -H ou -C₁₋₇alkyle, ou R^{v} et R^{w}, pris conjointement avec l'azote auquel ils sont liés, forment pipéridinyle, pyrrolidinyle, morpholinyle, thiomorpholinyle ou pipérazinyle, où chaque R^{v} et R^{w} est éventuellement et indépendamment substitué par -OH ou -C₁₋₇alkyle), -C(O)N(R^{v})R^{w}, -N(R^{x})C(O)R^{x}, -N(R^{x})SO₂C₁₋₆alkyle (où R^{x} est -H ou -C₁₋₇alkyle, ou deux R^{x} dans le même substituant, pris conjointement avec l'amide auquel ils sont liés, forment un cycle de 4 à 6 chaînons par ailleurs aliphatique), -N-(SO₂C₁₋₆alkyle)₂, -S(O)₀₋₂-C₁₋₆alkyle, -SO₂N (R^{v}) R^{w}, -SCF₃, -C(O)C₁₋₆alkyle, -NO₂, -CN, -COOH et -COOC₁₋₇alkyle ;
ii) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à un atome de carbone supplémentaire éventuellement remplacé par -N=, éventuellement mono-ou disubstitué par R^{u} ;
iii) un groupement hydrocarboné aromatique monocyclique à six atomes de cycle, ayant un atome de carbone comme point de liaison, ayant un ou deux atomes de carbone remplacés par -N=, éventuellement mono- ou disubstitué par R^{u} ; et
iv) un cycle hétérocyclique non aromatique de 4 à 8 chaînons, ledit cycle ayant 0, 1 ou 2 chaînons d'hétéroatomes non adjacents sélectionnés dans le groupe constitué par O, S, -N=, >NH et >N(C₁₋₄alkyl), ayant 0, 1 ou 2 doubles liaisons, ayant 0, 1 ou 2 chaînons de carbone s'agissant de carbonyle, éventuellement ayant un chaînon de carbone qui forme un pont, ayant 0 à 5 substituants R^{u}, et où, quand q représente 0, ledit cycle comporte un atome de carbone comme point de liaison ;
ainsi que les énantiomères, diastéréoisomères, hydrates, solvates et sels pharmaceutiquement acceptables, esters et amides de celui-ci ;
pour la fabrication d'un médicament destiné au traitement ou à la prévention d'un trouble du SNC choisi dans le groupe constitué par les troubles du sommeil, la dépression/anxiété, le trouble de l'anxiété généralisée, la schizophrénie, les troubles bipolaires, les troubles psychotiques, le trouble obsessionnel-compulsif, les troubles de l'humeur, le stress post-traumatique et autres troubles liés au stress, la migraine, les douleurs, les troubles de l'alimentation, l'obésité, les dysfonctionnements sexuels, les perturbations métaboliques, les déséquilibres hormonaux, l'alcoolisme, les troubles de dépendance, la nausée, les inflammations, l'hypertension centralement médiée, les troubles du sommeil/réveil, les symptômes du décalage horaire, et les anomalies du rythme veille-sommeil chez les mammifères ;
au traitement ou à la prévention d'une maladie ou d'une condition choisie dans le groupe constitué par l'hypotension, les troubles vasculaires périphériques, le choc cardio-vasculaire, les troubles rénaux, la motilité gastrique, la diarrhée, le côlon spastique, le trouble du côlon irritable, les ischémies, le choc septique, l'incontinence urinaire, et d'autres troubles liés aux systèmes gastro-intestinaux et vasculaires chez les mammifères ;
au traitement ou à la prévention d'un trouble oculaire choisi dans le groupe constitué par le glaucome, la névrite optique, la rétinopathie diabétique, l'oedème rétinien, et la dégénérescence maculaire liée à l'âge chez les mammifères.

34. Utilisation selon la revendication 33, dans laquelle ledit trouble du SNC est choisi dans le groupe constitué par la dépression/anxiété, les troubles du sommeil, et les anomalies du rythme veille-sommeil.

35. Composé de Formule (I) ou (II) : 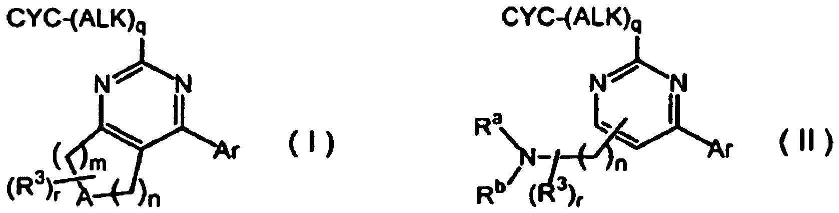 dans laquelle
m vaut 1, 2 ou 3 ;
n vaut 1, 2 ou 3 ;
où lorsque m et n sont tous deux présents, m + n est supérieur ou égal à 2, et est inférieur ou égal à 4 ;
R^{a} et R^{b} sont indépendamment -H, - C₁₋₇alkyle, ou -C₃₋₇cycloalkyle, ou R^{a} et R^{b}, pris conjointement avec l'azote auquel ils sont liés, forment pipéridinyle, pyrrolidinyle, morpholinyle, thiomorpholinyle ou pipérazinyle, où chaque R^{a} et R^{b} est éventuellement et indépendamment substitué par -C₁₋₄alkyle
q vaut 0 ou 1 ;
A représente >NR¹, >CHNR^{c}R^{d}, >CHOH, ou -CH₂-, où
R¹ est sélectionné dans le groupe constitué par -H, -C₁₋₇alkyle, -C₃₋₇cycloalkyle et benzyle, où chaque alkyle, cycloalkyle ou benzyle est éventuellement mono-, di- ou trisubstitué par R^{e} ;
R^{e} est sélectionné dans le groupe constitué par -C₁₋₄alkyle, -C₂₋₄alcényle, -C₂₋₄alcynyle, -C₃₋₆cycloalkyle, halogéno, -CF₃, -OH, -OC₁₋₄alkyle, -OCF₃, -N(R^{f})R^{g} (où R^{f} et R^{g} sont indépendamment -H ou -C₁₋₄alkyle, ou R^{f} et R^{g}, pris conjointement avec l'azote auquel ils sont liés, forment pipéridinyle, pyrrolidinyle, morpholinyle, thiomorpholinyle ou pipérazinyle), -C(O)N(R^{f})R^{g}, - N(R^{h})C(O)R^{h}, -N(R^{h})SO₂C₁₋₇alkyle (où R^{h} est -H ou -C₁₋₄alkyle, ou deux R^{h} dans le même substituant, pris conjointement avec l'amide auquel ils sont liés, forment un cycle de 4 à 6 chaînons par ailleurs aliphatique), -S (O)₀₋₂-C₁₋₄alkyle, -SO₂N (R^{f}) R^{g}, -SCF₃, - C(O)C₁₋₄alkyle, -CN, -COOH et -COOC₁₋₄alkyle ;
R^{c} et R^{d} sont sélectionnés indépendamment dans le groupe constitué par -H, -C₁₋₇alkyle, -C₃₋₇alcényle, -C₃₋₇alcynyle, -C₃₋₇cycloalkyle, -C₁₋₇alkylC₃₋₇cycloalkyle et -C₃₋₇cycloalkylC₁₋₇alkyle, ou R^{c} et R^{d}, pris conjointement avec l'azote auquel ils sont liés, forment pipéridinyle, pyrrolidinyle, morpholinyle, thiomorpholinyle ou pipérazinyle, où chaque R^{c} et R^{d} est éventuellement et indépendamment substitué par R^{e} ;
R³ est -C₁₋₄alkyle, -C₁₋₄alcényle ou benzyle, chacun éventuellement substitué par -C₁₋₃alkyle, -OH ou halogéno, ou deux substituants R³ pris conjointement forment C₂₋₅alkylène éventuellement substitué par -C₁₋₃alkyle, -OH ou halogéno ;
r vaut 0 ou est un entier inférieur ou égal à m + n + 1 ;
Ar est un cycle aryle ou hétéroaryle sélectionné dans le groupe constitué par :
a) phényle, éventuellement mono-, di- ou trisubstitué par Rⁱ ou disubstitué sur des carbones adjacents par -OC₁₋₄alkylèneO-, -(CH₂)₂₋₃NH-, -(CH₂)₁₋₂NH (CH₂)-, -(CH₂)₂₋₃N (C₁₋₄alkyl)-, ou -(CH₂)₁₋₂N(C₁₋₄alkyl)(CH₂)- ;
Rⁱ est sélectionné dans le groupe constitué par -C₁₋₇ alkyle, -C₂₋₇alcényle, -C₂₋₇alcynyle, -C₃₋₇cycloalkyle, halogéno, -CF₃, -OH, -OC₁₋₇alkyle, -OCF₃, -OC₃₋₇alcényle, -OC₃₋₇alcynyle, -N(R^{j})R^{k} (où R^{j} et R^{k} sont indépendamment -H ou -C₁₋₄alkyle), -C(O)N(R^{j})R^{k}, -N(R^{j})C(O)R^{k}, -N (R^{j}) SO₂C₁₋₆alkyle, -S (O)₀₋₂-C₁₋₆alkyle, -SO₂N(R^{j})R^{k}, -SCF₃, -C(O)C₁₋₆alkyle, -NO₂, -CN, -COOH et -COOC₁₋₇alkyle ;
b) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à deux atomes de carbone supplémentaires éventuellement remplacés par -N=, éventuellement mono-ou disubstitué par Rⁱ ;
c) un groupement hydrocarboné aromatique monocyclique à six atomes de cycle, ayant un atome de carbone comme point de liaison, ayant un ou deux atomes de carbone remplacés par -N=, éventuellement mono- ou disubstitué par Rⁱ ; et
d) phényle ou pyridyle, substitué par un substituant sélectionné dans le groupe constitué par phényle, phénoxy, pyridyle, thiophényle, oxazolyle et tétrazolyle, où le motif substitué résultant est éventuellement en outre mono-, di- ou trisubstitué par Rⁱ ;
ALK est C₁₋₇alkylène, C₂₋₇alcénylène, C₂₋₇alcynylène, C₃₋₇cycloalkylène, ou C₃₋₇cycloalcénylène ramifié ou non ramifié, éventuellement mono-, di- ou trisubstitué par R^{m} ;
R^{m} est sélectionné dans le groupe constitué par halogéno, -CF₃, -OH, -OC₁₋₇alkyle, -OC₃₋₇cycloalkyle, -OCF₃, -N(R^{p})R^{s} (où R^{p} et R^{s} sont indépendamment -H ou -C₁₋₇alkyle), -C(O)N(R^{p})R^{s}, -N(R^{t})C(O)R^{t}, -N(R^{t})SO₂C₁₋₆alkyle (où R^{t} est -H ou -C₁₋₇alkyle), -S(O)₀₋₂-C₁₋₆alkyle, -SO₂N(R^{p})R^{s}, -SCF₃, -CN, -NO₂, -C(O)C₁₋₇alkyle, -COOH et -COOC₁₋₇alkyle ;
CYC est -H ou est un noyau sélectionné dans le groupe constitué par :
i) phényle, éventuellement mono-, di- ou trisubstitué par R^{u} ou disubstitué sur des carbones adjacents par -OC₁₋₄alkylèneO-, -(CH₂)₂₋₃NH-, -(CH₂)₁₋₂NH(CH₂)-, -(CH₂)₂₋₃N(C₁₋₄alkyl)-, ou -(CH₂)₁₋₂N(C₁₋₄alkyl) (CH₂) - ;
R^{u} est sélectionné dans le groupe constitué par -C₁₋₇alkyle, -C₃₋₇cycloalkyle, phényle, benzyle, halogéno, -CF₃, -OH, -OC₁₋₇alkyle, -OC₃₋₇cycloalkyle, -Ophényle, -Obenzyle, -OCF₃, -N(R^{v})R^{w} (où R^{v} et R^{w} sont indépendamment -H ou -C₁₋₇alkyle, ou R^{v} et R^{w}, pris conjointement avec l'azote auquel ils sont liés, forment pipéridinyle, pyrrolidinyle, morpholinyle, thiomorpholinyle ou pipérazinyle, où chaque R^{v} et R^{w} est éventuellement et indépendamment substitué par -OH ou -C₁₋₇alkyle), -C(O)N(R^{v})R^{w}, -N(R^{x})C(O)R^{x}, -N(R^{x})SO₂C₁₋₆alkyle (où R^{x} est -H ou -C₁₋₇alkyle, ou deux R^{x} dans le même substituant, pris conjointement avec l'amide auquel ils sont liés, forment un cycle de 4 à 6 chaînons par ailleurs aliphatique), -N-(SO₂C₁₋₆alkyle)₂, -S(O)₀₋₂-C₁₋₆alkyle, -SO₂N(R^{v})R^{w}, -SCF₃, -C(O)C₁₋₆alkyle, -NO₂, -CN, -COOH et -COOC₁₋₇alkyle ;
ii) un groupement hydrocarboné aromatique monocyclique à cinq atomes de cycle, ayant un atome de carbone comme point de liaison, ayant un atome de carbone remplacé par >O, >S, >NH, ou >N(C₁₋₄alkyl), ayant jusqu'à un atome de carbone supplémentaire éventuellement remplacé par -N=, éventuellement mono-ou disubstitué par R^{u} ;
iii) un groupement hydrocarboné aromatique monocyclique à six atomes de cycle, ayant un atome de carbone comme point de liaison, ayant un ou deux atomes de carbone remplacés par -N=, éventuellement mono- ou disubstitué par R^{u} ; et
iv) un cycle hétérocyclique non aromatique de 4 à 8 chaînons, ledit cycle ayant 0, 1 ou 2 chaînons d'hétéroatomes non adjacents sélectionnés dans le groupe constitué par O, S, -N=, >NH et >N(C₁₋₄alkyl), ayant 0, 1 ou 2 doubles liaisons, ayant 0, 1 ou 2 chaînons de carbone s'agissant de carbonyle, éventuellement ayant un chaînon de carbone qui forme un pont, ayant 0 à 5 substituants R^{u}, et où, quand q représente 0, ledit cycle comporte un atome de carbone comme point de liaison ;
ainsi que les énantiomères, diastéréoisomères, hydrates, solvates et sels pharmaceutiquement acceptables, esters et amides de celui-ci ;
pour une utilisation dans le traitement ou la prévention d'un trouble du SNC choisi dans le groupe constitué par les troubles du sommeil, la dépression/anxiété, le trouble de l'anxiété généralisée, la schizophrénie, les troubles bipolaires, les troubles psychotiques, le trouble obsessionnel-compulsif, les troubles de l'humeur, le stress post-traumatique et autres troubles liés au stress, la migraine, les douleurs, les troubles de l'alimentation, l'obésité, les dysfonctionnements sexuels, les perturbations métaboliques, les déséquilibres hormonaux, l'alcoolisme, les troubles de dépendance, la nausée, les inflammations, l'hypertension centralement médiée, les troubles du sommeil/réveil, les symptômes du décalage horaire, et les anomalies du rythme veille-sommeil chez les mammifères ;
pour une utilisation dans le traitement ou la prévention d'une maladie ou d'une condition choisie dans le groupe constitué par l'hypotension, les troubles vasculaires périphériques, le choc cardio-vasculaire, les troubles rénaux, la motilité gastrique, la diarrhée, le côlon spastique, le trouble du côlon irritable, les ischémies, le choc septique, l'incontinence urinaire, et d'autres troubles liés aux systèmes gastro-intestinaux et vasculaires chez les mammifères ;
pour une utilisation dans le traitement ou la prévention d'un trouble oculaire choisi dans le groupe constitué par le glaucome, la névrite optique, la rétinopathie diabétique, l'oedème rétinien, et la dégénérescence maculaire liée à l'âge chez les mammifères.

36. Composé de Formule (I) ou (II) tel que défini selon la revendication 31, marqué isotopiquement afin de pouvoir être détecté par PET ou SPECT.

37. Méthode d'étude de troubles médiés par la sérotonine, comprenant l'étape consistant à utiliser un composé de Formule (I) ou (II) tel que défini selon la revendication 31, marqué par ¹⁸F ou ¹¹C, comme sonde moléculaire pour tomographie par émission de positons (PET), où ladite méthode n'est pas une méthode de traitement du corps humain ou animal par chirurgie ou thérapie ou une méthode de diagnostique pratiquée sur le corps humain ou animal.

38. Composé selon la revendication 1, sélectionné dans le groupe constitué par la {2-[2-tertio-butyl-6-(4-fluorophényl)pyrimidin-4-yl]éthyl}méthylamine et la {2-[2-tertio-butyl-6-(4-fluorophényl)pyrimidin-4-yl]-éthyl}diméthylamine.

39. Composé selon la revendication 1, qui est la 4-(4-fluorophényl)-2-isopropyl-5,6,7,8-tétrahydropyrido[4,3-d]pyrimidine, ou un sel pharmaceutiquement acceptable de celle-ci.
